(19) Europäisches Patentamt
European Patent Office
Office européen des brevets

(11) Publication number: 0 301 919 A2

(12) **EUROPEAN PATENT APPLICATION**

(21) Application number: 88307095.5

(22) Date of filing: 01.08.88

(51) Int. Cl.[4]: **C 07 D 487/04**
C 07 D 498/04, A 01 N 47/36
//(C07D487/04,237:00,235:00),
(C07D487/04,239:00,231:00),
(C07D487/04,251:00,231:00),
(C07D498/00,261:00,221:00)

(30) Priority: 31.07.87 US 80471 04.05.88 US 190242
04.05.88 US 190243

(43) Date of publication of application:
01.02.89 Bulletin 89/05

(84) Designated Contracting States: ES GR

(71) Applicant: **E.I. DU PONT DE NEMOURS AND COMPANY**
**Legal Department 1007 Market Street**
**Wilmington Delaware 19898 (US)**

(72) Inventor: **Tseng, Chi-Ping**
**1103 Artwin Road**
**Wilmington, Delaware 19803 (US)**

(74) Representative: **Hildyard, Edward Martin et al**
**Frank B. Dehn & Co. European Patent Attorneys Imperial House 15-19 Kingsway**
**London WC2B 6UZ (GB)**

(54) **Herbicidal sulfonamides.**

(57) The invention relates to sulfonylurea compounds of formula

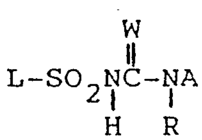

wherein L is a substituted or unsubstituted bicyclic ring group having one to five heteroatoms, W is O or S, R is H or $CH_3$ and A is a substituted or unsubstituted pyrimidinyl, triazolyl or triazinyl group. The compounds are preemergent and/or postemergent herbicides or plant growth regulators.

EP 0 301 919 A2

**Description**

## HERBICIDAL SULFONAMIDES

### Related Application

This application is a continuation-in-part of application U.S. Serial No. 080,471 filed July 31, 1987.

### Background of the Invention

This invention relates to novel sulfonylurea compounds, agriculturally suitable compositions thereof and a method of using them to control the growth of undesired vegetation.

New compounds effective for controlling the growth of undesired vegetation are in constant demand. In the most common situation, such compounds are sought to selectively control the growth of weeds in useful crops such as cotton, rice, corn, wheat and soybeans, to name a few. Unchecked weed growth in such crops can cause significant losses, reducing profit to the farmer and increasing costs to the consumer. In other situations, herbicides are desired which will control all plant growth. Examples of areas in which complete control of all vegetation is desired are areas around railroad tracks, and industrial storage areas. There are many products commercially available for these purposes, but the search continues for products which are more effective, less costly and environmentally safe.

The "sulfonylurea" herbicides are an extremely potent class of herbicides discovered within the last few years. A multitude of structural variations exist within this class of herbicides, but they generally consist of a sulfonylurea bridge, $-SO_2NHCONH-$, linking two aromatic or heteroaromatic rings.

U.S. 4,369,320 and U.S. 4,453,970 disclose herbicidal quinolinylsulfonylureas.

U.S. 4,391,627 discloses herbicidal benzothiophenesulfonylureas.

EP-A-70,698 published 1/26/83 discloses herbicidal indolesulfonylureas.

EP-A-146,263, published 6/26/85, discloses herbicidal sulfonylureas of the formula

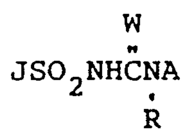

wherein

J is 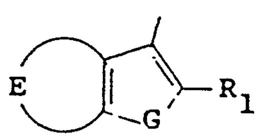 or 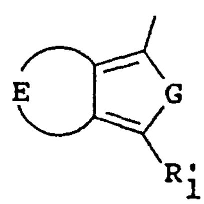 ;

E is a bridge of 3 or 4 atoms, which may be substituted or unsubstituted, containing 0 to 2 heteroatoms selected from the group consisting of oxygen, sulfur and nitrogen and also containing 1-4 atoms of carbon, said bridge together with two carbon attachment sites forming a partially saturated 5- to 6-membered carbocyclic or heterocyclic ring; or E is a bridge of 3 or 4 atoms which may be substituted or unsubstituted containing at least 1 heteroatom selected from 0-1 oxygen or sulfur or 0-2 nitrogen and 1-3 atoms of carbon, said bridge together with two carbon attachment sites forming an unsaturated 5- to 6-membered heterocyclic ring, with the proviso that when E contains two atoms of oxygen or sulfur, then they must be separated by at least one atom of carbon and that oxygen and sulfur are only linked to each other if the sulfur is in the form of SO or $SO_2$; in the bridging group E, nitrogen may take the form of N or N-O, sulfur may take the form of S, SO or $SO_2$, and one of the atoms of carbon may be a carbonyl, thiocarbonyl or the cyclic 5- and 6-membered ketals thereof; and

G is O, S, NH or $NCH_3$.

U.S. 4,622,062 discloses herbicidal sulfonylureas of the formula

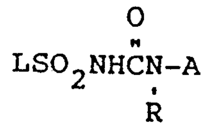

wherein

L, in part, is 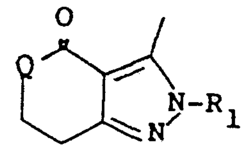, 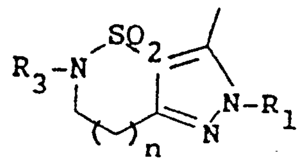,

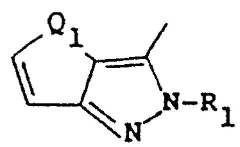 or 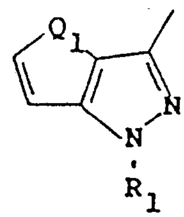

$Q_1$ is O, S or $SO_2$.

U.S. 4,643,759 discloses herbicidal compounds of the formula

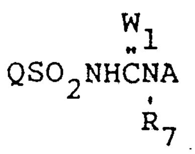

wherein

Q is 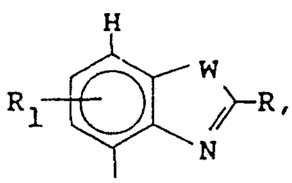 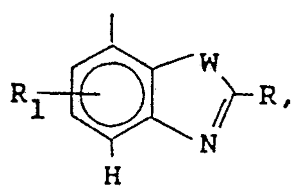

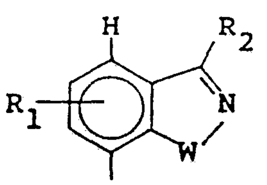 or 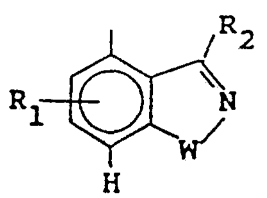 ; and

W is O, S or $NR_3$.

U.S. 4,678,499 discloses herbicidal compounds of the formula

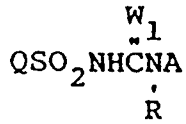

wherein

Q is 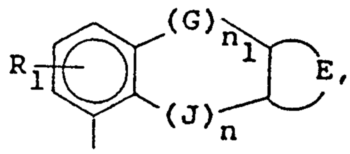 , 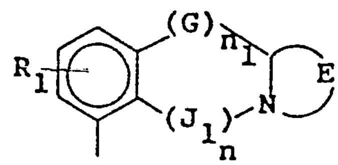 or

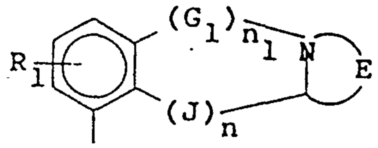 ;

G is $CH_2$, $CH_2CH_2$, O, S, NH, $NCH_3$ or CH=CH;
$G_1$ is $CH_2$, $CH_2CH_2$ or CH=CH;
J, in part, is $CH_2$, C(O), $S(O)_m$, O, NH or $NCH_3$;
n and $n_1$ are independently 0 or 1; and
E is a bridge of 3 or 4 atoms containing 0-2 heteroatoms selected from the group consisting of oxygen, sulfur and nitrogen, wherein 1 atom of sulfur may take the form of SO or $SO_2$ said bridge also containing 1 to 4 atoms of carbon wherein I atom of carbon may take the form of C=O, said bridge together with two attachment sites forming a non-aromatic heterocyclic or carbocyclic ring optionally substituted by 1 to 3 substituent groups selected from the group L, or E is a bridge of 3 or 4 atoms containing 0-1 heteroatoms of oxygen or sulfur and 0-3 heteroatoms of nitrogen, said bridge also containing 0-4 atoms of carbon, said bridge together with two attachment sites forming an aromatic heterocyclic or carbocyclic ring optionally substituted by 1 to 3 substituents selected from the group L, with the proviso that when E contains two oxygen atoms or two sulfur atoms said atoms must be separated by at least one atom of carbon and that oxygen and sulfur are only linked to each other if the sulfur is in the form of SO or $SO_2$;

U.S. 4,671,817 discloses herbicidal compounds of the formula

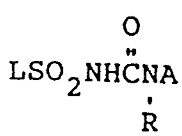

wherein

L is 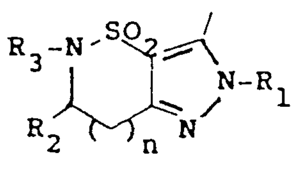 or 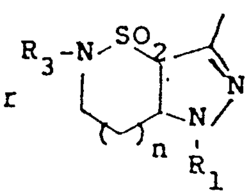

U.S. 4,723,987 discloses herbicidal compounds of the formula

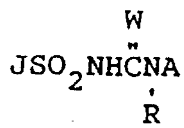

I

wherein

J is 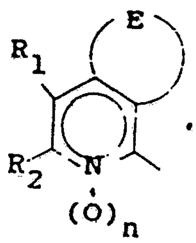 , 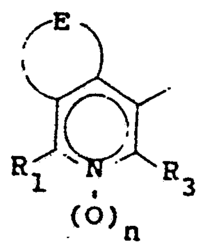 or 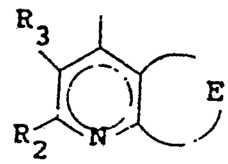 E; and

E is a bridge of 3 or 4 atoms, which may be substituted or unsubstituted, containing 0-2 heteroatoms selected from the group consisting of oxygen, sulfur and nitrogen, and also containing 1-4 atoms of carbon, said bridge together with two carbon attachment sites forming a partially saturated 5- or 6-membered carbocyclic or heterocyclic ring; or E is a bridge of 3 or 4 atoms, which may be substituted or unsubstituted, containing 0-1 heteroatoms selected from oxygen or sulfur, 0-2 heteroatoms of nitrogen and 1-4 atoms of car bon said bridge together with two carbon attachment sites forming a fully unsaturated 5- or 6-membered carbocyclic or heterocyclic ring, with the proviso that when E contains two atoms of oxygen or sulfur, they must be separated by at least one atom of carbon, and the oxygen and sulfur are only linked to each other if the sulfur is in the form of SO or $SO_2$; in the bridging group E, sulfur may take the form of S, SO or $SO_2$, and one of the atoms of carbon may be a carbonyl, thiocarbonyl or the cyclic 5- and 6-membered ketals thereof.

South African Patent Application 84/8844 (U.S. priority 5/30/85) and its equivalent U.S. 4,605,433, issued 8/12/86, disclose substituted 1,2,4-triazolo[1,5-a]pyrimidinesulfonamides of the following formula as herbicides and plant growth regulants.

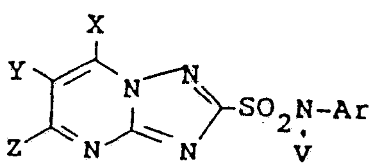

U.S. 4,685,958 (U.S. priority 5/6/87), discloses substituted 2-amino-1,2,4-triazolo[1,5-A]-1,3,5-triazines of the following formula as herbicides.

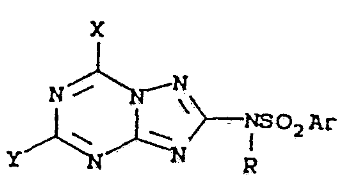

EP-A-244,097, published on November 4, 1987, discloses herbicidal pyrazolopyrimidine derivatives of the formula

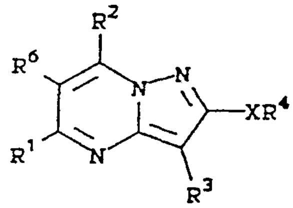

wherein
X is $-NR-SO_2-$, $-SO_2-O-$, $-SO_2-NR-$ or $-S(O)_nCR'R''-$.

EP-A-244,098, published on November 4, 1987, discloses herbicidal thiazolotriazolesulfonamides of the formula

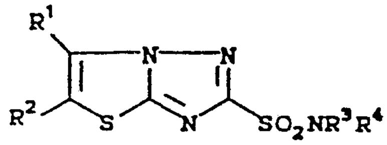

U.S.S.N. 07/039492 (allowed) discloses herbicidal benzotriazole sulfonylureas.

EP-A-244,166, published November 4, 1987, and U.S.S.N. 07/101,314 disclose herbicidal compounds of the formula

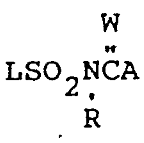 and 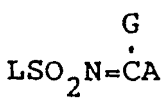

wherein
L, in part, is a benzene, naphthalene, pyrazole, thiophene, pyridine, benzothiophene or an indole moiety;
A, in part, is

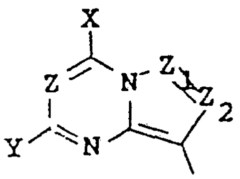, 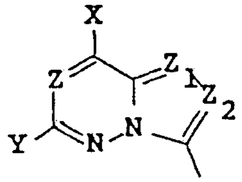, 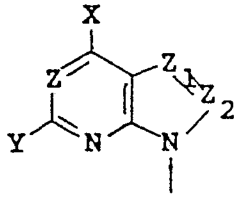,

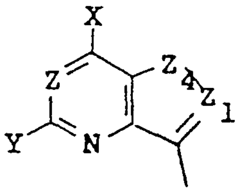, 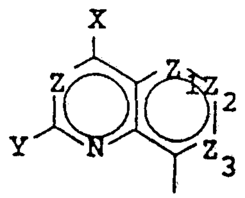, 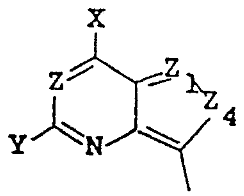,

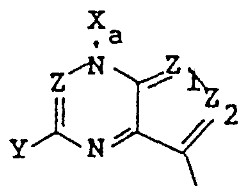, 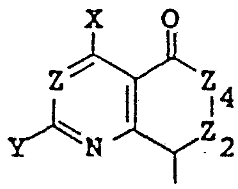, 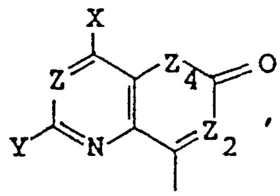,

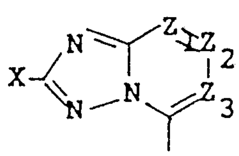, 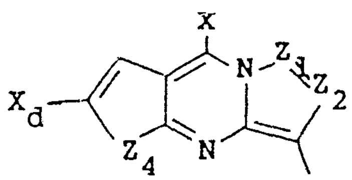, 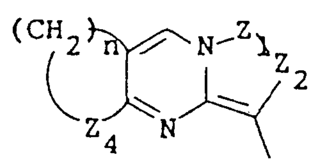,

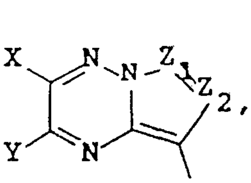, 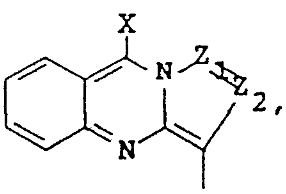, or 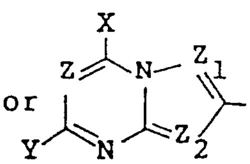;

Z is CH, N, $CCH_3$, CEt, CCl or CBr;
$Z_1$ is C-U, N or N-O;
$Z_2$ and $Z_3$ are independently N or C-U;
$Z_4$ is $NCH_3$, O, S or $CH_2$; and
U is H, F, Cl, Br, $C_1$-$C_2$ alkyl optionally substituted by F, Cl, Br or $OCH_3$, CN, $NO_2$, $NMe_2$, $OR_{22}$, or $SR_{22}$, or $CO_2CH_3$.

EP-A-238,070, published September 23, 1987, discloses compounds of the formula

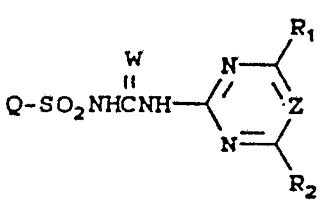

wherein
Q is a condensed heterocyclic group having N atom in the bridgehead which may be substituted;
W is O or S;
$R_1$ and $R_2$ are independently alkyl, alkoxy or halogen; and
Z is CH or N.

## Summary of the Invention

This invention relates to novel compounds of Formula I, their agriculturally suitable compositions and their method-of-use as preemergent and/or postemergent herbicides or plant growth regulants

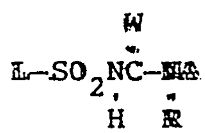

I

wherein

L is

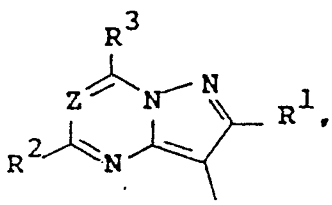,

L-1

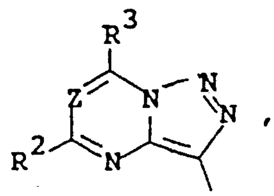,

L-2

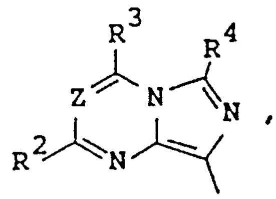,

L-3

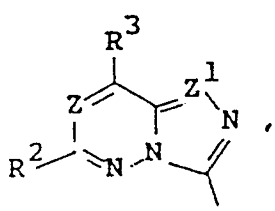,

L-4

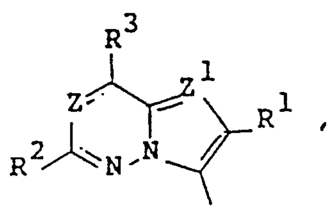,

L-5

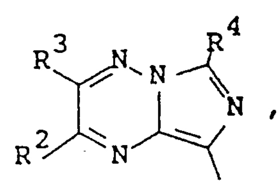,

L-6

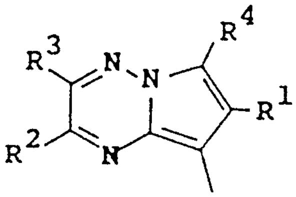,

L-7

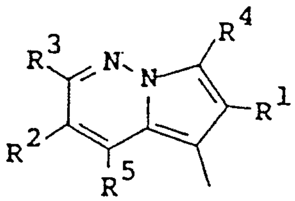,

L-8

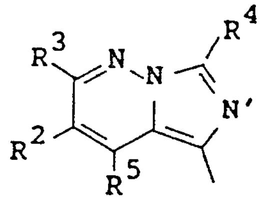,

L-9

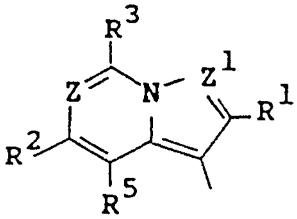,

L-10

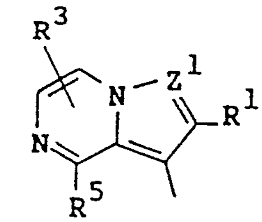,

L-11

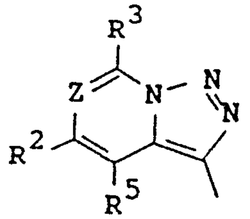,

L-12

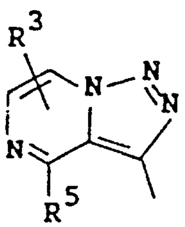,

L-13

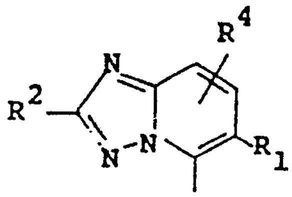,

L-14

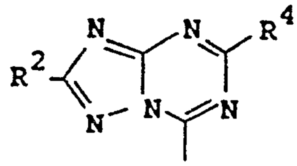,

L-15

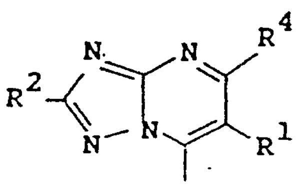,

L-16

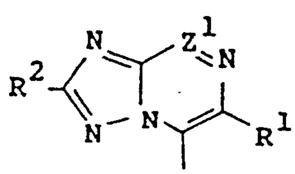,

L-17

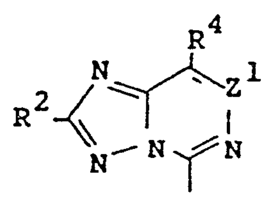,

L-18

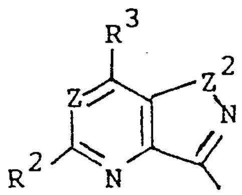 or 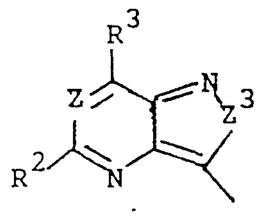 ;

L-19 L-20

$R^1$ is H, halogen, $C_1$-$C_3$ alkyl, $C_1$-$C_3$ haloalkyl, $OR^6$, $SR^6$, $S(O)R^6$, $S(O)_2R^6$, $CO_2R^7$, $C(O)NR^8R^9$, $S(O)_2NR^{10}R^{11}$, $C_2$-$C_3$ alkenyl, $C_2$-$C_3$ haloalkenyl, $C_2$-$C_3$ alkynyl, $NO_2$, CN, $C(O)R^{12}$, $C(R^{13})=NOR^{14}$ or $C_1$-$C_2$ alkyl substituted by $C_1$-$C_2$ alkoxy, $C_1$-$C_2$ alkylthio or CN;
$R^2$ is H, $C_1$-$C_3$ alkyl, $C_1$-$C_3$ alkoxy, $C_1$-$C_2$ haloalkoxy or $C_1$-$C_2$ alkyl substituted with $OCH_3$, $SCH_3$ or CN;
$R^3$ is H, $CH_3$ or $OCH_3$;
$R^4$ is H or $CH_3$;
$R^5$ is H, halogen, $C_1$-$C_3$ alkyl, $C_1$-$C_3$ haloalkyl, $OR^6$, $SR^6$, $S(O)R^6$, $S(O)_2R^6$, $CO_2R^7$, $C(O)NR^8R^9$, $S(O)_2NR^{10}R^{11}$, $C_2$-$C_3$ alkenyl, $C_2$-$C_3$ haloalkenyl, $C_2$-$C_3$ alkynyl, $NO_2$, CN, $C(O)R^{12}$, or $C_1$-$C_2$ alkyl substituted by $C_1$-$C_2$ alkoxy, $C_1$-$C_2$ alkylthio or CN;
$R^6$ is $C_1$-$C_3$ alkyl, $C_1$-$C_3$ haloalkyl, $C_3$ alkenyl, $C_2$-$C_3$ haloalkenyl, propargyl, cyclopropyl, cyclopropylmethyl or $-CH_2CH_2-$ substituted by OH, $C_1$-$C_2$ alkoxy, SH, $C_1$-$C_2$ thioalkyl or CN;
$R^7$ is $C_1$-$C_4$ alkyl, $C_2$-$C_3$ haloalkyl, allyl, propargyl, cyclopropyl, cyclopropylmethyl, or $-CH_2CH_2-$ substituted by OH, $OCH_3$, $SCH_3$ or CN;
$R^8$ is H or $C_1$-$C_2$ alkyl;
$R^9$ is $C_1$-$C_3$ alkyl, $C_1$-$C_3$ alkoxy, allyl, propargyl, cyclopropyl, $CH_2CN$, $CH_2CH_2CN$ or $CH_2CH_2OCH_3$;
$R^{10}$ is H or $C_1$-$C_3$ alkyl;
$R^{11}$ is $C_1$-$C_3$ alkyl, $C_1$-$C_3$ alkoxy, allyl, propargyl, cyclopropyl, cyclopropylmethyl, $CH_2CN$, $CH_2CH_2CN$ or $CH_2CH_2OCH_3$;
$R^{10}$ and $R^{11}$ can be taken together to form a ring consisting of $(-CH_2-)_4$, $(-CH_2-)_5$ or $(-CH_2CH_2-)_2O$;
$R^{12}$ is H, $C_1$-$C_3$ alkyl or cyclopropyl;
$R^{13}$ is H, $C_1$-$C_3$ alkyl, cyclopropyl, Cl, CN, $OCH_3$, $SCH_3$ or $N(CH_3)_2$;
$R^{14}$ is H or $C_1$-$C_3$ alkyl;
Z is N or CH;
$Z^1$ is N, CH or $CCH_3$;
$Z^2$ is O, S or $NCH_3$;
$Z^3$ is O, S or N-$R^{15}$;
$R^{15}$ is H, $C_1$-$C_2$ alkyl, $C_1$-$C_2$ haloalkyl, $CH_2CH_2OCH_3$, $CH_2CH_2SCH_3$, $CH_2CN$ or $CO_2CH_3$;
W is O or S;
R is H or $CH_3$;

A is 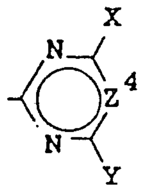 , 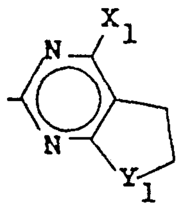 , 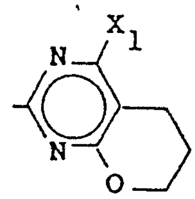 ,

A-1 A-2 A-3

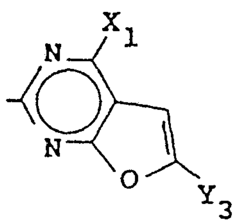 , 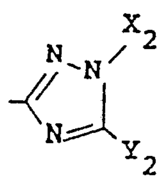 , 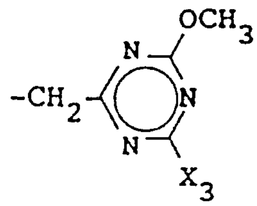

A-4 A-5 A-6

or 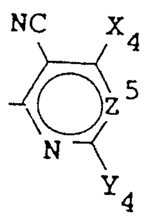 ;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino or di($C_1$-$C_3$ alkyl)amino;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkyl thioalkyl, $C_2$-$C_5$ alkylsulfinylalkyl, $C_2$-$C_5$ alkylsulfonylalkyl, $C_1$-$C_4$ haloalkyl, $C_2$-$C_4$ alkynyl, $C_3$-$C_5$ cycloalkyl, azido, cyano,

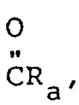 , 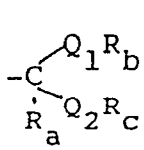 , 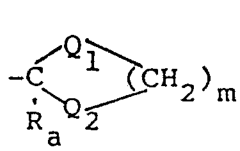 , 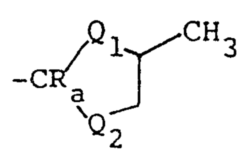

or $N(OCH_3)CH_3$;

m is 2 or 3;

$Q_1$ and $Q_2$ are independently O or S;

$R_a$ is H or $C_1$-$C_3$ alkyl;

$R_b$ and $R_c$ are independently $C_1$-$C_3$ alkyl;

$Z^4$ is CH, N, $CCH_3$, $CC_2H_5$, CCl or CBr;

$Z^5$ is CH or N;

$Y_1$ is O or $CH_2$;

$X_1$ is $CH_3$, $OCH_3$, $OC_2H_5$ or $OCF_2H$;

$X_2$ is $CH_3$, $C_2H_5$ or $CH_2CF_3$;

$Y_2$ is $OCH_3$, $OC_2H_5$, $SCH_3$, $SC_2H_5$, $CH_3$ or $CH_2CH_3$;

$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl;
$Y_4$ is $CH_3$, $OCH_3$, $OC_2H_5$ or Cl;
and their agriculturally suitable salts;
provided that

1) when X is halogen, then $Z^4$ is CH and Y is $OCH_3$, $OC_2H_5$, $NH_2$, $NHCH_3$, $N(CH_3)_2$, $OCF_2H$, $OCF_2Br$ or $N(OCH_3)CH_3$;
2) when X or Y is $C_1$ haloalkoxy, then $Z^4$ is CH;
3) when W is S, then R is H, A is A-1, $Z^4$ is CH or N, and Y is $CH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$,.

$CH(OCH_3)_2$ or 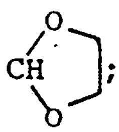;

4) when the total number of carbon atoms of X and Y is greater than four, then the greatest combined number of carbons of any two substituents on an L, selected from $R^1$, $R^2$, $R^3$, $R^4$, $R^5$ and $R^{15}$ is less than or equal to six;
5) $X_4$ and $Y_4$ are not simultaneously Cl;
6) the total number of carbon atoms of $R^{10}$ and $R^{11}$ is less than or equal to five.

In the above definitions, the term "alkyl", used either alone or in compound words such as "alkylthio" or "haloalkyl", denotes straight chain or branched alkyl, e.g. methyl, ethyl, n-propyl, isopropyl or the different butyl isomers.

Alkoxy denotes methoxy, ethoxy, n-propyloxy, isopropyloxy and the different butyloxy isomers.

Alkenyl denotes straight chain or branched alkenes, e.g. 1-propenyl, 2-propenyl, 3-propenyl and the different butenyl isomers.

Alkynyl denotes straight chain or branched alkynes, e.g. ethynyl, 1-propynyl, 2-propynyl and the different butynyl isomers.

Alkylsulfonyl denotes methylsulfonyl, ethylsulfonyl and the different propylsulfonyl isomers.

Alkylthio, alkylsulfinyl, alkylamino, etc. are defined analogously to the above examples.

Cycloalkyl denotes cyclopropyl, cyclobutyl and cyclopentyl.

The term "halogen," either alone or in compound words such as "haloalkyl," denotes fluorine, chlorine, bromine or iodine. Further, when used in compound words such as "haloalkyl" said alkyl may be partially halogenated or fully substituted with halogen atoms and said halogen atoms may be the same or different. Examples of haloalkyl include $CH_2CH_2F$, $CF_2CF_3$ and $CH_2CHFCl$.

The total number of carbon atoms in a substituent group is indicated by the $C_i$-$C_j$ prefix where i and j are numbers from 1 to 5. For example, $C_1$-$C_3$ alkylsulfonyl would designate methylsulfonyl through propylsulfonyl, $C_2$ alkoxyalkoxy would designate $OCH_2OCH_3$, $C_2$ cyanoalkyl would designate $CH_2CN$ and $C_3$ cyanoalkyl would designate $CH_2CH_2CN$ and $CH(CN)CH_3$.

Compounds of the invention preferred for reasons of increased ease of synthesis and/or greater herbicidal efficacy are:

1. Compounds of Formula I where L is L-1, L-3, L-4, L-5, L-6, L-7, L-8, L-9, L-10, L-11, L-14, L-19 or L-20.
2. Compounds of Preferred 1 where W is O and $Z^4$ is CH or N.
3. Compounds of Preferred 2 where

X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$;
Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $OCH_2CH_2OCH_3$,

$CH_2SCH_3$, 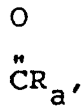, 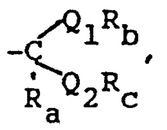, 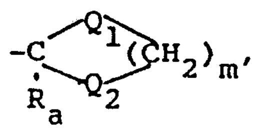,

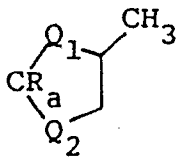, $OCF_2H$, $OCF_2Br$, $SCF_2H$, cyclopropyl, $C{\equiv}CH$ or $C{\equiv}CCH_3$;

$R_a$ is H or $CH_3$; and

$R_b$ and $R_c$ are independently $CH_3$ or $CH_2CH_3$.

4. Compounds of Preferred 3 where $R^1$ is H, halogen, $C_1$-$C_2$ alkyl, $C_1$-$C_2$ haloalkyl, $OR^6$, $SR^6$, $S(O)R^6$, $S(O)_2R^6$, $CO_2R^7$, $C(O)NR^8R^9$, $S(O)_2NR^{10}R^{11}$, $C_2$-$C_3$ alkenyl, $C_2$-$C_3$ haloalkenyl, $NO_2$, CN, $C(O)R^{12}$, $C(R^{13}){=}NOR^{14}$ or $C_1$-$C_2$ alkyl substituted by $C_1$-$C_2$ alkoxy, $C_1$-$C_2$ alkylthio or CN;

$R^2$ is H, $CH_3$ or $OCH_3$;

$R^5$ is H, halogen, $C_1$-$C_2$ alkyl, $C_1$-$C_2$ haloalkyl, $OR^6$, $SR^6$, $S(O)R^6$, $S(O)_2R^6$, $CO_2R^7$, $C(O)NR^8R^9$, $S(O)_2NR^{10}R^{11}$, $C_2$-$C_3$ alkenyl, $C_2$-$C_3$ haloalkenyl, $NO_2$, CN, $C(O)R^{12}$, or $C_1$-$C_2$ alkyl substituted by $C_1$-$C_2$ alkoxy, $C_1$-$C_2$ alkylthio or CN;

$R^6$ is $C_1$-$C_3$ alkyl, $C_1$-$C_3$ haloalkyl, $C_3$ alkenyl, $C_2$-$C_3$ haloalkenyl, propargyl, cyclopropyl or cyclopropylmethyl.

5. Compounds of Preferred 4 where

A is A-1;

X is $CH_3$, $OCH_3$, $OCH_2CH_3$, Cl or $OCF_2H$;

Y is $CH_3$, $OCH_3$, $C_2H_5$, $CH_2OCH_3$, $NHCH_3$, $CH(OCH_3)_2$ or cyclopropyl; and

R is H.

6. Compounds of Preferred 5 where L is L-1.
7. Compounds of Preferred 5 where L is L-3.
8. Compounds of Preferred 5 where L is L-4.
9. Compounds of Preferred 5 where L is L-5.
10. Compounds of Preferred 5 where L is L-6.
11. Compounds of Preferred 5 where L is L-7.
12. Compounds of Preferred 5 where L is L-8.
13. Compounds of Preferred 5 where L is L-9.
14. Compounds of Preferred 5 where L is L-10.
15. Compounds of Preferred 5 where L is L-11.
16. Compounds of Preferred 5 where L is L-14.
17. Compounds of Preferred 5 where L is L-19.
18. Compounds of Preferred 5 where L is L-20.

Compounds of the invention specifically preferred for reasons of greatest ease of synthesis and/or greatest herbicidal efficacy are N-[(4,6-dimethoxypyrimidin-2-yl)aminocarbonyl]-5,7-dimethylpyrazolo[1,5-A]-pyrimidine-3-sulfonamide and N-[(4,6-dimethoxytriazin-2-yl)aminocarbonyl]-5,7-dimethylpyrazolo[1,5-A]-pyrimidine-3-sulfonamide.

## DETAILED DESCRIPTION OF THE INVENTION

### Synthesis

Compounds of Formula I can be prepared by one or more of the procedures shown in Equations 1, 4, and 5. L, R, and A are as previously defined. The requisite intermediates for these reactions can be prepared by one or more of the methods described in this section. In some cases, substituents on the starting materials may be incompatible with the reaction conditions described. It will be readily apparent to one skilled in the art to use either standard protecting groups (e.g., ketal as a protecting group for ketone) or one of the alternative methods described.

Equation 1

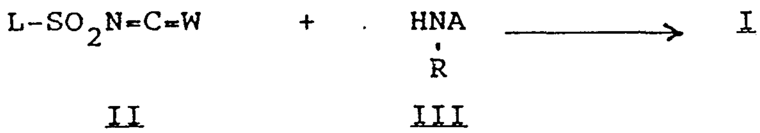


The reaction of Equation 1 is best carried out in an inert aprotic organic solvent such as dichloromethane, 1,2-dichloroethane, tetrahydrofuran, or acetonitrile, at a temperature between 20° and 85°C. The order of addition is not critical; however, it is often convenient to add the sulfonyl isocyanate or a solution of it in the reaction solvent, to a stirred suspension of the amine.

In some cases, the desired product is insoluble in the reaction solvent at ambient temperature and crystallizes from it in pure form. Products soluble in the reaction solvent are isolated by evaporation of the solvent. Compounds of Formula I then may be purified by trituration of the evaporation residue with solvents such as 1-chlorobutane or ethyl ether and filtration, by recrystallization from mixtures of solvents such as 1,2-dichloroethane, 1-chlorobutane and heptane or by chromatography on silica gel.

Sulfonyl isocyanates (II, W is O) can be prepared from the corresponding sulfonamides (IV) by one of the following two general methods.

Equation 2

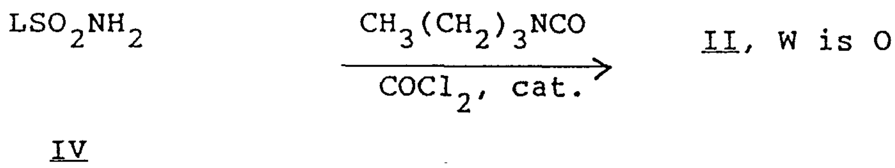


The sulfonamide IV is reacted with an alkyl isocyanate (e.g., n-butyl isocyanate) in a solvent whose boiling point is above 135°C, such as xylene. The reaction can optionally be carried out in the presence of a catalytic amount of 1,4-diaza[2.2.2]bicyclooctane (DABCO). The reaction mixture is heated to 135-140°C and held at that temperature for 5-60 minutes, after which phosgene is slowly added at such a rate that the temperature remains between 133 and 135°C. When the consumption of phosgene has ceased, the mixture is cooled and filtered to remove insoluble material. Finally, the solvent, alkyl isocyanate, and excess phosgene are evaporated, leaving the sulfonyl isocyanate (II).

If desired, the alkyl isocyanate-sulfonamide adduct can be made and isolated before reaction with the phosgene. In this case the sulfonamide (IV), alkyl isocyanate, and anhydrous base (e.g. $K_2CO_3$) or acetonitrile) are mixed and heated under reflux for 1 to 6 hours. The reaction mixture is then diluted with water, and the pH is adjusted to about 3 with acid (e.g. HCl, $H_2SO_4$). The adduct is filtered out and dried, and then reacted with phosgene as described above. This procedure modification is especially useful when sulfonamide (IV) is high melting and has low solubility in the phosgenation solvent.

Sulfonyl isocyanates (II, W is O) can also be prepared by the following method.

Equation 3

(a) $$\underline{IV} \xrightarrow{SOCl_2} LSO_2NSO \quad \underline{V}$$

(b) $$\underline{V} \xrightarrow[\text{pyridine cat.}]{COCl_2,} \underline{II}, \text{ W is O}$$

The sulfonamide (IV) is heated at reflux in an excess of thionyl chloride. The reaction is continued until the sulfonamide protons are no longer detectable in the proton magnetic resonance spectrum. From 16 hours to 5 days is typically sufficient for complete conversion to the thionylamide (V) (Equation 3a).

The thionyl chloride is evaporated and the residue is treated with an inert solvent (e.g. toluene) containing at least one equivalent (typically 2-3 equivalents) of phosgene. A catalytic amount of pyridine. (typically 0.1 equivalent) is added, and the mixture is heated to about 60-140° C, with 80-100° C preferred. Conversion to the isocyanate (II, W is O) is usually substantially complete within 15 minutes to 3 hours (Equation 3b). The mixture is then cooled and filtered, and the solvent is evaporated, leaving the sulfonyl isocyanate (II. W is O).

Sulfonyl isothiocyanates (II, W is S) can be prepared from the corresponding sulfonamides (IV) by reaction with carbon disulfide and potassium hydroxide followed by treatment of the resulting dipotassium salt VI with phosgene. Such a procedure is described in Arch. Pharm., 299, 174 (1966).

Many of the compounds of Formula I can be prepared by the procedure shown in Equation 4.

Equation 4

$$\underset{\underline{VI}}{LSO_2NH\overset{W}{\overset{\|}{C}}OC_6H_5} \quad + \quad \underline{III} \longrightarrow \underline{I}$$

The reaction of Equation 4 can be carried out by contacting phenylcarbamates or phenylthiocarbamates of Formula VI with aminoheterocycles of Formula III in an inert organic solvent such as dioxane or tetrahydrofuran at temperatures of about 20-100° C for a period of about one-half to twenty-four hours. The product can be isolated by evaporation of the reaction solvent and purified by methods previously described.

Phenylcarbamates and phenylthiocarbamates of Formula VI can be prepared by the methods described, or modifications thereof known to those skilled in the art, in U.S. 4,443,243.

Alternatively, many of the compounds of Formula I can be prepared by the method described in Equation 5.

Equation 5

$$\underline{IV} \quad + \quad \underset{\underline{VII}}{C_6H_5O\overset{W}{\overset{\|}{C}}\underset{R}{N}A} \longrightarrow \underline{I}$$

The reaction of Equation 5 can be carried out by contacting equimolar amounts of a sulfonamide of Formula IV with a heterocyclic phenylcarbamate or phenylthiocarbamate of Formula VII in the presence of a base such as 1,8-diazabicyclo[5.4.0]undec-7-ene (DBU), by methods analogous to those described in South African Patent Application 83/0441. The phenylcarbamates and phenylthiocarbamates of Formula VII can be prepared

by methods, or modifications thereof known to those skilled in the art, described in South African Patent Application 82/5671 and South African Patent Application 82/5045.

The sulfonamides IV of this invention may be prepared in a variety of ways some of which are described in Equations 6 through 15.

The sulfonamides IV of this invention can be prepared from the corresponding sulfonyl chloride of Formula VIII. The preparation of sulfonamides from sulfonyl chlorides is widely reported in the literature; for reviews see: F. Hawking and J.S. Lawrence. "The Sulfonamides". H.K. Lewis and Co., London, 1950 and E.H. Northey, "The Sulfonamides and Allied Compound", Reinhold Publishing Corp., New York, 1984.

$L-SO_2Cl$

VIII

The requisite sulfonyl chloride of Formula VIII may be synthesized from IX, X, or XI by known methods or with slight modification thereof, by one skilled in the art.

| | | |
|---|---|---|
| L'-H | LU | $L'-NO_2$ |
| IX | X | XI |

wherein L is as previously defined.

U is Cl, Br or I

L' is L-1, L-2, L-3, L-4, L-5, L-6, L-7, L-8, L-9, L-10, L-11, L-12, L-13, L-19 or L-20.

Several representative teaching are listed below. ·Aromatic nitro group may be transformed into sulfonyl chloride by reduction, diazotization and coupling with sulfur dioxide/cupric chloride as taught in U.S. Patent 4,310,346.

·European Publication No. 94,821 (published 11/23/83) describes the displacement of aromatic halide with thiolate anions and subsequent oxidative chlorination to yield sulfonyl chlorides.

·Halogen metal exchange of aromatic halides or proton-metal exchange of aromatic followed by quenching with sulfur dioxide give sulfinate salts.

These salts yield sulfonyl chlorides upon reaction with N-chlorosuccinimide as taught in U.S. Patent 4,481,029 (issued 11/6/84).

Directed proton-metal exchange of aromatic compounds has been reviewed by Gschweard and Rodriquez, Org. Reactions, 26, (1979). Also aryllithiums may be converted directly to arylsulfonyl chloride with sulfonyl chloride as described by S.N. Bhattacharya, et. al, in J. Chem. Soc. C., 1265 (1968). ·Electrophilic chlorosulfonation of an aromatic ring to give a sulfonyl chloride is well known in the literature. Its application is described by E.H. Huntress, et al., in J. Am. Chem. Soc., 62, 511-14 and 603-4 (1940), and W.E. Kirkpatrick, et al., in J. Med. Chem., 20, 386 (1977).

The compounds of Formula XI, where L' is L-1, L-2, L-3, L-4, L-5, L-6, L-7, L-8, L-9, L-10, L-11, L-12, L-13 L-19 or L-20, may be synthesized from compounds of Formula IX by nitration as taught by B.M. Lynch, et al., in Can. J. Chem., 53, 119 (1975), W.E. Kirkpatrick, et al., in J. Med. Chem., 20, 386 (1977) and B. Stanovnik in Lect. Heterocyclic. Chem., 2, 27.

The compounds of Formula $IX_1$, can be synthesized by the methods shown below in Equation 6 through 10.

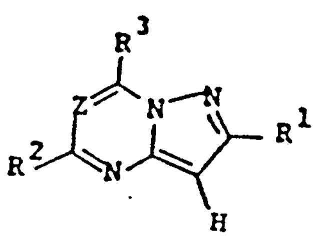

wherein

$R^1$, $R^2$, $R^3$ and Z are as previously defined.

Equation 6 illustrates the reaction of β-dicarbonyl compounds of Formula XII (or their equivalent) with an

aminopyrazole of Formula $XIII_1$ to give the desired heterocycles of Formula $IX_1$ Z is CH,

Equation 6

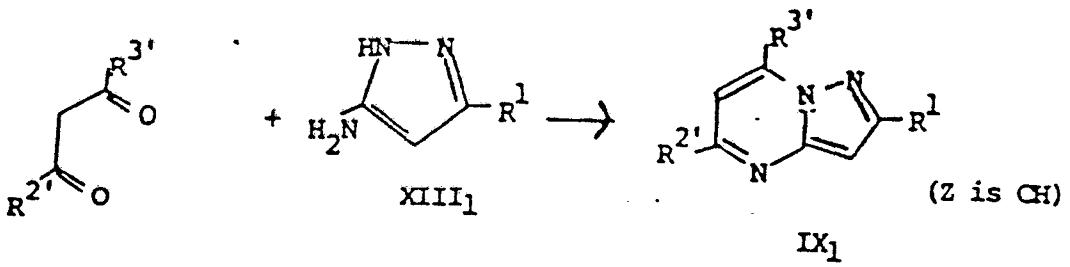

XIII1

IX1

(Z is CH)

XII

wherein $R^1$ is as previously defined, $R^{2'}$ is H, $C_1$-$C_3$ alkyl or $C_1$-$C_2$ alkyl substituted with $OCH_3$, $SCH_3$ or CN; $R^3$ is H or $CH_3$.

The reaction of Equation 6 is best carried out under the conditions taught by W.E. Kirkpatrick, et al., in J. Med. Chem., 20, 386 (1977), J.S. Bajwa, et al., in J. Chem. Soc. Perkin I., 3085 (1979) and B.M. Lynch, et al., in Can. J. Chem., 53, 119 (1975).

Equation 7 illustrates the reaction of aminopyrazole of Formula $XIII_1$ with compounds of Formula XIV to give the desired heterocycles of Formula $IX_1$, Z is N.

Equation 7

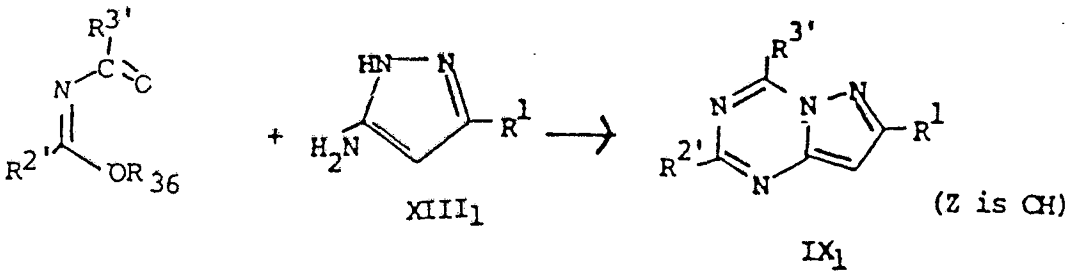

XIII1

IX1

(Z is CH)

wherein
$R^1$, $R^{2'}$, and $R^{3'}$ are as previously defined;
and
$R_{36}$ is $C_1$-$C_4$ alkyl.

The reaction of Equation 7 is best carried out in an inert solvent such as toluene in the presence of an acid such as p-toluenesulfonic acid at a temperature between about 0°C to 111°C. The products may be isolated by washing the reaction solution with an aqueous sodium carbonate solution, drying ($MgSO_4$) and concentrating the reaction solution. Crystallization and chromatography may be used for further purification of the products.

Equation 8 illustrates the reaction of aminopyrazole of Formula $XIII_1$, with an imidate of Formula XV to give the amidines of Formula XVI and the reaction of the amidines of Formula XVI with ortho esters of Formula XVII to give the desired heterocycles of Formula IX, Z is N.

Equation 8

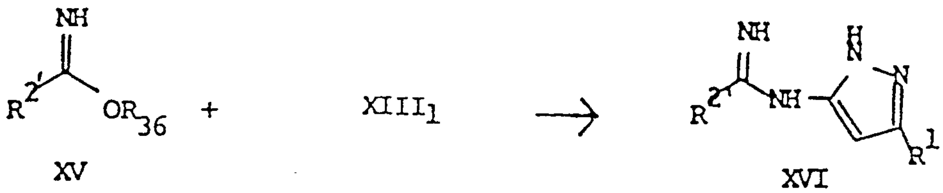

XV XVI

$R^{3'}-C(OR_{37})_3$

XVII

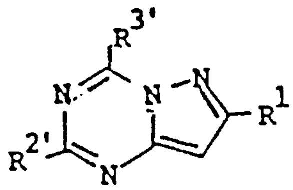

$IX_1$

(Z is N)

wherein

$R^1$, $R^{2'}$, and $R^{3'}$ and $R_{36}$ are as previously defined and

$R_{37}$ is $C_1$-$C_4$ alkyl.

The reaction of Equation 8 is best carried out under the conditions taught by Keitara Senga, et al., in J. Med. Chem., 25, 243 (1982).

Compounds of Formula $IX_1$ ($R^3$ is $OCH_3$) can be prepared as shown below in Equation 9 by reaction of chlorosubstituted heterocycles of Formula XVIII with methoxides of Formula XIX.

Equation 9

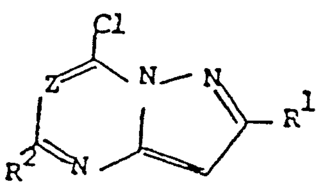

$+M^{+-}OCH_3$

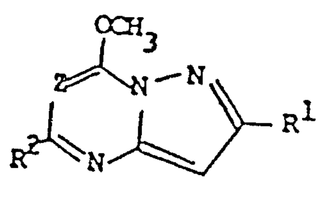

($R^3$ is $OCH_3$)

XVIII XIX $IX_1$

wherein

$R^1$ and $R^2$ are as previously defined; M is Na or K.

The reaction of Equation 9 is best carried out in an inert solvent such as dimethylformamide or an alcohol. The products may be isolated by evaporating the reaction solvent and washing the residue with water. Chromatography may be used for further purification of the products.

Compounds of Formula $IX_1$, ($R^2$ is $R^{2''}$), can be prepared as shown below in Equation 10 by reacting chlorosubstituted heterocycles of Formula XX with nucleophiles of Formula $^{-}R^{2''}$.

Equation 10

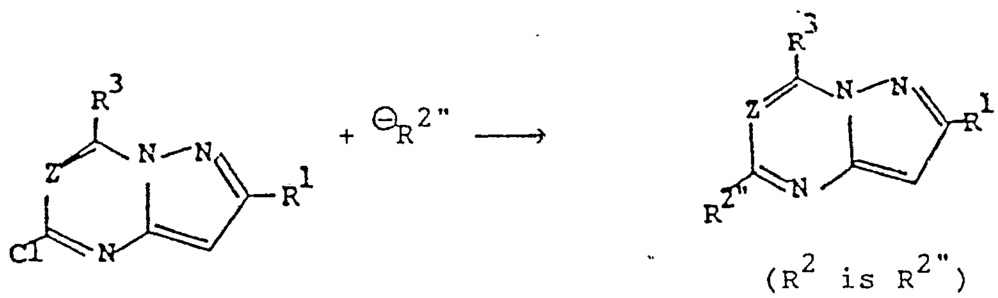

($R^2$ is $R^{2''}$)

XX IX$_1$

wherein $R^1$, $R^3$, and Z are as previously defined; $R^{2''}$ is $C_1$-$C_3$ alkoxy, or $C_1$-$C_2$ haloalkoxy.

The reaction of Equation 10 is best carried out in an inert solvent such as dimethylformamide or an alcohol. The products can be isolated by evaporating the reaction solvent and washing the residue with water. Chromatography may be used for further purification of the products.

Compounds XVIII and XX can be prepared from dichloroheterocycles of Formula XXI by nucleophilic displacement reactions with an appropriate nucleophile by methods in the art or by obvious modifications of these known methods.

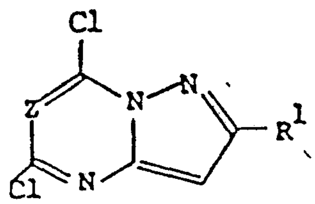

XXI

wherein
Z and $R^1$ are as previously defined.

Alternatively, they can be prepared by reacting the corresponding hydroxy compounds of Formula XXII or XXIII with $POCl_3$ or by other well-known methods or by obvious modification of these known methods.

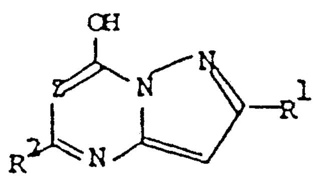

XXII

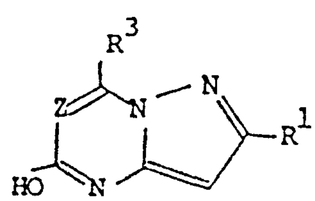

XXIII

wherein
Z, $R^1$, $R^2$ and $R^3$ are as previously defined.

Compounds XXI can be prepared by reacting the corresponding dihydroxy compounds of Formula XXIV with $POCl_3$ by methods well known in the art.

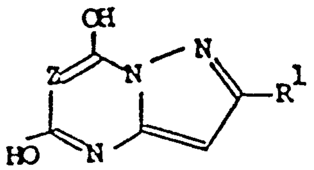

XXIV

wherein
$R^1$ and Z are as previously defined.

The compounds XXII (Z is C-H). XXIII (Z is C-H) or XXIV (Z is C-H) can be prepared by condensation of a β-keto ester, a malonatediester or one of their equivalents with 3-amino-pyrazoles of Formula XIII, as taught by:

1. Yasuo Makisumi in Chem. Pharm. Bull., 10, 612 (1962);
2. Keitara Senga et al. in J. Med. Chem., 24(5), 61a (1971);
3. Alfred Dornow and Klaus Dehmer in Chem. Ber., 100(8), 2577 (1967);
4. Mohamed Elnagdi in Arch. Pharm., 316(8), 713 (1983);
5. B. B. Gavrilenko in Zh. Org. Khim., 18(5), 1079 (1982); or by modifications of these methods (e.g., reaction of 3-amino-pyrazoles of Formula XIII, with diketene).

The compounds XXII (Z is N), XXIII (Z is N) or XXIV (Z is N) can be similarly prepared by condensation of carbonylisocyanates of Formula XXV or the compounds of Formula XXVI with aminopyrazoles of Formula $XIII_1$.

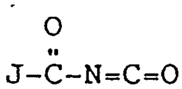

XXV :

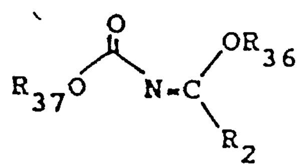

XXVI .

wherein
J is H, Cl, $CH_3$, $C_1$-$C_4$ alkoxy,
$R^2$, $R_{36}$ and $R_{37}$ are as previously defined.

The haloheterocycles of Formula $X_1$ can be synthesized by the same methods as those for the preparation of the heterocycles of Formula $IX_1$ using the aminohalopyrazoles of Formula $XXVII_1$ instead of the aminopyrazoles of Formula $XIII_1$ as starting material.

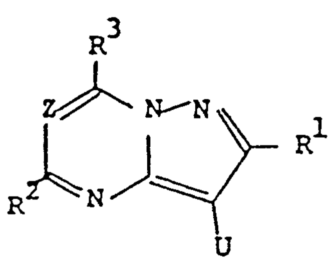

$X_1$

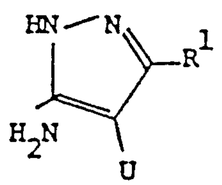

$XXVII_1$ .

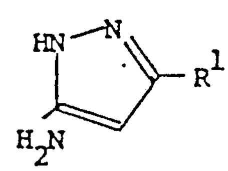

$XIII_1$

wherein
$R^1$, $R^2$, $R^3$, U and Z are as previously defined.

Alternatively, the haloheterocycles of Formula $X_1$ can be synthesized by halogenation of the heterocycles of

Formula $IX_1$ with a halogenating agent such as bromine, N-bromosuccinimide, chlorine, N-chlorosuccinimide or iodine as shown in Equation 11.

Equation 11

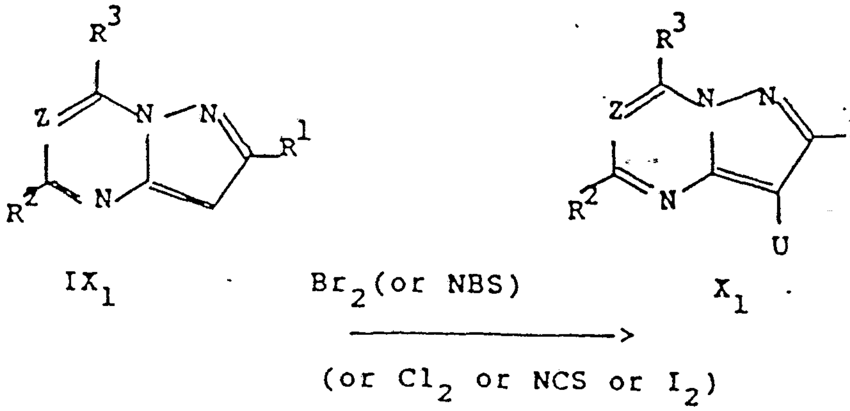


$IX_1$ $X_1$

wherein
$R^1$, $R^2$, $R^3$, Z and U are as previously defined.

The reaction of Equation 11 is best carried out in an inert solvent such as chloroform or carbon tetrachloride at a temperature between 0°C to 77°C. The products can then be isolated by filtration, concentration of the filtrate and washing the residue with water. Crystallization or chromatography may be used for further purification if desired.

By similar methods as discussed for the preparation of the heterocycles of Formula $IX_1$ and the haloheterocycles of Formula $X_1$ from the aminopyrazoles of Formula $XIII_1$ and the aminohalopyrazoles of Formula $XXVII_1$, following compounds can be prepared:

- The heterocycles of Formula $IX_2$ and the haloheterocycles of Formula $X_2$ can be prepared from the amino triazoles of Formula $XIII_2$ and the aminohalotriazoles of Formula $XXVII_2$;
- The heterocycles of Formula $IX_3$ and the haloheterocycles of Formula $X_3$ can be prepared from the aminoimidazoles of Formula $XIII_3$ and the aminohaloimidazoles of Formula $XXVII_3$;
- The heterocycles of Formula $IX_4$ and the haloheterocycles of Formula $X_4$ can be prepared from the N-aminoazoles of Formula $XIII_4$ and the N-aminohaloazoles of Formula $XXVII_4$;
- The heterocycles of Formula $IX_5$ (where $Z^1$ is CH or $CCH_3$) and the haloheterocycles of Formula $X_5$ can be prepared from the N-aminoazoles of Formula $XIII_5$ (where $Z^1$ is CH or $CCH_3$) and the N-aminohaloazoles of Formula $XXVII_5$.

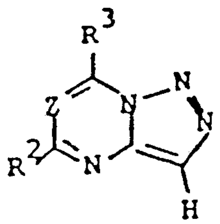

, 

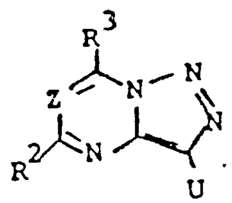

, 

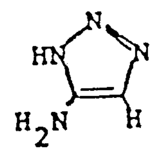

, 

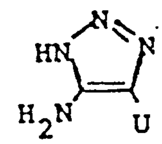

IX₂ X₂ XIII₂ XXVII₂

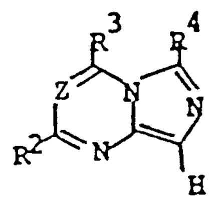

, 

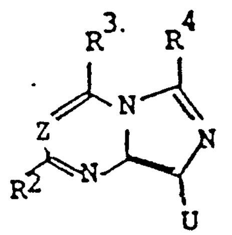

, 

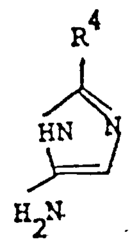

, 

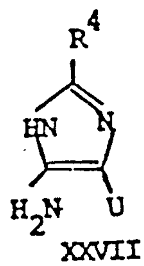

IX₃ X₃ XIII₃ XXVII₃

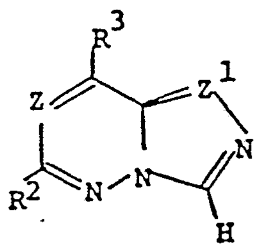

, 

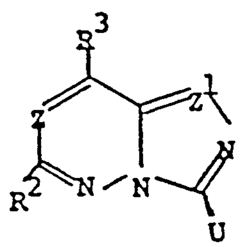

, 

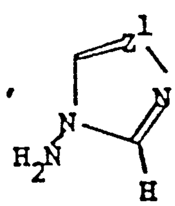

, 

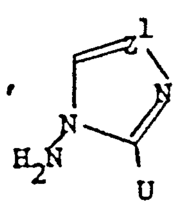

IX₄ X₄ XIII₄ XXVII₄

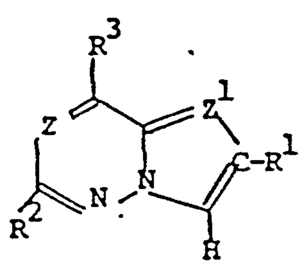

, 

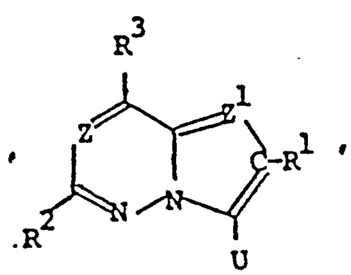

, 

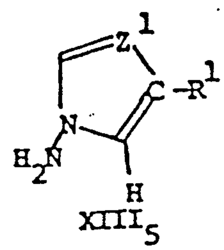

or

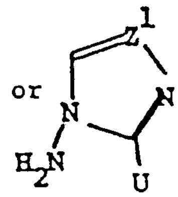

IX₅ X₅ XIII₅ XXVII₅

wherein
$R^1$, $R^2$, $R^3$, $R^4$, Z and N are as previously defined.

The haloheterocycles of Formula $X_2$, $X_3$, $X_4$, and $X_5$, can also be prepared by halogenation of the heterocycles of Formula $IX_2$, $IX_3$, $IX_4$ and $IX_5$.
The heterocycles of Formula $IX_4$ ($Z^1$ is N,) can also be prepared by the reaction of compounds of Formula XXXXIII with orthoformate as shown below in Equation 12.

Equation 12

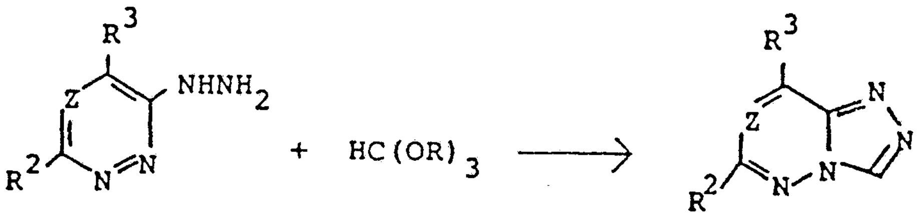


XXXXIII IX$_4$

wherein

$R^2$, $R^3$ and Z are as previously defined and R is lower alkyl.

The reaction of Equation 12 is best carried out in an inert solvent such as toluene at a temperature between about 0°C to 120°C. The product can be isolated by evaporation of the reaction solvent and orthoformate. Crystallization of chromatography may be used for further purification of the products.

The heterocycles of Formula IX$_5$ ($Z_1$ is N,) can be prepared by the reaction of compounds of Formula XXXXIV with an α-halocarbonyl compound of Formula XXXXV as shown in Equation 13.

Equation 13

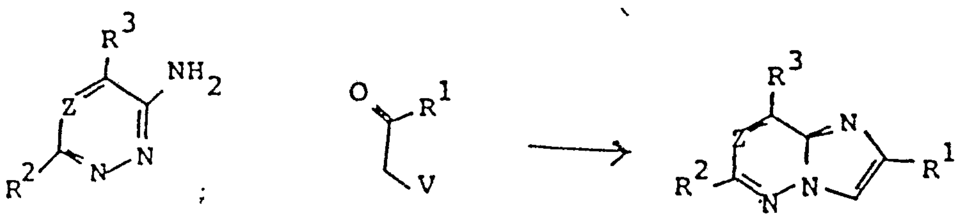

XXXXIV XXXXV IX$_5$

wherein

$R^1$, $R^2$, $R^3$, Z and V are as previously defined.

The reaction of Equation 13 is best carried out in an inert solvent such as N,N-dimethylformamide at a temperature between about 0°C to 160°C. The products can be isolated by evaporation of the reaction solvent and washing the residue with other solvent such as hexane or ether. Crystallization or chromatography may be used for further purification, if desired.

The heterocycles of Formula IX$_6$ can be prepared from compounds of Formula XXVIII. For example, reaction of some of the compounds of Formula XXVIII with 2,3-butadione gives the desired products of Formula IX$_6$ ($R_2$ is $CH_3$, $R_3$ is $CH_3$) as shown below in Equation 14.

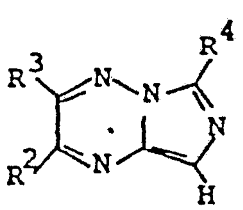

,

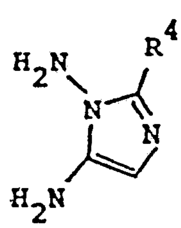

IX$_6$ XXVIII.

wherein

$R^2$, $R^3$, and $R^4$ are as previously defined.

Equation 14

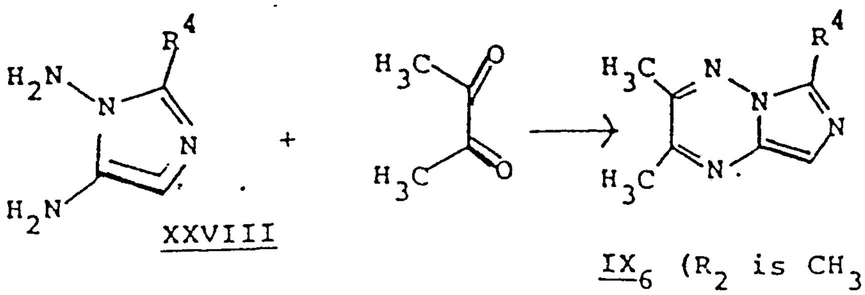

XXVIII

$\underline{IX}_6$ ($R_2$ is $CH_3$, $R_3$ is $CH_3$)

wherein
$R^4$ is as previously defined.

The reaction of Equation 14 is best carried out in an inert solvent such as toluene in the presence of an acid catalyst such as p-toluenesulfonic acid, if necessary, at a temperature between 0° C to 120° C. The Products may be isolated by evaporation of solvent, followed by chromatography or recrystallization of the residue.

The haloheterocycles of Formula $X_6$ can be synthesized by the same method as those for the preparation of the heterocycles of Formula $IX_6$ using the halocompounds of Formula XXIX instead of the compounds of Formula XXVIII as starting material.

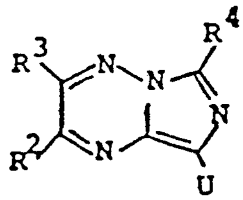

$\underline{X}_6$

,

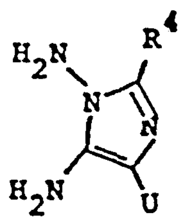

XXIX

wherein
$R^2$, $R^3$, $R^4$ and U are as previously defined.

Alternatively the haloheterocycles of Formula $X_6$ can be prepared by halogenation of the heterocycles of Formula $IX_6$ with a halogenating agent such as bromine, N-bromosuccinimide, chlorine, N-chlorosuccinimide or iodine.

The heterocycles of Formula $IX_7$ and the haloheterocycles of Formula $X_7$ can be synthesized from compounds of Formula XXX and Formula XXXI by the same methods as those for the preparation of compounds of Formula $IX_6$ and $X_6$ from compounds of Formula XXVIII and Formula XXIX.

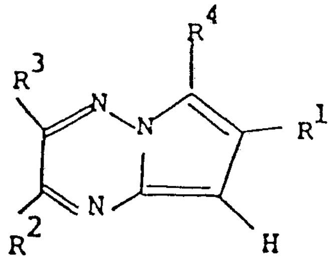

$IX_7$

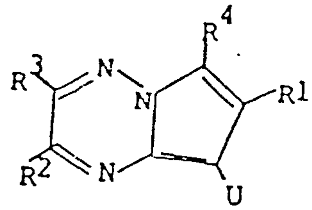

$X_7$

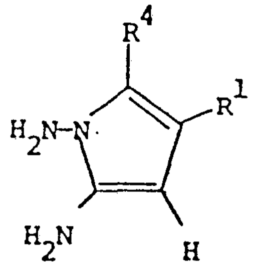

XXX

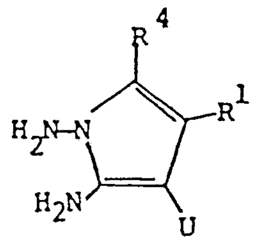

XXXI

wherein
$R^1$, $R^2$, $R^3$, $R^4$ and U are as previously defined.

The heterocycles of Formula $IX_{19}$ can be synthesized from the aminoheterocycles of Formula XXXII by similar methods as discussed for the preparation of the heterocycles of Formula $IX_1$ from aminopyrazole of Formula $XIII_1$. For example, reaction of the aminoheterocycles of Formula XXXII with a β-dicarbonyl compound of Formula XXII should give the desired heterocycles of Formula $IX_{19}$ ($R^2$ is $R^{2'}$, $R^3$ is $R^{3'}$ and Z is C-H) as shown below in Equation 15.

Equation 15

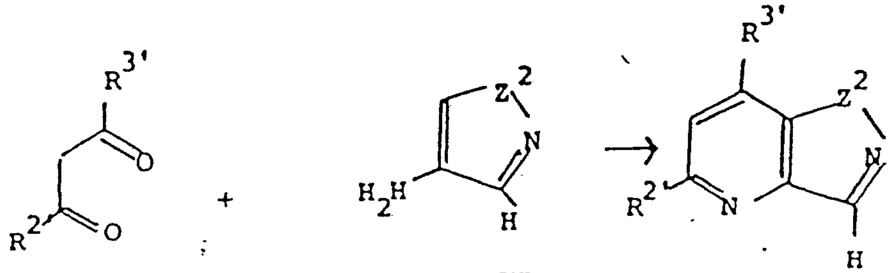

XII XXXII $IX_{19}$

(Z is CH; R is $R^{2'}$ and $R^3$ is $R^{3'}$)

wherein
$R^{2'}$, $R^{3'}$, and $Z^2$ are as previously defined.

The reaction of Equation 15 is best carried out under the conditions taught by A. Z. Britten, et al. in Chem. Ind., 6, 278 (1973).

Reaction of compounds of Formula XXXIII with $HCO_2NH_4$ gives the desired heterocycles of Formula $IX_{19}$ (Z is N, $R^2$ is $CH_3$, $R^3$ is $CH_3$) as shown in Equation 16.

Equation 16

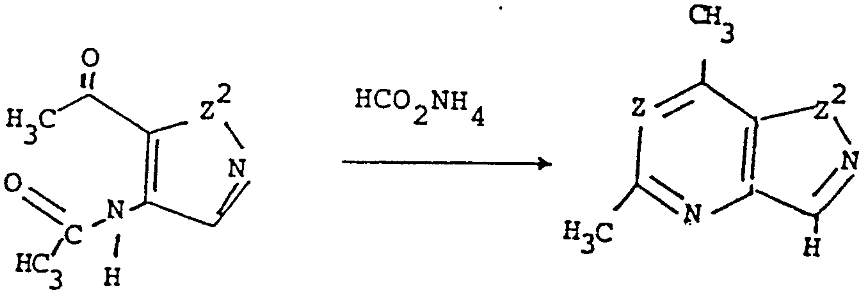


XXXIII IX19

$(R_2 \text{ is } CH_3, R^3 \text{ is } CH_3, Z \text{ is } N)$

The reaction of Equation 14 is best carried out under the conditions taught by Guy Ah-Kon, et al., in C. R. Acad. Sci., Ser. C., 278 (26), 1513 (1974).

The haloheterocycles of Formula $X_{19}$ be synthesized from starting materials such as compounds of Formula XXXIV and XXXV in the same way as for the preparation of the heterocycles of Formula $IX_{19}$ from compounds of Formula XXXII and Formula XXXIII. Alternatively, compounds of Formula $IX_{19}$ can be converted to the haloheterocycles of Formula $X_{19}$ by halogenation.

The haloheterocycles of Formula $X_{20}$ can be prepared from starting materials such as compounds of Formula XXXVI and formula XXXVII by similar chemistry as taught previously for the preparation of heterocycles of Formula $IX_{19}$.

The heterocycles of Formula $IX_{20}$ can be synthesized by dehalogenating the haloheterocycles of Formula $X_{20}$.

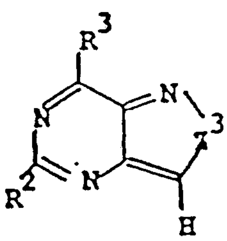 , 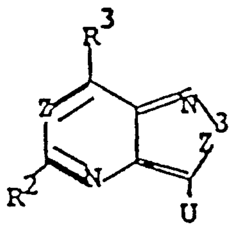 , 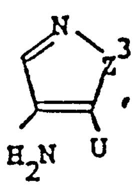 , 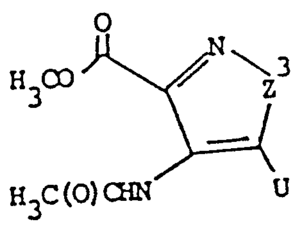

$IX_{20}$ $X_{20}$ XXXVI XXXVII

wherein
$R^2$, $R^3$, Z, $Z^3$ and U are as previously defined.

The haloheterocycles of Formula $X_{14}$ can be prepared from compounds of Formula XXXVIII. For example, reaction of compounds of Formula XXXVIII with triethylorthoacetate gives the desired haloheterocycles of Formula $X_{14}$ ($R^2$ is $CH_3$) as shown in Equation 17.

Equation 17

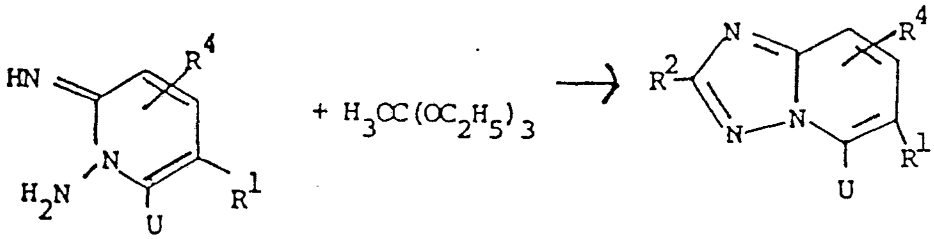


XXXVIII

$X_{14}$

($R^2$ is $CH_3$)

wherein
$R^1$, $R^4$, and U are as previously defined.

The reaction of Equation 17 is best carried out in an inert solvent such as acetic acid at a temperature between 20°C to 118°C. The products may be isolated by evaporation of the solvent, followed by chromatography or crystallization of the residue.

By similar methods as those described for the preparation of haloheterocycles of Formula $X_{14}$ from compounds of Formula XXXVIII, the following haloheterocycles can be prepared: ·The haloheterocycles of Formula $X_{15}$ can be prepared from compounds of Formula XXXIX.
·The haloheterocycles of Formula $X_{16}$ can be prepared from compounds of Formula XXXX.
·The haloheterocycles of Formula $X_{17}$ can be prepared from compounds of Formula XXXXI.
·The haloheterocycles of Formula $X_{18}$ can be prepared from the compounds of Formula XXXXII.

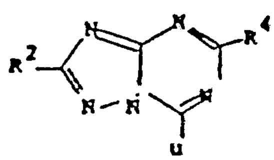
,
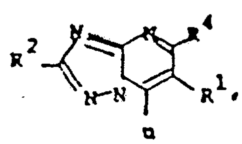
,
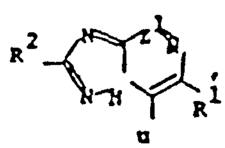
,
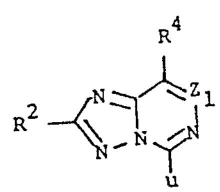

$X_{15}$ $X_{16}$ $X_{17}$ $X_{18}$

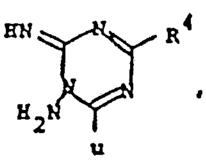
,
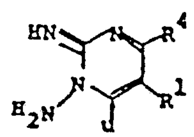
,
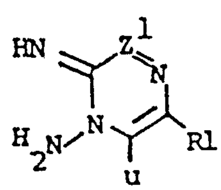
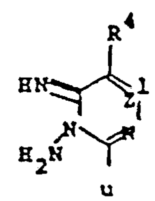

XXXIX XXXX XXXXI XXXXII

wherein
$R^1$, $R^2$, $R^4$, $Z^1$ and U are as previously defined.

The synthesis of compounds of Formula $IX_8$ and $X_8$ has been reviewed by D.E. Kuhla and J.G. Lombardino in "Adv. Hetercycl. Chem.", Vol. 21 (a series published by Academic Press, Inc., New York and London) and by B. Stanovnik in "Lect. Heterocycl. Chem.", Vol. 2 (a series published by Hetero Corporation, Orem, Utah).

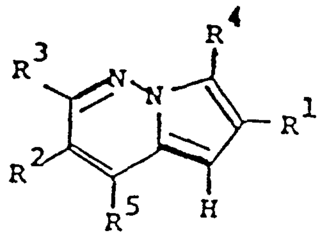

$IX_8$

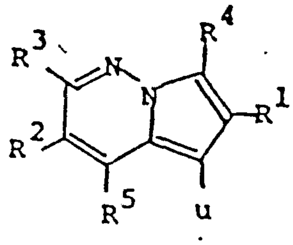

$X_8$

Compounds of Formula $IX_9$ and $X_9$ may be synthesized by known methods or with slight modifications thereof, by one skilled in the arts. Several representative teachings are taught by L. J. S. Knutsen, et al., in J. Chem. Soc., Perkin Trans., 1, 621 (1985) and 229 (1984), M. Iwao, et al. in J. Heterocycl. Chem., 16, 689 (1979) and R.W. Clarke, in Ger. Offen 2804909 (published August 10, 1978).

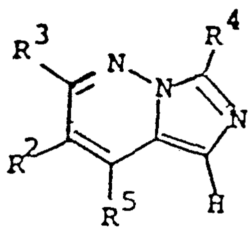

$IX_9$ -

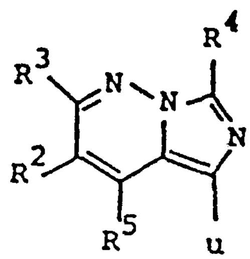

$X_9$

wherein
$R^2$, $R^3$, $R^4$, $R^5$ and U are as previously defined.

Heterocycles of Formula $IX_{10}$ can be synthesized by literature methods (or by modification of these methods) such as those taught by:
- T. Tsuchiya, et al., in Chem. Pharm. Bull., 31, 4568 (1983).
- A. Kakehi, et al., in J. Org. Chem., 42, 443 (1977).
- T. Tsuchiya, et al., in J. Chem. Soc., Chem. Commun., 1109 (1980).
- A. Ohsawa, et al., in J. Org. Chem., 47, 3497 (1982).
- D.E. Kuhla, et al., in "Adv. Heterocycle Chem.", 21, 1-63 (1977).
- U. Burger, et al., in Helv. Chim. Acta., 62, 540 (1975).
- A. Kakehi, et al., in J. Org. Chem., 43, 4837 (1978).
- G. Maury, et al., in Chem. Heterocycl. Compd., 30, 179 (1977).
- H.L. Blewitt in Chem. Heterocycl. Compd., 30, 117 (1977).
- T. Uchida, et al., in Synthesis, 209 (1976)
- E. Kranz, et al., in Ger. Offen. 2220186 (published November 8, 1973), Ger. Offen. 2058500 (published May 31, 1972) and in Chem. Ber., 105 (1972)

The haloheterocycles of Formula $X_{10}$ can be synthesized by halogenating the heterocycles of Formula $IX_{10}$ with halogenating agents.

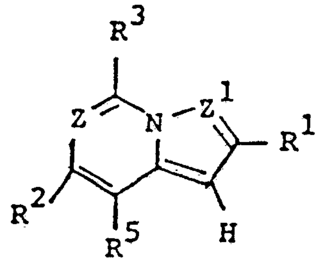

IX10

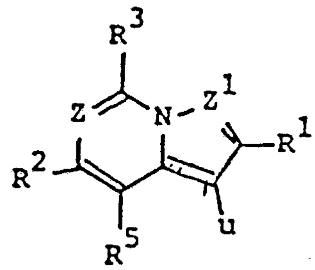

X10

wherein
$R^1$, $R^2$, $R^3$, $R^5$, Z, $Z^1$ and U are as previously defined.

The heterocycles of Formula $IX_{11}$ can be synthesized by literature methods (or by modification of these methods) such as those taught by K. Kasuga, et al., in Yakugaka Zasshi., 94, 952 (1974), M. Muehlstaedt, et al., in J. Prakt. Chem., 311, 363 (1969), and D.E. Kuhla, et al., in Adv. Heterocycl. Chem., 21, (1977).
Jpn. Kokai 84/2053801 also describes the synthesis of some heterocycles of Formula $IX_{11}$. The haloheterocycles of Formula $X_{11}$ can be prepared by halogenating the heterocycles of Formula $IX_{11}$

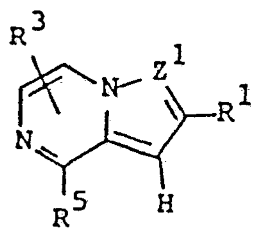

IX11

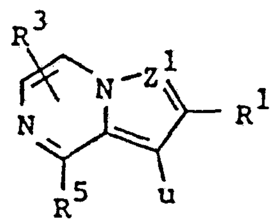

X11

wherein
$R^1$, $R^3$, $R^5$, $Z^1$ and U are as previously defined
The heterocycles of Formula $IX_{12}$ can be synthesized by literature methods (or by modification of these methods) such as those taught by G. Maury, et al., in J. Heterocycl. Chem., 15, 1041 (1978), C. Wentrup in Helv. Chim. Acta., 61, 1755 (1978), S. Mineo, et al., in Synth Commun., 6, 69 (1976) and Y. Tamura, et al., in J. Heterocycl. Chem., 12, 481 (1975).
$X_{12}$ can be prepared by halogenation of the heterocycles of Formula $IX_{12}$ with halogenating agents.

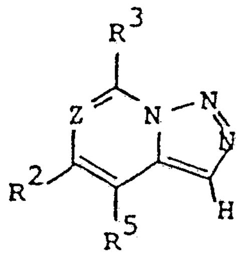

IX12

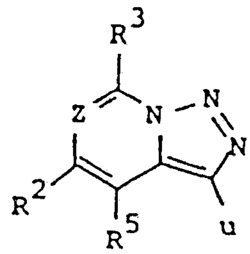

X12

Heterocycles of Formula $IX_{13}$ can be synthesized by literature methods (or by modification of these methods) such as those taught by G. Maury, et al., in Bull. Soc., Chim. Belg., 91, 153 (1982) and C. Wentrup in Helv. Chim. Acta., 61, 1755 (1978). The haloheterocycles of Formula $X_{13}$ can be prepared by halogenation of

the heterocycles of Formula $IX_{13}$.

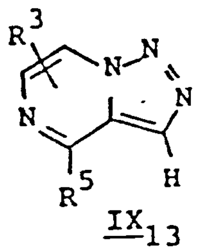

$IX_{13}$

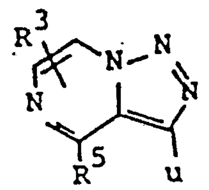

$X_{13}$

The intermediates of Formula XII, $XIII_1$, XIV, XV, XXV, XXVI, $XXVII_1$, $XIII_2$, $XIII_3$, $XXVII_3$, $XIII_4$, $XXVII_4$, $XIII_5$, $XXVII_5$, XXVIII, XXIX, XXX, XXXI, XXXII, XXXIII, XXXIV, XXXV, XXXVI, XXXVII, XXXVIII, XXXIX, XXXX, XXXXI, XXXXII, XXXXIII, XXXXIV and XXXXV can be prepared by methods known in the art or by obvious modification of these known methods.

The synthesis of heterocyclic amines such as those represented by Formula III has been reviewed in "The Chemistry of Heterocyclic Compounds," a series published by Interscience Publ., New York and London. Aminopyrimidines are described by D. J. Brown in "The Pyrimidines," Vol. XVI of the series mentioned above which is herein incorporated by reference. The 2-amino-1,3,5-triazines of Formula III, where A is A-1 and $Z^4$ is N, can be prepared according to methods described by E. M. Smolin and L. Rappaport in "s-Triazines and Derivatives," Vol. XIII.

Pyrimidines of Formula III, where A is A-1 and Y is an acetal or thioacetal substituent, can be prepared by methods taught in European Patent Application No. 84,224 (published July 27, 1983).

Pyrimidines of Formula III, where A is A-1 and Y is cyclopropyl or $OCF_2H$ can be synthesized according to the methods taught in United States Patent 4,515,626 and United States Patent 4,540,782, respectively.

Compounds of Formula III, where A is A-2 or A-3, can be prepared by procedures disclosed in United States Patent 4,339,267.

Compounds of Formula III, where A is A-4, can be prepared by methods taught in United States Patent 4,487,626.

Additional references dealing with the synthesis ob bicyclic pyrimidines of Formula III, where A is A-2, A-3, or A-4 are Braker, Sheehan, Spitzmiller and Lott, J. Am. Chem. Soc., 69, 3072 (1947); Mitler and Bhattacharya, Quart. J. Indian Chem. Soc., 4, 152 (1927); Shrage and Hitchings, J. Org. Chem., 16, 1153 (1951); Caldwell, Kornfeld and Donnell, J. Am. Chem. Soc., 63, 2188 (1941); and Fissekis, Myles and Brown, J. Org. Chem., 29, 2670 (1964).

Compounds of Formula III, where A is A-5, can be prepared by methods taught in the United States Patent 4,421,550.

Compounds of Formula III, where A is A-6, can be prepared by methods taught in the United States Patent 4,496,392.

## Example 1

N-[[4,6-Dimethoxy-2-pyrimidinyl)amino]carbonyl]-5,7-dimethylpyrazolo[1,5-a]pyrimidine-3-sulfonamide

To a suspension of 0.18 g of 5,7-dimethylpyrazole[1,5-a] pyrimidine-3-sulfonamide and 0.22 g of phenyl 4,6-dimethoxy-2-pyrimidinylcarbamate in 4 ml of anhydrous acetonitrile was added 0.12 ml of 1,8-diazabicyclo[5,4,0]undec-7-ene (DBU) dropwise with stirring. The resulting solution was then stirred at room temperature for 2 hours. To this solution was added 4 ml of water. The resulting solution was then acidified with 10% HCl aqueous solution until pH$\sim$3. The solid was collected by filtration, washed with water and ether, and dried in oven (for 12 hours at 50°C) to give 0.21 g of the desired product. mp 166-168°C, NMR ($CDCl_3$) $\delta$:
2.54 (s. 3H);
2.78 (s. 3H);
4.03 (s. 6H);
5.79 (s. 1H);
6.8 (s. 1H);
7.2 (s. 1H);
8.62 (s. 1H);
12.9 (s. 1H).

Example 2

5,7-dimethylpyrazolo[1,5-a]pyrimidine-3-sulfonamide[1]

To a 3-neck round bottom flask equipped with dry ice condenser and thermometer was added 3.55 g of 5,7-dimethylpyrazolo[1,5-a]pyrimidine-3-sulfonylchloride and 70 ml of anhydrous tetrahydrofuran. To the resulting solution was added 6 ml of anhydrous ammonia through dry ice condenser. The resulting mixture was stirred at ~0°C for 2 hours and then at room temperature for 11 hours. The solid from the reaction mixture was collected by filtration, washed with tetrahydrofuran, water and then ether, and dried in oven (at 50°C for 12 hours) to give 1.82 g of the desired product; m.p. 213-215°C. The tetrahydrofuran filtrate and wash were combined, concentrated, and dried in oven (at 50°C for 12 hours) to give additional 1.02 g of the desired product. m.p. 207-208°C, NMR (DMSO-$d_6$) δ: 2.58 (s. 3H);
2.71 (s. 3H);
7.13 (s. 1H);
7.28 (s. 2H);
8.36 (s. 1H).

[1] 5,7-Dimethylpyrazolo[1,5-a]pyrimidine-3-sulfonamide may also be prepared by the method disclosed by W.E. Kirkpatrick, et al., in J. Med. Chem. 20(3) 386 (1977).

## General Formulas For Tables

### General Formula 1

O
(L-1)$SO_2$NHCN(A-1)
R

### General Formula 2

O
(L-2)$SO_2$NHCN(A-1)
R

### General Formula 3

O
(L-3)$SO_2$NHCN(A-1)
R

### General Formula 4

O
(L-4)$SO_2$NHCN(A-1)
R

### General Formula 5

O
(L-5)$SO_2$NHCN(A-1)
R

### General Formula 6

O
(L-6)$SO_2$NHCN(A-1)
R

### General Formula 7

O
(L-7)$SO_2$NHCN(A-1)
R

## General Formulas For Tables

### General Formula 8

O
(L-8)$SO_2$NHCN(A-1)
R

### General Formula 9

O
(L-9)$SO_2$NHCN(A-1)
R

### General Formula 10

O
(L-10)$SO_2$NHCN(A-1)
R

### General Formula 11

O
(L-11)$SO_2$NHCN(A-1)
R

### General Formula 12

O
(L-12)$SO_2$NHCN(A-1)
R

### General Formula 13

O
(L-13)$SO_2$NHCN(A-1)
R

### General Formula 14

O
(L-14)$SO_2$NHCN(A-1)
R

## General Formulas For Tables

### General Formula 15

$$(L\text{-}15)SO_2NH\overset{O}{\overset{\|}{C}}\underset{R}{N}(A\text{-}1)$$

### General Formula 16

$$(L\text{-}16)SO_2NH\overset{O}{\overset{\|}{C}}\underset{R}{N}(A\text{-}1)$$

### General Formula 17

$$(L\text{-}17)SO_2NH\overset{O}{\overset{\|}{C}}\underset{R}{N}(A\text{-}1)$$

### General Formula 18

$$(L\text{-}18)SO_2NH\overset{O}{\overset{\|}{C}}\underset{R}{N}(A\text{-}1)$$

### General Formula 19

$$(L\text{-}19)SO_2NH\overset{O}{\overset{\|}{C}}\underset{R}{N}(A\text{-}1)$$

### General Formula 20

$$(L\text{-}20)SO_2NH\overset{O}{\overset{\|}{C}}\underset{R}{N}(A\text{-}1)$$

Table 1

General Formula 1

| Z | $R^1$ | $R^2$ | $R^3$ | R | X | Y | $Z^4$ |
|---|---|---|---|---|---|---|---|
| CH | H | $CH_3$ | $CH_3$ | H | $OCH_3$ | $OCH_3$ | CH |
| CH | H | $CH_3$ | $CH_3$ | H | $OCH_3$ | $CH_3$ | CH |
| CH | H | $CH_3$ | $CH_3$ | H | Cl | $OCH_3$ | CH |
| CH | H | $CH_3$ | $CH_3$ | H | $CH_3$ | $CH_3$ | CH |
| CH | H | $CH_3$ | $CH_3$ | H | $OCH_3$ | $OCH_3$ | N |
| CH | H | $CH_3$ | $CH_3$ | H | $OCH_3$ | $CH_3$ | N |
| CH | H | $CH_3$ | $CH_3$ | H | $OC_2H_5$ | $NHCH_3$ | N |
| CH | H | $CH_3$ | $CH_3$ | $CH_3$ | $OCH_3$ | $CH_3$ | N |
| CH | Cl | $CH_3$ | $CH_3$ | H | $OCH_3$ | $OCH_3$ | CH |
| CH | Cl | $CH_3$ | $CH_3$ | H | $OCH_3$ | $CH_3$ | CH |
| CH | Cl | $CH_3$ | $CH_3$ | H | Cl | $OCH_3$ | CH |
| CH | Cl | $CH_3$ | $CH_3$ | H | $CH_3$ | $CH_3$ | CH |
| CH | Cl | $CH_3$ | $CH_3$ | H | $OCH_2CF_3$ | $N(CH_3)_2$ | N |
| CH | Cl | $CH_3$ | $CH_3$ | H | $OCH_3$ | $OCH_3$ | N |
| CH | Cl | $CH_3$ | $CH_3$ | H | $OCH_3$ | $N(CH_3)_2$ | N |
| CH | Cl | $CH_3$ | $CH_3$ | H | $OCH_3$ | $CH_3$ | N |
| CH | Cl | $CH_3$ | $CH_3$ | H | $OC_2H_5$ | $OC_2H_5$ | N |
| CH | Cl | $CH_3$ | $CH_3$ | H | $OCH_2CF_3$ | $NHCH_3$ | N |
| CH | Cl | $CH_3$ | $CH_3$ | H | $OC_2H_5$ | $NHCH_3$ | N |
| CH | Cl | $CH_3$ | $CH_3$ | H | $OC_2H_5$ | $N(CH_3)_2$ | N |
| CH | Cl | $CH_3$ | $CH_3$ | $CH_3$ | $OCH_3$ | $CH_3$ | N |
| CH | $SO_2CH_3$ | $CH_3$ | $CH_3$ | H | $OCH_3$ | $CH_3$ | CH |
| CH | $SO_2CH_3$ | $CH_3$ | $CH_3$ | H | Cl | $OCH_3$ | CH |
| CH | $SO_2CH_3$ | $CH_3$ | $CH_3$ | H | $CH_3$ | $CH_3$ | CH |
| CH | $SO_2CH_3$ | $CH_3$ | $CH_3$ | H | $OCH_3$ | $OCH_3$ | N |
| CH | $SO_2CH_3$ | $CH_3$ | $CH_3$ | H | $OCH_3$ | $CH_3$ | N |
| CH | $SO_2CH_3$ | $CH_3$ | $CH_3$ | H | $OC_2H_5$ | $NHCH_3$ | N |

Table 1 (continued)

General Formula 1

| Z | $R^1$ | $R^2$ | $R^3$ | R | X | Y | $Z^4$ |
|---|---|---|---|---|---|---|---|
| CH | $SO_2CH_3$ | $CH_3$ | $CH_3$ | $CH_3$ | $OCH_3$ | $CH_3$ | N |
| CH | $SO_2CH_3$ | $CH_3$ | $CH_3$ | H | $OCH_3$ | $OCH_3$ | CH |
| CH | $SCH_3$ | $CH_3$ | $CH_3$ | H | $CH_3$ | $OCH_3$ | N |
| CH | $SCH_3$ | $CH_3$ | $CH_3$ | H | $OCH_3$ | $OCH_3$ | N |
| CH | $SCH_3$ | $CH_3$ | $CH_3$ | H | Cl | $OCH_3$ | CH |
| CH | $SCH_3$ | $CH_3$ | $CH_3$ | H | $CH_3$ | $OCH_3$ | CH |
| CH | $SCH_3$ | $CH_3$ | $CH_3$ | H | $OCH_3$ | $OCH_3$ | CH |
| CH | $SCH_2CH_2CH_3$ | $CH_3$ | $CH_3$ | H | $OCH_3$ | $OCH_3$ | CH |
| CH | $SCH_2CH_2CH_3$ | $CH_3$ | $CH_3$ | H | $CH_3$ | $OCH_3$ | CH |
| CH | $SHC_2CH_2CH_3$ | $CH_3$ | $CH_3$ | H | Cl | $OCH_3$ | CH |
| CH | $SCH_2CH_2CH_3$ | $CH_3$ | $CH_3$ | H | $CH_3$ | $CH_3$ | CH |
| CH | $SCH_2CH_2CH_3$ | $CH_3$ | $CH_3$ | H | $CH_3$ | $OCH_3$ | N |
| CH | $SO_2CH_2CH_2CH_3$ | $CH_3$ | $CH_3$ | H | $OCH_3$ | $OCH_3$ | CH |
| CH | $SO_2CH_2CH_2CH_3$ | $CH_3$ | $CH_3$ | H | $CH_3$ | $OCH_3$ | CH |
| CH | $SO_2CH_2CH_2CH_3$ | $CH_3$ | $CH_3$ | H | Cl | $OCH_3$ | CH |
| CH | $SO_2CH_2CH_2CH_3$ | $CH_3$ | $CH_3$ | H | $CH_3$ | $CH_3$ | CH |
| CH | $SO_2CH_2CH_2CH_3$ | $CH_3$ | $CH_3$ | H | $OCH_3$ | $OCH_3$ | N |
| CH | $SO_2CH_2CH_2CH_3$ | $CH_3$ | $CH_3$ | H | $CH_3$ | $OCH_3$ | N |
| CH | $SCH_2CH_3$ | $CH_3$ | $CH_3$ | H | $OCH_3$ | $OCH_3$ | CH |
| CH | $SCH_2CH_3$ | $CH_3$ | $CH_3$ | H | $CH_3$ | $OCH_3$ | CH |
| CH | $SCH_2CH_3$ | $CH_3$ | $CH_3$ | H | Cl | $OCH_3$ | CH |
| CH | $SCH_2CH_3$ | $CH_3$ | $CH_3$ | H | $CH_3$ | $CH_3$ | CH |
| CH | $SCH_2CH_3$ | $CH_3$ | $CH_3$ | H | $OCH_3$ | $OCH_3$ | N |
| CH | $SCH_2CH_3$ | $CH_3$ | $CH_3$ | H | $CH_3$ | $OCH_3$ | N |
| CH | $SO_2CH_2CH_3$ | $CH_3$ | $CH_3$ | H | $OCH_3$ | $OCH_3$ | CH |
| CH | $SO_2CH_2CH_3$ | $CH_3$ | $CH_3$ | H | $CH_3$ | $OCH_3$ | CH |
| CH | $SO_2CH_2CH_3$ | $CH_3$ | $CH_3$ | H | Cl | $OCH_3$ | CH |
| CH | $SO_2CH_2CH_3$ | $CH_3$ | $CH_3$ | H | $OCH_3$ | $OCH_3$ | N |
| CH | $SO_2CH_2CH_3$ | $CH_3$ | $CH_3$ | H | $CH_3$ | $OCH_3$ | N |
| CH | $SO_2CH_2CH_3$ | $CH_3$ | $CH_3$ | H | $CH_3$ | $CH_3$ | CH |

Table 1 (continued)
General Formula 1

| Z | $R^1$ | $R^2$ | $R^3$ | R | X | Y | $Z^4$ |
|---|---|---|---|---|---|---|---|
| CH | $SO_2N(CH_3)_2$ | $CH_3$ | $CH_3$ | H | $OCH_3$ | $OCH_3$ | CH |
| CH | $SO_2N(CH_3)_2$ | $CH_3$ | $CH_3$ | H | $OCH_3$ | $CH_3$ | CH |
| CH | $SO_2N(CH_3)_2$ | $CH_3$ | $CH_3$ | H | Cl | $OCH_3$ | CH |
| CH | $SO_2N(CH_3)_2$ | $CH_3$ | $CH_3$ | H | $OCH_3$ | $OCH_3$ | N |
| CH | $SO_2N(CH_3)_2$ | $CH_3$ | $CH_3$ | H | $OCH_3$ | $CH_3$ | N |
| CH | H | H | H | H | $OCH_3$ | $OCH_3$ | CH |
| CH | H | H | H | H | $OCH_3$ | $CH_3$ | CH |
| CH | H | H | H | H | Cl | $OCH_3$ | CH |
| CH | H | H | H | H | $CH_3$ | $CH_3$ | CH |
| CH | H | H | H | H | $OCH_3$ | $OCH_3$ | N |
| CH | H | H | H | H | $OCH_3$ | $CH_3$ | N |
| CH | H | H | H | H | $OC_2H_5$ | $NHCH_3$ | N |
| CH | H | H | H | $CH_3$ | $OCH_3$ | $CH_3$ | N |
| CH | Cl | H | H | H | $OCH_3$ | $OCH_3$ | CH |
| CH | Cl | H | H | H | $OCH_3$ | $CH_3$ | CH |
| CH | Cl | H | H | H | Cl | $OCH_3$ | CH |
| CH | Cl | H | H | H | $CH_3$ | $CH_3$ | CH |
| CH | Cl | H | H | H | $OCH_3$ | $OCH_3$ | N |
| CH | Cl | H | H | H | $OCH_3$ | $CH_3$ | N |
| CH | Cl | H | H | H | $OCH_3$ | $CH_3$ | N |
| CH | Cl | H | H | H | $OC_2H_5$ | $NHCH_3$ | N |

Table 1 (continued)

General Formula 1

| Z | $R^1$ | $R^2$ | $R^3$ | R | X | Y | $Z^4$ |
|---|---|---|---|---|---|---|---|
| CH | Cl | H | H | $CH_3$ | $OCH_3$ | $CH_3$ | N |
| CH | $SO_2CH_3$ | H | H | H | $OCH_3$ | $OCH_3$ | CH |
| CH | $SO_2CH_3$ | H | H | H | $OCH_3$ | $CH_3$ | CH |
| CH | $SO_2CH_3$ | H | H | H | Cl | $OCH_3$ | CH |
| CH | $SO_2CH_3$ | H | H | H | $CH_3$ | $CH_3$ | CH |
| CH | $SO_2CH_3$ | H | H | H | $OCH_3$ | $OCH_3$ | N |
| CH | $SO_2CH_3$ | H | H | H | $OCH_3$ | $CH_3$ | N |
| CH | $SO_2CH_3$ | H | H | N | $OC_2H_5$ | $NHCH_3$ | N |
| CH | $SO_2CH_3$ | H | H | $CH_3$ | $OCH_3$ | $CH_3$ | N |
| CH | $SO_2N(CH_3)_2$ | H | H | H | $OCH_3$ | $OCH_3$ | CH |
| CH | $SO_2N(CH_3)_2$ | H | H | H | $OCH_3$ | $CH_3$ | CH |
| CH | $SO_2N(CH_3)_2$ | H | H | H | Cl | $OCH_3$ | CH |
| CH | $SO_2N(CH_3)_2$ | H | H | H | $OCH_3$ | $OCH_3$ | N |
| CH | $SO_2N(CH_3)_2$ | H | H | H | $OCH_3$ | $CH_3$ | N |
| CH | H | H | $CH_3$ | H | $OCH_3$ | $OCH_3$ | CH |
| CH | H | H | $CH_3$ | H | $OCH_3$ | $CH_3$ | CH |
| CH | H | H | $CH_3$ | H | Cl | $OCH_3$ | CH |
| CH | H | H | $CH_3$ | H | $CH_3$ | $CH_3$ | CH |
| CH | H | H | $CH_3$ | H | $OCH_3$ | $OCH_3$ | N |
| CH | H | H | $CH_3$ | H | $OCH_3$ | $CH_3$ | N |
| CH | H | H | $CH_3$ | H | $OC_2H_5$ | $NHCH_3$ | N |
| CH | H | H | $CH_3$ | H | $OCH_3$ | $CH_3$ | N |
| CH | Cl | H | $CH_3$ | H | $OCH_3$ | $OCH_3$ | CH |

Table 1 (continued)

General Formula 1

| Z | $R^1$ | $R^2$ | $R^3$ | R | X | Y | $Z^4$ |
|---|---|---|---|---|---|---|---|
| CH | Cl | H | $CH_3$ | H | $OCH_3$ | $CH_3$ | CH |
| CH | Cl | H | $CH_3$ | H | Cl | $OCH_3$ | CH |
| CH | Cl | H | $CH_3$ | H | $CH_3$ | $CH_3$ | CH |
| CH | Cl | H | $CH_3$ | H | $OCH_3$ | $OCH_3$ | N |
| CH | Cl | H | $CH_3$ | H | $OCH_3$ | $CH_3$ | N |
| CH | Cl | H | $CH_3$ | H | $OC_2H_5$ | $NHCH_3$ | N |
| CH | Cl | H | $CH_3$ | $CH_3$ | $OCH_3$ | $CH_3$ | N |
| CH | $SO_2CH_3$ | H | $CH_3$ | H | $OCH_3$ | $OCH_3$ | CH |
| CH | $SO_2CH_3$ | H | $CH_3$ | H | $OCH_3$ | $CH_3$ | CH |
| CH | $SO_2CH_3$ | H | $CH_3$ | H | Cl | $OCH_3$ | CH |
| CH | $SO_2CH_3$ | H | $CH_3$ | H | $CH_3$ | $CH_3$ | CH |
| CH | $SO_2CH_3$ | H | $CH_3$ | H | $OCH_3$ | $OCH_3$ | N |
| CH | $SO_2CH_3$ | H | $CH_3$ | H | $OCH_3$ | $CH_3$ | N |
| CH | $SO_2CH_3$ | H | $CH_3$ | H | $OC_2H_5$ | $NHCH_3$ | N |
| CH | $SO_2CH_3$ | H | $CH_3$ | $CH_3$ | $OCH_3$ | $CH_3$ | N |
| CH | $SO_2N(CH_3)_2$ | H | $CH_3$ | H | $OCH_3$ | $OCH_3$ | CH |
| CH | $SO_2N(CH_3)_2$ | H | $CH_3$ | H | $OCH_3$ | $CH_3$ | CH |
| CH | $SO_2N(CH_3)_2$ | H | $CH_3$ | H | Cl | $OCH_3$ | CH |
| CH | $SO_2N(CH_3)_2$ | H | $CH_3$ | H | $OCH_3$ | $OCH_3$ | N |
| CH | $SO_2N(CH_3)_2$ | H | $CH_3$ | H | $OCH_3$ | $CH_3$ | N |
| CH | H | $CH_3$ | H | H | $OCH_3$ | $OCH_3$ | CH |
| CH | H | $CH_3$ | H | H | $OCH_3$ | $CH_3$ | CH |

Table 1 (continued)

General Formula 1

| Z | $R^1$ | $R^2$ | $R^3$ | R | X | Y | $Z^4$ |
|---|---|---|---|---|---|---|---|
| CH | H | $CH_3$ | H | H | Cl | $OCH_3$ | CH |
| CH | H | $CH_3$ | H | H | $CH_3$ | $CH_3$ | CH |
| CH | H | $CH_3$ | H | H | $OCH_3$ | $OCH_3$ | N |
| CH | H | $CH_3$ | H | H | $OCH_3$ | $CH_3$ | N |
| CH | H | $CH_3$ | H | H | $OC_2H_5$ | $NHCH_3$ | N |
| CH | H | $CH_3$ | H | H | $OCH_3$ | $CH_3$ | N |
| CH | Cl | $CH_3$ | H | H | $OCH_3$ | $OCH_3$ | CH |
| CH | Cl | $CH_3$ | H | H | $OCH_3$ | $CH_3$ | CH |
| CH | Cl | $CH_3$ | H | H | Cl | $OCH_3$ | CH |
| CH | Cl | $CH_3$ | H | H | $CH_3$ | $CH_3$ | CH |
| CH | Cl | $CH_3$ | H | H | $OCH_3$ | $OCH_3$ | N |
| CH | Cl | $CH_3$ | H | H | $OCH_3$ | $CH_3$ | N |
| CH | Cl | $CH_3$ | H | H | $OC_2H_5$ | $NHCH_3$ | N |
| CH | Cl | $CH_3$ | H | $CH_3$ | $OCH_3$ | $CH_3$ | N |
| CH | $SO_2CH_3$ | CH | H | H | $OCH_3$ | $OCH_3$ | CH |
| CH | $SO_2CH_3$ | $CH_3$ | H | H | $OCH_3$ | $CH_3$ | CH |
| CH | $SO_2CH_3$ | $CH_3$ | H | H | Cl | $OCH_3$ | CH |
| CH | $SO_2CH_3$ | $CH_3$ | H | H | $CH_3$ | $CH_3$ | CH |
| CH | $SO_2CH_3$ | $CH_3$ | H | H | $OCH_3$ | $OCH_3$ | N |
| CH | $SO_2CH_3$ | $CH_3$ | H | H | $OCH_3$ | $CH_3$ | N |
| CH | $SO_2CH_3$ | $CH_3$ | H | H | $OC_2H_5$ | $NHCH_3$ | N |
| CH | $SO_2CH_3$ | $CH_3$ | H | $CH_3$ | $OCH_3$ | $CH_3$ | N |

Table 1 (continued)

General Formula 1

| Z | $R^1$ | $R^2$ | $R^3$ | R | X | Y | $Z^4$ |
|---|---|---|---|---|---|---|---|
| CH | $SO_2N(CH_3)_2$ | CH | H | H | $OCH_3$ | $OCH_3$ | CH |
| CH | $SO_2N(CH_3)_2$ | $CH_3$ | H | H | $OCH_3$ | $CH_3$ | CH |
| CH | $SO_2N(CH_3)_2$ | $CH_3$ | H | H | Cl | $OCH_3$ | CH |
| CH | $SO_2N(CH_3)_2$ | $CH_3$ | H | H | $OCH_3$ | $OCH_3$ | N |
| CH | $SO_2N(CH_3)_2$ | $CH_3$ | H | H | OCH | $CH_3$ | N |
| N | H | $CH_3$ | $CH_3$ | H | $OCH_3$ | $OCH_3$ | CH |
| N | H | $CH_3$ | $CH_3$ | H | $OCH_3$ | $CH_3$ | CH |
| N | H | $CH_3$ | $CH_3$ | H | Cl | $OCH_3$ | CH |
| N | H | $CH_3$ | $CH_3$ | H | $CH_3$ | $CH_3$ | CH |
| N | H | $CH_3$ | $CH_3$ | H | $OCH_3$ | $OCH_3$ | N |
| N | H | $CH_3$ | $CH_3$ | H | $OCH_3$ | $CH_3$ | N |
| N | H | $CH_3$ | $CH_3$ | H | $OC_2H_5$ | $NHCH_3$ | N |
| N | H | $CH_3$ | $CH_3$ | CH | $OCH_3$ | $CH_3$ | N |
| N | Cl | $CH_3$ | $CH_3$ | H | $OCH_3$ | $OCH_3$ | CH |
| N | Cl | $CH_3$ | $CH_3$ | H | $OCH_3$ | $CH_3$ | CH |
| N | Cl | $CH_3$ | $CH_3$ | H | Cl | $OCH_3$ | CH |
| N | Cl | $CH_3$ | $CH_3$ | H | $CH_3$ | $CH_3$ | CH |
| N | Cl | $CH_3$ | $CH_3$ | H | $OCH_3$ | $OCH_3$ | N |
| N | Cl | $CH_3$ | $CH_3$ | H | $OCH_3$ | $CH_3$ | N |
| N | Cl | $CH_3$ | $CH_3$ | H | $OC_2H_5$ | $NHCH_3$ | N |
| N | Cl | $CH_3$ | $CH_3$ | $CH_3$ | $OCH_3$ | $CH_3$ | N |
| N | $SO_2CH_3$ | $CH_3$ | $CH_3$ | H | $OCH_3$ | $OCH_3$ | CH |
| N | $SO_2CH_3$ | CH | CH | H | $OCH_3$ | $CH_3$ | CH |

Table 1 (continued)

General Formula 1

| Z | $R^1$ | $R^2$ | $R^3$ | R | X | Y | $Z^4$ |
|---|---|---|---|---|---|---|---|
| N | $SO_2CH_3$ | $CH_3$ | $CH_3$ | H | Cl | $OCH_3$ | CH |
| N | $SO_2CH_3$ | $CH_3$ | $CH_3$ | H | $CH_3$ | $CH_3$ | CH |
| N | $SO_2CH_3$ | $CH_3$ | $CH_3$ | H | $OCH_3$ | $OCH_3$ | N |
| N | $SO_2CH_3$ | $CH_3$ | $CH_3$ | H | $OCH_3$ | $CH_3$ | N |
| N | $SO_2CH_3$ | $CH_3$ | $CH_3$ | H | $OC_2H_5$ | $NHCH_3$ | N |
| N | $SO_2CH_3$ | $CH_3$ | $CH_3$ | $CH_3$ | $OCH_3$ | $CH_3$ | N |
| N | $SO_2N(CH_3)_2$ | $CH_3$ | $CH_3$ | H | $OCH_3$ | $OCH_3$ | CH |
| N | $SO_2N(CH_3)_2$ | $CH_3$ | $CH_3$ | H | $OCH_3$ | $CH_3$ | CH |
| N | $SO_2N(CH_3)_2$ | $CH_3$ | $CH_3$ | H | Cl | $OCH_3$ | CH |
| N | $SO_2N(CH_3)_2$ | $CH_3$ | $CH_3$ | H | $OCH_3$ | $OCH_3$ | N |
| N | $SO_2N(CH_3)_2$ | $CH_3$ | $CH_3$ | H | $OCH_3$ | $CH_3$ | N |
| N | H | H | H | H | $OCH_3$ | $OCH_3$ | CH |
| N | H | H | H | H | $OCH_3$ | $CH_3$ | CH |
| N | H | H | H | H | Cl | $OCH_3$ | CH |
| N | H | H | H | H | $CH_3$ | $CH_3$ | CH |
| N | H | H | H | H | $OCH_3$ | $OCH_3$ | N |
| N | H | H | H | H | $OCH_3$ | $CH_3$ | N |
| N | H | H | H | H | $OC_2H_5$ | $NHCH_3$ | N |
| N | H | H | H | $CH_3$ | $OCH_3$ | $CH_3$ | N |
| N | Cl | H | H | H | $OCH_3$ | $OCH_3$ | CH |
| N | Cl | H | H | H | $OCH_3$ | $CH_3$ | CH |
| N | Cl | H | H | H | Cl | $OCH_3$ | CH |

Table 1 (continued)

General Formula 1

| Z | $R^1$ | $R^2$ | $R^3$ | R | X | Y | $Z^4$ |
|---|---|---|---|---|---|---|---|
| N | Cl | H | H | H | $CH_3$ | $CH_3$ | CH |
| N | Cl | H | H | H | $OCH_3$ | $OCH_3$ | N |
| N | Cl | H | H | H | $OCH_3$ | $CH_3$ | N |
| N | Cl | H | H | H | $OC_2H_5$ | $NHCH_3$ | N |
| N | Cl | H | H | $CH_3$ | $OCH_3$ | $CH_3$ | N |
| N | $SO_2CH_3$ | H | H | H | $OCH_3$ | $OCH_3$ | CH |
| N | $SO_2CH_3$ | H | H | H | $OCH_3$ | $CH_3$ | CH |
| N | $SO_2CH_3$ | H | H | H | Cl | $OCH_3$ | CH |
| N | $SO_2CH_3$ | H | H | H | $CH_3$ | $CH_3$ | CH |
| N | $SO_2CH_3$ | H | H | H | $OCH_3$ | $OCH_3$ | N |
| N | $SO_2CH_3$ | H | H | H | $OCH_3$ | $CH_3$ | N |
| N | $SO_2CH_3$ | H | H | H | $OC_2H_5$ | $NHCH_3$ | N |
| N | $SO_2CH_3$ | H | H | $CH_3$ | $OCH_3$ | $CH_3$ | N |
| N | $SO_2N(CH_3)_2$ | H | H | H | $OCH_3$ | $OCH_3$ | CH |
| N | $SO_2N(CH_3)_2$ | H | H | H | $OCH_3$ | $CH_3$ | CH |
| N | $SO_2N(CH_3)_2$ | H | H | H | Cl | $OCH_3$ | CH |
| N | $SO_2N(CH_3)_2$ | H | H | H | $OCH_3$ | $OCH_3$ | N |
| N | $SO_2N(CH_3)_2$ | H | H | H | $OCH_3$ | $CH_3$ | N |
| N | H | H | $CH_3$ | H | $OCH_3$ | $OCH_3$ | CH |
| N | H | H | $CH_3$ | H | $OCH_3$ | $CH_3$ | CH |
| N | H | H | $CH_3$ | H | Cl | $OCH_3$ | CH |
| N | H | H | $CH_3$ | H | $CH_3$ | $CH_3$ | CH |

Table 1 (continued)

General Formula 1

| Z | $R^1$ | $R^2$ | $R^3$ | R | X | Y | $Z^4$ |
|---|---|---|---|---|---|---|---|
| N | H | H | $CH_3$ | H | $OCH_3$ | $OCH_3$ | N |
| N | H | H | $CH_3$ | H | $OCH_3$ | $CH_3$ | N |
| N | H | H | $CH_3$ | H | $OC_2H_5$ | $NHCH_3$ | N |
| N | H | H | $CH_3$ | H | $OCH_3$ | $CH_3$ | N |
| N | Cl | H | $CH_3$ | H | $OCH_3$ | $OCH_3$ | CH |
| N | Cl | H | $CH_3$ | H | $OCH_3$ | $CH_3$ | CH |
| N | Cl | H | $CH_3$ | H | Cl | $OCH_3$ | CH |
| N | Cl | H | $CH_3$ | H | $CH_3$ | $CH_3$ | CH |
| N | Cl | H | $CH_3$ | H | $OCH_3$ | $OCH_3$ | N |
| N | Cl | H | $CH_3$ | H | $OCH_3$ | $CH_3$ | N |
| N | Cl | H | $CH_3$ | H | $OC_2H_5$ | $NHCH_3$ | N |
| N | Cl | H | $CH_3$ | $CH_3$ | $OCH_3$ | $CH_3$ | N |
| N | $CO_2CH_3$ | H | $CH_3$ | H | $OCH_3$ | $OCH_3$ | CH |
| N | $CO_2CH_3$ | H | $CH_3$ | H | $OCH_3$ | $CH_3$ | CH |
| N | $CO_2CH_3$ | H | $CH_3$ | H | Cl | $OCH_3$ | CH |
| N | $CO_2CH_3$ | H | $CH_3$ | H | $CH_3$ | $CH_3$ | CH |
| N | $CO_2CH_3$ | H | $CH_3$ | H | $OCH_3$ | $OCH_3$ | N |
| N | $CO_2CH_3$ | H | $CH_3$ | H | $OCH_3$ | $CH_3$ | N |
| N | $CO_2CH_3$ | H | $CH_3$ | H | $OC_2H_5$ | $NHCH_3$ | N |
| N | $CO_2CH_3$ | H | $CH_3$ | $CH_3$ | $OCH_3$ | $CH_3$ | N |
| N | $SO_2N(CH_3)_2$ | H | $CH_3$ | H | $OCH_3$ | $OCH_3$ | CH |
| N | $SO_2N(CH_3)_2$ | H | $CH_3$ | H | $OCH_3$ | $CH_3$ | CH |

Table 1 (continued)

General Formula 1

| Z | $R^1$ | $R^2$ | $R^3$ | R | X | Y | $Z^1$ |
|---|---|---|---|---|---|---|---|
| N | $SO_2N(CH_3)_2$ | H | $CH_3$ | H | Cl | $OCH_3$ | CH |
| N | $SO_2N(CH_3)_2$ | H | $CH_3$ | H | $OCH_3$ | $OCH_3$ | N |
| N | $SO_2N(CH_3)_2$ | H | $CH_3$ | H | $OCH_3$ | $CH_3$ | N |
| N | H | $CH_3$ | H | H | $OCH_3$ | $OCH_3$ | CH |
| N | H | $CH_3$ | H | H | $OCH_3$ | $CH_3$ | CH |
| N | H | $CH_3$ | H | H | Cl | $OCH_3$ | CH |
| N | H | $CH_3$ | H | H | $CH_3$ | $CH_3$ | CH |
| N | H | $CH_3$ | H | H | $OCH_3$ | $OCH_3$ | N |
| N | H | $CH_3$ | H | H | $OCH_3$ | $CH_3$ | N |
| N | H | $CH_3$ | H | H | $OC_2H_5$ | $NHCH_3$ | N |
| N | H | $CH_3$ | H | $CH_3$ | $OCH_3$ | $CH_3$ | N |
| N | Cl | $CH_3$ | H | H | $OCH_3$ | $OCH_3$ | CH |
| N | Cl | $CH_3$ | H | H | $OCH_3$ | $CH_3$ | CH |
| N | Cl | $CH_3$ | H | H | Cl | $OCH_3$ | CH |
| N | Cl | $CH_3$ | H | H | $CH_3$ | $CH_3$ | CH |
| N | Cl | $CH_3$ | H | H | $OCH_3$ | $OCH_3$ | N |
| N | Cl | $CH_3$ | H | H | $OCH_3$ | $CH_3$ | N |
| N | Cl | $CH_3$ | H | H | $OC_2H_5$ | $NHCH_3$ | N |
| N | Cl | $CH_3$ | H | $CH_3$ | $OCH_3$ | $CH_3$ | N |
| N | $SO_2CH_3$ | $CH_3$ | H | H | $OCH_3$ | $OCH_3$ | CH |
| N | $SO_2CH_3$ | $CH_3$ | H | H | $OCH_3$ | $CH_3$ | CH |
| N | $SO_2CH_3$ | $CH_3$ | H | H | Cl | $OCH_3$ | CH |
| N | $SO_2CH_3$ | $CH_3$ | H | H | $CH_3$ | $CH_3$ | CH |

Table 1 (continued)

General Formula 1

| Z | $R^1$ | $R^2$ | $R^3$ | R | X | Y | $Z^4$ |
|---|---|---|---|---|---|---|---|
| N | $SO_2CH_3$ | $CH_3$ | H | H | $OCH_3$ | $OCH_3$ | N |
| N | $SO_2CH_3$ | $CH_3$ | H | H | $OCH_3$ | $CH_3$ | N |
| N | $SO_2CH_3$ | $CH_3$ | H | H | $OC_2H_5$ | $NHCH_3$ | N |
| N | $SO_2CH_3$ | $CH_3$ | H | $CH_3$ | $OCH_3$ | $CH_3$ | N |
| N | $SO_2N(CH_3)_2$ | $CH_3$ | H | H | $OCH_3$ | $OCH_3$ | CH |
| N | $SO_2N(CH_3)_2$ | $CH_3$ | H | H | $OCH_3$ | $CH_3$ | CH |
| N | $SO_2N(CH_3)_2$ | $CH_3$ | H | H | Cl | $OCH_3$ | CH |
| N | $SO_2N(CH_3)_2$ | $CH_3$ | H | H | $OCH_3$ | $OCH_3$ | N |
| N | $SO_2N(CH_3)_2$ | $CH_3$ | H | H | $OCH_3$ | $CH_3$ | N |
| CH | $CO_2CH_3$ | $CH_3$ | $CH_3$ | H | $OCH_3$ | $OCH_3$ | CH |
| CH | $CO_2CH_3$ | $CH_3$ | $CH_3$ | H | $OCH_3$ | $CH_3$ | CH |
| CH | $CO_2CH_3$ | $CH_3$ | $CH_3$ | H | Cl | $OCH_3$ | CH |
| CH | $CO_2CH_3$ | $CH_3$ | $CH_3$ | H | $CH_3$ | $CH_3$ | CH |
| CH | $CO_2CH_3$ | $CH_3$ | $CH_3$ | H | $OCH_3$ | $OCH_3$ | N |
| CH | $CO_2CH_3$ | $CH_3$ | $CH_3$ | H | $OCH_3$ | $CH_3$ | N |
| CH | $CO_2CH_3$ | $CH_3$ | $CH_3$ | H | $OC_2H_5$ | $NHCH_3$ | N |
| CH | $CO_2CH_3$ | $CH_3$ | $CH_3$ | $CH_3$ | $OCH_3$ | $CH_3$ | N |
| CH | $CO_2CH_3$ | $CH_3$ | H | H | $OCH_3$ | $OCH_3$ | CH |
| CH | $CO_2CH_3$ | $CH_3$ | H | H | $OCH_3$ | $CH_3$ | CH |
| CH | $CO_2CH_3$ | $CH_3$ | H | H | Cl | $OCH_3$ | CH |
| CH | $CO_2CH_3$ | $CH_3$ | H | H | $CH_3$ | $CH_3$ | CH |

Table 1 (continued)

General Formula I

| Z | $R^1$ | $R^2$ | $R^3$ | R | X | Y | $Z^4$ |
|---|---|---|---|---|---|---|---|
| CH | $CO_2CH_3$ | $CH_3$ | H | H | $OCH_3$ | $OCH_3$ | N |
| CH | $CO_2CH_3$ | $CH_3$ | H | H | $OCH_3$ | $CH_3$ | N |
| CH | $CO_2CH_3$ | $CH_3$ | H | H | $OC_2H_5$ | $NHCH_3$ | N |
| CH | $CO_2CH_3$ | $CH_3$ | H | $CH_3$ | $OCH_3$ | $CH_3$ | N |
| CH | $CO_2CH_3$ | H | $CH_3$ | H | $OCH_3$ | $OCH_3$ | CH |
| CH | $CO_2CH_3$ | H | $CH_3$ | H | $OCH_3$ | $CH_3$ | CH |
| CH | $CO_2CH_3$ | H | $CH_3$ | H | Cl | $OCH_3$ | CH |
| CH | $CO_2CH_3$ | H | $CH_3$ | H | $CH_3$ | $CH_3$ | CH |
| CH | $CO_2CH_3$ | H | $CH_3$ | H | $OCH_3$ | $OCH_3$ | N |
| CH | $CO_2CH_3$ | H | $CH_3$ | H | $OCH_3$ | $CH_3$ | N |
| CH | $CO_2CH_3$ | H | $CH_3$ | H | $OC_2H_5$ | $NHCH_3$ | N |
| CH | $CO_2CH_3$ | H | $CH_3$ | $CH_3$ | $OCH_3$ | $CH_3$ | N |
| CH | $CO_2CH_3$ | H | H | H | $OCH_3$ | $OCH_3$ | CH |
| CH | $CO_2CH_3$ | H | H | H | $OCH_3$ | $CH_3$ | CH |
| CH | $CO_2CH_3$ | H | H | H | Cl | $OCH_3$ | CH |
| CH | $CO_2CH_3$ | H | H | H | $CH_3$ | $CH_3$ | CH |
| CH | $CO_2CH_3$ | H | H | H | $OCH_3$ | $OCH_3$ | N |
| CH | $CO_2CH_3$ | H | H | H | $OCH_3$ | $CH_3$ | N |
| CH | $CO_2CH_3$ | H | H | H | $OC_2H_5$ | $NHCH_3$ | N |
| CH | $CO_2CH_3$ | H | H | $CH_3$ | $OCH_3$ | $CH_3$ | N |
| CH | $CO_2C_2H_5$ | H | H | H | $OCH_3$ | $OCH_3$ | CH |
| CH | $CO_2C_2H_5$ | H | H | H | $OCH_3$ | $CH_3$ | CH |

Table 1 (continued)

General Formula 1

| Z | $R^1$ | $R^2$ | $R^3$ | R | X | Y | $Z^1$ |
|---|---|---|---|---|---|---|---|
| CH | $CO_2C_2H_5$ | H | H | H | Cl | $OCH_3$ | CH |
| CH | $CO_2C_2H_5$ | H | H | H | $CH_3$ | $CH_3$ | CH |
| CH | $CO_2C_2H_5$ | H | H | H | $OCH_3$ | $OCH_3$ | N |
| CH | $CO_2C_2H_5$ | H | H | H | $OCH_3$ | $CH_3$ | N |
| CH | $CO_2C_2H_5$ | H | H | H | $OC_2H_5$ | $NHCH_3$ | N |
| CH | $CO_2C_2H_5$ | H | H | $CH_3$ | $OCH_3$ | $CH_3$ | N |
| CH | $CO_2C_2H_5$ | $CH_3$ | $CH_3$ | H | $OCH_3$ | $OCH_3$ | CH |
| CH | $CO_2C_2H_5$ | $CH_3$ | $CH_3$ | H | $OCH_3$ | $CH_3$ | CH |
| CH | $CO_2C_2H_5$ | $CH_3$ | $CH_3$ | H | Cl | $OCH_3$ | CH |
| CH | $CO_2C_2H_5$ | $CH_3$ | $CH_3$ | H | $CH_3$ | $CH_3$ | CH |
| CH | $CO_2C_2H_5$ | $CH_3$ | $CH_3$ | H | $OCH_3$ | $OCH_3$ | N |
| CH | $CO_2C_2H_5$ | $CH_3$ | $CH_3$ | H | $OCH_3$ | $CH_3$ | N |
| CH | $CO_2C_2H_5$ | $CH_3$ | $CH_3$ | H | $OC_2H_5$ | $NHCH_3$ | N |
| CH | $CO_2C_2H_5$ | $CH_3$ | $CH_3$ | $CH_3$ | $OCH_3$ | $CH_3$ | N |
| N | $CO_2CH_3$ | $CH_3$ | $CH_3$ | H | $OCH_3$ | $OCH_3$ | CH |
| N | $CO_2CH_3$ | $CH_3$ | $CH_3$ | H | $OCH_3$ | $CH_3$ | CH |
| N | $CO_2CH_3$ | $CH_3$ | $CH_3$ | H | Cl | $OCH_3$ | CH |
| N | $CO_2CH_3$ | $CH_3$ | $CH_3$ | H | $CH_3$ | $CH_3$ | CH |
| N | $CO_2CH_3$ | $CH_3$ | $CH_3$ | H | $OCH_3$ | $OCH_3$ | N |
| N | $CO_2CH_3$ | $CH_3$ | $CH_3$ | H | $OCH_3$ | $CH_3$ | N |
| N | $CO_2CH_3$ | $CH_3$ | $CH_3$ | H | $OC_2H_5$ | $NHCH_3$ | N |
| N | $CO_2CH_3$ | $CH_3$ | $CH_3$ | $CH_3$ | $OCH_3$ | $CH_3$ | N |

Table 1 (continued)

General Formula 1

| Z | $R^1$ | $R^2$ | $R^3$ | R | X | Y | $Z^4$ |
|---|---|---|---|---|---|---|---|
| N | $CO_2CH_3$ | $CH_3$ | H | H | $OCH_3$ | $OCH_3$ | CH |
| N | $CO_2CH_3$ | $CH_3$ | H | H | $OCH_3$ | $CH_3$ | CH |
| N | $CO_2CH_3$ | $CH_3$ | H | H | Cl | $OCH_3$ | CH |
| N | $CO_2CH_3$ | $CH_3$ | H | H | $CH_3$ | $CH_3$ | CH |
| N | $CO_2CH_3$ | $CH_3$ | H | H | $OCH_3$ | $OCH_3$ | N |
| N | $CO_2CH_3$ | $CH_3$ | H | H | $OCH_3$ | $CH_3$ | N |
| N | $CO_2CH_3$ | $CH_3$ | H | H | $OC_2H_5$ | $NHCH_3$ | N |
| N | $CO_2CH_3$ | $CH_3$ | H | $CH_3$ | $OCH_3$ | $CH_3$ | N |
| N | $CO_2CH_3$ | H | $CH_3$ | H | $OCH_3$ | $OCH_3$ | CH |
| N | $CO_2CH_3$ | H | $CH_3$ | H | $OCH_3$ | $CH_3$ | CH |
| N | $CO_2CH_3$ | H | $CH_3$ | H | Cl | $OCH_3$ | CH |
| N | $CO_2CH_3$ | H | $CH_3$ | H | $CH_3$ | $CH_3$ | CH |
| N | $CO_2CH_3$ | H | $CH_3$ | H | $OCH_3$ | $OCH_3$ | N |
| N | $CO_2CH_3$ | H | $CH_3$ | H | $OCH_3$ | $CH_3$ | N |
| N | $CO_2CH_3$ | H | $CH_3$ | H | $OC_2H_5$ | $NHCH_3$ | N |
| N | $CO_2CH_3$ | H | $CH_3$ | $CH_3$ | $OCH_3$ | $CH_3$ | N |
| CH | $CO_2CH_3$ | H | H | H | $OCH_3$ | $OCH_3$ | CH |
| CH | $CO_2CH_3$ | H | H | H | $OCH_3$ | $CH_3$ | CH |
| CH | $CO_2CH_3$ | H | H | H | Cl | $OCH_3$ | CH |
| CH | $CO_2CH_3$ | H | H | H | $CH_3$ | $CH_3$ | CH |
| CH | $CO_2CH_3$ | H | H | H | $OCH_3$ | $OCH_3$ | N |
| CH | $CO_2CH_3$ | H | H | H | $OCH_3$ | $CH_3$ | N |

Table 1 (continued)

General Formula 1

| Z | $R^1$ | $R^2$ | $R^3$ | R | X | Y | $Z^4$ |
|---|---|---|---|---|---|---|---|
| CH | $CO_2CH_3$ | H | H | H | $OC_2H_5$ | $NHCH_3$ | N |
| CH | $CO_2CH_3$ | H | H | $CH_3$ | $OCH_3$ | $CH_3$ | N |
| CH | $CO_2C_2H_5$ | H | H | H | $OCH_3$ | $OCH_3$ | CH |
| CH | $CO_2C_2H_5$ | H | H | H | $OCH_3$ | $CH_3$ | CH |
| CH | $CO_2C_2H_5$ | H | H | H | Cl | $OCH_3$ | CH |
| CH | $CO_2C_2H_5$ | H | H | H | $CH_3$ | $CH_3$ | CH |
| CH | $CO_2C_2H_5$ | H | H | H | $OCH_3$ | $OCH_3$ | N |
| CH | $CO_2C_2H_5$ | H | H | H | $OCH_3$ | $CH_3$ | N |
| CH | $CO_2C_2H_5$ | H | H | H | $OC_2H_5$ | $NHCH_3$ | N |
| CH | $CO_2C_2H_5$ | H | H | $CH_3$ | $OCH_3$ | $CH_3$ | N |
| CH | $CO_2C_2H_5$ | $CH_3$ | $CH_3$ | H | $OCH_3$ | $OCH_3$ | CH |
| CH | $CO_2C_2H_5$ | $CH_3$ | $CH_3$ | H | $OCH_3$ | $CH_3$ | CH |
| CH | $CO_2C_2H_5$ | $CH_3$ | $CH_3$ | H | Cl | $OCH_3$ | CH |
| CH | $CO_2C_2H_5$ | $CH_3$ | $CH_3$ | H | $CH_3$ | $CH_3$ | CH |
| CH | $CO_2C_2H_5$ | $CH_3$ | $CH_3$ | H | $OCH_3$ | $OCH_3$ | N |
| CH | $CO_2C_2H_5$ | $CH_3$ | $CH_3$ | H | $OCH_3$ | $CH_3$ | N |
| CH | $CO_2C_2H_5$ | $CH_3$ | $CH_3$ | H | $OC_2H_5$ | $NHCH_3$ | N |
| CH | $CO_2C_2H_5$ | $CH_3$ | $CH_3$ | $CH_3$ | $OCH_3$ | $CH_3$ | N |

Table 2

General Formula 2

| Z | $R^2$ | $R^3$ | R | X | Y | $Z^4$ |
|---|---|---|---|---|---|---|
| CH | $CH_3$ | $CH_3$ | H | $OCH_3$ | $OCH_3$ | CH |
| CH | $CH_3$ | $CH_3$ | H | $OCH_3$ | $CH_3$ | CH |
| CH | $CH_3$ | $CH_3$ | H | Cl | $OCH_3$ | CH |
| CH | $CH_3$ | $CH_3$ | H | $CH_3$ | $CH_3$ | CH |
| CH | $CH_3$ | $CH_3$ | H | $OCH_3$ | $OCH_3$ | N |
| CH | $CH_3$ | $CH_3$ | H | $OCH_3$ | $CH_3$ | N |
| CH | $CH_3$ | $CH_3$ | H | $OC_2H_5$ | $NHCH_3$ | N |
| CH | $CH_3$ | $CH_3$ | $CH_3$ | $OCH_3$ | $CH_3$ | N |
| N | $CH_3$ | $CH_3$ | H | $OCH_3$ | $OCH_3$ | CH |
| N | $CH_3$ | $CH_3$ | H | $OCH_3$ | $CH_3$ | CH |
| N | $CH_3$ | $CH_3$ | H | Cl | $OCH_3$ | CH |
| N | $CH_3$ | $CH_3$ | H | $CH_3$ | $CH_3$ | CH |
| N | $CH_3$ | $CH_3$ | H | $OCH_3$ | $OCH_3$ | N |
| N | $CH_3$ | $CH_3$ | H | $OCH_3$ | $CH_3$ | N |
| N | $CH_3$ | $CH_3$ | H | $OC_2H_5$ | $NHCH_3$ | N |
| N | $CH_3$ | $CH_3$ | $CH_3$ | $OCH_3$ | $CH_3$ | N |
| CH | $CH_3$ | H | H | $OCH_3$ | $OCH_3$ | CH |
| CH | $CH_3$ | H | H | $OCH_3$ | $CH_3$ | CH |
| CH | $CH_3$ | H | H | Cl | $OCH_3$ | CH |
| CH | $CH_3$ | H | H | $CH_3$ | $CH_3$ | CH |
| CH | $CH_3$ | H | H | $OCH_3$ | $OCH_3$ | N |
| CH | $CH_3$ | H | H | $OCH_3$ | $CH_3$ | N |

Table 2 (continued)

General Formula 2

| Z | $R^2$ | $R^3$ | R | X | Y | $Z^4$ |
|---|---|---|---|---|---|---|
| CH | $CH_3$ | H | H | $OC_2H_5$ | $NHCH_3$ | N |
| CH | $CH_3$ | H | $CH_3$ | $OCH_3$ | $CH_3$ | N |
| N | $CH_3$ | H | H | $OCH_3$ | $OCH_3$ | CH |
| N | $CH_3$ | H | H | $OCH_3$ | $CH_3$ | CH |
| N | $CH_3$ | H | H | Cl | $OCH_3$ | CH |
| N | $CH_3$ | H | H | $CH_3$ | $CH_3$ | CH |
| N | $CH_3$ | H | H | $OCH_3$ | $OCH_3$ | N |
| N | $CH_3$ | H | H | $OCH_3$ | $CH_3$ | N |
| N | $CH_3$ | H | H | $OC_2H_5$ | $NHCH_3$ | N |
| N | $CH_3$ | H | $CH_3$ | $OCH_3$ | $CH_3$ | N |
| CH | H | $CH_3$ | H | $OCH_3$ | $OCH_3$ | CH |
| CH | H | $CH_3$ | H | $OCH_3$ | $CH_3$ | CH |
| CH | H | $CH_3$ | H | Cl | $OCH_3$ | CH |
| CH | H | $CH_3$ | H | $CH_3$ | $CH_3$ | CH |
| CH | H | $CH_3$ | H | $OCH_3$ | $OCH_3$ | N |
| CH | H | $CH_3$ | H | $OCH_3$ | $CH_3$ | N |
| CH | H | $CH_3$ | H | $OC_2H_5$ | $NHCH_3$ | N |
| CH | H | $CH_3$ | $CH_3$ | $OCH_3$ | $CH_3$ | N |
| N | H | $CH_3$ | H | $OCH_3$ | $OCH_3$ | CH |
| N | H | $CH_3$ | H | $OCH_3$ | $CH_3$ | CH |
| N | H | $CH_3$ | H | Cl | $OCH_3$ | CH |
| N | H | $CH_3$ | H | $CH_3$ | $CH_3$ | CH |
| N | H | CH | H | $OCH_3$ | $OCH_3$ | N |

Table 2 (continued)

General Formula 2

| Z | $R^2$ | $R^3$ | R | X | Y | $Z^4$ |
|---|---|---|---|---|---|---|
| N | H | $CH_3$ | H | $OCH_3$ | $CH_3$ | N |
| N | H | $CH_3$ | H | $OC_2H_5$ | $NHCH_3$ | N |
| N | H | $CH_3$ | $CH_3$ | $OCH_3$ | $CH_3$ | N |
| CH | H | H | H | $OCH_3$ | $OCH_3$ | CH |
| CH | H | H | H | $OCH_3$ | $CH_3$ | CH |
| CH | H | H | H | Cl | $OCH_3$ | CH |
| CH | H | H | H | $CH_3$ | $CH_3$ | CH |
| CH | H | H | H | $OCH_3$ | $OCH_3$ | N |
| CH | H | H | H | $OCH_3$ | $CH_3$ | N |
| CH | H | H | H | $OC_2H_5$ | $NHCH_3$ | N |
| CH | H | H | $CH_3$ | $OCH_3$ | $CH_3$ | N |
| N | H | H | H | $OCH_3$ | $OCH_3$ | CH |
| N | H | H | H | $OCH_3$ | $CH_3$ | CH |
| N | H | H | H | Cl | $OCH_3$ | CH |
| N | H | H | H | $CH_3$ | $CH_3$ | CH |
| N | H | H | H | $OCH_3$ | $OCH_3$ | N |
| N | H | H | H | $OCH_3$ | $CH_3$ | N |
| N | H | H | H | $OC_2H_5$ | $NHCH_3$ | N |
| N | H | H | $CH_3$ | $OCH_3$ | $CH_3$ | N |

Table 3

General Formula 3

| Z | $R^2$ | $R^3$ | $R^4$ | R | X | Y | $Z^4$ |
|---|---|---|---|---|---|---|---|
| CH | $CH_3$ | $CH_3$ | H | H | $OCH_3$ | $OCH_3$ | CH |
| CH | $CH_3$ | $CH_3$ | H | H | $OCH_3$ | $CH_3$ | CH |
| CH | $CH_3$ | $CH_3$ | H | H | Cl | $OCH_3$ | CH |
| CH | $CH_3$ | $CH_3$ | H | H | $CH_3$ | $CH_3$ | CH |
| CH | $CH_3$ | $CH_3$ | H | H | $OCH_3$ | $OCH_3$ | N |
| CH | $CH_3$ | $CH_3$ | H | H | $OCH_3$ | $CH_3$ | N |
| CH | $CH_3$ | $CH_3$ | H | H | $OC_2H_5$ | $NHCH_3$ | N |
| CH | $CH_3$ | $CH_3$ | H | $CH_3$ | $OCH_3$ | $CH_3$ | N |
| N | $CH_3$ | $CH_3$ | H | H | $OCH_3$ | $OCH_3$ | CH |
| N | $CH_3$ | $CH_3$ | H | H | $OCH_3$ | $CH_3$ | CH |
| N | $CH_3$ | $CH_3$ | H | H | Cl | $OCH_3$ | CH |
| N | $CH_3$ | $CH_3$ | H | H | $CH_3$ | $CH_3$ | CH |
| N | $CH_3$ | $CH_3$ | H | H | $OCH_3$ | $OCH_3$ | N |
| N | $CH_3$ | $CH_3$ | H | H | $OC_2H_5$ | $NHCH_3$ | N |
| N | $CH_3$ | $CH_3$ | H | $CH_3$ | $OCH_3$ | $CH_3$ | N |
| CH | $CH_3$ | H | H | H | $OCH_3$ | $OCH_3$ | CH |
| CH | $CH_3$ | H | H | H | $OCH_3$ | $CH_3$ | CH |
| CH | $CH_3$ | H | H | H | Cl | $OCH_3$ | CH |
| CH | $CH_3$ | H | H | H | $CH_3$ | $CH_3$ | CH |
| CH | $CH_3$ | H | H | H | $OCH_3$ | $OCH_3$ | N |
| CH | $CH_3$ | H | H | H | $OCH_3$ | $CH_3$ | N |
| CH | $CH_3$ | H | H | H | $OC_2H_5$ | $NHCH_3$ | N |
| CH | $CH_3$ | H | H | $CH_3$ | $OCH_3$ | $CH_3$ | N |

Table 3 (continued)

General Formula 3

| Z | $R^2$ | $R^3$ | $R^4$ | R | X | Y | $Z^4$ |
|---|---|---|---|---|---|---|---|
| N | $CH_3$ | H | H | H | $OCH_3$ | $OCH_3$ | CH |
| N | $CH_3$ | H | H | H | $OCH_3$ | $CH_3$ | CH |
| N | $CH_3$ | H | H | H | Cl | $OCH_3$ | CH |
| N | $CH_3$ | H | H | H | $CH_3$ | $CH_3$ | CH |
| N | $CH_3$ | H | H | H | $OCH_3$ | $OCH_3$ | N |
| N | $CH_3$ | H | H | H | $OCH_3$ | $CH_3$ | N |
| N | $CH_3$ | H | H | H | $OC_2H_5$ | $NHCH_3$ | N |
| N | $CH_3$ | H | H | $CH_3$ | $OCH_3$ | $CH_3$ | N |
| CH | H | $CH_3$ | H | H | $OCH_3$ | $OCH_3$ | CH |
| CH | H | $CH_3$ | H | H | $OCH_3$ | $CH_3$ | CH |
| CH | H | $CH_3$ | H | H | Cl | $OCH_3$ | CH |
| CH | H | $CH_3$ | H | H | $CH_3$ | $CH_3$ | CH |
| CH | H | $CH_3$ | H | H | $OCH_3$ | $OCH_3$ | N |
| CH | H | $CH_3$ | H | H | $OCH_3$ | $CH_3$ | N |
| CH | H | $CH_3$ | H | H | $OC_2H_5$ | $NHCH_3$ | N |
| CH | H | $CH_3$ | H | $CH_3$ | $OCH_3$ | $CH_3$ | N |
| N | H | $CH_3$ | H | H | $OCH_3$ | $OCH_3$ | CH |
| N | H | $CH_3$ | H | H | $OCH_3$ | $CH_3$ | CH |
| N | H | $CH_3$ | H | H | Cl | $OCH_3$ | CH |
| N | H | $CH_3$ | H | H | $CH_3$ | $CH_3$ | CH |
| N | H | $CH_3$ | H | H | $OCH_3$ | $OCH_3$ | N |
| N | H | $CH_3$ | H | H | $OCH_3$ | $CH_3$ | N |
| N | H | $CH_3$ | H | H | $OC_2H_5$ | $NHCH_3$ | N |

Table 3 (continued)

General Formula 3

| $Z$ | $R^2$ | $R^3$ | $R^4$ | $R$ | $X$ | $Y$ | $Z^4$ |
|---|---|---|---|---|---|---|---|
| N | H | $CH_3$ | H | $CH_3$ | $OCH_3$ | $CH_3$ | N |
| CH | H | H | H | H | $OCH_3$ | $OCH_3$ | CH |
| CH | H | H | H | H | $OCH_3$ | $CH_3$ | CH |
| CH | H | H | H | H | Cl | $OCH_3$ | CH |
| CH | H | H | H | H | $CH_3$ | $CH_3$ | CH |
| CH | H | H | H | H | $OCH_3$ | $OCH_3$ | N |
| CH | H | H | H | H | $OCH_3$ | $CH_3$ | N |
| CH | H | H | H | H | $OC_2H_5$ | $NHCH_3$ | N |
| CH | H | H | H | $CH_3$ | $OCH_3$ | $CH_3$ | N |
| N | H | H | H | H | $OCH_3$ | $OCH_3$ | CH |
| N | H | H | H | H | $OCH_3$ | $CH_3$ | CH |
| N | H | H | H | H | Cl | $OCH_3$ | CH |
| N | H | H | H | H | $CH_3$ | $CH_3$ | CH |
| N | H | H | H | H | $OCH_3$ | $OCH_3$ | N |
| N | H | H | H | H | $OCH_3$ | $CH_3$ | N |
| N | H | H | H | H | $OC_2H_5$ | $NHCH_3$ | N |
| N | H | H | H | $CH_3$ | $OCH_3$ | $CH_3$ | N |

Table 4

General Formula 4

| Z | $Z^1$ | $R^2$ | $R^3$ | R | X | Y | $Z^4$ |
|---|---|---|---|---|---|---|---|
| CH | CH | $CH_3$ | $CH_3$ | H | $OCH_3$ | $OCH_3$ | CH |
| CH | CH | $CH_3$ | $CH_3$ | H | $OCH_3$ | $CH_3$ | CH |
| CH | CH | $CH_3$ | $CH_3$ | H | Cl | $OCH_3$ | CH |
| CH | CH | $CH_3$ | $CH_3$ | H | $CH_3$ | $CH_3$ | CH |
| CH | CH | $CH_3$ | $CH_3$ | H | $OCH_3$ | $OCH_3$ | N |
| CH | CH | $CH_3$ | $CH_3$ | H | $OCH_3$ | $CH_3$ | N |
| CH | CH | $CH_3$ | $CH_3$ | H | $OC_2H_5$ | $NHCH_3$ | N |
| CH | CH | $CH_3$ | $CH_3$ | $CH_3$ | $OCH_3$ | $CH_3$ | N |
| N | CH | $CH_3$ | $CH_3$ | H | $OCH_3$ | $OCH_3$ | CH |
| N | CH | $CH_3$ | $CH_3$ | H | $OCH_3$ | $CH_3$ | CH |
| N | CH | $CH_3$ | $CH_3$ | H | Cl | $OCH_3$ | CH |
| N | CH | $CH_3$ | $CH_3$ | H | $CH_3$ | $CH_3$ | CH |
| N | CH | $CH_3$ | $CH_3$ | H | $OCH_3$ | $OCH_3$ | N |
| N | CH | $CH_3$ | $CH_3$ | H | $OCH_3$ | $CH_3$ | N |
| N | CH | $CH_3$ | $CH_3$ | H | $OC_2H_5$ | $NHCH_3$ | N |
| N | CH | $CH_3$ | $CH_3$ | $CH_3$ | $OCH_3$ | $CH_3$ | N |
| CH | CH | H | H | H | $OCH_3$ | $OCH_3$ | CH |
| CH | CH | H | H | H | $OCH_3$ | $CH_3$ | CH |
| CH | CH | H | H | H | Cl | $OCH_3$ | CH |
| CH | CH | H | H | H | $CH_3$ | $CH_3$ | CH |
| CH | CH | H | H | H | $OCH_3$ | $OCH_3$ | N |
| CH | CH | H | H | H | $OCH_3$ | $CH_3$ | N |
| CH | CH | H | H | H | $OC_2H_5$ | $NHCH_3$ | N |

Table 4 (continued)

General Formula 4

| Z | $Z^1$ | $R^2$ | $R^3$ | R | X | Y | $Z^4$ |
|---|---|---|---|---|---|---|---|
| CH | CH | H | H | $CH_3$ | $OCH_3$ | $CH_3$ | N |
| N | CH | H | H | H | $OCH_3$ | $OCH_3$ | CH |
| N | CH | H | H | H | $OCH_3$ | $CH_3$ | CH |
| N | CH | H | H | H | Cl | $OCH_3$ | CH |
| N | CH | H | H | H | $CH_3$ | $CH_3$ | CH |
| N | CH | H | H | H | $OCH_3$ | $OCH_3$ | N |
| N | CH | H | H | H | $OCH_3$ | $CH_3$ | N |
| N | CH | H | H | H | $OC_2H_5$ | $NHCH_3$ | N |
| N | CH | H | H | $CH_3$ | $OCH_3$ | $CH_3$ | N |
| CH | N | $CH_3$ | $CH_3$ | H | $OCH_3$ | $OCH_3$ | CH |
| CH | N | $CH_3$ | $CH_3$ | H | $OCH_3$ | $CH_3$ | CH |
| CH | N | $CH_3$ | $CH_3$ | H | Cl | $OCH_3$ | CH |
| CH | N | $CH_3$ | $CH_3$ | H | $CH_3$ | $CH_3$ | CH |
| CH | N | $CH_3$ | $CH_3$ | H | $OCH_3$ | $OCH_3$ | N |
| CH | N | $CH_3$ | $CH_3$ | H | $OCH_3$ | $CH_3$ | N |
| CH | N | $CH_3$ | $CH_3$ | H | $OC_2H_5$ | $NHCH_3$ | N |
| CH | N | $CH_3$ | $CH_3$ | $CH_3$ | $OCH_3$ | $CH_3$ | N |
| N | N | $CH_3$ | $CH_3$ | H | $OCH_3$ | $OCH_3$ | CH |
| N | N | $CH_3$ | $CH_3$ | H | $OCH_3$ | $CH_3$ | CH |
| N | N | $CH_3$ | $CH_3$ | H | Cl | $OCH_3$ | CH |
| N | N | $CH_3$ | $CH_3$ | H | $CH_3$ | $CH_3$ | CH |
| N | N | $CH_3$ | $CH_3$ | H | $OCH_3$ | $OCH_3$ | N |
| N | N | $CH_3$ | $CH_3$ | H | $OCH_3$ | $CH_3$ | N |

Table 4 (continued)

General Formula 4

| Z | $Z^1$ | $R^2$ | $R^3$ | R | X | Y | $Z^4$ |
|---|---|---|---|---|---|---|---|
| N | N | $CH_3$ | $CH_3$ | H | $OC_2H_5$ | $NHCH_3$ | N |
| N | N | $CH_3$ | $CH_3$ | $CH_3$ | $OCH_3$ | $CH_3$ | N |
| CH | N | $CH_3$ | H | H | $OCH_3$ | $OCH_3$ | CH |
| CH | N | $CH_3$ | H | H | $OCH_3$ | $CH_3$ | CH |
| CH | N | $CH_3$ | H | H | Cl | $OCH_3$ | CH |
| CH | N | $CH_3$ | H | H | $CH_3$ | $CH_3$ | CH |
| CH | N | $CH_3$ | H | H | $OCH_3$ | $OCH_3$ | N |
| CH | N | $CH_3$ | H | H | $OCH_3$ | $CH_3$ | N |
| CH | N | $CH_3$ | H | H | $OC_2H_5$ | $NHCH_3$ | N |
| CH | N | $CH_3$ | H | $CH_3$ | $OCH_3$ | $CH_3$ | N |
| N | N | $CH_3$ | H | H | $OCH_3$ | $OCH_3$ | CH |
| N | N | $CH_3$ | H | H | $OCH_3$ | $CH_3$ | CH |
| N | N | $CH_3$ | H | H | Cl | $OCH_3$ | CH |
| N | N | $CH_3$ | H | H | $CH_3$ | $CH_3$ | CH |
| N | N | $CH_3$ | H | H | $OCH_3$ | $OCH_3$ | N |
| N | N | $CH_3$ | H | H | $OCH_3$ | $CH_3$ | N |
| N | N | $CH_3$ | H | H | $OC_2H_5$ | $NHCH_3$ | N |
| N | N | $CH_3$ | H | $CH_3$ | $OCH_3$ | $CH_3$ | N |
| CH | N | H | $CH_3$ | H | $OCH_3$ | $OCH_3$ | CH |
| CH | N | H | $CH_3$ | H | $OCH_3$ | $CH_3$ | CH |
| CH | N | H | $CH_3$ | H | Cl | $OCH_3$ | CH |
| CH | N | H | $CH_3$ | H | $CH_3$ | $CH_3$ | CH |
| CH | N | H | $CH_3$ | H | $OCH_3$ | $OCH_3$ | N |

Table 4 (continued)

General Formula 4

| Z | $Z^1$ | $R^2$ | $R^3$ | R | X | Y | $Z^4$ |
|---|---|---|---|---|---|---|---|
| CH | N | H | $CH_3$ | H | $OCH_3$ | $CH_3$ | N |
| CH | N | H | $CH_3$ | H | $OC_2H_5$ | $NHCH_3$ | N |
| CH | N | H | $CH_3$ | $CH_3$ | $OCH_3$ | $CH_3$ | N |
| N | N | H | $CH_3$ | H | $OCH_3$ | $OCH_3$ | CH |
| N | N | H | $CH_3$ | H | $OCH_3$ | $CH_3$ | CH |
| N | N | H | $CH_3$ | H | Cl | $OCH_3$ | CH |
| N | N | H | $CH_3$ | H | $CH_3$ | $CH_3$ | CH |
| N | N | H | $CH_3$ | H | $OCH_3$ | $OCH_3$ | N |
| N | N | H | $CH_3$ | H | $OCH_3$ | $CH_3$ | N |
| N | N | H | $CH_3$ | H | $OC_2H_5$ | $NHCH_3$ | N |
| N | N | H | $CH_3$ | $CH_3$ | $OCH_3$ | $CH_3$ | N |
| CH | N | H | H | H | $OCH_3$ | $OCH_3$ | CH |
| CH | N | H | H | H | $OCH_3$ | $CH_3$ | CH |
| CH | N | H | H | H | Cl | $OCH_3$ | CH |
| CH | N | H | H | H | $CH_3$ | $CH_3$ | CH |
| CH | N | H | H | H | $OCH_3$ | $OCH_3$ | N |
| CH | N | H | H | H | $OCH_3$ | $CH_3$ | N |
| CH | N | H | H | H | $OC_2H_5$ | $NHCH_3$ | N |
| CH | N | H | H | $CH_3$ | $OCH_3$ | $CH_3$ | N |
| N | N | H | H | H | $OCH_3$ | $OCH_3$ | CH |
| N | N | H | H | H | $OCH_3$ | $CH_3$ | CH |
| N | N | H | H | H | Cl | $OCH_3$ | CH |
| N | N | H | H | H | $CH_3$ | $CH_3$ | CH |

Table 4 (continued)

General Formula 4

| Z | $Z^1$ | $R^2$ | $R^3$ | R | X | Y | $Z^4$ |
|---|---|---|---|---|---|---|---|
| N | N | H | H | H | $OCH_3$ | $OCH_3$ | N |
| N | N | H | H | H | $OCH_3$ | $CH_3$ | N |
| N | N | H | H | H | $OC_2H_5$ | $NHCH_3$ | N |
| N | N | H | H | $CH_3$ | $OCH_3$ | $CH_3$ | N |

Table 5

General Formula 5

| Z | $Z^1$ | $R^1$ | $R^2$ | $R^3$ | R | X | Y | $Z^4$ |
|---|---|---|---|---|---|---|---|---|
| CH | CH | H | $CH_3$ | $CH_3$ | H | $OCH_3$ | $OCH_3$ | CH |
| CH | CH | H | $CH_3$ | $CH_3$ | H | $OCH_3$ | $CH_3$ | CH |
| CH | CH | H | $CH_3$ | $CH_3$ | H | Cl | $OCH_3$ | CH |
| CH | CH | H | $CH_3$ | $CH_3$ | H | $CH_3$ | $CH_3$ | CH |
| CH | CH | H | $CH_3$ | $CH_3$ | H | $OCH_3$ | $OCH_3$ | N |
| CH | CH | H | $CH_3$ | $CH_3$ | H | $OCH_3$ | $CH_3$ | N |
| CH | CH | H | $CH_3$ | $CH_3$ | H | $OC_2H_5$ | $NHCH_3$ | N |
| CH | CH | H | $CH_3$ | $CH_3$ | $CH_3$ | $OCH_3$ | $CH_3$ | N |
| CH | CH | Cl | $CH_3$ | $CH_3$ | H | $OCH_3$ | $OCH_3$ | CH |
| CH | CH | Cl | $CH_3$ | $CH_3$ | H | $OCH_3$ | $CH_3$ | CH |
| CH | CH | Cl | $CH_3$ | $CH_3$ | H | Cl | $OCH_3$ | CH |
| CH | CH | Cl | $CH_3$ | $CH_3$ | H | $CH_3$ | $CH_3$ | CH |
| CH | CH | Cl | $CH_3$ | $CH_3$ | H | $OCH_3$ | $OCH_3$ | N |
| CH | CH | Cl | $CH_3$ | $CH_3$ | H | $OCH_3$ | $CH_3$ | N |
| CH | CH | Cl | $CH_3$ | $CH_3$ | H | $OC_2H_5$ | $NHCH_3$ | N |
| CH | CH | Cl | $CH_3$ | $CH_3$ | $CH_3$ | $OCH_3$ | $CH_3$ | N |
| CH | CH | $SO_2CH_3$ | $CH_3$ | $CH_3$ | H | $OCH_3$ | $OCH_3$ | CH |
| CH | CH | $SO_2CH_3$ | $CH_3$ | $CH_3$ | H | $OCH_3$ | $CH_3$ | CH |
| CH | CH | $SO_2CH_3$ | $CH_3$ | $CH_3$ | H | Cl | $OCH_3$ | CH |
| CH | CH | $SO_2CH_3$ | $CH_3$ | $CH_3$ | H | $CH_3$ | $CH_3$ | CH |
| CH | CH | $SO_2CH_3$ | $CH_3$ | $CH_3$ | H | $OCH_3$ | $OCH_3$ | N |
| CH | CH | $SO_2CH_3$ | $CH_3$ | $CH_3$ | H | $OCH_3$ | $CH_3$ | N |
| CH | CH | $SO_2CH_3$ | $CH_3$ | $CH_3$ | H | $OC_2H_5$ | $NHCH_3$ | N |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | CH | $SO_2CH_3$ | $CH_3$ | $CH_3$ | $CH_3$ | $OCH_3$ | $CH_3$ | N |
| CH | CH | $SO_2N(CH_3)_2$ | $CH_3$ | $CH_3$ | H | $OCH_3$ | $OCH_3$ | CH |
| CH | CH | $SO_2N(CH_3)_2$ | $CH_3$ | $CH_3$ | H | $OCH_3$ | $CH_3$ | CH |
| CH | CH | $SO_2N(CH_3)_2$ | $CH_3$ | $CH_3$ | H | Cl | $OCH_3$ | CH |
| CH | CH | $SO_2N(CH_3)_2$ | $CH_3$ | $CH_3$ | H | $OCH_3$ | $OCH_3$ | N |
| CH | CH | $SO_2N(CH_3)_2$ | $CH_3$ | $CH_3$ | H | $OCH_3$ | $CH_3$ | N |
| CH | CH | H | H | H | H | $OCH_3$ | $OCH_3$ | CH |
| CH | CH | H | H | H | H | $OCH_3$ | $CH_3$ | CH |
| CH | CH | H | H | H | H | Cl | $OCH_3$ | CH |
| CH | CH | H | H | H | H | $CH_3$ | $CH_3$ | CH |
| CH | CH | H | H | H | H | $OCH_3$ | $OCH_3$ | N |
| CH | CH | H | H | H | H | $OCH_3$ | $CH_3$ | N |
| CH | CH | H | H | H | H | $OC_2H_5$ | $NHCH_3$ | N |
| CH | CH | H | H | H | $CH_3$ | $OCH_3$ | $CH_3$ | N |
| CH | CH | Cl | H | H | H | $OCH_3$ | $OCH_3$ | CH |
| CH | CH | Cl | H | H | H | $OCH_3$ | $CH_3$ | CH |
| CH | CH | Cl | H | H | H | Cl | $OCH_3$ | CH |
| CH | CH | Cl | H | H | H | $CH_3$ | $CH_3$ | CH |
| CH | CH | Cl | H | H | H | $OCH_3$ | $OCH_3$ | N |
| CH | CH | Cl | H | H | H | $OCH_3$ | $CH_3$ | N |
| CH | CH | Cl | H | H | H | $OC_2H_5$ | $NHCH_3$ | N |
| CH | CH | Cl | H | H | $CH_3$ | $OCH_3$ | $CH_3$ | N |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | CH | $SO_2CH_3$ | H | H | H | $OCH_3$ | $OCH_3$ | CH |
| CH | CH | $SO_2CH_3$ | H | H | H | $OCH_3$ | $CH_3$ | CH |
| CH | CH | $SO_2CH_3$ | H | H | H | Cl | $OCH_3$ | CH |
| CH | CH | $SO_2CH_3$ | H | H | H | $CH_3$ | $CH_3$ | CH |
| CH | CH | $SO_2CH_3$ | H | H | H | $OCH_3$ | $OCH_3$ | N |
| CH | CH | $SO_2CH_3$ | H | H | H | $OCH_3$ | $CH_3$ | N |
| CH | CH | $SO_2CH_3$ | H | H | H | $OC_2H_5$ | $NHCH_3$ | N |
| CH | CH | $SO_2CH_3$ | H | H | $CH_3$ | $OCH_3$ | $CH_3$ | N |
| CH | CH | $SO_2N(CH_3)_2$ | H | H | H | $OCH_3$ | $OCH_3$ | CH |
| CH | CH | $SO_2N(CH_3)_2$ | H | H | H | $OCH_3$ | $CH_3$ | CH |
| CH | CH | $SO_2N(CH_3)_2$ | H | H | H | Cl | $OCH_3$ | CH |
| CH | CH | $SO_2N(CH_3)_2$ | H | H | H | $OCH_3$ | $OCH_3$ | N |
| CH | CH | $SO_2N(CH_3)_2$ | H | H | H | $OCH_3$ | $CH_3$ | N |
| CH | CH | H | H | $CH_3$ | H | $OCH_3$ | $OCH_3$ | CH |
| CH | CH | H | H | $CH_3$ | H | $OCH_3$ | $CH_3$ | CH |
| CH | CH | H | H | $CH_3$ | H | Cl | $OCH_3$ | CH |
| CH | CH | H | H | $CH_3$ | H | $CH_3$ | $CH_3$ | CH |
| CH | CH | H | H | $CH_3$ | H | $OCH_3$ | $OCH_3$ | N |
| CH | CH | H | H | $CH_3$ | H | $OCH_3$ | $CH_3$ | N |
| CH | CH | H | H | $CH_3$ | H | $OC_2H_5$ | $NHCH_3$ | N |
| CH | CH | H | H | $CH_3$ | $CH_3$ | $OCH_3$ | $CH_3$ | N |
| CH | CH | Cl | H | $CH_3$ | H | $OCH_3$ | $OCH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | CH | Cl | H | $CH_3$ | H | $OCH_3$ | $CH_3$ | CH |
| CH | CH | Cl | H | $CH_3$ | H | Cl | $OCH_3$ | CH |
| CH | CH | Cl | H | $CH_3$ | H | $CH_3$ | $CH_3$ | CH |
| CH | CH | Cl | H | $CH_3$ | H | $OCH_3$ | $OCH_3$ | N |
| CH | CH | Cl | H | $CH_3$ | H | $OCH_3$ | $CH_3$ | N |
| CH | CH | Cl | H | $CH_3$ | H | $OC_2H_5$ | $NHCH_3$ | N |
| CH | CH | Cl | H | $CH_3$ | $CH_3$ | $OCH_3$ | $CH_3$ | N |
| CH | CH | $SO_2CH_3$ | H | $CH_3$ | H | $OCH_3$ | $OCH_3$ | CH |
| CH | CH | $SO_2CH_3$ | H | $CH_3$ | H | $OCH_3$ | $CH_3$ | CH |
| CH | CH | $SO_2CH_3$ | H | $CH_3$ | H | Cl | $OCH_3$ | CH |
| CH | CH | $SO_2CH_3$ | H | $CH_3$ | H | $CH_3$ | $CH_3$ | CH |
| CH | CH | $SO_2CH_3$ | H | $CH_3$ | H | $OCH_3$ | $OCH_3$ | N |
| CH | CH | $SO_2CH_3$ | H | $CH_3$ | H | $OCH_3$ | $CH_3$ | N |
| CH | CH | $SO_2CH_3$ | H | $CH_3$ | H | $OC_2H_5$ | $NHCH_3$ | N |
| CH | CH | $SO_2CH_3$ | H | $CH_3$ | $CH_3$ | $OCH_3$ | $CH_3$ | N |
| CH | CH | $SO_2N(CH_3)_2$ | H | $CH_3$ | H | $OCH_3$ | $OCH_3$ | CH |
| CH | CH | $SO_2N(CH_3)_2$ | H | $CH_3$ | H | $OCH_3$ | $CH_3$ | CH |
| CH | CH | $SO_2N(CH_3)_2$ | H | $CH_3$ | H | Cl | $OCH_3$ | CH |
| CH | CH | $SO_2N(CH_3)_2$ | H | $CH_3$ | H | $OCH_3$ | $OCH_3$ | N |
| CH | CH | $SO_2N(CH_3)_2$ | H | $CH_3$ | H | $OCH_3$ | $CH_3$ | N |
| CH | CH | H | $CH_3$ | H | H | $OCH_3$ | $OCH_3$ | CH |
| CH | CH | H | $CH_3$ | H | H | $OCH_3$ | $CH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | CH | H | $CH_3$ | H | H | Cl | $OCH_3$ | CH |
| CH | CH | H | $CH_3$ | H | H | $CH_3$ | $CH_3$ | CH |
| CH | CH | H | $CH_3$ | H | H | $OCH_3$ | $OCH_3$ | N |
| CH | CH | H | $CH_3$ | H | H | $OCH_3$ | $CH_3$ | N |
| CH | CH | H | $CH_3$ | H | H | $OC_2H_5$ | $NHCH_3$ | N |
| CH | CH | H | $CH_3$ | H | $CH_3$ | $OCH_3$ | $CH_3$ | N |
| CH | CH | Cl | $CH_3$ | H | H | $OCH_3$ | $OCH_3$ | CH |
| CH | CH | Cl | $CH_3$ | H | H | $OCH_3$ | $CH_3$ | CH |
| CH | CH | Cl | $CH_3$ | H | H | Cl | $OCH_3$ | CH |
| CH | CH | Cl | $CH_3$ | H | H | $CH_3$ | $CH_3$ | CH |
| CH | CH | Cl | $CH_3$ | H | H | $OCH_3$ | $OCH_3$ | N |
| CH | CH | Cl | $CH_3$ | H | H | $OCH_3$ | $CH_3$ | N |
| CH | CH | Cl | $CH_3$ | H | H | $OC_2H_5$ | $NHCH_3$ | N |
| CH | CH | Cl | $CH_3$ | H | $CH_3$ | $OCH_3$ | $CH_3$ | N |
| CH | CH | $SO_2CH_3$ | $CH_3$ | H | H | $OCH_3$ | $OCH_3$ | CH |
| CH | CH | $SO_2CH_3$ | $CH_3$ | H | H | $OCH_3$ | $CH_3$ | CH |
| CH | CH | $SO_2CH_3$ | $CH_3$ | H | H | Cl | $OCH_3$ | CH |
| CH | CH | $SO_2CH_3$ | $CH_3$ | H | H | $CH_3$ | $CH_3$ | CH |
| CH | CH | $SO_2CH_3$ | $CH_3$ | H | H | $OCH_3$ | $OCH_3$ | N |
| CH | CH | $SO_2CH_3$ | $CH_3$ | H | H | $OCH_3$ | $CH_3$ | N |
| CH | CH | $SO_2CH_3$ | $CH_3$ | H | H | $OC_2H_5$ | $NHCH_3$ | N |
| CH | CH | $SO_2CH_3$ | $CH_3$ | H | $CH_3$ | $OCH_3$ | $CH_3$ | N |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | CH | $SO_2N(CH_3)_2$ | $CH_3$ | H | H | $OCH_3$ | $OCH_3$ | CH |
| CH | CH | $SO_2N(CH_3)_2$ | $CH_3$ | H | H | $OCH_3$ | $CH_3$ | CH |
| CH | CH | $SO_2N(CH_3)_2$ | $CH_3$ | H | H | Cl | $OCH_3$ | CH |
| CH | CH | $SO_2N(CH_3)_2$ | $CH_3$ | H | H | $OCH_3$ | $OCH_3$ | N |
| CH | CH | $SO_2N(CH_3)_2$ | $CH_3$ | H | H | $OCH_3$ | $CH_3$ | N |
| CH | N | H | $CH_3$ | $CH_3$ | H | $OCH_3$ | $OCH_3$ | CH |
| CH | N | H | $CH_3$ | $CH_3$ | H | $OCH_3$ | $CH_3$ | CH |
| CH | N | H | $CH_3$ | $CH_3$ | H | Cl | $OCH_3$ | CH |
| CH | N | H | $CH_3$ | $CH_3$ | H | $CH_3$ | $CH_3$ | CH |
| CH | N | H | $CH_3$ | $CH_3$ | H | $OCH_3$ | $OCH_3$ | N |
| CH | N | H | $CH_3$ | $CH_3$ | H | $OCH_3$ | $CH_3$ | N |
| CH | N | H | $CH_3$ | $CH_3$ | H | $OC_2H_5$ | $NHCH_3$ | N |
| CH | N | H | $CH_3$ | $CH_3$ | $CH_3$ | $OCH_3$ | $CH_3$ | N |
| CH | N | Cl | $CH_3$ | $CH_3$ | H | $OCH_3$ | $OCH_3$ | CH |
| CH | N | Cl | $CH_3$ | $CH_3$ | H | $OCH_3$ | $CH_3$ | CH |
| CH | N | Cl | $CH_3$ | $CH_3$ | H | Cl | $OCH_3$ | CH |
| CH | N | Cl | $CH_3$ | $CH_3$ | H | $CH_3$ | $CH_3$ | CH |
| CH | N | Cl | $CH_3$ | $CH_3$ | H | $OCH_3$ | $OCH_3$ | N |
| CH | N | Cl | $CH_3$ | $CH_3$ | H | $OCH_3$ | $CH_3$ | N |
| CH | N | Cl | $CH_3$ | $CH_3$ | H | $OC_2H_5$ | $NHCH_3$ | N |
| CH | N | Cl | $CH_3$ | $CH_3$ | $CH_3$ | $OCH_3$ | $CH_3$ | N |
| CH | N | $SO_2CH_3$ | $CH_3$ | $CH_3$ | H | $OCH_3$ | $OCH_3$ | CH |
| CH | N | $SO_2CH_3$ | $CH_3$ | $CH_3$ | H | $OCH_3$ | $CH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | N | $SO_2CH_3$ | $CH_3$ | $CH_3$ | H | Cl | $OCH_3$ | CH |
| CH | N | $SO_2CH_3$ | $CH_3$ | $CH_3$ | H | $CH_3$ | $CH_3$ | CH |
| CH | N | $SO_2CH_3$ | $CH_3$ | $CH_3$ | H | $OCH_3$ | $OCH_3$ | N |
| CH | N | $SO_2CH_3$ | $CH_3$ | $CH_3$ | H | $OCH_3$ | $CH_3$ | N |
| CH | N | $SO_2CH_3$ | $CH_3$ | $CH_3$ | H | $OC_2H_5$ | $NHCH_3$ | N |
| CH | N | $SO_2CH_3$ | $CH_3$ | $CH_3$ | $CH_3$ | $OCH_3$ | $CH_3$ | N |
| CH | N | $SO_2N(CH_3)_2$ | $CH_3$ | $CH_3$ | H | $OCH_3$ | $OCH_3$ | CH |
| CH | N | $SO_2N(CH_3)_2$ | $CH_3$ | $CH_3$ | H | $OCH_3$ | $CH_3$ | CH |
| CH | N | $SO_2N(CH_3)_2$ | $CH_3$ | $CH_3$ | H | Cl | $OCH_3$ | CH |
| CH | N | $SO_2N(CH_3)_2$ | $CH_3$ | $CH_3$ | H | $OCH_3$ | $OCH_3$ | N |
| CH | N | $SO_2N(CH_3)_2$ | $CH_3$ | $CH_3$ | H | $OCH_3$ | $CH_3$ | N |
| CH | N | H | H | H | H | $OCH_3$ | $OCH_3$ | CH |
| CH | N | H | H | H | H | $OCH_3$ | $CH_3$ | CH |
| CH | N | H | H | H | H | Cl | $OCH_3$ | CH |
| CH | N | H | H | H | H | $CH_3$ | $CH_3$ | CH |
| CH | N | H | H | H | H | $OCH_3$ | $OCH_3$ | N |
| CH | N | H | H | H | H | $OCH_3$ | $CH_3$ | N |
| CH | N | H | H | H | H | $OC_2H_5$ | $NHCH_3$ | N |
| CH | N | H | H | H | $CH_3$ | $OCH_3$ | $CH_3$ | N |
| CH | N | Cl | H | H | H | $OCH_3$ | $OCH_3$ | CH |
| CH | N | Cl | H | H | H | $OCH_3$ | $CH_3$ | CH |
| CH | N | Cl | H | H | H | Cl | $OCH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | N | Cl | H | H | H | $CH_3$ | $CH_3$ | CH |
| CH | N | Cl | H | H | H | $OCH_3$ | $OCH_3$ | N |
| CH | N | Cl | H | H | H | $OCH_3$ | $CH_3$ | N |
| CH | N | Cl | H | H | H | $OC_2H_5$ | $NHCH_3$ | N |
| CH | N | Cl | H | H | $CH_3$ | $OCH_3$ | $CH_3$ | N |
| CH | N | $SO_2CH_3$ | H | H | H | $OCH_3$ | $OCH_3$ | CH |
| CH | N | $SO_2CH_3$ | H | H | H | $OCH_3$ | $CH_3$ | CH |
| CH | N | $SO_2CH_3$ | H | H | H | Cl | $OCH_3$ | CH |
| CH | N | $SO_2CH_3$ | H | H | H | $CH_3$ | $CH_3$ | CH |
| CH | N | $SO_2CH_3$ | H | H | H | $OCH_3$ | $OCH_3$ | N |
| CH | N | $SO_2CH_3$ | H | H | H | $OCH_3$ | $CH_3$ | N |
| CH | N | $SO_2CH_3$ | H | H | H | $OC_2H_5$ | $NHCH_3$ | N |
| CH | N | $SO_2CH_3$ | H | H | $CH_3$ | $OCH_3$ | $CH_3$ | N |
| CH | N | $SO_2N(CH_3)_2$ | H | H | H | $OCH_3$ | $OCH_3$ | CH |
| CH | N | $SO_2N(CH_3)_2$ | H | H | H | $OCH_3$ | $CH_3$ | CH |
| CH | N | $SO_2N(CH_3)_2$ | H | H | H | Cl | $OCH_3$ | CH |
| CH | N | $SO_2N(CH_3)_2$ | H | H | H | $OCH_3$ | $OCH_3$ | N |
| CH | N | $SO_2N(CH_3)_2$ | H | H | H | $OCH_3$ | $CH_3$ | N |
| CH | N | H | H | $CH_3$ | H | $OCH_3$ | $OCH_3$ | CH |
| CH | N | H | H | $CH_3$ | H | $OCH_3$ | $CH_3$ | CH |
| CH | N | H | H | $CH_3$ | H | Cl | $OCH_3$ | CH |
| CH | N | H | H | $CH_3$ | H | $CH_3$ | $CH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | N | H | H | $CH_3$ | H | $OCH_3$ | $OCH_3$ | N |
| CH | N | H | H | $CH_3$ | H | $OCH_3$ | $CH_3$ | N |
| CH | N | H | H | $CH_3$ | H | $OC_2H_5$ | $NHCH_3$ | N |
| CH | N | H | H | $CH_3$ | $CH_3$ | $OCH_3$ | $CH_3$ | N |
| CH | N | Cl | H | $CH_3$ | H | $OCH_3$ | $OCH_3$ | CH |
| CH | N | Cl | H | $CH_3$ | H | $OCH_3$ | $CH_3$ | CH |
| CH | N | Cl | H | $CH_3$ | H | Cl | $OCH_3$ | CH |
| CH | N | Cl | H | $CH_3$ | H | $CH_3$ | $CH_3$ | CH |
| CH | N | Cl | H | $CH_3$ | H | $OCH_3$ | $OCH_3$ | N |
| CH | N | Cl | H | $CH_3$ | H | $OCH_3$ | $CH_3$ | N |
| CH | N | Cl | H | $CH_3$ | H | $OC_2H_5$ | $NHCH_3$ | N |
| CH | N | Cl | H | $CH_3$ | $CH_3$ | $OCH_3$ | $CH_3$ | N |
| CH | N | $SO_2CH_3$ | H | $CH_3$ | H | $OCH_3$ | $OCH_3$ | CH |
| CH | N | $SO_2CH_3$ | H | $CH_3$ | H | $OCH_3$ | $CH_3$ | CH |
| CH | N | $SO_2CH_3$ | H | $CH_3$ | H | Cl | $OCH_3$ | CH |
| CH | N | $SO_2CH_3$ | H | $CH_3$ | H | $CH_3$ | $CH_3$ | CH |
| CH | N | $SO_2CH_3$ | H | $CH_3$ | H | $OCH_3$ | $OCH_3$ | N |
| CH | N | $SO_2CH_3$ | H | $CH_3$ | H | $OCH_3$ | $CH_3$ | N |
| CH | N | $SO_2CH_3$ | H | $CH_3$ | H | $OC_2H_5$ | $NHCH_3$ | N |
| CH | N | $SO_2CH_3$ | H | $CH_3$ | $CH_3$ | $OCH_3$ | $CH_3$ | N |
| CH | N | $SO_2N(CH_3)_2$ | H | $CH_3$ | H | $OCH_3$ | $OCH_3$ | CH |
| CH | N | $SO_2N(CH_3)_2$ | H | $CH_3$ | H | $OCH_3$ | $CH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | N | $SO_2N(CH_3)_2$ | H | $CH_3$ | H | Cl | $OCH_3$ | CH |
| CH | N | $SO_2N(CH_3)_2$ | H | $CH_3$ | H | $OCH_3$ | $OCH_3$ | N |
| CH | N | $SO_2N(CH_3)_2$ | H | $CH_3$ | H | $OCH_3$ | $CH_3$ | N |
| CH | N | H | $CH_3$ | H | H | $OCH_3$ | $OCH_3$ | CH |
| CH | N | H | $CH_3$ | H | H | $OCH_3$ | $CH_3$ | CH |
| CH | N | H | $CH_3$ | H | H | Cl | $OCH_3$ | CH |
| CH | N | H | $CH_3$ | H | H | $CH_3$ | $CH_3$ | CH |
| CH | N | H | $CH_3$ | H | H | $OCH_3$ | $OCH_3$ | N |
| CH | N | H | $CH_3$ | H | H | $OCH_3$ | $CH_3$ | N |
| CH | N | H | $CH_3$ | H | H | $OC_2H_5$ | $NHCH_3$ | N |
| CH | N | H | $CH_3$ | H | $CH_3$ | $OCH_3$ | $CH_3$ | N |
| CH | N | Cl | $CH_3$ | H | H | $OCH_3$ | $OCH_3$ | CH |
| CH | N | Cl | $CH_3$ | H | H | $OCH_3$ | $CH_3$ | CH |
| CH | N | Cl | $CH_3$ | H | H | Cl | $OCH_3$ | CH |
| CH | N | Cl | $CH_3$ | H | H | $CH_3$ | $CH_3$ | CH |
| CH | N | Cl | $CH_3$ | H | H | $OCH_3$ | $OCH_3$ | N |
| CH | N | Cl | $CH_3$ | H | H | $OCH_3$ | $CH_3$ | N |
| CH | N | Cl | $CH_3$ | H | H | $OC_2H_5$ | $NHCH_3$ | N |
| CH | N | Cl | $CH_3$ | H | $CH_3$ | $OCH_3$ | $CH_3$ | N |
| CH | N | $SO_2CH_3$ | $CH_3$ | H | H | $OCH_3$ | $OCH_3$ | CH |
| CH | N | $SO_2CH_3$ | $CH_3$ | H | H | $OCH_3$ | $CH_3$ | CH |
| CH | N | $SO_2CH_3$ | $CH_3$ | H | H | Cl | $OCH_3$ | CH |
| CH | N | $SO_2CH_3$ | $CH_3$ | H | H | $CH_3$ | $CH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | N | $SO_2CH_3$ | $CH_3$ | H | H | $OCH_3$ | $OCH_3$ | N |
| CH | N | $SO_2CH_3$ | $CH_3$ | H | H | $OCH_3$ | $CH_3$ | N |
| CH | N | $SO_2CH_3$ | $CH_3$ | H | H | $OC_2H_5$ | $NHCH_5$ | N |
| CH | N | $SO_2CH_3$ | $CH_3$ | H | $CH_3$ | $OCH_3$ | $CH_3$ | N |
| CH | N | $SO_2N(CH_3)_2$ | $CH_3$ | H | H | $OCH_3$ | $OCH_3$ | CH |
| CH | N | $SO_2N(CH_3)_2$ | $CH_3$ | H | H | $OCH_3$ | $CH_3$ | CH |
| CH | N | $SO_2N(CH_3)_2$ | $CH_3$ | H | H | Cl | $OCH_3$ | CH |
| CH | N | $SO_2N(CH_3)_2$ | $CH_3$ | H | H | $OCH_3$ | $OCH_3$ | N |
| CH | N | $SO_2N(CH_3)_2$ | $CH_3$ | H | H | $OCH_3$ | $CH_3$ | N |
| CH | CH | $CO_2CH_3$ | $CH_3$ | $CH_3$ | H | $OCH_3$ | $OCH_3$ | CH |
| CH | CH | $CO_2CH_3$ | $CH_3$ | $CH_3$ | H | $OCH_3$ | $CH_3$ | CH |
| CH | CH | $CO_2CH_3$ | $CH_3$ | $CH_3$ | H | Cl | $OCH_3$ | CH |
| CH | CH | $CO_2CH_3$ | $CH_3$ | $CH_3$ | H | $CH_3$ | $CH_3$ | CH |
| CH | CH | $CO_2CH_3$ | $CH_3$ | $CH_3$ | H | $OCH_3$ | $OCH_3$ | N |
| CH | CH | $CO_2CH_3$ | $CH_3$ | $CH_3$ | H | $OCH_3$ | $CH_3$ | N |
| CH | CH | $CO_2CH_3$ | $CH_3$ | $CH_3$ | H | $OC_2H_5$ | $NHCH_3$ | N |
| CH | CH | $CO_2CH_3$ | $CH_3$ | $CH_3$ | $CH_3$ | $OCH_3$ | $CH_3$ | N |
| CH | CH | $CO_2CH_3$ | $CH_3$ | H | H | $OCH_3$ | $OCH_3$ | CH |
| CH | CH | $CO_2CH_3$ | $CH_3$ | H | H | $OCH_3$ | $CH_3$ | CH |
| CH | CH | $CO_2CH_3$ | $CH_3$ | H | H | Cl | $OCH_3$ | CH |
| CH | CH | $CO_2CH_3$ | $CH_3$ | H | H | $CH_3$ | $CH_3$ | CH |
| CH | CH | $CO_2CH_3$ | $CH_3$ | H | H | $OCH_3$ | $OCH_3$ | N |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | CH | $CO_2CH_3$ | $CH_3$ | H | H | $OCH_3$ | $CH_3$ | N |
| CH | CH | $CO_2CH_3$ | $CH_3$ | H | H | $OC_2H_5$ | $NHCH_3$ | N |
| CH | CH | $CO_2CH_3$ | $CH_3$ | H | $CH_3$ | $OCH_3$ | $CH_3$ | N |
| CH | CH | $CO_2CH_3$ | H | $CH_3$ | H | $OCH_3$ | $OCH_3$ | CH |
| CH | CH | $CO_2CH_3$ | H | $CH_3$ | H | $OCH_3$ | $CH_3$ | CH |
| CH | CH | $CO_2CH_3$ | H | $CH_3$ | H | Cl | $OCH_3$ | CH |
| CH | CH | $CO_2CH_3$ | H | $CH_3$ | H | $CH_3$ | $CH_3$ | CH |
| CH | CH | $CO_2CH_3$ | H | $CH_3$ | H | $OCH_3$ | $OCH_3$ | N |
| CH | CH | $CO_2CH_3$ | H | $CH_3$ | H | $OCH_3$ | $CH_3$ | N |
| CH | CH | $CO_2CH_3$ | H | $CH_3$ | H | $OC_2H_5$ | $NHCH_3$ | N |
| CH | CH | $CO_2CH_3$ | H | $CH_3$ | $CH_3$ | $OCH_3$ | $CH_3$ | N |
| CH | CH | $CO_2CH_3$ | H | H | H | $OCH_3$ | $OCH_3$ | CH |
| CH | CH | $CO_2CH_3$ | H | H | H | $OCH_3$ | $CH_3$ | CH |
| CH | CH | $CO_2CH_3$ | H | H | H | Cl | $OCH_3$ | CH |
| CH | CH | $CO_2CH_3$ | H | H | H | $CH_3$ | $CH_3$ | CH |
| CH | CH | $CO_2CH_3$ | H | H | H | $OCH_3$ | $OCH_3$ | N |
| CH | CH | $CO_2CH_3$ | H | H | H | $OCH_3$ | $CH_3$ | N |
| CH | CH | $CO_2CH_3$ | H | H | H | $OC_2H_5$ | $NHCH_3$ | N |
| CH | CH | $CO_2CH_3$ | H | H | $CH_3$ | $OCH_3$ | $CH_3$ | N |
| CH | CH | $CO_2C_2H_5$ | H | H | H | $OCH_3$ | $OCH_3$ | CH |
| CH | CH | $CO_2C_2H_5$ | H | H | H | $OCH_3$ | $CH_3$ | CH |
| CH | CH | $CO_2C_2H_5$ | H | H | H | Cl | $OCH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | CH | $CO_2C_2H_5$ | H | H | H | $CH_3$ | $CH_3$ | CH |
| CH | CH | $CO_2C_2H_5$ | H | H | H | $OCH_3$ | $OCH_3$ | N |
| CH | CH | $CO_2C_2H_5$ | H | H | H | $OCH_3$ | $CH_3$ | N |
| CH | CH | $CO_2C_2H_5$ | H | H | H | $OC_2H_5$ | $NHCH_3$ | N |
| CH | CH | $CO_2C_2H_5$ | H | H | $CH_3$ | $OCH_3$ | $CH_3$ | N |
| CH | CH | $CO_2C_2H_5$ | $CH_3$ | $CH_3$ | H | $OCH_3$ | $OCH_3$ | CH |
| CH | CH | $CO_2C_2H_5$ | $CH_3$ | $CH_3$ | H | $OCH_3$ | $CH_3$ | CH |
| CH | CH | $CO_2C_2H_5$ | $CH_3$ | $CH_3$ | H | Cl | $OCH_3$ | CH |
| CH | CH | $CO_2C_2H_5$ | $CH_3$ | $CH_3$ | H | $CH_3$ | $CH_3$ | CH |
| CH | CH | $CO_2C_2H_5$ | $CH_3$ | $CH_3$ | H | $OCH_3$ | $OCH_3$ | N |
| CH | CH | $CO_2C_2H_5$ | $CH_3$ | $CH_3$ | H | $OCH_3$ | $CH_3$ | N |
| CH | CH | $CO_2C_2H_5$ | $CH_3$ | $CH_3$ | H | $OC_2H_5$ | $NHCH_3$ | N |
| CH | CH | $CO_2C_2H_5$ | $CH_3$ | $CH_3$ | $CH_3$ | $OCH_3$ | $CH_3$ | N |
| CH | N | $CO_2CH_3$ | $CH_3$ | $CH_3$ | H | $OCH_3$ | $OCH_3$ | CH |
| CH | N | $CO_2CH_3$ | $CH_3$ | $CH_3$ | H | $OCH_3$ | $CH_3$ | CH |
| CH | N | $CO_2CH_3$ | $CH_3$ | $CH_3$ | H | Cl | $OCH_3$ | CH |
| CH | N | $CO_2CH_3$ | $CH_3$ | $CH_3$ | H | $CH_3$ | $CH_3$ | CH |
| CH | N | $CO_2CH_3$ | $CH_3$ | $CH_3$ | H | $OCH_3$ | $OCH_3$ | N |
| CH | N | $CO_2CH_3$ | $CH_3$ | $CH_3$ | H | $OCH_3$ | $CH_3$ | N |
| CH | N | $CO_2CH_3$ | $CH_3$ | $CH_3$ | H | $OC_2H_5$ | $NHCH_3$ | N |
| CH | N | $CO_2CH_3$ | $CH_3$ | $CH_3$ | $CH_3$ | $OCH_3$ | $CH_3$ | N |
| CH | N | $CO_2CH_3$ | $CH_3$ | H | H | $OCH_3$ | $OCH_3$ | CH |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | N | $CO_2CH_3$ | $CH_3$ | H | H | $OCH_3$ | $CH_3$ | CH |
| CH | N | $CO_2CH_3$ | $CH_3$ | H | H | Cl | $OCH_3$ | CH |
| CH | N | $CO_2CH_3$ | $CH_3$ | H | H | $CH_3$ | $CH_3$ | CH |
| CH | N | $CO_2CH_3$ | $CH_3$ | H | H | $OCH_3$ | $OCH_3$ | N |
| CH | N | $CO_2CH_3$ | $CH_3$ | H | H | $OCH_3$ | $CH_3$ | N |
| CH | N | $CO_2CH_3$ | $CH_3$ | H | H | $OC_2H_5$ | $NHCH_3$ | N |
| CH | N | $CO_2CH_3$ | $CH_3$ | H | $CH_3$ | $OCH_3$ | $CH_3$ | N |
| CH | N | $CO_2CH_3$ | H | $CH_3$ | H | $OCH_3$ | $OCH_3$ | CH |
| CH | N | $CO_2CH_3$ | H | $CH_3$ | H | $OCH_3$ | $CH_3$ | CH |
| CH | N | $CO_2CH_3$ | H | $CH_3$ | H | Cl | $OCH_3$ | CH |
| CH | N | $CO_2CH_3$ | H | $CH_3$ | H | $CH_3$ | $CH_3$ | CH |
| CH | N | $CO_2CH_3$ | H | $CH_3$ | H | $OCH_3$ | $OCH_3$ | N |
| CH | N | $CO_2CH_3$ | H | $CH_3$ | H | $OCH_3$ | $CH_3$ | N |
| CH | N | $CO_2CH_3$ | H | $CH_3$ | H | $OC_2H_5$ | $NHCH_3$ | N |
| CH | N | $CO_2CH_3$ | H | $CH_3$ | $CH_3$ | $OCH_3$ | $CH_3$ | N |
| CH | N | $CO_2CH_3$ | H | H | H | $OCH_3$ | $OCH_3$ | CH |
| CH | N | $CO_2CH_3$ | H | H | H | $OCH_3$ | $CH_3$ | CH |
| CH | N | $CO_2CH_3$ | H | H | H | Cl | $OCH_3$ | CH |
| CH | N | $CO_2CH_3$ | H | H | H | $CH_3$ | $CH_3$ | CH |
| CH | N | $CO_2CH_3$ | H | H | H | $OCH_3$ | $OCH_3$ | N |
| CH | N | $CO_2CH_3$ | H | H | H | $OCH_3$ | $CH_3$ | N |
| CH | N | $CO_2CH_3$ | H | H | H | $OC_2H_5$ | $NHCH_3$ | N |

Table 5 (continued)

General Formula 5

| Z | $Z^1$ | $R^1$ | $R^2$ | $R^3$ | R | X | Y | $Z^4$ |
|---|---|---|---|---|---|---|---|---|
| CH | N | $CO_2CH_3$ | H | H | $CH_3$ | $OCH_3$ | $CH_3$ | N |
| CH | N | $CO_2C_2H_5$ | H | H | H | $OCH_3$ | $OCH_3$ | CH |
| CH | N | $CO_2C_2H_5$ | H | H | H | $OCH_3$ | $CH_3$ | CH |
| CH | N | $CO_2C_2H_5$ | H | H | H | Cl | $OCH_3$ | CH |
| CH | N | $CO_2C_2H_5$ | H | H | H | $CH_3$ | $CH_3$ | CH |
| CH | N | $CO_2C_2H_5$ | H | H | H | $OCH_3$ | $OCH_3$ | N |
| CH | N | $CO_2C_2H_5$ | H | H | H | $OCH_3$ | $CH_3$ | N |
| CH | N | $CO_2C_2H_5$ | H | H | H | $OC_2H_5$ | $NHCH_3$ | N |
| CH | N | $CO_2C_2H_5$ | H | H | $CH_3$ | $OCH_3$ | $CH_3$ | N |
| CH | N | $CO_2C_2H_5$ | $CH_3$ | $CH_3$ | H | $OCH_3$ | $OCH_3$ | CH |
| CH | N | $CO_2C_2H_5$ | $CH_3$ | $CH_3$ | H | $OCH_3$ | $CH_3$ | CH |
| CH | N | $CO_2C_2H_5$ | $CH_3$ | $CH_3$ | H | Cl | $OCH_3$ | CH |
| CH | N | $CO_2C_2H_5$ | $CH_3$ | $CH_3$ | H | $CH_3$ | $CH_3$ | CH |
| CH | N | $CO_2C_2H_5$ | $CH_3$ | $CH_3$ | H | $OCH_3$ | $OCH_3$ | N |
| CH | N | $CO_2C_2H_5$ | $CH_3$ | $CH_3$ | H | $OCH_3$ | $CH_3$ | N |
| CH | N | $CO_2C_2H_5$ | $CH_3$ | $CH_3$ | H | $OC_2H_5$ | $NHCH_3$ | N |
| CH | N | $CO_2C_2H_5$ | $CH_3$ | $CH_3$ | $CH_3$ | $OCH_3$ | $CH_3$ | N |

Table 6
General Formula 6

| $R^2$ | $R^3$ | $R^4$ | R | X | Y | $Z^4$ |
|---|---|---|---|---|---|---|
| $CH_3$ | $CH_3$ | H | H | $OCH_3$ | $OCH_3$ | CH |
| $CH_3$ | $CH_3$ | H | H | $OCH_3$ | $CH_3$ | CH |
| $CH_3$ | $CH_3$ | H | H | Cl | $OCH_3$ | CH |
| $CH_3$ | $CH_3$ | H | H | $CH_3$ | $CH_3$ | CH |
| $CH_3$ | $CH_3$ | H | H | $OCH_3$ | $OCH_3$ | N |
| $CH_3$ | $CH_3$ | H | H | $OCH_3$ | $CH_3$ | N |
| $CH_3$ | $CH_3$ | H | H | $OC_2H_5$ | $NHCH_3$ | N |
| $CH_3$ | $CH_3$ | H | $CH_3$ | $OCH_3$ | $CH_3$ | N |
| H | $CH_3$ | H | H | $OCH_3$ | $OCH_3$ | CH |
| H | $CH_3$ | H | H | $OCH_3$ | $CH_3$ | CH |
| H | $CH_3$ | H | H | Cl | $OCH_3$ | CH |
| H | $CH_3$ | H | H | $CH_3$ | $CH_3$ | CH |
| H | $CH_3$ | H | H | $OCH_3$ | $OCH_3$ | N |
| H | $CH_3$ | H | H | $OCH_3$ | $CH_3$ | N |
| H | $CH_3$ | H | H | $OC_2H_5$ | $NHCH_3$ | N |
| H | $CH_3$ | H | $CH_3$ | $OCH_3$ | $CH_3$ | N |
| $CH_3$ | H | H | H | $OCH_3$ | $OCH_3$ | CH |
| $CH_3$ | H | H | H | $OCH_3$ | $CH_3$ | CH |
| $CH_3$ | H | H | H | Cl | $OCH_3$ | CH |
| $CH_3$ | H | H | H | $CH_3$ | $CH_3$ | CH |
| $CH_3$ | H | H | H | $OCH_3$ | $OCH_3$ | N |
| $CH_3$ | H | H | H | $OCH_3$ | $CH_3$ | N |
| $CH_3$ | H | H | H | $OC_2H_5$ | $NHCH_3$ | N |

Table 6 (continued)

General Formula 6

| $R^2$ | $R^3$ | $R^4$ | R | X | Y | $Z^4$ |
|---|---|---|---|---|---|---|
| $CH_3$ | H | H | $CH_3$ | $OCH_3$ | $CH_3$ | N |
| H | H | H | H | $OCH_3$ | $OCH_3$ | CH |
| H | H | H | H | $OCH_3$ | $CH_3$ | CH |
| H | H | H | H | Cl | $OCH_3$ | CH |
| H | H | H | H | $CH_3$ | $CH_3$ | CH |
| H | H | H | H | $OCH_3$ | $OCH_3$ | N |
| H | H | H | H | $OCH_3$ | $CH_3$ | N |
| H | H | H | H | $OC_2H_5$ | $NHCH_3$ | N |
| H | H | H | $CH_3$ | $OCH_3$ | $CH_3$ | N |

Table 7

General Formula 7

| $R^4$ | $R^1$ | $R^2$ | $R^3$ | R | X | Y | $Z^4$ |
|---|---|---|---|---|---|---|---|
| H | H | $CH_3$ | $CH_3$ | H | $OCH_3$ | $OCH_3$ | CH |
| H | H | $CH_3$ | $CH_3$ | H | $OCH_3$ | $CH_3$ | CH |
| H | H | $CH_3$ | $CH_3$ | H | Cl | $OCH_3$ | CH |
| H | H | $CH_3$ | $CH_3$ | H | $CH_3$ | $CH_3$ | CH |
| H | H | $CH_3$ | $CH_3$ | H | $OCH_3$ | $OCH_3$ | N |
| H | H | $CH_3$ | $CH_3$ | H | $OCH_3$ | $CH_3$ | N |
| H | H | $CH_3$ | $CH_3$ | H | $OC_2H_5$ | $NHCH_3$ | N |
| H | H | $CH_3$ | $CH_3$ | $CH_3$ | $OCH_3$ | $CH_3$ | N |
| H | Cl | $CH_3$ | $CH_3$ | H | $OCH_3$ | $OCH_3$ | CH |
| H | Cl | $CH_3$ | $CH_3$ | H | $OCH_3$ | $CH_3$ | CH |
| H | Cl | $CH_3$ | $CH_3$ | H | Cl | $OCH_3$ | CH |
| H | Cl | $CH_3$ | $CH_3$ | H | $CH_3$ | $CH_3$ | CH |
| H | Cl | $CH_3$ | $CH_3$ | H | $OCH_3$ | $OCH_3$ | N |
| H | Cl | $CH_3$ | $CH_3$ | H | $OCH_3$ | $CH_3$ | N |
| H | Cl | $CH_3$ | $CH_3$ | H | $OC_2H_5$ | $NHCH_3$ | N |
| H | Cl | $CH_3$ | $CH_3$ | $CH_3$ | $OCH_3$ | $CH_3$ | N |
| H | $SO_2CH_3$ | $CH_3$ | $CH_3$ | H | $OCH_3$ | $OCH_3$ | CH |
| H | $SO_2CH_3$ | $CH_3$ | $CH_3$ | H | $OCH_3$ | $CH_3$ | CH |
| H | $SO_2CH_3$ | $CH_3$ | $CH_3$ | H | Cl | $OCH_3$ | CH |
| H | $SO_2CH_3$ | $CH_3$ | $CH_3$ | H | $CH_3$ | $CH_3$ | CH |
| H | $SO_2CH_3$ | $CH_3$ | $CH_3$ | H | $OCH_3$ | $OCH_3$ | N |
| H | $SO_2CH_3$ | $CH_3$ | $CH_3$ | H | $OCH_3$ | $CH_3$ | N |

Table 7 (continued)

General Formula 7

| $R^4$ | $R^1$ | $R^2$ | $R^3$ | R | X | Y | $Z^4$ |
|---|---|---|---|---|---|---|---|
| H | $SO_2CH_3$ | $CH_3$ | $CH_3$ | H | $OC_2H_5$ | $NHCH_3$ | N |
| H | $SO_2CH_3$ | $CH_3$ | $CH_3$ | $CH_3$ | $OCH_3$ | $CH_3$ | N |
| H | $SO_2N(CH_3)_2$ | $CH_3$ | $CH_3$ | H | $OCH_3$ | $OCH_3$ | CH |
| H | $SO_2N(CH_3)_2$ | $CH_3$ | $CH_3$ | H | $OCH_3$ | $CH_3$ | CH |
| H | $SO_2N(CH_3)_2$ | $CH_3$ | $CH_3$ | H | Cl | $OCH_3$ | CH |
| H | $SO_2N(CH_3)_2$ | $CH_3$ | $CH_3$ | H | $OCH_3$ | $OCH_3$ | N |
| H | $SO_2N(CH_3)_2$ | $CH_3$ | $CH_3$ | H | $OCH_3$ | $CH_3$ | N |
| H | H | H | H | H | $OCH_3$ | $OCH_3$ | CH |
| H | H | H | H | H | $OCH_3$ | $CH_3$ | CH |
| H | H | H | H | H | Cl | $OCH_3$ | CH |
| H | H | H | H | H | $CH_3$ | $CH_3$ | CH |
| H | H | H | H | H | $OCH_3$ | $OCH_3$ | N |
| H | H | H | H | H | $OCH_3$ | $CH_3$ | N |
| H | H | H | H | H | $OC_2H_5$ | $NHCH_3$ | N |
| H | H | H | H | $CH_3$ | $OCH_3$ | $CH_3$ | N |
| H | Cl | H | H | H | $OCH_3$ | $OCH_3$ | CH |
| H | Cl | H | H | H | $OCH_3$ | $CH_3$ | CH |
| H | Cl | H | H | H | Cl | $OCH_3$ | CH |
| H | Cl | H | H | H | $CH_3$ | $CH_3$ | CH |
| H | Cl | H | H | H | $OCH_3$ | $OCH_3$ | N |
| H | Cl | H | H | H | $OCH_3$ | $CH_3$ | N |

Table 7 (continued)

General Formula 7

| $R^4$ | $R^1$ | $R^2$ | $R^3$ | $R$ | $X$ | $Y$ | $Z^4$ |
|---|---|---|---|---|---|---|---|
| H | Cl | H | H | H | $OC_2H_5$ | $NHCH_3$ | N |
| H | Cl | H | H | $CH_3$ | $OCH_3$ | $CH_3$ | N |
| H | $SO_2CH_3$ | H | H | H | $OCH_3$ | $OCH_3$ | CH |
| H | $SO_2CH_3$ | H | H | H | $OCH_3$ | $CH_3$ | CH |
| H | $SO_2CH_3$ | H | H | H | Cl | $OCH_3$ | CH |
| H | $SO_2CH_3$ | H | H | H | $CH_3$ | $CH_3$ | CH |
| H | $SO_2CH_3$ | H | H | H | $OCH_3$ | $OCH_3$ | N |
| H | $SO_2CH_3$ | H | H | H | $OCH_3$ | $CH_3$ | N |
| H | $SO_2CH_3$ | H | H | H | $OC_2H_5$ | $NHCH_3$ | N |
| H | $SO_2CH_3$ | H | H | $CH_3$ | $OCH_3$ | $CH_3$ | N |
| H | $SO_2N(CH_3)_2$ | H | H | H | $OCH_3$ | $OCH_3$ | CH |
| H | $SO_2N(CH_3)_2$ | H | H | H | $OCH_3$ | $CH_3$ | CH |
| H | $SO_2N(CH_3)_2$ | H | H | H | Cl | $OCH_3$ | CH |
| H | $SO_2N(CH_3)_2$ | H | H | H | $OCH_3$ | $OCH_3$ | N |
| H | $SO_2N(CH_3)_2$ | H | H | H | $OCH_3$ | $CH_3$ | N |
| H | H | H | $CH_3$ | H | $OCH_3$ | $OCH_3$ | CH |
| H | H | H | $CH_3$ | H | $OCH_3$ | $CH_3$ | CH |
| H | H | H | $CH_3$ | H | Cl | $OCH_3$ | CH |
| H | H | H | $CH_3$ | H | $CH_3$ | $CH_3$ | CH |
| H | H | H | $CH_3$ | H | $OCH_3$ | $OCH_3$ | N |
| H | H | H | $CH_3$ | H | $OCH_3$ | $CH_3$ | N |

Table 7 (continued)

General Formula 7

| $R^4$ | $R^1$ | $R^2$ | $R^3$ | R | X | Y | $Z^4$ |
|---|---|---|---|---|---|---|---|
| H | H | H | $CH_3$ | H | $OC_2H_5$ | $NHCH_3$ | N |
| H | H | H | $CH_3$ | $CH_3$ | $OCH_3$ | $CH_3$ | N |
| H | Cl | H | $CH_3$ | H | $OCH_3$ | $OCH_3$ | CH |
| H | Cl | H | $CH_3$ | H | $OCH_3$ | $CH_3$ | CH |
| H | Cl | H | $CH_3$ | H | Cl | $OCH_3$ | CH |
| H | Cl | H | $CH_3$ | H | $CH_3$ | $CH_3$ | CH |
| H | Cl | H | $CH_3$ | H | $OCH_3$ | $OCH_3$ | N |
| H | Cl | H | $CH_3$ | H | $OCH_3$ | $CH_3$ | N |
| H | Cl | H | $CH_3$ | H | $OC_2H_5$ | $NHCH_3$ | N |
| H | Cl | H | $CH_3$ | $CH_3$ | $OCH_3$ | $CH_3$ | N |
| H | $SO_2CH_3$ | H | $CH_3$ | H | $OCH_3$ | $OCH_3$ | CH |
| H | $SO_2CH_3$ | H | $CH_3$ | H | $OCH_3$ | $CH_3$ | CH |
| H | $SO_2CH_3$ | H | $CH_3$ | H | Cl | $OCH_3$ | CH |
| H | $SO_2CH_3$ | H | $CH_3$ | H | $CH_3$ | $CH_3$ | CH |
| H | $SO_2CH_3$ | H | $CH_3$ | H | $OCH_3$ | $OCH_3$ | N |
| H | $SO_2CH_3$ | H | $CH_3$ | H | $OCH_3$ | $CH_3$ | N |
| H | $SO_2CH_3$ | H | $CH_3$ | H | $OC_2H_5$ | $NHCH_3$ | N |
| H | $SO_2CH_3$ | H | $CH_3$ | H | $OCH_3$ | $CH_3$ | N |
| H | $SO_2N(CH_3)_2$ | H | $CH_3$ | H | $OCH_3$ | $OCH_3$ | CH |
| H | $SO_2N(CH_3)_2$ | H | $CH_3$ | H | $OCH_3$ | $CH_3$ | CH |
| H | $SO_2N(CH_3)_2$ | H | $CH_3$ | H | Cl | $OCH_3$ | CH |

Table 7 (continued)

General Formula 7

| $R^4$ | $R^1$ | $R^2$ | $R^3$ | R | X | Y | $Z^4$ |
|---|---|---|---|---|---|---|---|
| H | $SO_2N(CH_3)_2$ | H | $CH_3$ | H | $OCH_3$ | $OCH_3$ | N |
| H | $SO_2N(CH_3)_2$ | H | $CH_3$ | H | $OCH_3$ | $CH_3$ | N |
| H | $CO_2CH_3$ | $CH_3$ | $CH_3$ | H | $OCH_3$ | $OCH_3$ | CH |
| H | $CO_2CH_3$ | $CH_3$ | $CH_3$ | H | $OCH_3$ | $CH_3$ | CH |
| H | $CO_2CH_3$ | $CH_3$ | $CH_3$ | H | Cl | $OCH_3$ | CH |
| H | $CO_2CH_3$ | $CH_3$ | $CH_3$ | H | $CH_3$ | $CH_3$ | CH |
| H | $CO_2CH_3$ | $CH_3$ | $CH_3$ | H | $OCH_3$ | $OCH_3$ | N |
| H | $CO_2CH_3$ | $CH_3$ | $CH_3$ | H | $OCH_3$ | $CH_3$ | N |
| H | $CO_2CH_3$ | $CH_3$ | $CH_3$ | H | $OC_2H_5$ | $NHCH_3$ | N |
| H | $CO_2CH_3$ | $CH_3$ | $CH_3$ | $CH_3$ | $OCH_3$ | $CH_3$ | N |
| H | $CO_2CH_3$ | $CH_3$ | H | H | $OCH_3$ | $OCH_3$ | CH |
| H | $CO_2CH_3$ | $CH_3$ | H | H | $OCH_3$ | $CH_3$ | CH |
| H | $CO_2CH_3$ | $CH_3$ | H | H | Cl | $OCH_3$ | CH |
| H | $CO_2CH_3$ | $CH_3$ | H | H | $CH_3$ | $CH_3$ | CH |
| H | $CO_2CH_3$ | $CH_3$ | H | H | $OCH_3$ | $OCH_3$ | N |
| H | $CO_2CH_3$ | $CH_3$ | H | H | $OCH_3$ | $CH_3$ | N |
| H | $CO_2CH_3$ | $CH_3$ | H | H | $OC_2H_5$ | $NHCH_3$ | N |
| H | $CO_2CH_3$ | $CH_3$ | H | $CH_3$ | $OCH_3$ | $CH_3$ | N |
| H | $CO_2CH_3$ | H | $CH_3$ | H | $OCH_3$ | $OCH_3$ | CH |
| H | $CO_2CH_3$ | H | $CH_3$ | H | $OCH_3$ | $CH_3$ | CH |
| H | $CO_2CH_3$ | H | $CH_3$ | H | Cl | $OCH_3$ | CH |
| H | $CO_2CH_3$ | H | $CH_3$ | H | $CH_3$ | $CH_3$ | CH |

Table 7 (continued)

General Formula 7

| $R^4$ | $R^1$ | $R^2$ | $R^3$ | R | X | Y | $Z^4$ |
|---|---|---|---|---|---|---|---|
| H | $CO_2CH_3$ | H | $CH_3$ | H | $OCH_3$ | $OCH_3$ | N |
| H | $CO_2CH_3$ | H | $CH_3$ | H | $OCH_3$ | $CH_3$ | N |
| H | $CO_2CH_3$ | H | $CH_3$ | H | $OC_2H_5$ | $NHCH_3$ | N |
| H | $CO_2CH_3$ | H | $CH_3$ | $CH_3$ | $OCH_3$ | $CH_3$ | N |
| H | $CO_2CH_3$ | H | $CH_3$ | H | $OCH_3$ | $OCH_3$ | CH |
| H | $CO_2CH_3$ | H | H | H | $OCH_3$ | $OCH_3$ | CH |
| H | $CO_2CH_3$ | H | H | H | $OCH_3$ | $CH_3$ | CH |
| H | $CO_2CH_3$ | H | H | H | Cl | $OCH_3$ | CH |
| H | $CO_2CH_3$ | H | H | H | $CH_3$ | $CH_3$ | CH |
| H | $CO_2CH_3$ | H | H | H | $OCH_3$ | $OCH_3$ | N |
| H | $CO_2CH_3$ | H | H | H | $OCH_3$ | $CH_3$ | N |
| H | $CO_2CH_3$ | H | H | H | $OC_2H_5$ | $NHCH_3$ | N |
| H | $CO_2CH_3$ | H | H | $CH_3$ | $OCH_3$ | $CH_3$ | N |
| H | $CO_2C_2H_5$ | H | H | H | $OCH_3$ | $OCH_3$ | CH |
| H | $CO_2C_2H_5$ | H | H | H | $OCH_3$ | $CH_3$ | CH |
| H | $CO_2C_2H_5$ | H | H | H | Cl | $OCH_3$ | CH |
| H | $CO_2C_2H_5$ | H | H | H | $CH_3$ | $CH_3$ | CH |
| H | $CO_2C_2H_5$ | H | H | H | $OCH_3$ | $OCH_3$ | N |
| H | $CO_2C_2H_5$ | H | H | H | $OCH_3$ | $CH_3$ | N |
| H | $CO_2C_2H_5$ | H | H | H | $OC_2H_5$ | $NHCH_3$ | N |
| H | $CO_2C_2H_5$ | H | H | $CH_3$ | $OCH_3$ | $CH_3$ | N |
| H | $CO_2C_2H_5$ | $CH_3$ | $CH_3$ | H | $OCH_3$ | $OCH_3$ | CH |

Table 7 (continued)

General Formula 7

| $R^4$ | $R^1$ | $R^2$ | $R^3$ | R | X | Y | $Z^4$ |
|---|---|---|---|---|---|---|---|
| H | $CO_2C_2H_5$ | $CH_3$ | $CH_3$ | H | $OCH_3$ | $CH_3$ | CH |
| H | $CO_2C_2H_5$ | $CH_3$ | $CH_3$ | H | Cl | $OCH_3$ | CH |
| H | $CO_2C_2H_5$ | $CH_3$ | $CH_3$ | H | $CH_3$ | $CH_3$ | CH |
| H | $CO_2C_2H_5$ | $CH_3$ | $CH_3$ | H | $OCH_3$ | $OCH_3$ | N |
| H | $CO_2C_2H_5$ | $CH_3$ | $CH_3$ | H | $OCH_3$ | $CH_3$ | N |
| H | $CO_2C_2H_5$ | $CH_3$ | $CH_3$ | H | $OC_2H_5$ | $NHCH_3$ | N |
| H | $CO_2C_2H_5$ | $CH_3$ | $CH_3$ | $CH_3$ | $OCH_3$ | $CH_3$ | N |
| H | $CO_2C_2H_5$ | $CH_3$ | $CH_3$ | H | $OCH_3$ | $OCH_3$ | CH |
| H | H | $CH_3$ | H | H | $OCH_3$ | $OCH_3$ | CH |
| H | H | $CH_3$ | H | H | $OCH_3$ | $CH_3$ | CH |
| H | H | $CH_3$ | H | H | Cl | $OCH_3$ | CH |
| H | H | $CH_3$ | H | H | $CH_3$ | $CH_3$ | CH |
| H | H | $CH_3$ | H | H | $OCH_3$ | $OCH_3$ | N |
| N | H | $CH_3$ | H | H | $OCH_3$ | $CH_3$ | N |
| N | H | $CH_3$ | H | H | $OC_2H_5$ | $NHCH_3$ | N |
| N | H | $CH_3$ | H | $CH_3$ | $OCH_3$ | $CH_3$ | N |
| N | Cl | $CH_3$ | H | H | $OCH_3$ | $OCH_3$ | CH |
| N | Cl | $CH_3$ | H | H | $OCH_3$ | $CH_3$ | CH |
| N | Cl | $CH_3$ | H | H | Cl | $OCH_3$ | CH |
| N | Cl | $CH_3$ | H | H | $CH_3$ | $CH_3$ | CH |
| N | Cl | $CH_3$ | H | H | $OCH_3$ | $OCH_3$ | N |
| N | Cl | $CH_3$ | H | H | $OCH_3$ | $CH_3$ | N |
| N | Cl | $CH_3$ | H | H | $OC_2H_5$ | $NHCH_3$ | N |

Table 7 (continued)

General Formula 7

| $R^4$ | $R^1$ | $R^2$ | $R^3$ | R | X | Y | $Z^4$ |
|---|---|---|---|---|---|---|---|
| N | Cl | $CH_3$ | H | $CH_3$ | $OCH_3$ | $CH_3$ | N |
| N | $SO_2CH_3$ | $CH_3$ | H | H | $OCH_3$ | $OCH_3$ | CH |
| N | $SO_2CH_3$ | $CH_3$ | H | H | $OCH_3$ | $CH_3$ | CH |
| N | $SO_2CH_3$ | $CH_3$ | H | H | Cl | $OCH_3$ | CH |
| N | $SO_2CH_3$ | $CH_3$ | H | H | $CH_3$ | $CH_3$ | CH |
| N | $SO_2CH_3$ | $CH_3$ | H | H | $OCH_3$ | $OCH_3$ | N |
| N | $SO_2CH_3$ | $CH_3$ | H | H | $OCH_3$ | $CH_3$ | N |
| N | $SO_2CH_3$ | $CH_3$ | H | H | $OC_2H_5$ | $NHCH_3$ | N |
| N | $SO_2CH_3$ | $CH_3$ | H | $CH_3$ | $OCH_3$ | $CH_3$ | N |
| N | $SO_2N(CH_3)_2$ | $CH_3$ | H | H | $OCH_3$ | $OCH_3$ | CH |
| N | $SO_2N(CH_3)_2$ | $CH_3$ | H | H | $OCH_3$ | $CH_3$ | CH |
| N | $SO_2N(CH_3)_2$ | $CH_3$ | H | H | Cl | $OCH_3$ | CH |
| N | $SO_2N(CH_3)_2$ | $CH_3$ | H | H | $OCH_3$ | $OCH_3$ | N |
| N | $SO_2N(CH_3)_2$ | $CH_3$ | H | H | $OCH_3$ | $CH_3$ | N |

Table 8

General Formula 8

| $R^4$ | $R^1$ | $R^2$ | $R^3$ | $R^5$ | R | X | Y | Z |
|---|---|---|---|---|---|---|---|---|
| H | H | $CH_3$ | $CH_3$ | H | H | $OCH_3$ | $OCH_3$ | CH |
| H | H | $CH_3$ | $CH_3$ | H | H | $OCH_3$ | $CH_3$ | CH |
| H | H | $CH_3$ | $CH_3$ | H | H | Cl | $OCH_3$ | CH |
| H | H | $CH_3$ | $CH_3$ | H | H | $CH_3$ | $CH_3$ | CH |
| H | H | $CH_3$ | $CH_3$ | H | H | $OCH_3$ | $OCH_3$ | N |
| H | H | $CH_3$ | $CH_3$ | H | H | $OCH_3$ | $CH_3$ | N |
| H | H | $CH_3$ | $CH_3$ | H | H | $OC_2H_5$ | $NHCH_3$ | N |
| H | H | $CH_3$ | $CH_3$ | H | $CH_3$ | $OCH_3$ | $CH_3$ | N |
| H | Cl | $CH_3$ | $CH_3$ | H | H | $OCH_3$ | $OCH_3$ | CH |
| H | Cl | $CH_3$ | $CH_3$ | H | H | $OCH_3$ | $CH_3$ | CH |
| H | Cl | $CH_3$ | $CH_3$ | H | H | Cl | $OCH_3$ | CH |
| H | Cl | $CH_3$ | $CH_3$ | H | H | $CH_3$ | $CH_3$ | CH |
| H | Cl | $CH_3$ | $CH_3$ | H | H | $OCH_3$ | $OCH_3$ | N |
| H | Cl | $CH_3$ | $CH_3$ | H | H | $OCH_3$ | $CH_3$ | N |
| H | Cl | $CH_3$ | $CH_3$ | H | H | $OC_2H_5$ | $NHCH_3$ | N |
| H | Cl | $CH_3$ | $CH_3$ | H | $CH_3$ | $OCH_3$ | $CH_3$ | N |
| H | $SO_2CH_3$ | $CH_3$ | $CH_3$ | H | H | $OCH_3$ | $OCH_3$ | CH |
| H | $SO_2CH_3$ | $CH_3$ | $CH_3$ | H | H | $OCH_3$ | $CH_3$ | CH |
| H | $SO_2CH_3$ | $CH_3$ | $CH_3$ | H | H | Cl | $OCH_3$ | CH |
| H | $SO_2CH_3$ | $CH_3$ | $CH_3$ | H | H | $CH_3$ | $CH_3$ | CH |
| H | $SO_2CH_3$ | $CH_3$ | $CH_3$ | H | H | $OCH_3$ | $OCH_3$ | N |
| H | $SO_2CH_3$ | $CH_3$ | $CH_3$ | H | H | $OCH_3$ | $CH_3$ | N |
| H | $SO_2CH_3$ | $CH_3$ | $CH_3$ | H | H | $OC_2H_5$ | $NHCH_3$ | N |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $SO_2CH_3$ | $CH_3$ | $CH_3$ | H | $CH_3$ | $OCH_3$ | $CH_3$ | N |
| H | $SO_2N(CH_3)_2$ | $CH_3$ | $CH_3$ | H | H | $OCH_3$ | $OCH_3$ | CH |
| H | $SO_2N(CH_3)_2$ | $CH_3$ | $CH_3$ | H | H | $OCH_3$ | $CH_3$ | CH |
| H | $SO_2N(CH_3)_2$ | $CH_3$ | $CH_3$ | H | H | Cl | $OCH_3$ | CH |
| H | $SO_2N(CH_3)_2$ | $CH_3$ | $CH_3$ | H | H | $OCH_3$ | $OCH_3$ | N |
| H | $SO_2N(CH_3)_2$ | $CH_3$ | $CH_3$ | H | H | $OCH_3$ | $CH_3$ | N |
| H | H | H | H | H | H | $OCH_3$ | $OCH_3$ | CH |
| H | H | H | H | H | H | $OCH_3$ | $CH_3$ | CH |
| H | H | H | H | H | H | Cl | $OCH_3$ | CH |
| H | H | H | H | H | H | $CH_3$ | $CH_3$ | CH |
| H | H | H | H | H | H | $OCH_3$ | $OCH_3$ | N |
| H | H | H | H | H | H | $OCH_3$ | $CH_3$ | N |
| H | H | H | H | H | H | $OC_2H_5$ | $NHCH_3$ | N |
| H | H | H | H | H | $CH_3$ | $OCH_3$ | $CH_3$ | N |
| H | Cl | H | H | H | H | $OCH_3$ | $OCH_3$ | CH |
| H | Cl | H | H | H | H | $OCH_3$ | $CH_3$ | CH |
| H | Cl | H | H | H | H | Cl | $OCH_3$ | CH |
| H | Cl | H | H | H | H | $CH_3$ | $CH_3$ | CH |
| H | Cl | H | H | H | H | $OCH_3$ | $OCH_3$ | N |
| H | Cl | H | H | H | H | $OCH_3$ | $CH_3$ | N |
| H | Cl | H | H | H | H | $OC_2H_5$ | $NHCH_3$ | N |
| H | Cl | H | H | H | $CH_3$ | $OCH_3$ | $CH_3$ | N |

Table 8 (continued)

General Formula 8

| $R^6$ | $R^1$ | $R^2$ | $R^3$ | $R^5$ | R | X | Y | $Z^4$ |
|---|---|---|---|---|---|---|---|---|
| H | $SO_2CH_3$ | H | H | H | H | $OCH_3$ | $OCH_3$ | CH |
| H | $SO_2CH_3$ | H | H | H | H | $OCH_3$ | $CH_3$ | CH |
| H | $SO_2CH_3$ | H | H | H | H | Cl | $OCH_3$ | CH |
| H | $SO_2CH_3$ | H | H | H | H | $CH_3$ | $CH_3$ | CH |
| H | $SO_2CH_3$ | H | H | H | H | $OCH_3$ | $OCH_3$ | N |
| H | $SO_2CH_3$ | H | H | H | H | $OCH_3$ | $CH_3$ | N |
| H | $SO_2CH_3$ | H | H | H | H | $OC_2H_5$ | $NHCH_3$ | N |
| H | $SO_2CH_3$ | H | H | H | $CH_3$ | $OCH_3$ | $CH_3$ | N |
| H | $SO_2N(CH_3)_2$ | H | H | H | H | $OCH_3$ | $OCH_3$ | CH |
| H | $SO_2N(CH_3)_2$ | H | H | H | H | $OCH_3$ | $CH_3$ | CH |
| H | $SO_2N(CH_3)_2$ | H | H | H | H | Cl | $OCH_3$ | CH |
| H | $SO_2N(CH_3)_2$ | H | H | H | H | $OCH_3$ | $OCH_3$ | N |
| H | $SO_2N(CH_3)_2$ | H | H | H | H | $OCH_3$ | $CH_3$ | N |
| H | H | H | $CH_3$ | H | H | $OCH_3$ | $OCH_3$ | CH |
| H | H | H | $CH_3$ | H | H | $OCH_3$ | $CH_3$ | CH |
| H | H | H | $CH_3$ | H | H | Cl | $OCH_3$ | CH |
| H | H | H | $CH_3$ | H | H | $CH_3$ | $CH_3$ | CH |
| H | H | H | $CH_3$ | H | H | $OCH_3$ | $OCH_3$ | N |
| H | H | H | $CH_3$ | H | H | $OCH_3$ | $CH_3$ | N |
| H | H | H | $CH_3$ | H | H | $OC_2H_5$ | $NHCH_3$ | N |
| H | H | H | $CH_3$ | H | $CH_3$ | $OCH_3$ | $CH_3$ | N |
| H | Cl | H | $CH_3$ | H | H | $OCH_3$ | $OCH_3$ | CH |

Table 8 (continued)

General Formula 8

| $R^4$ | $R^1$ | $R^2$ | $R^3$ | $R^5$ | R | X | Y | Z |
|---|---|---|---|---|---|---|---|---|
| H | Cl | H | $CH_3$ | H | H | $OCH_3$ | $CH_3$ | CH |
| H | Cl | H | $CH_3$ | H | H | Cl | $OCH_3$ | CH |
| H | Cl | H | $CH_3$ | H | H | $CH_3$ | $CH_3$ | CH |
| H | Cl | H | $CH_3$ | H | H | $OCH_3$ | $OCH_3$ | N |
| H | Cl | H | $CH_3$ | H | H | $OCH_3$ | $CH_3$ | N |
| H | Cl | H | $CH_3$ | H | H | $OC_2H_5$ | $NHCH_3$ | N |
| H | Cl | H | $CH_3$ | H | H | $OCH_3$ | $CH_3$ | N |
| H | $SO_2CH_3$ | H | $CH_3$ | H | H | $OCH_3$ | $OCH_3$ | CH |
| H | $SO_2CH_3$ | H | $CH_3$ | H | H | $OCH_3$ | $CH_3$ | CH |
| H | $SO_2CH_3$ | H | $CH_3$ | H | H | Cl | $OCH_3$ | CH |
| H | $SO_2CH_3$ | H | $CH_3$ | H | H | $CH_3$ | $CH_3$ | CH |
| H | $SO_2CH_3$ | H | $CH_3$ | H | H | $OCH_3$ | $OCH_3$ | N |
| H | $SO_2CH_3$ | H | $CH_3$ | H | H | $OCH_3$ | $CH_3$ | N |
| H | $SO_2CH_3$ | H | $CH_3$ | H | H | $OC_2H_5$ | $NHCH_3$ | N |
| H | $SO_2CH_3$ | H | $CH_3$ | H | $CH_3$ | $OCH_3$ | $CH_3$ | N |
| H | $SO_2N(CH_3)_2$ | H | $CH_3$ | H | H | $OCH_3$ | $OCH_3$ | CH |
| H | $SO_2N(CH_3)_2$ | H | $CH_3$ | H | H | $OCH_3$ | $CH_3$ | CH |
| H | $SO_2N(CH_3)_2$ | H | $CH_3$ | H | H | Cl | $OCH_3$ | CH |
| H | $SO_2N(CH_3)_2$ | H | $CH_3$ | H | H | $OCH_3$ | $OCH_3$ | N |
| H | $SO_2N(CH_3)_2$ | H | $CH_3$ | H | H | $OCH_3$ | $CH_3$ | N |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | H | $CH_3$ | H | H | H | $OCH_3$ | $OCH_3$ | CH |
| H | H | $CH_3$ | H | H | H | $OCH_3$ | $CH_3$ | CH |
| H | H | $CH_3$ | H | H | H | Cl | $OCH_3$ | CH |
| H | H | $CH_3$ | H | H | H | $CH_3$ | $CH_3$ | CH |
| H | H | $CH_3$ | H | H | H | $OCH_3$ | $OCH_3$ | N |
| H | H | $CH_3$ | H | H | H | $OCH_3$ | $CH_3$ | N |
| H | H | $CH_3$ | H | H | H | $OC_2H_5$ | $NHCH_3$ | N |
| H | H | $CH_3$ | H | H | $CH_3$ | $OCH_3$ | $CH_3$ | N |
| H | Cl | $CH_3$ | H | H | H | $OCH_3$ | $OCH_3$ | CH |
| H | Cl | $CH_3$ | H | H | H | $OCH_3$ | $CH_3$ | CH |
| H | Cl | $CH_3$ | H | H | H | Cl | $OCH_3$ | CH |
| H | Cl | $CH_3$ | H | H | H | $CH_3$ | $CH_3$ | CH |
| H | Cl | $CH_3$ | H | H | H | $OCH_3$ | $OCH_3$ | N |
| H | Cl | $CH_3$ | H | H | H | $OCH_3$ | $CH_3$ | N |
| H | Cl | $CH_3$ | H | H | H | $OC_2H_5$ | $NHCH_3$ | N |
| H | Cl | $CH_3$ | H | H | H | $OCH_3$ | $CH_3$ | N |
| H | $SO_2CH_3$ | $CH_3$ | H | H | H | $OCH_3$ | $OCH_3$ | CH |
| H | $SO_2CH_3$ | $CH_3$ | H | H | H | $OCH_3$ | $CH_3$ | CH |
| H | $SO_2CH_3$ | $CH_3$ | H | H | H | Cl | $OCH_3$ | CH |
| H | $SO_2CH_3$ | $CH_3$ | H | H | H | $CH_3$ | $CH_3$ | CH |
| H | $SO_2CH_3$ | $CH_3$ | H | H | H | $OCH_3$ | $OCH_3$ | N |
| H | $SO_2CH_3$ | $CH_3$ | H | H | H | $OCH_3$ | $CH_3$ | N |
| H | $SO_2CH_3$ | $CH_3$ | H | H | H | $OC_2H_5$ | $NHCH_3$ | N |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $SO_2CH_3$ | $CH_3$ | H | H | $CH_3$ | $OCH_3$ | $CH_3$ | N |
| H | $SO_2N(CH_3)_2$ | $CH_3$ | H | H | H | $OCH_3$ | $OCH_3$ | CH |
| H | $SO_2N(CH_3)_2$ | $CH_3$ | H | H | H | $OCH_3$ | $CH_3$ | CH |
| H | $SO_2N(CH_3)_2$ | $CH_3$ | H | H | H | Cl | $OCH_3$ | CH |
| H | $SO_2N(CH_3)_2$ | $CH_3$ | H | H | H | $OCH_3$ | $OCH_3$ | N |
| H | $SO_2N(CH_3)_2$ | CH | H | H | H | $OCH_3$ | $CH_3$ | N |
| H | H | $CH_3$ | $CH_3$ | H | H | $OCH_3$ | $OCH_3$ | CH |
| H | H | $CH_3$ | $CH_3$ | H | H | $OCH_3$ | $CH_3$ | CH |
| H | H | $CH_3$ | $CH_3$ | H | H | Cl | $OCH_3$ | CH |
| H | H | $CH_3$ | $CH_3$ | H | H | $CH_3$ | $CH_3$ | CH |
| H | H | $CH_3$ | $CH_3$ | H | H | $OCH_3$ | $OCH_3$ | N |
| H | H | $CH_3$ | $CH_3$ | H | H | $OCH_3$ | $CH_3$ | N |
| H | H | $CH_3$ | $CH_3$ | H | H | $OC_2H_5$ | $NHCH_3$ | N |
| H | H | $CH_3$ | $CH_3$ | H | $CH_3$ | $OCH_3$ | $CH_3$ | N |
| H | Cl | $CH_3$ | $CH_3$ | H | H | $OCH_3$ | $OCH_3$ | CH |
| H | Cl | $CH_3$ | $CH_3$ | H | H | $OCH_3$ | $CH_3$ | CH |
| H | Cl | $CH_3$ | $CH_3$ | H | H | Cl | $OCH_3$ | CH |
| H | Cl | $CH_3$ | $CH_3$ | H | H | $CH_3$ | $CH_3$ | CH |
| H | Cl | $CH_3$ | $CH_3$ | H | H | $OCH_3$ | $OCH_3$ | N |
| H | Cl | $CH_3$ | $CH_3$ | H | H | $OCH_3$ | $CH_3$ | N |
| H | Cl | $CH_3$ | $CH_3$ | H | H | $OC_2H_5$ | $NHCH_3$ | N |
| H | Cl | $CH_3$ | $CH_3$ | H | H | $OCH_3$ | $CH_3$ | N |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $SO_2CH_3$ | $CH_3$ | $CH_3$ | H | H | $OCH_3$ | $OCH_3$ | CH |
| H | $SO_2CH_3$ | $CH_3$ | $CH_3$ | H | H | $OCH_3$ | $CH_3$ | CH |
| H | $SO_2CH_3$ | $CH_3$ | $CH_3$ | H | H | Cl | $OCH_3$ | CH |
| H | $SO_2CH_3$ | $CH_3$ | $CH_3$ | H | H | $CH_3$ | $CH_3$ | CH |
| H | $SO_2CH_3$ | $CH_3$ | $CH_3$ | H | H | $OCH_3$ | $OCH_3$ | N |
| H | $SO_2CH_3$ | $CH_3$ | $CH_3$ | H | H | $OCH_3$ | $CH_3$ | N |
| H | $SO_2CH_3$ | $CH_3$ | $CH_3$ | H | H | $OC_2H_5$ | $NHCH_3$ | N |
| H | $SO_2CH_3$ | $CH_3$ | $CH_3$ | H | $CH_3$ | $OCH_3$ | $CH_3$ | N |
| H | $SO_2N(CH_3)_2$ | $CH_3$ | $CH_3$ | H | H | $OCH_3$ | $OCH_3$ | CH |
| H | $SO_2N(CH_3)_2$ | $CH_3$ | $CH_3$ | H | H | $OCH_3$ | $CH_3$ | CH |
| H | $SO_2N(CH_3)_2$ | $CH_3$ | $CH_3$ | H | H | Cl | $OCH_3$ | CH |
| H | $SO_2N(CH_3)_2$ | $CH_3$ | $CH_3$ | H | H | $OCH_3$ | $OCH_3$ | N |
| H | $SO_2N(CH_3)_2$ | $CH_3$ | $CH_3$ | H | H | $OCH_3$ | $CH_3$ | N |
| H | H | H | H | H | H | $OCH_3$ | $OCH_3$ | CH |
| H | H | H | H | H | H | $OCH_3$ | $CH_3$ | CH |
| H | H | H | H | H | H | Cl | $OCH_3$ | CH |
| H | H | H | H | H | H | $CH_3$ | $CH_3$ | CH |
| H | H | H | H | H | H | $OCH_3$ | $OCH_3$ | N |
| H | H | H | H | H | H | $OCH_3$ | $CH_3$ | N |
| H | H | H | H | H | H | $OC_2H_5$ | $NHCH_3$ | N |
| H | H | H | H | H | $CH_3$ | $OCH_3$ | $CH_3$ | N |
| H | Cl | H | H | H | H | $OCH_3$ | $OCH_3$ | CH |

Table 8 (continued)

General Formula 8

| $R^4$ | $R^1$ | $R^2$ | $R^3$ | $R^5$ | R | X | Y | Z |
|---|---|---|---|---|---|---|---|---|
| H | Cl | H | H | H | H | $OCH_3$ | $CH_3$ | CH |
| H | Cl | H | H | H | H | Cl | $OCH_3$ | CH |
| H | Cl | H | H | H | H | $CH_3$ | $CH_3$ | CH |
| H | Cl | H | H | H | H | $OCH_3$ | $OCH_3$ | N |
| H | Cl | H | H | H | H | $OCH_3$ | $CH_3$ | N |
| H | Cl | H | H | H | H | $OC_2H_5$ | $NHCH_3$ | N |
| H | Cl | H | H | H | H | $OCH_3$ | $CH_3$ | N |
| H | $SO_2CH_3$ | H | H | H | H | $OCH_3$ | $OCH_3$ | CH |
| H | $SO_2CH_3$ | H | H | H | H | $OCH_3$ | $CH_3$ | CH |
| H | $SO_2CH_3$ | H | H | H | H | Cl | $OCH_3$ | CH |
| H | $SO_2CH_3$ | H | H | H | H | $CH_3$ | $CH_3$ | CH |
| H | $SO_2CH_3$ | H | H | H | H | $OCH_3$ | $OCH_3$ | N |
| H | $SO_2CH_3$ | H | H | H | H | $OCH_3$ | $CH_3$ | N |
| H | $SO_2CH_3$ | H | H | H | H | $OC_2H_5$ | $NHCH_3$ | N |
| H | $SO_2CH_3$ | H | H | H | $CH_3$ | $OCH_3$ | $CH_3$ | N |
| H | $SO_2N(CH_3)_2$ | H | H | H | H | $OCH_3$ | $OCH_3$ | CH |
| H | $SO_2N(CH_3)_2$ | H | H | H | H | $OCH_3$ | $CH_3$ | CH |
| H | $SO_2N(CH_3)_2$ | H | H | H | H | Cl | $OCH_3$ | CH |
| H | $SO_2N(CH_3)_2$ | H | H | H | H | $OCH_3$ | $OCH_3$ | N |
| H | $SO_2N(CH_3)_2$ | H | H | H | H | $OCH_3$ | $CH_3$ | N |
| H | H | H | $CH_3$ | H | H | $OCH_3$ | $OCH_3$ | CH |
| H | H | H | $CH_3$ | H | H | $OCH_3$ | $CH_3$ | CH |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | H | H | $CH_3$ | H | H | Cl | $OCH_3$ | CH |
| H | H | H | $CH_3$ | H | H | $CH_3$ | $CH_3$ | CH |
| H | H | H | $CH_3$ | H | H | $OCH_3$ | $OCH_3$ | N |
| H | H | H | $CH_3$ | H | H | $OCH_3$ | $CH_3$ | N |
| H | H | H | $CH_3$ | H | H | $OC_2H_5$ | $NHCH_3$ | N |
| H | H | H | $CH_3$ | H | $CH_3$ | $OCH_3$ | $CH_3$ | N |
| H | Cl | H | $CH_3$ | H | H | $OCH_3$ | $OCH_3$ | CH |
| H | Cl | H | $CH_3$ | H | H | $OCH_3$ | $CH_3$ | CH |
| H | Cl | H | $CH_3$ | H | H | Cl | $OCH_3$ | CH |
| H | Cl | H | $CH_3$ | H | H | $CH_3$ | $CH_3$ | CH |
| H | Cl | H | $CH_3$ | H | H | $OCH_3$ | $OCH_3$ | N |
| H | Cl | H | $CH_3$ | H | H | $OCH_3$ | $CH_3$ | N |
| H | Cl | H | $CH_3$ | H | H | $OC_2H_5$ | $NHCH_3$ | N |
| H | Cl | H | $CH_3$ | H | H | $OCH_3$ | $CH_3$ | N |
| H | $SO_2CH_3$ | H | $CH_3$ | H | H | $OCH_3$ | $OCH_3$ | CH |
| H | $SO_2CH_3$ | H | $CH_3$ | H | H | $OCH_3$ | $CH_3$ | CH |
| H | $SO_2CH_3$ | H | $CH_3$ | H | H | Cl | $OCH_3$ | CH |
| H | $SO_2CH_3$ | H | $CH_3$ | H | H | $CH_3$ | $CH_3$ | CH |
| H | $SO_2CH_3$ | H | $CH_3$ | H | H | $OCH_3$ | $OCH_3$ | N |
| H | $SO_2CH_3$ | H | $CH_3$ | H | H | $OCH_3$ | $CH_3$ | N |
| H | $SO_2CH_3$ | H | $CH_3$ | H | H | $OC_2H_5$ | $NHCH_3$ | N |
| H | $SO_2CH_3$ | H | $CH_3$ | H | $CH_3$ | $OCH_3$ | $CH_3$ | N |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $SO_2N(CH_3)_2$ | H | $CH_3$ | H | H | $OCH_3$ | $OCH_3$ | CH |
| H | $SO_2N(CH_3)_2$ | H | $CH_3$ | H | H | $OCH_3$ | $CH_3$ | CH |
| H | $SO_2N(CH_3)_2$ | H | $CH_3$ | H | H | Cl | $OCH_3$ | CH |
| H | $SO_2N(CH_3)_2$ | H | $CH_3$ | H | H | $OCH_3$ | $OCH_3$ | N |
| H | $SO_2N(CH_3)_2$ | H | $CH_3$ | H | H | $OCH_3$ | $CH_3$ | N |
| H | H | $CH_3$ | H | H | H | $OCH_3$ | $OCH_3$ | CH |
| H | H | $CH_3$ | H | H | H | $OCH_3$ | $CH_3$ | CH |
| H | H | $CH_3$ | H | H | H | Cl | $OCH_3$ | CH |
| H | H | $CH_3$ | H | H | H | $CH_3$ | $CH_3$ | CH |
| H | H | $CH_3$ | H | H | H | $OCH_3$ | $OCH_3$ | N |
| H | H | $CH_3$ | H | H | H | $OCH_3$ | $CH_3$ | N |
| H | H | $CH_3$ | H | H | H | $OC_2H_5$ | $NHCH_3$ | N |
| H | H | $CH_3$ | H | H | $CH_3$ | $OCH_3$ | $CH_3$ | N |
| H | Cl | $CH_3$ | H | H | H | $OCH_3$ | $OCH_3$ | CH |
| H | Cl | $CH_3$ | H | H | H | $OCH_3$ | $CH_3$ | CH |
| H | Cl | $CH_3$ | H | H | H | Cl | $OCH_3$ | CH |
| H | Cl | $CH_3$ | H | H | H | $CH_3$ | $CH_3$ | CH |
| H | Cl | $CH_3$ | H | H | H | $OCH_3$ | $OCH_3$ | N |
| H | Cl | $CH_3$ | H | H | H | $OCH_3$ | $CH_3$ | N |
| H | Cl | $CH_3$ | H | H | H | $OC_2H_5$ | $NHCH_3$ | N |
| H | Cl | $CH_3$ | H | H | H | $OCH_3$ | $CH_3$ | N |
| H | $SO_2CH_3$ | $CH_3$ | H | H | H | $OCH_3$ | $OCH_3$ | CH |
| H | $SO_2CH_3$ | $CH_3$ | H | H | H | $OCH_3$ | $CH_3$ | CH |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $SO_2CH_3$ | $CH_3$ | H | H | H | Cl | $OCH_3$ | CH |
| H | $SO_2CH_3$ | $CH_3$ | H | H | H | $CH_3$ | $CH_3$ | CH |
| H | $SO_2CH_3$ | $CH_3$ | H | H | H | $OCH_3$ | $OCH_3$ | N |
| H | $SO_2CH_3$ | $CH_3$ | H | H | H | $OCH_3$ | $CH_3$ | N |
| H | $SO_2CH_3$ | $CH_3$ | H | H | H | $OC_2H_5$ | $NHCH_3$ | N |
| H | $SO_2CH_3$ | $CH_3$ | H | H | $CH_3$ | $OCH_3$ | $CH_3$ | N |
| H | $SO_2N(CH_3)_2$ | $CH_3$ | H | H | H | $OCH_3$ | $OCH_3$ | CH |
| H | $SO_2N(CH_3)_2$ | $CH_3$ | H | H | H | $OCH_3$ | $CH_3$ | CH |
| N | $SO_2N(CH_3)_2$ | $CH_3$ | H | H | H | Cl | $OCH_3$ | CH |
| N | $SO_2N(CH_3)_2$ | $CH_3$ | H | H | H | $OCH_3$ | $OCH_3$ | N |
| N | $SO_2N(CH_3)_2$ | $CH_3$ | H | H | H | $OCH_3$ | $CH_3$ | N |
| H | $CO_2CH_3$ | $CH_3$ | $CH_3$ | H | H | $OCH_3$ | $OCH_3$ | CH |
| H | $CO_2CH_3$ | $CH_3$ | $CH_3$ | H | H | $OCH_3$ | $CH_3$ | CH |
| H | $CO_2CH_3$ | $CH_3$ | $CH_3$ | H | H | Cl | $OCH_3$ | CH |
| H | $CO_2CH_3$ | $CH_3$ | $CH_3$ | H | H | $CH_3$ | $CH_3$ | CH |
| H | $CO_2CH_3$ | $CH_3$ | $CH_3$ | H | H | $OCH_3$ | $OCH_3$ | N |
| H | $CO_2CH_3$ | $CH_3$ | $CH_3$ | H | H | $OCH_3$ | $CH_3$ | N |
| H | $CO_2CH_3$ | $CH_3$ | $CH_3$ | H | H | $OC_2H_5$ | $NHCH_3$ | N |
| H | $CO_2CH_3$ | $CH_3$ | $CH_3$ | H | $CH_3$ | $OCH_3$ | $CH_3$ | N |
| H | $CO_2CH_3$ | $CH_3$ | H | H | H | $OCH_3$ | $OCH_3$ | CH |
| H | $CO_2CH_3$ | $CH_3$ | H | H | H | $OCH_3$ | $CH_3$ | CH |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $CO_2CH_3$ | $CH_3$ | H | H | H | Cl | $OCH_3$ | CH |
| H | $CO_2CH_3$ | $CH_3$ | H | H | H | $CH_3$ | $CH_3$ | CH |
| H | $CO_2CH_3$ | $CH_3$ | H | H | H | $OCH_3$ | $OCH_3$ | N |
| H | $CO_2CH_3$ | $CH_3$ | H | H | H | $OCH_3$ | $CH_3$ | N |
| H | $CO_2CH_3$ | $CH_3$ | H | H | H | $OC_2H_5$ | $NHCH_3$ | N |
| H | $CO_2CH_3$ | $CH_3$ | H | H | $CH_3$ | $OCH_3$ | $CH_3$ | N |
| H | $CO_2CH_3$ | H | $CH_3$ | H | H | $OCH_3$ | $OCH_3$ | CH |
| H | $CO_2CH_3$ | H | $CH_3$ | H | H | $OCH_3$ | $CH_3$ | CH |
| H | $CO_2CH_3$ | H | $CH_3$ | H | H | Cl | $OCH_3$ | CH |
| H | $CO_2CH_3$ | H | $CH_3$ | H | H | $CH_3$ | $CH_3$ | CH |
| H | $CO_2CH_3$ | H | $CH_3$ | H | H | $OCH_3$ | $OCH_3$ | N |
| H | $CO_2CH_3$ | H | $CH_3$ | H | H | $OCH_3$ | $CH_3$ | N |
| H | $CO_2CH_3$ | H | $CH_3$ | H | H | $OC_2H_5$ | $NHCH_3$ | N |
| H | $CO_2CH_3$ | H | $CH_3$ | H | $CH_3$ | $OCH_3$ | $CH_3$ | N |
| H | $CO_2CH_3$ | H | H | H | H | $OCH_3$ | $OCH_3$ | CH |
| H | $CO_2CH_3$ | H | H | H | H | $OCH_3$ | $CH_3$ | CH |
| H | $CO_2CH_3$ | H | H | H | H | Cl | $OCH_3$ | CH |
| H | $CO_2CH_3$ | H | H | H | H | $CH_3$ | $CH_3$ | CH |
| H | $CO_2CH_3$ | H | H | H | H | $OCH_3$ | $OCH_3$ | N |
| H | $CO_2CH_3$ | H | H | H | H | $OCH_3$ | $CH_3$ | N |
| H | $CO_2CH_3$ | H | H | H | H | $OC_2H_5$ | $NHCH_3$ | N |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $CO_2CH_3$ | H | H | H | $CH_3$ | $OCH_3$ | $CH_3$ | N |
| H | $CO_2C_2H_5$ | H | H | H | H | $OCH_3$ | $OCH_3$ | CH |
| H | $CO_2C_2H_5$ | H | H | H | H | $OCH_3$ | $CH_3$ | CH |
| H | $CO_2C_2H_5$ | H | H | H | H | Cl | $OCH_3$ | CH |
| H | $CO_2C_2H_5$ | H | H | H | H | $CH_3$ | $CH_3$ | CH |
| H | $CO_2C_2H_5$ | H | H | H | H | $OCH_3$ | $OCH_3$ | N |
| H | $CO_2C_2H_5$ | H | H | H | H | $OCH_3$ | $CH_3$ | N |
| H | $CO_2C_2H_5$ | H | H | H | H | $OC_2H_5$ | $NHCH_3$ | N |
| H | $CO_2C_2H_5$ | H | H | H | $CH_3$ | $OCH_3$ | $CH_3$ | N |
| H | $CO_2C_2H_5$ | $CH_3$ | $CH_3$ | H | H | $OCH_3$ | $OCH_3$ | CH |
| H | $CO_2C_2H_5$ | $CH_3$ | $CH_3$ | H | H | $OCH_3$ | $CH_3$ | CH |
| H | $CO_2C_2H_5$ | $CH_3$ | $CH_3$ | H | H | Cl | $OCH_3$ | CH |
| H | $CO_2C_2H_5$ | $CH_3$ | $CH_3$ | H | H | $CH_3$ | $CH_3$ | CH |
| H | $CO_2C_2H_5$ | $CH_3$ | $CH_3$ | H | H | $OCH_3$ | $OCH_3$ | N |
| H | $CO_2C_2H_5$ | $CH_3$ | $CH_3$ | H | H | $OCH_3$ | $CH_3$ | N |
| H | $CO_2C_2H_5$ | $CH_3$ | $CH_3$ | H | H | $OC_2H_5$ | $NHCH_3$ | N |
| H | $CO_2C_2H_5$ | $CH_3$ | $CH_3$ | H | $CH_3$ | $OCH_3$ | $CH_3$ | N |
| H | $CO_2CH_3$ | $CH_3$ | $CH_3$ | H | H | $OCH_3$ | $OCH_3$ | CH |
| H | $CO_2CH_3$ | $CH_3$ | $CH_3$ | H | H | $OCH_3$ | $CH_3$ | CH |
| H | $CO_2CH_3$ | $CH_3$ | $CH_3$ | H | H | Cl | $OCH_3$ | CH |
| H | $CO_2CH_3$ | $CH_3$ | $CH_3$ | H | H | $CH_3$ | $CH_3$ | CH |
| H | $CO_2CH_3$ | $CH_3$ | $CH_3$ | H | H | $OCH_3$ | $OCH_3$ | N |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $CO_2CH_3$ | $CH_3$ | $CH_3$ | H | H | $OCH_3$ | $CH_3$ | N |
| H | $CO_2CH_3$ | $CH_3$ | $CH_3$ | H | H | $OC_2H_5$ | $NHCH_3$ | N |
| H | $CO_2CH_3$ | $CH_3$ | $CH_3$ | H | $CH_3$ | $OCH_3$ | $CH_3$ | N |
| H | $CO_2CH_3$ | $CH_3$ | H | H | H | $OCH_3$ | $OCH_3$ | CH |
| H | $CO_2CH_3$ | $CH_3$ | H | H | H | $OCH_3$ | $CH_3$ | CH |
| H | $CO_2CH_3$ | $CH_3$ | H | H | H | Cl | $OCH_3$ | CH |
| H | $CO_2CH_3$ | $CH_3$ | H | H | H | $CH_3$ | $CH_3$ | CH |
| H | $CO_2CH_3$ | $CH_3$ | H | H | H | $OCH_3$ | $OCH_3$ | N |
| H | $CO_2CH_3$ | $CH_3$ | H | H | H | $OCH_3$ | $CH_3$ | N |
| H | $CO_2CH_3$ | $CH_3$ | H | H | H | $OC_2H_5$ | $NHCH_3$ | N |
| H | $CO_2CH_3$ | $CH_3$ | H | H | $CH_3$ | $OCH_3$ | $CH_3$ | N |
| H | $CO_2CH_3$ | H | $CH_3$ | H | H | $OCH_3$ | $OCH_3$ | CH |
| H | $CO_2CH_3$ | H | $CH_3$ | H | H | $OCH_3$ | $CH_3$ | CH |
| H | $CO_2CH_3$ | H | $CH_3$ | H | H | Cl | $OCH_3$ | CH |
| H | $CO_2CH_3$ | H | $CH_3$ | H | H | $CH_3$ | $CH_3$ | CH |
| H | $CO_2CH_3$ | H | $CH_3$ | H | H | $OCH_3$ | $OCH_3$ | N |
| H | $CO_2CH_3$ | H | $CH_3$ | H | H | $OCH_3$ | $CH_3$ | N |
| H | $CO_2CH_3$ | H | $CH_3$ | H | H | $OC_2H_5$ | $NHCH_3$ | N |
| H | $CO_2CH_3$ | H | $CH_3$ | H | $CH_3$ | $OCH_3$ | $CH_3$ | N |
| H | $CO_2CH_3$ | H | H | H | H | $OCH_3$ | $OCH_3$ | CH |
| H | $CO_2CH_3$ | H | H | H | H | $OCH_3$ | $CH_3$ | CH |
| H | $CO_2CH_3$ | H | H | H | H | Cl | $OCH_3$ | CH |

Table 8 (continued)

General Formula 8

| $R^4$ | $R^1$ | $R^2$ | $R^3$ | $R^5$ | R | X | Y | $Z^4$ |
|---|---|---|---|---|---|---|---|---|
| H | $CO_2CH_3$ | H | H | H | H | $CH_3$ | $CH_3$ | CH |
| H | $CO_2CH_3$ | H | H | H | H | $OCH_3$ | $OCH_3$ | N |
| H | $CO_2CH_3$ | H | H | H | H | $OCH_3$ | $CH_3$ | N |
| H | $CO_2CH_3$ | H | H | H | H | $OC_2H_5$ | $NHCH_3$ | N |
| H | $CO_2CH_3$ | H | H | H | $CH_3$ | $OCH_3$ | $CH_3$ | N |
| H | $CO_2C_2H_5$ | H | H | H | H | $OCH_3$ | $OCH_3$ | CH |
| H | $CO_2C_2H_5$ | H | H | H | H | $OCH_3$ | $CH_3$ | CH |
| H | $CO_2C_2H_5$ | H | H | H | H | Cl | $OCH_3$ | CH |
| H | $CO_2C_2H_5$ | H | H | H | H | $CH_3$ | $CH_3$ | CH |
| H | $CO_2C_2H_5$ | H | H | H | H | $OCH_3$ | $OCH_3$ | N |
| H | $CO_2C_2H_5$ | H | H | H | H | $OCH_3$ | $CH_3$ | N |
| H | $CO_2C_2H_5$ | H | H | H | H | $OC_2H_5$ | $NHCH_3$ | N |
| H | $CO_2C_2H_5$ | H | H | H | $CH_3$ | $OCH_3$ | $CH_3$ | N |
| H | $CO_2C_2H_5$ | $CH_3$ | $CH_3$ | H | H | $OCH_3$ | $CH_3$ | CH |
| H | $CO_2C_2H_5$ | $CH_3$ | $CH_3$ | H | H | Cl | $OCH_3$ | CH |
| H | $CO_2C_2H_5$ | $CH_3$ | $CH_3$ | H | H | $CH_3$ | $CH_3$ | CH |
| H | $CO_2C_2H_5$ | $CH_3$ | $CH_3$ | H | H | $OCH_3$ | $OCH_3$ | N |
| H | $CO_2C_2H_5$ | $CH_3$ | $CH_3$ | H | H | $OCH_3$ | $CH_3$ | N |
| H | $CO_2C_2H_5$ | $CH_3$ | $CH_3$ | H | H | $OC_2H_5$ | $NHCH_3$ | N |
| H | $CO_2C_2H_5$ | $CH_3$ | $CH_3$ | H | $CH_3$ | $OCH_3$ | $CH_3$ | N |
| H | $CO_2C_2H_5$ | $CH_3$ | $CH_3$ | H | H | $OCH_3$ | $OCH_3$ | CH |

Table 9

General Formula 9

| $R^4$ | $R^5$ | $R^2$ | $R^3$ | R | X | Y | $Z^4$ |
|---|---|---|---|---|---|---|---|
| H | H | $CH_3$ | $CH_3$ | H | $OCH_3$ | $OCH_3$ | CH |
| H | H | $CH_3$ | $CH_3$ | H | $OCH_3$ | $CH_3$ | CH |
| H | H | $CH_3$ | $CH_3$ | H | Cl | $OCH_3$ | CH |
| H | H | $CH_3$ | $CH_3$ | H 3 | $CH_3$ | CH | CH |
| H | H | $CH_3$ | $CH_3$ | H | $OCH_3$ | $OCH_3$ | N |
| H | H | $CH_3$ | $CH_3$ | H | $OCH_3$ | $CH_3$ | N |
| H | H | $CH_3$ | $CH_3$ | H | $OC_2H_5$ | $NHCH_3$ | N |
| H | H | $CH_3$ | $CH_3$ | $CH_3$ | $OCH_3$ | $CH_3$ | N |
| H | Cl | $CH_3$ | $CH_3$ | H | $OCH_3$ | $OCH_3$ | CH |
| H | Cl | $CH_3$ | $CH_3$ | H | $OCH_3$ | $CH_3$ | CH |
| H | Cl | $CH_3$ | $CH_3$ | H | Cl | $OCH_3$ | CH |
| H | Cl | $CH_3$ | $CH_3$ | H | $CH_3$ | $CH_3$ | CH |
| H | Cl | $CH_3$ | $CH_3$ | H | $OCH_3$ | $OCH_3$ | N |
| H | Cl | $CH_3$ | $CH_3$ | H | $OCH_3$ | $CH_3$ | N |
| H | Cl | $CH_3$ | $CH_3$ | H | $OC_2H_5$ | $NHCH_3$ | N |
| H | Cl | $CH_3$ | $CH_3$ | $CH_3$ | $OCH_3$ | $CH_3$ | N |
| H | $SO_2CH_3$ | $CH_3$ | $CH_3$ | H | $OCH_3$ | $OCH_3$ | CH |
| H | $SO_2CH_3$ | $CH_3$ | $CH_3$ | H | $OCH_3$ | $CH_3$ | CH |
| H | $SO_2CH_3$ | $CH_3$ | $CH_3$ | H | Cl | $OCH_3$ | CH |
| H | $SO_2CH_3$ | $CH_3$ | $CH_3$ | H | $CH_3$ | $CH_3$ | CH |
| H | $SO_2CH_3$ | $CH_3$ | $CH_3$ | H | $OCH_3$ | $OCH_3$ | N |
| H | $SO_2CH_3$ | $CH_3$ | $CH_3$ | H | $OCH_3$ | $CH_3$ | N |
| H | $SO_2CH_3$ | $CH_3$ | $CH_3$ | H | $OC_2H_5$ | $NHCH_3$ | N |

Table 9 (continued)

General Formula 9

| $R^4$ | $R^5$ | $R^2$ | $R^3$ | R | X | Y | $Z^4$ |
|---|---|---|---|---|---|---|---|
| H | $SO_2CH_3$ | $CH_3$ | $CH_3$ | $CH_3$ | $OCH_3$ | $CH_3$ | N |
| H | $SO_2N(CH_3)_2$ | $CH_3$ | $CH_3$ | H | $OCH_3$ | $OCH_3$ | CH |
| H | $SO_2N(CH_3)_2$ | $CH_3$ | $CH_3$ | H | $OCH_3$ | $CH_3$ | CH |
| H | $SO_2N(CH_3)_2$ | $CH_3$ | $CH_3$ | H | Cl | $OCH_3$ | CH |
| H | $SO_2N(CH_3)_2$ | $CH_3$ | $CH_3$ | H | $OCH_3$ | $OCH_3$ | N |
| H | $SO_2N(CH_3)_2$ | $CH_3$ | $CH_3$ | H | $OCH_3$ | $CH_3$ | N |
| H | H | H | H | H | $OCH_3$ | $OCH_3$ | CH |
| H | H | H | H | H | $OCH_3$ | $CH_3$ | CH |
| H | H | H | H | H | Cl | $OCH_3$ | CH |
| H | H | H | H | H | $CH_3$ | $CH_3$ | CH |
| H | H | H | H | H | $OCH_3$ | $OCH_3$ | N |
| H | H | H | H | H | $OCH_3$ | $CH_3$ | N |
| H | H | H | H | H | $OC_2H_5$ | $NHCH_3$ | N |
| H | H | H | H | $CH_3$ | $OCH_3$ | $CH_3$ | N |
| H | Cl | H | H | H | $OCH_3$ | $OCH_3$ | CH |
| H | Cl | H | H | H | $OCH_3$ | $CH_3$ | CH |
| H | Cl | H | H | H | Cl | $OCH_3$ | CH |
| H | Cl | H | H | H | $CH_3$ | $CH_3$ | CH |
| H | Cl | H | H | H | $OCH_3$ | $OCH_3$ | N |
| H | Cl | H | H | H | $OCH_3$ | $CH_3$ | N |
| H | Cl | H | H | H | $OC_2H_5$ | $NHCH_3$ | N |
| H | Cl | H | H | $CH_3$ | $OCH_3$ | $CH_3$ | N |
| H | $SO_2CH_3$ | H | H | H | $OCH_3$ | $OCH_3$ | CH |
| H | $SO_2CH_3$ | H | H | H | $OCH_3$ | $CH_3$ | CH |
| H | $SO_2CH_3$ | H | H | H | Cl | $OCH_3$ | CH |

Table 9 (continued)

General Formula 9

| $R^4$ | $R^5$ | $R^2$ | $R^3$ | R | X | Y | $Z^4$ |
|---|---|---|---|---|---|---|---|
| H | $SO_2CH_3$ | H | H | H | $CH_3$ | $CH_3$ | CH |
| H | $SO_2CH_3$ | H | H | H | $OCH_3$ | $OCH_3$ | N |
| H | $SO_2CH_3$ | H | H | H | $OCH_3$ | $CH_3$ | N |
| H | $SO_2CH_3$ | H | H | H | $OC_2H_5$ | $NHCH_3$ | N |
| H | $SO_2CH_3$ | H | H | $CH_3$ | $OCH_3$ | $CH_3$ | N |
| H | $SO_2N(CH_3)_2$ | H | H | H | $OCH_3$ | $OCH_3$ | CH |
| H | $SO_2N(CH_3)_2$ | H | H | H | $OCH_3$ | $CH_3$ | CH |
| H | $SO_2N(CH_3)_2$ | H | H | H | Cl | $OCH_3$ | CH |
| H | $SO_2N(CH_3)_2$ | H | H | H | $OCH_3$ | $OCH_3$ | N |
| H | $SO_2N(CH_3)_2$ | H | H | H | $OCH_3$ | $CH_3$ | N |
| H | H | H | $CH_3$ | H | $OCH_3$ | $OCH_3$ | CH |
| H | H | H | $CH_3$ | H | $OCH_3$ | $CH_3$ | CH |
| H | H | H | $CH_3$ | H | Cl | $OCH_3$ | CH |
| H | H | H | $CH_3$ | H | $CH_3$ | $CH_3$ | CH |
| H | H | H | $CH_3$ | H | $OCH_3$ | $OCH_3$ | N |
| H | H | H | $CH_3$ | H | $OCH_3$ | $CH_3$ | N |
| H | H | H | $CH_3$ | H | $OC_2H_5$ | $NHCH_3$ | N |
| H | H | H | $CH_3$ | $CH_3$ | $OCH_3$ | $CH_3$ | N |
| H | Cl | H | $CH_3$ | H | $OCH_3$ | $OCH_3$ | CH |
| H | Cl | H | $CH_3$ | H | $OCH_3$ | $CH_3$ | CH |
| H | Cl | H | $CH_3$ | H | Cl | $OCH_3$ | CH |
| H | Cl | H | $CH_3$ | H | $CH_3$ | $CH_3$ | CH |
| H | Cl | H | $CH_3$ | H | $OCH_3$ | $OCH_3$ | N |
| H | Cl | H | $CH_3$ | H | $OCH_3$ | $CH_3$ | N |
| H | Cl | H | $CH_3$ | H | $OC_2H_5$ | $NHCH_3$ | N |

Table 9 (continued)

General Formula 9

| $R^4$ | $R^5$ | $R^2$ | $R^3$ | R | X | Y | $Z^4$ |
|---|---|---|---|---|---|---|---|
| H | Cl | H | $CH_3$ | $CH_3$ | $OCH_3$ | $CH_3$ | N |
| H | $SO_2CH_3$ | H | $CH_3$ | H | $OCH_3$ | $OCH_3$ | CH |
| H | $SO_2CH_3$ | H | $CH_3$ | H | $OCH_3$ | $CH_3$ | CH |
| H | $SO_2CH_3$ | H | $CH_3$ | H | Cl | $OCH_3$ | CH |
| H | $SO_2CH_3$ | H | $CH_3$ | H | $CH_3$ | $CH_3$ | CH |
| H | $SO_2CH_3$ | H | $CH_3$ | H | $OCH_3$ | $OCH_3$ | N |
| H | $SO_2CH_3$ | H | $CH_3$ | H | $OCH_3$ | $CH_3$ | N |
| H | $SO_2CH_3$ | H | $CH_3$ | H | $OC_2H_5$ | $NHCH_3$ | N |
| H | $SO_2CH_3$ | H | $CH_3$ | $CH_3$ | $OCH_3$ | $CH_3$ | N |
| H | $SO_2N(CH_3)_2$ | H | $CH_3$ | H | $OCH_3$ | $OCH_3$ | CH |
| H | $SO_2N(CH_3)_2$ | H | $CH_3$ | H | $OCH_3$ | $CH_3$ | CH |
| H | $SO_2N(CH_3)_2$ | H | $CH_3$ | H | Cl | $OCH_3$ | CH |
| H | $SO_2N(CH_3)_2$ | H | $CH_3$ | H | $OCH_3$ | $OCH_3$ | N |
| H | $SO_2N(CH_3)_2$ | H | $CH_3$ | H | $OCH_3$ | $CH_3$ | N |
| H | H | $CH_3$ | H | H | $OCH_3$ | $OCH_3$ | CH |
| H | H | $CH_3$ | H | H | $OCH_3$ | $CH_3$ | CH |
| H | H | $CH_3$ | H | H | Cl | $OCH_3$ | CH |
| H | H | $CH_3$ | H | H | $CH_3$ | $CH_3$ | CH |
| H | H | $CH_3$ | H | H | $OCH_3$ | $OCH_3$ | N |
| H | H | $CH_3$ | H | H | $OCH_3$ | $CH_3$ | N |
| H | H | $CH_3$ | H | H | $OC_2H_5$ | $NHCH_3$ | N |
| H | H | $CH_3$ | H | $CH_3$ | $OCH_3$ | $CH_3$ | N |
| H | Cl | $CH_3$ | H | H | $OCH_3$ | $OCH_3$ | CH |
| H | Cl | $CH_3$ | H | H | $OCH_3$ | $CH_3$ | CH |
| H | Cl | $CH_3$ | H | HC | Cl | $OCH_3$ | CH |

Table 9 (continued)

General Formula 9

| $R^4$ | $R^5$ | $R^2$ | $R^3$ | R | X | Y | $Z^4$ |
|---|---|---|---|---|---|---|---|
| H | Cl | $CH_3$ | H | H | $CH_3$ | $CH_3$ | CH |
| H | Cl | $CH_3$ | H | H | $OCH_3$ | $OCH_3$ | N |
| H | Cl | $CH_3$ | H | H | $OCH_3$ | $CH_3$ | N |
| H | Cl | $CH_3$ | H | H | $OC_2H_5$ | $NHCH_3$ | N |
| H | Cl | $CH_3$ | H | $CH_3$ | $OCH_3$ | $CH_3$ | N |
| H | $SO_2CH_3$ | $CH_3$ | H | H | $OCH_3$ | $OCH_3$ | CH |
| H | $SO_2CH_3$ | $CH_3$ | H | H | $OCH_3$ | $CH_3$ | CH |
| H | $SO_2CH_3$ | $CH_3$ | H | H | Cl | $OCH_3$ | CH |
| H | $SO_2CH_3$ | $CH_3$ | H | H | $CH_3$ | $CH_3$ | CH |
| H | $SO_2CH_3$ | $CH_3$ | H | H | $OCH_3$ | $OCH_3$ | N |
| H | $SO_2CH_3$ | $CH_3$ | H | H | $OCH_3$ | $CH_3$ | N |
| H | $SO_2CH_3$ | $CH_3$ | H | H | $OC_2H_5$ | $NHCH_3$ | N |
| H | $SO_2CH_3$ | $CH_3$ | H | $CH_3$ | $OCH_3$ | $CH_3$ | N |
| H | $SO_2N(CH_3)_2$ | $CH_3$ | H | H | $OCH_3$ | $OCH_3$ | CH |
| H | $SO_2N(CH_3)_2$ | $CH_3$ | H | H | $OCH_3$ | $CH_3$ | CH |
| H | $SO_2N(CH_3)_2$ | $CH_3$ | H | H | Cl | $OCH_3$ | CH |
| H | $SO_2N(CH_3)_2$ | $CH_3$ | H | H | $OCH_3$ | $OCH_3$ | N |
| H | $SO_2N(CH_3)_2$ | $CH_3$ | H | H | $OCH_3$ | $CH_3$ | N |
| H | $CO_2CH_3$ | $CH_3$ | $CH_3$ | H | $OCH_3$ | $OCH_3$ | CH |
| H | $CO_2CH_3$ | $CH_3$ | $CH_3$ | H | $OCH_3$ | $CH_3$ | CH |
| H | $CO_2CH_3$ | $CH_3$ | $CH_3$ | H | Cl | $OCH_3$ | CH |
| H | $CO_2CH_3$ | $CH_3$ | $CH_3$ | H | $CH_3$ | $CH_3$ | CH |
| H | $CO_2CH_3$ | $CH_3$ | $CH_3$ | H | $OCH_3$ | $OCH_3$ | N |
| H | $CO_2CH_3$ | $CH_3$ | $CH_3$ | H | $OCH_3$ | $CH_3$ | N |
| H | $CO_2CH_3$ | $CH_3$ | $CH_3$ | H | $OC_2H_5$ | $NHCH_3$ | N |

Table 9 (continued)

General Formula 9

| $R^4$ | $R^5$ | $R^2$ | $R^3$ | R | X | Y | $Z^4$ |
|---|---|---|---|---|---|---|---|
| H | $CO_2CH_3$ | $CH_3$ | $CH_3$ | $CH_3$ | $OCH_3$ | $CH_3$ | N |
| H | $CO_2CH_3$ | $CH_3$ | H | H | $OCH_3$ | $OCH_3$ | CH |
| H | $CO_2CH_3$ | $CH_3$ | H | H | $OCH_3$ | $CH_3$ | CH |
| H | $CO_2CH_3$ | $CH_3$ | H | H | Cl | $OCH_3$ | CH |
| H | $CO_2CH_3$ | $CH_3$ | H | H | $CH_3$ | $CH_3$ | CH |
| H | $CO_2CH_3$ | $CH_3$ | H | H | $OCH_3$ | $OCH_3$ | N |
| H | $CO_2CH_3$ | $CH_3$ | H | H | $OCH_3$ | $CH_3$ | N |
| H | $CO_2CH_3$ | $CH_3$ | H | H | $OC_2H_5$ | $NHCH_3$ | N |
| H | $CO_2CH_3$ | $CH_3$ | H | $CH_3$ | $OCH_3$ | $CH_3$ | N |
| H | $CO_2CH_3$ | H | $CH_3$ | H | $OCH_3$ | $OCH_3$ | CH |
| H | $CO_2CH_3$ | H | $CH_3$ | H | $OCH_3$ | $CH_3$ | CH |
| H | $CO_2CH_3$ | H | $CH_3$ | H | Cl | $OCH_3$ | CH |
| H | $CO_2CH_3$ | H | $CH_3$ | H | $CH_3$ | $CH_3$ | CH |
| H | $CO_2CH_3$ | H | $CH_3$ | H | $OCH_3$ | $OCH_3$ | N |
| H | $CO_2CH_3$ | H | $CH_3$ | H | $OCH_3$ | $CH_3$ | N |
| H | $CO_2CH_3$ | H | $CH_3$ | H | $OC_2H_5$ | $NHCH_3$ | N |
| H | $CO_2CH_3$ | H | $CH_3$ | $CH_3$ | $OCH_3$ | $CH_3$ | N |
| H | $CO_2CH_3$ | H | H | H | $OCH_3$ | $OCH_3$ | CH |
| H | $CO_2CH_3$ | H | H | H | $OCH_3$ | $CH_3$ | CH |
| H | $CO_2CH_3$ | H | H | H | Cl | $OCH_3$ | CH |
| H | $CO_2CH_3$ | H | H | H | $CH_3$ | $CH_3$ | CH |
| H | $CO_2CH_3$ | H | H | H | $OCH_3$ | $OCH_3$ | N |
| H | $CO_2CH_3$ | H | H | H | $OCH_3$ | $CH_3$ | N |
| H | $CO_2CH_3$ | H | H | H | $OC_2H_5$ | $NHCH_3$ | N |
| H | $CO_2CH_3$ | H | H | $CH_3$ | $OCH_3$ | $CH_3$ | N |

Table 9 (continued)

General Formula 9

| $R^4$ | $R^5$ | $R^2$ | $R^3$ | R | X | Y | $Z^4$ |
|---|---|---|---|---|---|---|---|
| H | $CO_2C_2H_5$ | H | H | H | $OCH_3$ | $OCH_3$ | CH |
| H | $CO_2C_2H_5$ | H | H | H | $OCH_3$ | $CH_3$ | CH |
| H | $CO_2C_2H_5$ | H | H | H | Cl | $OCH_3$ | CH |
| H | $CO_2C_2H_5$ | H | H | H | $CH_3$ | $CH_3$ | CH |
| H | $CO_2C_2H_5$ | H | H | H | $OCH_3$ | $OCH_3$ | N |
| H | $CO_2C_2H_5$ | H | H | H | $OCH_3$ | $CH_3$ | N |
| H | $CO_2C_2H_5$ | H | H | H | $OC_2H_5$ | $NHCH_3$ | N |
| H | $CO_2C_2H_5$ | H | H | $CH_3$ | $OCH_3$ | $CH_3$ | N |
| H | $CO_2C_2H_5$ | $CH_3$ | $CH_3$ | H | $OCH_3$ | $OCH_3$ | CH |
| H | $CO_2C_2H_5$ | $CH_3$ | $CH_3$ | H | $OCH_3$ | $CH_3$ | CH |
| H | $CO_2C_2H_5$ | $CH_3$ | $CH_3$ | H | Cl | $OCH_3$ | CH |
| H | $CO_2C_2H_5$ | $CH_3$ | $CH_3$ | H | $CH_3$ | $CH_3$ | CH |
| H | $CO_2C_2H_5$ | $CH_3$ | $CH_3$ | H | $OCH_3$ | $OCH_3$ | N |
| H | $CO_2C_2H_5$ | $CH_3$ | $CH_3$ | H | $OCH_3$ | $CH_3$ | N |
| H | $CO_2C_2H_5$ | $CH_3$ | $CH_3$ | H | $OC_2H_5$ | $NHCH_3$ | N |
| H | $CO_2C_2H_5$ | $CH_3$ | $CH_3$ | $CH_3$ | $OCH_3$ | $CH_3$ | N |

Table 10

General Formula 10

| Z | $Z^1$ | $R^1$ | $R^2$ | $R^3$ | $R^5$ | R | X | Y | $Z^4$ |
|---|---|---|---|---|---|---|---|---|---|
| N | CH | H | $CH_3$ | $CH_3$ | H | H | $OCH_3$ | $OCH_3$ | CH |
| N | CH | H | $CH_3$ | $CH_3$ | H | H | $OCH_3$ | $CH_3$ | CH |
| N | CH | H | $CH_3$ | $CH_3$ | H | H | Cl | $OCH_3$ | CH |
| N | CH | H | $CH_3$ | $CH_3$ | H | H | $CH_3$ | $CH_3$ | CH |
| N | CH | H | $CH_3$ | $CH_3$ | H | H | $OCH_3$ | $OCH_3$ | N |
| N | CH | H | $CH_3$ | $CH_3$ | H | H | $OCH_3$ | $CH_3$ | N |
| N | CH | H | $CH_3$ | $CH_3$ | H | H | $OC_2H_5$ | $NHCH_3$ | N |
| N | CH | H | $CH_3$ | $CH_3$ | H | $CH_3$ | $OCH_3$ | $CH_3$ | N |
| N | CH | Cl | $CH_3$ | $CH_3$ | H | H | $OCH_3$ | $OCH_3$ | CH |
| N | CH | Cl | $CH_3$ | $CH_3$ | H | H | $OCH_3$ | $CH_3$ | CH |
| N | CH | Cl | $CH_3$ | $CH_3$ | H | H | Cl | $OCH_3$ | CH |
| N | CH | Cl | $CH_3$ | $CH_3$ | H | H | $CH_3$ | $CH_3$ | CH |
| N | CH | Cl | $CH_3$ | $CH_3$ | H | H | $OCH_3$ | $OCH_3$ | N |
| N | CH | Cl | $CH_3$ | $CH_3$ | H | H | $OCH_3$ | $CH_3$ | N |
| N | CH | Cl | $CH_3$ | $CH_3$ | H | H | $OC_2H_5$ | $NHCH_3$ | N |
| N | CH | Cl | $CH_3$ | $CH_3$ | H | $CH_3$ | $OCH_3$ | $CH_3$ | N |
| N | CH | $SO_2CH_3$ | $CH_3$ | $CH_3$ | H | H | $OCH_3$ | $OCH_3$ | CH |
| N | CH | $SO_2CH_3$ | $CH_3$ | $CH_3$ | H | H | $OCH_3$ | $CH_3$ | CH |
| N | CH | $SO_2CH_3$ | $CH_3$ | $CH_3$ | H | H | Cl | $OCH_3$ | CH |
| N | CH | $SO_2CH_3$ | $CH_3$ | $CH_3$ | H | H | $CH_3$ | $CH_3$ | CH |
| N | CH | $SO_2CH_3$ | $CH_3$ | $CH_3$ | H | H | $OCH_3$ | $OCH_3$ | N |
| N | CH | $SO_2CH_3$ | $CH_3$ | $CH_3$ | H | H | $OCH_3$ | $CH_3$ | N |
| N | CH | $SO_2CH_3$ | $CH_3$ | $CH_3$ | H | H | $OC_2H_5$ | $NHCH_3$ | N |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| N | CH | $SO_2CH_3$ | $CH_3$ | $CH_3$ | H | $CH_3$ | $OCH_3$ | $CH_3$ | N |
| N | CH | $SO_2N(CH_3)_2$ | $CH_3$ | $CH_3$ | H | H | $OCH_3$ | $OCH_3$ | CH |
| N | CH | $SO_2N(CH_3)_2$ | $CH_3$ | $CH_3$ | H | H | $OCH_3$ | $CH_3$ | CH |
| N | CH | $SO_2N(CH_3)_2$ | $CH_3$ | $CH_3$ | H | H | Cl | $OCH_3$ | CH |
| N | CH | $SO_2N(CH_3)_2$ | $CH_3$ | $CH_3$ | H | H | $OCH_3$ | $OCH_3$ | N |
| N | CH | $SO_2N(CH_3)_2$ | $CH_3$ | $CH_3$ | H | H | $OCH_3$ | $CH_3$ | N |
| CH | N | H | H | H | H | H | $OCH_3$ | $OCH_3$ | CH |
| CH | N | H | H | H | H | H | $OCH_3$ | $CH_3$ | CH |
| CH | N | H | H | H | H | H | Cl | $OCH_3$ | CH |
| CH | N | H | H | H | H | H | CH | $CH_3$ | $CH_3$ |
| CH | N | H | H | H | H | H | $OCH_3$ | $OCH_3$ | N |
| CH | N | H | H | H | H | H | $OCH_3$ | $CH_3$ | N |
| CH | N | H | H | H | H | H | $OC_2H_5$ | $NHCH_3$ | N |
| CH | N | H | H | H | H | $CH_3$ | $OCH_3$ | $CH_3$ | N |
| CH | N | Cl | H | H | H | H | $OCH_3$ | $OCH_3$ | CH |
| CH | N | Cl | H | H | H | H | $OCH_3$ | $CH_3$ | CH |
| CH | N | Cl | H | H | H | H | Cl | $OCH_3$ | CH |
| CH | N | Cl | H | H | H | H | $CH_3$ | $CH_3$ | CH |
| CH | N | Cl | H | H | H | H | $OCH_3$ | $OCH_3$ | N |
| CH | N | Cl | H | H | H | H | $OCH_3$ | $CH_3$ | N |
| CH | N | Cl | H | H | H | H | $OC_2H_5$ | $NHCH_3$ | N |
| CH | N | Cl | H | H | H | $CH_3$ | $OCH_3$ | $CH_3$ | N |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| CH | N | $SO_2CH_3$ | H | H | H | H | $OCH_3$ | $OCH_3$ | CH |
| CH | N | $SO_2CH_3$ | H | H | H | H | $OCH_3$ | $CH_3$ | CH |
| CH | N | $SO_2CH_3$ | H | H | H | H | Cl | $OCH_3$ | CH |
| CH | N | $SO_2CH_3$ | H | H | H | H | $CH_3$ | $CH_3$ | CH |
| CH | N | $SO_2CH_3$ | H | H | H | H | $OCH_3$ | $OCH_3$ | N |
| CH | N | $SO_2CH_3$ | H | H | H | H | $OCH_3$ | $CH_3$ | N |
| CH | N | $SO_2CH_3$ | H | H | H | H | $OC_2H_5$ | $NHCH_3$ | N |
| CH | N | $SO_2CH_3$ | H | H | H | $CH_3$ | $OCH_3$ | $CH_3$ | N |
| CH | N | $SO_2N(CH_3)_2$ | H | H | H | H | $OCH_3$ | $OCH_3$ | CH |
| CH | N | $SO_2N(CH_3)_2$ | H | H | H | H | $OCH_3$ | $CH_3$ | CH |
| CH | N | $SO_2N(CH_3)_2$ | H | H | H | H | Cl | $OCH_3$ | CH |
| CH | N | $SO_2N(CH_3)_2$ | H | H | H | H | $OCH_3$ | $OCH_3$ | N |
| CH | N | $SO_2N(CH_3)_2$ | H | H | H | H | $OCH_3$ | $CH_3$ | N |
| CH | CH | H | $CH_3$ | $CH_3$ | H | H | $OCH_3$ | $OCH_3$ | CH |
| CH | CH | H | $CH_3$ | $CH_3$ | H | H | $OCH_3$ | $CH_3$ | CH |
| CH | CH | H | $CH_3$ | $CH_3$ | H | H | Cl | $OCH_3$ | CH |
| CH | CH | H | $CH_3$ | $CH_3$ | H | H | $CH_3$ | $CH_3$ | CH |
| CH | CH | H | $CH_3$ | $CH_3$ | H | H | $OCH_3$ | $OCH_3$ | N |
| CH | CH | H | $CH_3$ | $CH_3$ | H | H | $OCH_3$ | $CH_3$ | N |
| CH | CH | H | $CH_3$ | $CH_3$ | H | H | $OC_2H_5$ | $NHCH_3$ | N |
| CH | CH | H | $CH_3$ | $CH_3$ | H | $CH_3$ | $OCH_3$ | $CH_3$ | N |
| CH | CH | Cl | $CH_3$ | $CH_3$ | H | H | $OCH_3$ | $OCH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| CH | CH | Cl | $CH_3$ | $CH_3$ | H | H | $OCH_3$ | $CH_3$ | CH |
| CH | CH | Cl | $CH_3$ | $CH_3$ | H | H | Cl | $OCH_3$ | CH |
| CH | CH | Cl | $CH_3$ | $CH_3$ | H | H | $CH_3$ | $CH_3$ | CH |
| CH | CH | Cl | $CH_3$ | $CH_3$ | H | H | $OCH_3$ | $OCH_3$ | N |
| CH | CH | Cl | $CH_3$ | $CH_3$ | H | H | $OCH_3$ | $CH_3$ | N |
| CH | CH | Cl | $CH_3$ | $CH_3$ | H | H | $OC_2H_5$ | $NHCH_3$ | N |
| CH | CH | Cl | $CH_3$ | $CH_3$ | H | $CH_3$ | $OCH_3$ | $CH_3$ | N |
| CH | CH | $SO_2CH_3$ | $CH_3$ | $CH_3$ | H | H | $OCH_3$ | $OCH_3$ | CH |
| CH | CH | $SO_2CH_3$ | $CH_3$ | $CH_3$ | H | H | $OCH_3$ | $CH_3$ | CH |
| CH | CH | $SO_2CH_3$ | $CH_3$ | $CH_3$ | H | H | Cl | $OCH_3$ | CH |
| CH | CH | $SO_2CH_3$ | $CH_3$ | $CH_3$ | H | H | $CH_3$ | $CH_3$ | CH |
| CH | CH | $SO_2CH_3$ | $CH_3$ | $CH_3$ | H | H | $OCH_3$ | $OCH_3$ | N |
| CH | CH | $SO_2CH_3$ | $CH_3$ | $CH_3$ | H | H | $OCH_3$ | $CH_3$ | N |
| CH | CH | $SO_2CH_3$ | $CH_3$ | $CH_3$ | H | H | $OC_2H_5$ | $NHCH_3$ | N |
| CH | CH | $SO_2CH_3$ | $CH_3$ | $CH_3$ | H | $CH_3$ | $OCH_3$ | $CH_3$ | N |
| CH | CH | $SO_2N(CH_3)_2$ | $CH_3$ | $CH_3$ | H | H | $OCH_3$ | $OCH_3$ | CH |
| CH | CH | $SO_2N(CH_3)_2$ | $CH_3$ | $CH_3$ | H | H | $OCH_3$ | $CH_3$ | CH |
| CH | CH | $SO_2N(CH_3)_2$ | $CH_3$ | $CH_3$ | H | H | Cl | $OCH_3$ | CH |
| CH | CH | $SO_2N(CH_3)_2$ | $CH_3$ | $CH_3$ | H | H | $OCH_3$ | $OCH_3$ | N |
| CH | CH | $SO_2N(CH_3)_2$ | $CH_3$ | $CH_3$ | H | H | $OCH_3$ | $CH_3$ | N |
| CH | CH | H | H | H | H | H | $OCH_3$ | $OCH_3$ | CH |
| CH | CH | H | H | H | H | H | $OCH_3$ | $CH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| CH | CH | H | H | H | H | H | Cl | $OCH_3$ | CH |
| CH | CH | H | H | H | H | H | $CH_3$ | $CH_3$ | CH |
| CH | CH | H | H | H | H | H | $OCH_3$ | $OCH_3$ | N |
| CH | CH | H | H | H | H | H | $OCH_3$ | $CH_3$ | N |
| CH | CH | H | H | H | H | H | $OC_2H_5$ | $NHCH_3$ | N |
| CH | CH | H | H | H | H | $CH_3$ | $OCH_3$ | $CH_3$ | N |
| CH | CH | Cl | H | H | H | H | $OCH_3$ | $OCH_3$ | CH |
| CH | CH | Cl | H | H | H | H | $OCH_3$ | $CH_3$ | CH |
| CH | CH | Cl | H | H | H | H | Cl | $OCH_3$ | CH |
| CH | CH | Cl | H | H | H | H | $CH_3$ | $CH_3$ | CH |
| CH | CH | Cl | H | H | H | H | $OCH_3$ | $OCH_3$ | N |
| CH | CH | Cl | H | H | H | H | $OCH_3$ | $CH_3$ | N |
| CH | CH | Cl | H | H | H | H | $OC_2H_5$ | $NHCH_3$ | N |
| CH | CH | Cl | H | H | H | $CH_3$ | $OCH_3$ | $CH_3$ | N |
| CH | CH | $SO_2CH_3$ | H | H | H | H | $OCH_3$ | $OCH_3$ | CH |
| CH | CH | $SO_2CH_3$ | H | H | H | H | $OCH_3$ | $CH_3$ | CH |
| CH | CH | $SO_2CH_3$ | H | H | H | H | Cl | $OCH_3$ | CH |
| CH | CH | $SO_2CH_3$ | H | H | H | H | $CH_3$ | $CH_3$ | CH |
| CH | CH | $SO_2CH_3$ | H | H | H | H | $OCH_3$ | $OCH_3$ | N |
| CH | CH | $SO_2CH_3$ | H | H | H | H | $OCH_3$ | $CH_3$ | N |
| CH | CH | $SO_2CH_3$ | H | H | H | H | $OC_2H_5$ | $NHCH_3$ | N |
| CH | CH | $SO_2CH_3$ | H | H | H | $CH_3$ | $OCH_3$ | $CH_3$ | N |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| CH | CH | $SO_2N(CH_3)_2$ | H | H | H | H | $OCH_3$ | $OCH_3$ | CH |
| CH | CH | $SO_2N(CH_3)_2$ | H | H | H | H | $OCH_3$ | $CH_3$ | CH |
| CH | CH | $SO_2N(CH_3)_2$ | H | H | H | H | Cl | $OCH_3$ | CH |
| CH | CH | $SO_2N(CH_3)_2$ | H | H | H | H | $OCH_3$ | $OCH_3$ | N |
| CH | CH | $SO_2N(CH_3)_2$ | H | H | H | H | $OCH_3$ | $CH_3$ | N |
| N | N | H | $CH_3$ | $CH_3$ | H | H | $OCH_3$ | $OCH_3$ | CH |
| N | N | H | $CH_3$ | $CH_3$ | H | H | $OCH_3$ | $CH_3$ | CH |
| N | N | H | $CH_3$ | $CH_3$ | H | H | Cl | $OCH_3$ | CH |
| N | N | H | $CH_3$ | $CH_3$ | H | H | $CH_3$ | $CH_3$ | CH |
| N | N | H | $CH_3$ | $CH_3$ | H | H | $OCH_3$ | $OCH_3$ | N |
| N | N | H | $CH_3$ | $CH_3$ | H | H | $OCH_3$ | $CH_3$ | N |
| N | N | H | $CH_3$ | $CH_3$ | H | H | $OC_2H_5$ | $NHCH_3$ | N |
| N | N | H | $CH_3$ | $CH_3$ | H | $CH_3$ | $OCH_3$ | $CH_3$ | N |
| N | N | Cl | $CH_3$ | $CH_3$ | H | H | $OCH_3$ | $OCH_3$ | CH |
| N | N | Cl | $CH_3$ | $CH_3$ | H | H | $OCH_3$ | $CH_3$ | CH |
| N | N | Cl | $CH_3$ | $CH_3$ | H | H | Cl | $OCH_3$ | CH |
| N | N | Cl | $CH_3$ | $CH_3$ | H | H | $CH_3$ | $CH_3$ | CH |
| N | N | Cl | $CH_3$ | $CH_3$ | H | H | $OCH_3$ | $OCH_3$ | N |
| N | N | Cl | $CH_3$ | $CH_3$ | H | H | $OCH_3$ | $CH_3$ | N |
| N | N | Cl | $CH_3$ | $CH_3$ | H | H | $OC_2H_5$ | $NHCH_3$ | N |
| N | N | Cl | $CH_3$ | $CH_3$ | H | $CH_3$ | $OCH_3$ | $CH_3$ | N |
| N | N | $SO_2CH_3$ | $CH_3$ | $CH_3$ | H | H | $OCH_3$ | $OCH_3$ | CH |
| N | N | $SO_2CH_3$ | $CH_3$ | $CH_3$ | H | H | $OCH_3$ | $CH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| N | N | $SO_2CH_3$ | $CH_3$ | $CH_3$ | H | H | Cl | $OCH_3$ | CH |
| N | N | $SO_2CH_3$ | $CH_3$ | $CH_3$ | H | H | $CH_3$ | $CH_3$ | CH |
| N | N | $SO_2CH_3$ | $CH_3$ | $CH_3$ | H | H | $OCH_3$ | $OCH_3$ | N |
| N | N | $SO_2CH_3$ | $CH_3$ | $CH_3$ | H | H | $OCH_3$ | $CH_3$ | N |
| N | N | $SO_2CH_3$ | $CH_3$ | $CH_3$ | H | H | $OC_2H_5$ | $NHCH_3$ | N |
| N | N | $SO_2CH_3$ | $CH_3$ | $CH_3$ | H | $CH_3$ | $OCH_3$ | $CH_3$ | N |
| N | N | $SO_2N(CH_3)_2$ | $CH_3$ | $CH_3$ | H | H | $OCH_3$ | $OCH_3$ | CH |
| N | N | $SO_2N(CH_3)_2$ | $CH_3$ | $CH_3$ | H | H | $OCH_3$ | $CH_3$ | CH |
| N | N | $SO_2N(CH_3)_2$ | $CH_3$ | $CH_3$ | H | H | Cl | $OCH_3$ | CH |
| N | N | $SO_2N(CH_3)_2$ | $CH_3$ | $CH_3$ | H | H | $OCH_3$ | $OCH_3$ | N |
| N | N | $SO_2N(CH_3)_2$ | $CH_3$ | $CH_3$ | H | H | $OCH_3$ | $CH_3$ | N |
| N | N | H | H | H | H | H | $OCH_3$ | $OCH_3$ | CH |
| N | N | H | H | H | H | H | $OCH_3$ | $CH_3$ | CH |
| N | N | H | H | H | H | H | Cl | $OCH_3$ | CH |
| N | N | H | H | H | H | H | $CH_3$ | $CH_3$ | CH |
| N | N | H | H | H | H | H | $OCH_3$ | $OCH_3$ | N |
| N | N | H | H | H | H | H | $OCH_3$ | $CH_3$ | N |
| N | N | H | H | H | H | H | $OC_2H_5$ | $NHCH_3$ | N |
| N | N | H | H | H | H | $CH_3$ | $OCH_3$ | $CH_3$ | N |
| N | N | Cl | H | H | H | H | $OCH_3$ | $OCH_3$ | CH |
| N | N | Cl | H | H | H | H | $OCH_3$ | $CH_3$ | CH |
| N | N | Cl | H | H | H | H | Cl | $OCH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| N | N | Cl | H | H | H | H | $CH_3$ | $CH_3$ | CH |
| N | N | Cl | H | H | H | H | $OCH_3$ | $OCH_3$ | N |
| N | N | Cl | H | H | H | H | $OCH_3$ | $CH_3$ | N |
| N | N | Cl | H | H | H | H | $OC_2H_5$ | $NHCH_3$ | N |
| N | N | Cl | H | H | H | $CH_3$ | $OCH_3$ | $CH_3$ | N |
| N | N | $SO_2CH_3$ | H | H | H | H | $OCH_3$ | $OCH_3$ | CH |
| N | N | $SO_2CH_3$ | H | H | H | H | $OCH_3$ | $CH_3$ | CH |
| N | N | $SO_2CH_3$ | H | H | H | H | Cl | $OCH_3$ | CH |
| N | N | $SO_2CH_3$ | H | H | H | H | $CH_3$ | $CH_3$ | CH |
| N | N | $SO_2CH_3$ | H | H | H | H | $OCH_3$ | $OCH_3$ | N |
| N | N | $SO_2CH_3$ | H | H | H | H | $OCH_3$ | $CH_3$ | N |
| N | N | $SO_2CH_3$ | H | H | H | H | $OC_2H_5$ | $NHCH_3$ | N |
| N | N | $SO_2CH_3$ | H | H | H | $CH_3$ | $OCH_3$ | $CH_3$ | N |
| N | N | $SO_2N(CH_3)_2$ | H | H | H | H | $OCH_3$ | $OCH_3$ | CH |
| N | N | $SO_2N(CH_3)_2$ | H | H | H | H | $OCH_3$ | $CH_3$ | CH |
| N | N | $SO_2N(CH_3)_2$ | H | H | H | H | Cl | $OCH_3$ | CH |
| N | N | $SO_2N(CH_3)_2$ | H | H | H | H | $OCH_3$ | $OCH_3$ | N |
| N | N | $SO_2N(CH_3)_2$ | H | H | H | H | $OCH_3$ | $CH_3$ | N |
| N | CH | H | H | H | H | H | $OCH_3$ | $OCH_3$ | CH |
| N | CH | H | H | H | H | H | $OCH_3$ | $CH_3$ | CH |
| N | CH | H | H | H | H | H | Cl | $OCH_3$ | CH |
| N | CH | H | H | H | H | H | $CH_3$ | $CH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| N | CH | H | H | H | H | H | $OCH_3$ | $OCH_3$ | N |
| N | CH | H | H | H | H | H | $OCH_3$ | $CH_3$ | N |
| N | CH | H | H | H | H | H | $OC_2H_5$ | $NHCH_3$ | N |
| N | CH | H | H | H | H | $CH_3$ | $OCH_3$ | $CH_3$ | N |
| N | CH | Cl | H | H | H | H | $OCH_3$ | $OCH_3$ | CH |
| N | CH | Cl | H | H | H | H | $OCH_3$ | $CH_3$ | CH |
| N | CH | Cl | H | H | H | H | Cl | $OCH_3$ | CH |
| N | CH | Cl | H | H | H | H | $CH_3$ | $CH_3$ | CH |
| N | CH | Cl | H | H | H | H | $OCH_3$ | $OCH_3$ | N |
| N | CH | Cl | H | H | H | H | $OCH_3$ | $CH_3$ | N |
| N | CH | Cl | H | H | H | H | $OC_2H_5$ | $NHCH_3$ | N |
| N | CH | Cl | H | H | H | $CH_3$ | $OCH_3$ | $CH_3$ | N |
| N | CH | $SO_2CH_3$ | H | H | H | H | $OCH_3$ | $OCH_3$ | CH |
| N | CH | $SO_2CH_3$ | H | H | H | H | $OCH_3$ | $CH_3$ | CH |
| N | CH | $SO_2CH_3$ | H | H | H | H | Cl | $OCH_3$ | CH |
| N | CH | $SO_2CH_3$ | H | H | H | H | $CH_3$ | $CH_3$ | CH |
| N | CH | $SO_2CH_3$ | H | H | H | H | $OCH_3$ | $OCH_3$ | N |
| N | CH | $SO_2CH_3$ | H | H | H | H | $OCH_3$ | $CH_3$ | N |
| N | CH | $SO_2CH_3$ | H | H | H | H | $OC_2H_5$ | $NHCH_3$ | N |
| N | CH | $SO_2CH_3$ | H | H | H | $CH_3$ | $OCH_3$ | $CH_3$ | N |
| N | CH | $SO_2N(CH_3)_2$ | H | H | H | H | $OCH_3$ | $OCH_3$ | CH |
| N | CH | $SO_2N(CH_3)_2$ | H | H | H | H | $OCH_3$ | $CH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| N | CH | $SO_2N(CH_3)_2$ | H | H | H | H | Cl | $OCH_3$ | CH |
| N | CH | $SO_2N(CH_3)_2$ | H | H | H | H | $OCH_3$ | $OCH_3$ | N |
| N | CH | $SO_2N(CH_3)_2$ | H | H | H | H | $OCH_3$ | $CH_3$ | N |
| CH | N | $CO_2CH_3$ | $CH_3$ | $CH_3$ | H | H | $OCH_3$ | $OCH_3$ | CH |
| CH | N | $CO_2CH_3$ | $CH_3$ | $CH_3$ | H | H | $OCH_3$ | $CH_3$ | CH |
| CH | N | $CO_2CH_3$ | $CH_3$ | $CH_3$ | H | H | Cl | $OCH_3$ | CH |
| CH | N | $CO_2CH_3$ | $CH_3$ | $CH_3$ | H | H | $CH_3$ | $CH_3$ | CH |
| CH | N | $CO_2CH_3$ | $CH_3$ | $CH_3$ | H | H | $OCH_3$ | $OCH_3$ | N |
| CH | N | $CO_2CH_3$ | $CH_3$ | $CH_3$ | H | H | $OCH_3$ | $OCH_3$ | N |
| CH | N | $CO_2CH_3$ | $CH_3$ | $CH_3$ | H | H | $OC_2H_5$ | $NHCH_3$ | N |
| CH | N | $CO_2CH_3$ | $CH_3$ | $CH_3$ | H | $CH_3$ | $OCH_3$ | $CH_3$ | N |
| CH | N | $CO_2CH_3$ | H | H | H | H | $OCH_3$ | $OCH_3$ | CH |
| CH | N | $CO_2CH_3$ | H | H | H | H | $OCH_3$ | $CH_3$ | CH |
| CH | N | $CO_2CH_3$ | H | H | H | H | Cl | $OCH_3$ | CH |
| CH | N | $CO_2CH_3$ | H | H | H | H | $CH_3$ | $CH_3$ | CH |
| CH | N | $CO_2CH_3$ | H | H | H | H | $OCH_3$ | $OCH_3$ | N |
| CH | N | $CO_2CH_3$ | H | H | H | H | $OCH_3$ | $CH_3$ | N |
| CH | N | $CO_2CH_3$ | H | H | H | H | $OC_2H_5$ | $NHCH_3$ | N |
| CH | N | $CO_2CH_3$ | H | H | H | $CH_3$ | $OCH_3$ | $CH_3$ | N |
| CH | CH | $CO_2CH_3$ | $CH_3$ | $CH_3$ | H | H | $OCH_3$ | $OCH_3$ | CH |
| CH | CH | $CO_2CH_3$ | $CH_3$ | $CH_3$ | H | H | $OCH_3$ | $CH_3$ | CH |
| CH | CH | $CO_2CH_3$ | $CH_3$ | $CH_3$ | H | H | Cl | $OCH_3$ | CH |
| CH | CH | $CO_2CH_3$ | $CH_3$ | $CH_3$ | H | H | $CH_3$ | $CH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| CH | CH | $CO_2CH_3$ | $CH_3$ | $CH_3$ | H | H | $OCH_3$ | $OCH_3$ | N |
| CH | CH | $CO_2CH_3$ | $CH_3$ | $CH_3$ | H | H | $OCH_3$ | $CH_3$ | N |
| CH | CH | $CO_2CH_3$ | $CH_3$ | $CH_3$ | H | H | $OC_2H_5$ | $NHCH_3$ | N |
| CH | CH | $CO_2CH_3$ | $CH_3$ | $CH_3$ | H | $CH_3$ | $OCH_3$ | $CH_3$ | N |
| CH | CH | $CO_2CH_3$ | H | H | H | H | $OCH_3$ | $OCH_3$ | CH |
| CH | CH | $CO_2CH_3$ | H | H | H | H | $OCH_3$ | $CH_3$ | CH |
| CH | CH | $CO_2CH_3$ | H | H | H | H | Cl | $OCH_3$ | CH |
| CH | CH | $CO_2CH_3$ | H | H | H | $CH_3$ | $CH_3$ | $CH_3$ | CH |
| CH | CH | $CO_2CH_3$ | H | H | H | H | $OCH_3$ | $OCH_3$ | N |
| CH | CH | $CO_2CH_3$ | H | H | H | H | $OCH_3$ | $CH_3$ | N |
| CH | CH | $CO_2CH_3$ | H | H | H | H | $OC_2H_5$ | $NHCH_3$ | N |
| CH | CH | $CO_2CH_3$ | H | H | H | $CH_3$ | $OCH_3$ | $CH_3$ | N |
| CH | N | $CO_2C_2H_5$ | H | H | H | H | $OCH_3$ | $OCH_3$ | CH |
| CH | N | $CO_2C_2H_5$ | H | H | H | H | $OCH_3$ | $CH_3$ | CH |
| CH | N | $CO_2C_2H_5$ | H | H | H | H | Cl | $OCH_3$ | CH |
| CH | N | $CO_2C_2H_5$ | H | H | H | H | $CH_3$ | $CH_3$ | CH |
| CH | N | $CO_2C_2H_5$ | H | H | H | H | $OCH_3$ | $OCH_3$ | N |
| CH | N | $CO_2C_2H_5$ | H | H | H | H | $OCH_3$ | $CH_3$ | N |
| CH | N | $CO_2C_2H_5$ | H | H | H | H | $OC_2H_5$ | $NHCH_3$ | N |
| CH | N | $CO_2C_2H_5$ | H | H | H | $CH_3$ | $OCH_3$ | $CH_3$ | N |
| CH | N | $CO_2C_2H_5$ | $CH_3$ | $CH_3$ | H | H | $OCH_3$ | $OCH_3$ | CH |
| CH | N | $CO_2C_2H_5$ | $CH_3$ | $CH_3$ | H | H | $OCH_3$ | $CH_3$ | CH |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| CH | N | $CO_2C_2H_5$ | $CH_3$ | $CH_3$ | H | H | Cl | $OCH_3$ | CH |
| CH | N | $CO_2C_2H_5$ | $H_3$ | $CH_3$ | H | H | $CH_3$ | $CH_3$ | CH |
| CH | N | $CO_2C_2H_5$ | $H_3$ | $CH_3$ | H | H | $OCH_3$ | $OCH_3$ | N |
| CH | N | $CO_2C_2H_5$ | $H_3$ | $CH_3$ | H | H | $OCH_3$ | $CH_3$ | N |
| CH | N | $CO_2C_2H_5$ | $H_3$ | $CH_3$ | H | H | $OC_2H_5$ | $NHCH_3$ | N |
| CH | N | $CO_2C_2H_5$ | $H_3$ | $CH_3$ | H | $CH_3$ | $OCH_3$ | CH | N |
| N | N | $CO_2CH_3$ | $H_3$ | $CH_3$ | H | H | $OCH_3$ | $OCH_3$ | CH |
| N | N | $CO_2CH_3$ | $H_3$ | $CH_3$ | H | H | $OCH_3$ | $CH_3$ | CH |
| [illegible] | [illegible] | $CO_2CH_3$ | $H_3$ | $CH_3$ | H | H | Cl | $OCH_3$ | CH |
| N | N | $CO_2CH_3$ | $H_3$ | $CH_3$ | H | H | $CH_3$ | $CH_3$ | CH |
| N | N | $CO_2CH_3$ | 3 | $CH_3$ | H | H | $OCH_3$ | $OCH_3$ | N |
| N | N | $CO_2CH_3$ | 3 | $CH_3$ | H | H | $OCH_3$ | $CH_3$ | N |
| N | N | $CO_2CH_3$ | 3 | $CH_3$ | H | H | $OC_2H_5$ | $NHCH_3$ | N |
| N | N | $CO_2CH_3$ | 3 | $CH_3$ | H | $CH_3$ | $OCH_3$ | $CH_3$ | N |
| [illegible] | N | $CO_2CH_3$ | | H | H | H | $OCH_3$ | $OCH_3$ | CH |
| [illegible] | N | $CO_2CH_3$ | | H | H | H | $OCH_3$ | $CH_3$ | CH |
| N | N | $CO_2CH_3$ | [illegible] | H | H | H | Cl | $OCH_3$ | CH |
| N | N | $CO_2CH_3$ | [illegible] | H | H | H | $CH_3$ | $CH_3$ | CH |
| N | N | $CO_2CH_3$ | [illegible] | H | H | H | $OCH_3$ | $OCH_3$ | N |
| N | N | $CO_2CH_3$ | [illegible] | H | H | H | $OCH_3$ | $CH_3$ | N |
| N | N | $CO_2CH_3$ | H | H | H | H | $OC_2H_5$ | $NHCH_3$ | N |
| N | N | $CO_2CH_3$ | H | H | H | $CH_3$ | $OCH_3$ | $CH_3$ | N |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| N | CH | $CO_2CH_3$ | $CH_3$ | $CH_3$ | H | H | $OCH_3$ | $OCH_3$ | CH |
| N | CH | $CO_2CH_3$ | $CH_3$ | $CH_3$ | H | H | $OCH_3$ | $CH_3$ | CH |
| N | CH | $CO_2CH_3$ | $CH_3$ | $CH_3$ | H | H | Cl | $OCH_3$ | CH |
| N | CH | $CO_2CH_3$ | $CH_3$ | $CH_3$ | H | H | $CH_3$ | $CH_3$ | CH |
| N | CH | $CO_2CH_3$ | $CH_3$ | $CH_3$ | H | H | $OCH_3$ | $OCH_3$ | N |
| N | CH | $CO_2CH_3$ | $CH_3$ | $CH_3$ | H | H | $OCH_3$ | $CH_3$ | N |
| N | CH | $CO_2CH_3$ | $CH_3$ | $CH_3$ | H | H | $OC_2H_5$ | $NHCH_3$ | N |
| N | CH | $CO_2CH_3$ | $CH_3$ | $CH_3$ | H | $CH_3$ | $OCH_3$ | $CH_3$ | N |
| N | CH | $CO_2CH_3$ | H | H | H | H | $OCH_3$ | $OCH_3$ | CH |
| N | CH | $CO_2CH_3$ | H | H | H | H | $OCH_3$ | $CH_3$ | CH |
| N | CH | $CO_2CH_3$ | H | H | H | H | Cl | $OCH_3$ | CH |
| N | CH | $CO_2CH_3$ | H | H | H | H | $CH_3$ | $CH_3$ | CH |
| N | CH | $CO_2CH_3$ | H | H | H | H | $OCH_3$ | $OCH_3$ | N |
| N | CH | $CO_2CH_3$ | H | H | H | H | $OCH_3$ | $CH_3$ | N |
| N | CH | $CO_2CH_3$ | H | H | H | H | $OC_2H_5$ | $NHCH_3$ | N |
| N | CH | $CO_2CH_3$ | H | H | H | $CH_3$ | $OCH_3$ | $CH_3$ | N |
| N | N | $CO_2C_2H_5$ | H | H | H | H | $OCH_3$ | $OCH_3$ | CH |
| N | N | $CO_2C_2H_5$ | H | H | H | H | $OCH_3$ | $CH_3$ | CH |
| N | N | $CO_2C_2H_5$ | H | H | H | H | Cl | $OCH_3$ | CH |
| N | N | $CO_2C_2H_5$ | H | H | H | H | $CH_3$ | $CH_3$ | CH |
| N | N | $CO_2C_2H_5$ | H | H | H | H | $OCH_3$ | $OCH_3$ | N |
| N | N | $CO_2C_2H_5$ | H | H | H | H | $OCH_3$ | $CH_3$ | N |

Table 10 (continued)

General Formula 10

| Z | $Z^1$ | $R^1$ | $R^2$ | $R^3$ | $R^5$ | R | X | Y | $Z^4$ |
|---|---|---|---|---|---|---|---|---|---|
| N | N | $CO_2C_2H_5$ | H | H | H | H | $OC_2H_5$ | $NHCH_3$ | N |
| N | N | $CO_2C_2H_5$ | H | H | H | $CH_3$ | $OCH_3$ | $CH_3$ | N |
| N | N | $CO_2C_2H_5$ | $CH_3$ | $CH_3$ | H | H | $OCH_3$ | $OCH_3$ | CH |
| N | N | $CO_2C_2H_5$ | $CH_3$ | $CH_3$ | H | H | $OCH_3$ | $CH_3$ | CH |
| N | N | $CO_2C_2H_5$ | $CH_3$ | $CH_3$ | H | H | Cl | $OCH_3$ | CH |
| N | N | $CO_2C_2H_5$ | $CH_3$ | $CH_3$ | H | H | $CH_3$ | $CH_3$ | CH |
| N | N | $CO_2C_2H_5$ | $CH_3$ | $CH_3$ | H | H | $OCH_3$ | $OCH_3$ | N |
| N | N | $CO_2C_2H_5$ | $CH_3$ | $CH_3$ | H | H | $OCH_3$ | $CH_3$ | N |
| N | N | $CO_2C_2H_5$ | $CH_3$ | $CH_3$ | H | H | $OC_2H_5$ | $NHCH_3$ | N |
| N | N | $CO_2C_2H_5$ | $CH_3$ | $CH_3$ | H | $CH_3$ | $OCH_3$ | $CH_3$ | N |
| CH | N | H | $CH_3$ | $CH_3$ | H | H | $OCH_3$ | $OCH_3$ | CH |
| CH | N | H | $CH_3$ | $CH_3$ | H | H | $OCH_3$ | $CH_3$ | CH |
| CH | N | H | $CH_3$ | $CH_3$ | H | H | Cl | $OCH_3$ | CH |
| CH | N | H | $CH_3$ | $CH_3$ | H | H | $CH_3$ | $CH_3$ | CH |
| CH | N | H | $CH_3$ | $CH_3$ | H | H | $OCH_3$ | $OCH_3$ | N |
| CH | N | H | $CH_3$ | $CH_3$ | H | H | $OCH_3$ | $CH_3$ | N |
| CH | N | H | $CH_3$ | $CH_3$ | H | H | $OC_2H_5$ | $NHCH_3$ | N |
| CH | N | H | $CH_3$ | $CH_3$ | H | $CH_3$ | $OCH_3$ | $CH_3$ | N |
| CH | N | Cl | $CH_3$ | $CH_3$ | H | H | $OCH_3$ | $OCH_3$ | CH |
| CH | N | Cl | $CH_3$ | $CH_3$ | H | H | $OCH_3$ | $CH_3$ | CH |
| CH | N | Cl | $CH_3$ | $CH_3$ | H | H | Cl | $OCH_3$ | CH |
| CH | N | Cl | $CH_3$ | $CH_3$ | H | H | $CH_3$ | $CH_3$ | CH |

Table 10 (continued)

General Formula 10

| Z | $Z^1$ | $R^1$ | $R^2$ | $R^3$ | $R^5$ | R | X | Y | $Z^2$ |
|---|---|---|---|---|---|---|---|---|---|
| CH | N | Cl | $CH_3$ | $CH_3$ | H | H | $OCH_3$ | $OCH_3$ | N |
| CH | N | Cl | $CH_3$ | $CH_3$ | H | H | $OCH_3$ | $CH_3$ | N |
| CH | N | Cl | $CH_3$ | $CH_3$ | H | H | $OC_2H_5$ | $NHCH_3$ | N |
| CH | N | Cl | $CH_3$ | $CH_3$ | H | $CH_3$ | $OCH_3$ | $CH_3$ | N |
| CH | N | $SO_2CH_3$ | $CH_3$ | $CH_3$ | H | H | $OCH_3$ | $OCH_3$ | CH |
| CH | N | $SO_2CH_3$ | $CH_3$ | $CH_3$ | H | H | $OCH_3$ | $CH_3$ | CH |
| CH | N | $SO_2CH_3$ | $CH_3$ | $CH_3$ | H | H | Cl | $OCH_3$ | CH |
| CH | N | $SO_2CH_3$ | $CH_3$ | $CH_3$ | H | H | $CH_3$ | $CH_3$ | CH |
| CH | N | $SO_2CH_3$ | $CH_3$ | $CH_3$ | H | H | $OCH_3$ | $OCH_3$ | N |
| CH | N | $SO_2CH_3$ | $CH_3$ | $CH_3$ | H | H | $OCH_3$ | $CH_3$ | N |
| CH | N | $SO_2CH_3$ | $CH_3$ | $CH_3$ | H | H | $OC_2H_5$ | $NHCH_3$ | N |
| CH | N | $SO_2CH_3$ | $CH_3$ | $CH_3$ | H | $CH_3$ | $OCH_3$ | $CH_3$ | N |
| CH | N | $SO_2N(CH_3)_2$ | $CH_3$ | $CH_3$ | H | H | $OCH_3$ | $OCH_3$ | CH |
| CH | N | $SO_2N(CH_3)_2$ | $CH_3$ | $CH_3$ | H | H | $OCH_3$ | $CH_3$ | CH |
| CH | N | $SO_2N(CH_3)_2$ | $CH_3$ | $CH_3$ | H | H | Cl | $OCH_3$ | CH |
| CH | N | $SO_2N(CH_3)_2$ | $CH_3$ | $CH_3$ | H | H | $OCH_3$ | $OCH_3$ | N |
| CH | N | $SO_2N(CH_3)_2$ | $CH_3$ | $CH_3$ | H | H | $OCH_3$ | $CH_3$ | N |

Table 11

General Formula 11

| $Z^1$ | $R^1$ | $R^3$ | $R^5$ | R | X | Y | $Z^2$ |
|---|---|---|---|---|---|---|---|
| CH | H | H | H | H | $OCH_3$ | $OCH_3$ | CH |
| CH | H | H | H | H | $OCH_3$ | $CH_3$ | CH |
| CH | H | H | H | H | Cl | $OCH_3$ | CH |
| CH | H | H | H | H | $CH_3$ | $CH_3$ | CH |
| CH | H | H | H | H | $OCH_3$ | $OCH_3$ | N |
| CH | H | H | H | H | $OCH_3$ | $CH_3$ | N |
| CH | H | H | H | H | $OC_2H_5$ | $NHCH_3$ | N |
| CH | H | H | H | $CH_3$ | $OCH_3$ | $CH_3$ | N |
| CH | Cl | H | H | H | $OCH_3$ | $OCH_3$ | CH |
| CH | Cl | H | H | H | $OCH_3$ | $CH_3$ | CH |
| CH | Cl | H | H | H | Cl | $OCH_3$ | CH |
| CH | Cl | H | H | H | $CH_3$ | $CH_3$ | CH |
| CH | Cl | H | H | H | $OCH_3$ | $OCH_3$ | N |
| CH | Cl | H | H | H | $OCH_3$ | $CH_3$ | N |
| CH | Cl | H | H | H | $OC_2H_5$ | $NHCH_3$ | N |
| CH | Cl | H | H | $CH_3$ | $OCH_3$ | $CH_3$ | N |
| CH | $SO_2CH_3$ | H | H | H | $OCH_3$ | $OCH_3$ | CH |
| CH | $SO_2CH_3$ | H | H | H | $OCH_3$ | $CH_3$ | CH |
| CH | $SO_2CH_3$ | H | H | H | Cl | $OCH_3$ | CH |
| CH | $SO_2CH_3$ | H | H | H | $CH_3$ | $CH_3$ | CH |
| CH | $SO_2CH_3$ | H | H | H | $OCH_3$ | $OCH_3$ | N |
| CH | $SO_2CH_3$ | H | H | H | $OCH_3$ | $CH_3$ | N |
| CH | $SO_2CH_3$ | H | H | H | $OC_2H_5$ | $NHCH_3$ | N |

Table 11 (continued)

General Formula 11

| $Z^1$ | $R^1$ | $R^3$ | $R^5$ | R | X | Y | $Z^4$ |
|---|---|---|---|---|---|---|---|
| CH | $SO_2CH_3$ | H | H | $CH_3$ | $OCH_3$ | $CH_3$ | N |
| CH | $CO_2CH_3$ | H | H | H | $OCH_3$ | $OCH_3$ | CH |
| CH | $CO_2CH_3$ | H | H | H | $OCH_3$ | $CH_3$ | CH |
| CH | $CO_2CH_3$ | H | H | H | Cl | $OCH_3$ | CH |
| CH | $CO_2CH_3$ | H | H | H | $CH_3$ | $CH_3$ | CH |
| CH | $CO_2CH_3$ | H | H | H | $OCH_3$ | $OCH_3$ | N |
| CH | $CO_2CH_3$ | H | H | H | $OCH_3$ | $CH_3$ | N |
| CH | $CO_2CH_3$ | H | H | H | $OC_2H_5$ | $NHCH_3$ | N |
| CH | $CO_2CH_3$ | H | H | $CH_3$ | $OCH_3$ | $CH_3$ | N |
| CH | $CO_2C_2H_5$ | H | H | H | $OCH_3$ | $OCH_3$ | CH |
| CH | $CO_2C_2H_5$ | H | H | H | $OCH_3$ | $CH_3$ | CH |
| CH | $CO_2C_2H_5$ | H | H | H | Cl | $OCH_3$ | CH |
| CH | $CO_2C_2H_5$ | H | H | H | $CH_3$ | $CH_3$ | CH |
| CH | $CO_2C_2H_5$ | H | H | H | $OCH_3$ | $OCH_3$ | N |
| CH | $CO_2C_2H_5$ | H | H | H | $OCH_3$ | $CH_3$ | N |
| CH | $CO_2C_2H_5$ | H | H | H | $OC_2H_5$ | $NHCH_3$ | N |
| CH | $CO_2C_2H_5$ | H | H | $CH_3$ | $OCH_3$ | $CH_3$ | N |
| CH | $SO_2N(CH_3)_2$ | H | H | H | $OCH_3$ | $OCH_3$ | CH |
| CH | $SO_2N(CH_3)_2$ | H | H | H | $OCH_3$ | $CH_3$ | CH |
| CH | $SO_2N(CH_3)_2$ | H | H | H | Cl | $OCH_3$ | CH |
| CH | $SO_2N(CH_3)_2$ | H | H | H | $CH_3$ | $CH_3$ | CH |
| CH | $SO_2N(CH_3)_2$ | H | H | H | $OCH_3$ | $OCH_3$ | N |

Table 11 (continued)

General Formula 11

| $Z^1$ | $R^1$ | $R^3$ | $R^5$ | R | X | Y | $Z^4$ |
|---|---|---|---|---|---|---|---|
| CH | $SO_2N(CH_3)_2$ | H | H | H | $OCH_3$ | $CH_3$ | N |
| CH | $SO_2N(CH_3)_2$ | H | H | H | $OC_2H_5$ | $NHCH_3$ | N |
| CH | $SO_2N(CH_3)_2$ | H | H | $CH_3$ | $OCH_3$ | $CH_3$ | N |
| CH | H | H | H | H | $OCH_3$ | $OCH_3$ | CH |
| CH | H | H | H | H | $OCH_3$ | $CH_3$ | CH |
| CH | H | H | H | H | Cl | $OCH_3$ | CH |
| CH | H | H | H | H | $CH_3$ | $CH_3$ | CH |
| CH | H | H | H | H | $OCH_3$ | $OCH_3$ | N |
| CH | H | H | H | H | $OCH_3$ | $CH_3$ | N |
| CH | H | H | H | H | $OC_2H_5$ | $NHCH_3$ | N |
| CH | H | H | H | $CH_3$ | $OCH_3$ | $CH_3$ | N |
| CH | H | H | Cl | H | $OCH_3$ | $OCH_3$ | CH |
| CH | H | H | Cl | H | $OCH_3$ | $CH_3$ | CH |
| CH | H | H | Cl | H | Cl | $OCH_3$ | CH |
| CH | H | H | Cl | H | $CH_3$ | $CH_3$ | CH |
| CH | H | H | Cl | H | $OCH_3$ | $OCH_3$ | N |
| CH | H | H | Cl | H | $OCH_3$ | $CH_3$ | N |
| CH | H | H | Cl | H | $OC_2H_5$ | $NHCH_3$ | N |
| CH | H | H | Cl | $CH_3$ | $OCH_3$ | $CH_3$ | N |
| CH | H | H | $SO_2CH_3$ | H | $OCH_3$ | $OCH_3$ | CH |
| CH | H | H | $SO_2CH_3$ | H | $OCH_3$ | $CH_3$ | CH |
| CH | H | H | $SO_2CH_3$ | H | Cl | $OCH_3$ | CH |

Table 11 (continued)

General Formula 11

| $Z^1$ | $R^1$ | $R^3$ | $R^5$ | R | X | Y | $Z^4$ |
|---|---|---|---|---|---|---|---|
| CH | H | H | $SO_2CH_3$ | H | CH | $CH_3$ | $CH_3$ |
| CH | H | H | $SO_2CH_3$ | H | $OCH_3$ | $OCH_3$ | N |
| CH | H | H | $SO_2CH_3$ | H | $OCH_3$ | $CH_3$ | N |
| CH | H | H | $SO_2CH_3$ | H | $OC_2H_5$ | $NHCH_3$ | N |
| CH | H | H | $SO_2CH_3$ | $CH_3$ | $OCH_3$ | $CH_3$ | N |
| CH | H | H | $CO_2CH_3$ | H | $OCH_3$ | $OCH_3$ | CH |
| CH | H | H | $CO_2CH_3$ | H | $OCH_3$ | $CH_3$ | CH |
| CH | H | H | $CO_2CH_3$ | H | Cl | $OCH_3$ | CH |
| CH | H | H | $CO_2CH_3$ | H | $CH_3$ | $CH_3$ | CH |
| CH | H | H | $CO_2CH_3$ | H | $OCH_3$ | $OCH_3$ | N |
| CH | H | H | $CO_2CH_3$ | H | $OCH_3$ | $CH_3$ | N |
| CH | H | H | $CO_2CH_3$ | H | $OC_2H_5$ | $NHCH_3$ | N |
| CH | H | H | $CO_2CH_3$ | $CH_3$ | $OCH_3$ | $CH_3$ | N |
| CH | H | H | $CO_2C_2H_5$ | H | $OCH_3$ | $OCH_3$ | CH |
| CH | H | H | $CO_2C_2H_5$ | H | $OCH_3$ | $CH_3$ | CH |
| CH | H | H | $CO_2C_2H_5$ | H | Cl | $OCH_3$ | CH |
| CH | H | H | $CO_2C_2H_5$ | H | $CH_3$ | $CH_3$ | CH |
| CH | H | H | $CO_2C_2H_5$ | H | $OCH_3$ | $OCH_3$ | N |
| CH | H | H | $CO_2C_2H_5$ | H | $OCH_3$ | $CH_3$ | N |
| CH | H | H | $CO_2C_2H_5$ | H | $OC_2H_5$ | $NHCH_3$ | N |
| CH | H | H | $CO_2C_2H_5$ | $CH_3$ | $OCH_3$ | $CH_3$ | N |

Table 11 (continued)

General Formula 11

| $Z^1$ | $R^1$ | $R^3$ | $R^5$ | R | X | Y | $Z^4$ |
|---|---|---|---|---|---|---|---|
| CH | H | H | $SO_2N(CH_3)_2$ | H | $OCH_3$ | $OCH_3$ | CH |
| CH | H | H | $SO_2N(CH_3)_2$ | H | $OCH_3$ | $CH_3$ | CH |
| CH | H | H | $SO_2N(CH_3)_2$ | H | Cl | $OCH_3$ | CH |
| CH | H | H | $SO_2N(CH_3)_2$ | H | $CH_3$ | $CH_3$ | CH |
| CH | H | H | $SO_2N(CH_3)_2$ | H | $OCH_3$ | $OCH_3$ | N |
| CH | H | H | $SO_2N(CH_3)_2$ | H | $OCH_3$ | $CH_3$ | N |
| CH | H | H | $SO_2N(CH_3)_2$ | H | $OC_2H_5$ | $NHCH_3$ | N |
| CH | H | H | $SO_2N(CH_3)_2$ | $CH_3$ | $OCH_3$ | $CH_3$ | N |
| N | H | H | H | H | $OCH_3$ | $OCH_3$ | CH |
| N | H | H | H | H | $OCH_3$ | $CH_3$ | CH |
| N | H | H | H | H | Cl | $OCH_3$ | CH |
| N | H | H | H | H | $CH_3$ | $CH_3$ | CH |
| N | H | H | H | H | $OCH_3$ | $OCH_3$ | N |
| N | H | H | H | H | $OCH_3$ | $CH_3$ | N |
| N | H | H | H | H | $OC_2H_5$ | $NHCH_3$ | N |
| N | H | H | H | $CH_3$ | $OCH_3$ | $CH_3$ | N |
| N | Cl | H | H | H | $OCH_3$ | $OCH_3$ | CH |
| N | Cl | H | H | H | $OCH_3$ | $CH_3$ | CH |
| N | Cl | H | H | H | Cl | $OCH_3$ | CH |
| N | Cl | H | H | H | $CH_3$ | $CH_3$ | CH |
| N | Cl | H | H | H | $OCH_3$ | $OCH_3$ | N |
| N | Cl | H | H | H | $OCH_3$ | $CH_3$ | N |
| N | Cl | H | H | H | $OC_2H_5$ | $NHCH_3$ | N |

Table 11 (continued)

General Formula 11

| $Z^1$ | $R^1$ | $R^3$ | $R^5$ | R | X | Y | $Z^4$ |
|---|---|---|---|---|---|---|---|
| N | Cl | H | H | $CH_3$ | $OCH_3$ | $CH_3$ | N |
| N | $SO_2CH_3$ | H | H | H | $OCH_3$ | $OCH_3$ | CH |
| N | $SO_2CH_3$ | H | H | H | $OCH_3$ | $CH_3$ | CH |
| N | $SO_2CH_3$ | H | H | H | Cl | $OCH_3$ | CH |
| N | $SO_2CH_3$ | H | H | H | $CH_3$ | $CH_3$ | CH |
| N | $SO_2CH_3$ | H | H | H | $OCH_3$ | $OCH_3$ | N |
| N | $SO_2CH_3$ | H | H | H | $OCH_3$ | $CH_3$ | N |
| N | $SO_2CH_3$ | H | H | H | $OC_2H_5$ | $NHCH_3$ | N |
| N | $SO_2CH_3$ | H | H | $CH_3$ | $OCH_3$ | $CH_3$ | N |
| N | $CO_2CH_3$ | H | H | H | $OCH_3$ | $OCH_3$ | CH |
| N | $CO_2CH_3$ | H | H | H | $OCH_3$ | $CH_3$ | CH |
| N | $CO_2CH_3$ | H | H | H | Cl | $OCH_3$ | CH |
| N | $CO_2CH_3$ | H | H | H | $CH_3$ | $CH_3$ | CH |
| N | $CO_2CH_3$ | H | H | H | $OCH_3$ | $OCH_3$ | N |
| N | $CO_2CH_3$ | H | H | H | $OCH_3$ | $CH_3$ | N |
| N | $CO_2CH_3$ | H | H | H | $OC_2H_5$ | $NHCH_3$ | N |
| N | $CO_2CH_3$ | H | H | $CH_3$ | $OCH_3$ | $CH_3$ | N |
| N | $CO_2C_2H_5$ | H | H | H | $OCH_3$ | $OCH_3$ | CH |
| N | $CO_2C_2H_5$ | H | H | H | $OCH_3$ | $CH_3$ | CH |
| N | $CO_2C_2H_5$ | H | H | H | Cl | $OCH_3$ | CH |
| N | $CO_2C_2H_5$ | H | H | H | $CH_3$ | $CH_3$ | CH |
| N | $CO_2C_2H_5$ | H | H | H | $OCH_3$ | $OCH_3$ | N |

Table 11 (continued)

General Formula 11

| $Z^1$ | $R^1$ | $R^3$ | $R^5$ | R | X | Y | $Z^2$ |
|---|---|---|---|---|---|---|---|
| N | $CO_2C_2H_5$ | H | H | H | $OCH_3$ | $CH_3$ | N |
| N | $CO_2C_2H_5$ | H | H | H | $OC_2H_5$ | $NHCH_3$ | N |
| N | $CO_2C_2H_5$ | H | H | $CH_3$ | $OCH_3$ | $CH_3$ | N |
| N | $SO_2N(CH_3)_2$ | H | H | H | $OCH_3$ | $OCH_3$ | CH |
| N | $SO_2N(CH_3)_2$ | H | H | H | $OCH_3$ | $CH_3$ | CH |
| N | $SO_2N(CH_3)_2$ | H | H | H | Cl | $OCH_3$ | CH |
| N | $SO_2N(CH_3)_2$ | H | H | H | $CH_3$ | $CH_3$ | CH |
| N | $SO_2N(CH_3)_2$ | H | H | H | $OCH_3$ | $OCH_3$ | N |
| N | $SO_2N(CH_3)_2$ | H | H | H | $OCH_3$ | $CH_3$ | N |
| N | $SO_2N(CH_3)_2$ | H | H | H | $OC_2H_5$ | $NHCH_3$ | N |
| N | $SO_2N(CH_3)_2$ | H | H | $CH_3$ | $OCH_3$ | $CH_3$ | N |
| N | H | H | H | H | $OCH_3$ | $OCH_3$ | CH |
| N | H | H | H | H | $OCH_3$ | $CH_3$ | CH |
| N | H | H | H | H | Cl | $OCH_3$ | CH |
| N | H | H | H | H | $CH_3$ | $CH_3$ | CH |
| N | H | H | H | H | $OCH_3$ | $OCH_3$ | N |
| N | H | H | H | H | $OCH_3$ | $CH_3$ | N |
| N | H | H | H | H | $OC_2H_5$ | $NHCH_3$ | N |
| N | H | H | H | $CH_3$ | $OCH_3$ | $CH_3$ | N |
| N | H | H | Cl | H | $OCH_3$ | $OCH_3$ | CH |
| N | H | H | Cl | H | $OCH_3$ | $CH_3$ | CH |
| N | H | H | Cl | H | Cl | $OCH_3$ | CH |

Table 11 (continued)

General Formula 11

| $Z^1$ | $R^1$ | $R^3$ | $R^5$ | R | X | Y | $Z^4$ |
|---|---|---|---|---|---|---|---|
| N | H | H | Cl | H | $CH_3$ | $CH_3$ | CH |
| N | H | H | Cl | H | $OCH_3$ | $OCH_3$ | N |
| N | H | H | Cl | H | $OCH_3$ | $CH_3$ | N |
| N | H | H | Cl | H | $OC_2H_5$ | $NHCH_3$ | N |
| N | H | H | Cl | $CH_3$ | $OCH_3$ | $CH_3$ | N |
| N | H | H | $SO_2CH_3$ | H | $OCH_3$ | $OCH_3$ | CH |
| N | H | H | $SO_2CH_3$ | H | $OCH_3$ | $CH_3$ | CH |
| N | H | H | $SO_2CH_3$ | H | Cl | $OCH_3$ | CH |
| N | H | H | $SO_2CH_3$ | H | $CH_3$ | $CH_3$ | CH |
| N | H | H | $SO_2CH_3$ | H | $OCH_3$ | $OCH_3$ | N |
| N | H | H | $SO_2CH_3$ | H | $OCH_3$ | $CH_3$ | N |
| N | H | H | $SO_2CH_3$ | H | $OC_2H_5$ | $NHCH_3$ | N |
| N | H | H | $SO_2CH_3$ | $CH_3$ | $OCH_3$ | $CH_3$ | N |
| N | H | H | $CO_2CH_3$ | H | $OCH_3$ | $OCH_3$ | CH |
| N | H | H | $CO_2CH_3$ | H | $OCH_3$ | $CH_3$ | CH |
| N | H | H | $CO_2CH_3$ | H | Cl | $OCH_3$ | CH |
| N | H | H | $CO_2CH_3$ | H | $CH_3$ | $CH_3$ | CH |
| N | H | H | $CO_2CH_3$ | H | $OCH_3$ | $OCH_3$ | N |
| N | H | H | $CO_2CH_3$ | H | $OCH_3$ | $CH_3$ | N |
| N | H | H | $CO_2CH_3$ | H | $OC_2H_5$ | $NHCH_3$ | N |
| N | H | H | $CO_2CH_3$ | $CH_3$ | $OCH_3$ | $CH_3$ | N |
| N | H | H | $CO_2C_2H_5$ | H | $OCH_3$ | $OCH_3$ | CH |

Table 11 (continued)

General Formula 11

| $Z^1$ | $R^1$ | $R^3$ | $R^5$ | R | X | Y | $Z^4$ |
|---|---|---|---|---|---|---|---|
| N | H | H | $CO_2C_2H_5$ | H | $OCH_3$ | $CH_3$ | CH |
| N | H | H | $CO_2C_2H_5$ | H | Cl | $OCH_3$ | CH |
| N | H | H | $CO_2C_2H_5$ | H | $CH_3$ | $CH_3$ | CH |
| N | H | H | $CO_2C_2H_5$ | H | $OCH_3$ | $OCH_3$ | N |
| N | H | H | $CO_2C_2H_5$ | H | $OCH_3$ | $CH_3$ | N |
| N | H | H | $CO_2C_2H_5$ | H | $OC_2H_5$ | $NHCH_3$ | N |
| N | H | H | $CO_2C_2H_5$ | $CH_3$ | $OCH_3$ | $CH_3$ | N |
| N | H | H | $SO_2N(CH_3)_2$ | H | $OCH_3$ | $OCH_3$ | CH |
| N | H | H | $SO_2N(CH_3)_2$ | H | $OCH_3$ | $CH_3$ | CH |
| N | H | H | $SO_2N(CH_3)_2$ | H | Cl | $OCH_3$ | CH |
| N | H | H | $SO_2N(CH_3)_2$ | H | $CH_3$ | $CH_3$ | CH |
| N | H | H | $SO_2N(CH_3)_2$ | H | $OCH_3$ | $OCH_3$ | N |
| N | H | H | $SO_2N(CH_3)_2$ | H | $OCH_3$ | $CH_3$ | N |
| N | H | H | $SO_2N(CH_3)_2$ | H | $OC_2H_5$ | $NHCH_3$ | N |
| N | H | H | $SO_2N(CH_3)_2$ | $CH_3$ | $OCH_3$ | $CH_3$ | N |

Table 12

General Formula 12

| Z | $R^2$ | $R^3$ | $R^5$ | R | X | Y | Z' |
|---|---|---|---|---|---|---|---|
| CH | H | H | H | H | $OCH_3$ | $OCH_3$ | CH |
| CH | H | H | H | H | $OCH_3$ | $CH_3$ | CH |
| CH | H | H | H | H | Cl | $OCH_3$ | CH |
| CH | H | H | H | H | $CH_3$ | $CH_3$ | CH |
| CH | H | H | H | H | $OCH_3$ | $OCH_3$ | N |
| CH | H | H | H | H | $OCH_3$ | $CH_3$ | N |
| CH | H | H | H | H | $OC_2H_5$ | $NHCH_3$ | N |
| CH | H | H | H | $CH_3$ | $OCH_3$ | CH | N |
| CH | H | H | Cl | H | $OCH_3$ | $OCH_3$ | CH |
| CH | H | H | Cl | H | $OCH_3$ | $CH_3$ | CH |
| CH | H | H | Cl | H | Cl | $OCH_3$ | CH |
| CH | H | H | Cl | H | $CH_3$ | $CH_3$ | CH |
| CH | H | H | Cl | H | $OCH_3$ | $OCH_3$ | N |
| CH | H | H | Cl | H | $OCH_3$ | $CH_3$ | N |
| CH | H | H | Cl | H | $OC_2H_5$ | $NHCH_3$ | N |
| CH | H | H | Cl | $CH_3$ | $OCH_3$ | CH | N |
| CH | H | H | $SO_2CH_3$ | H | $OCH_3$ | $OCH_3$ | CH |
| CH | H | H | $SO_2CH_3$ | H | $OCH_3$ | $CH_3$ | CH |
| CH | H | H | $SO_2CH_3$ | H | Cl | $OCH_3$ | CH |
| CH | H | H | $SO_2CH_3$ | H | $CH_3$ | $CH_3$ | CH |
| CH | H | H | $SO_2CH_3$ | H | $OCH_3$ | $OCH_3$ | N |
| CH | H | H | $SO_2CH_3$ | H | $OCH_3$ | $CH_3$ | N |
| CH | H | H | $SO_2CH_3$ | H | $OC_2H_5$ | $NHCH_3$ | N |
| CH | H | H | $SO_2CH_3$ | $CH_3$ | $OCH_3$ | $CH_3$ | N |

Table 12 (continued)

General Formula 12

| Z | $R^2$ | $R^3$ | $R^5$ | R | X | Y | Z' |
|---|---|---|---|---|---|---|---|
| N | H | H | H | H | $OCH_3$ | $OCH_3$ | CH |
| N | H | H | H | H | $OCH_3$ | $CH_3$ | CH |
| N | H | H | H | H | Cl | $OCH_3$ | CH |
| N | H | H | H | H | $CH_3$ | $CH_3$ | CH |
| N | H | H | H | H | $OCH_3$ | $OCH_3$ | N |
| N | H | H | H | H | $OCH_3$ | $CH_3$ | N |
| N | H | H | H | H | $OC_2H_5$ | $NHCH_3$ | N |
| N | H | H | $CH_3$ | H | $OCH_3$ | $CH_3$ | N |
| N | H | H | Cl | H | $OCH_3$ | $OCH_3$ | CH |
| N | H | H | Cl | H | $OCH_3$ | $CH_3$ | CH |
| N | H | H | Cl | H | Cl | $OCH_3$ | CH |
| N | H | H | Cl | H | $CH_3$ | $CH_3$ | CH |
| N | H | H | Cl | H | $OCH_3$ | $OCH_3$ | N |
| N | H | H | Cl | H | $OCH_3$ | $CH_3$ | N |
| N | H | H | Cl | H | $OC_2H_5$ | $NHCH_3$ | N |
| N | H | H | Cl | $CH_3$ | $OCH_3$ | $CH_3$ | N |
| N | H | H | $SO_2CH_3$ | H | $OCH_3$ | $OCH_3$ | CH |
| N | H | H | $SO_2CH_3$ | H | $OCH_3$ | $CH_3$ | CH |
| N | H | H | $SO_2CH_3$ | H | Cl | $OCH_3$ | CH |
| N | H | H | $SO_2CH_3$ | H | $CH_3$ | $CH_3$ | CH |
| N | H | H | $SO_2CH_3$ | H | $OCH_3$ | $OCH_3$ | N |
| N | H | H | $SO_2CH_3$ | H | $OCH_3$ | $CH_3$ | N |
| N | H | H | $SO_2CH_3$ | H | $OC_2H_5$ | $NHCH_3$ | N |
| N | H | H | $SO_2CH_3$ | $CH_3$ | $OCH_3$ | $CH_3$ | N |

Table 12 (continued)

General Formula 12

| Z | $R^2$ | $R^3$ | $R^5$ | R | X | Y | $Z^4$ |
|---|---|---|---|---|---|---|---|
| CH | H | H | $CO_2CH_3$ | H | $OCH_3$ | $OCH_3$ | CH |
| CH | H | H | $CO_2CH_3$ | H | $OCH_3$ | $CH_3$ | CH |
| CH | H | H | $CO_2CH_3$ | H | Cl | $OCH_3$ | CH |
| CH | H | H | $CO_2CH_3$ | H | $CH_3$ | $CH_3$ | CH |
| CH | H | H | $CO_2CH_3$ | H | $OCH_3$ | $OCH_3$ | N |
| CH | H | H | $CO_2CH_3$ | H | $OCH_3$ | $CH_3$ | N |
| CH | H | H | $CO_2CH_3$ | H | $OC_2H_5$ | $NHCH_3$ | N |
| CH | H | H | $CO_2CH_3$ | $CH_3$ | $OCH_3$ | $CH_3$ | N |
| CH | H | H | $CO_2C_2H_5$ | H | $OCH_3$ | $OCH_3$ | CH |
| CH | H | H | $CO_2C_2H_5$ | H | $OCH_3$ | $CH_3$ | CH |
| CH | H | H | $CO_2C_2H_5$ | H | Cl | $OCH_3$ | CH |
| CH | H | H | $CO_2C_2H_5$ | H | $CH_3$ | $CH_3$ | CH |
| CH | H | H | $CO_2C_2H_5$ | H | $OCH_3$ | $OCH_3$ | N |
| CH | H | H | $CO_2C_2H_5$ | H | $OCH_3$ | $CH_3$ | N |
| CH | H | H | $CO_2C_2H_5$ | H | $OC_2H_5$ | $NHCH_3$ | N |
| CH | H | H | $CO_2C_2H_5$ | $CH_3$ | $OCH_3$ | $CH_3$ | N |
| CH | H | H | $SO_2N(CH_3)_2$ | H | $OCH_3$ | $OCH_3$ | CH |
| CH | H | H | $SO_2N(CH_3)_2$ | H | $OCH_3$ | $CH_3$ | CH |
| CH | H | H | $SO_2N(CH_3)_2$ | H | Cl | $OCH_3$ | CH |
| CH | H | H | $SO_2N(CH_3)_2$ | H | $CH_3$ | $CH_3$ | CH |
| CH | H | H | $SO_2N(CH_3)_2$ | H | $OCH_3$ | $OCH_3$ | N |
| CH | H | H | $SO_2N(CH_3)_2$ | H | $OCH_3$ | $CH_3$ | N |
| CH | H | H | $SO_2N(CH_3)_2$ | H | $OC_2H_5$ | $NHCH_3$ | N |
| CH | H | H | $SO_2N(CH_3)_2$ | $CH_3$ | $OCH_3$ | $CH_3$ | N |

Table 12 (continued)

General Formula 12

| Z | $R^2$ | $R^3$ | $R^5$ | R | X | Y | $Z^4$ |
|---|---|---|---|---|---|---|---|
| N | H | H | $CO_2CH_3$ | H | $OCH_3$ | $OCH_3$ | CH |
| N | H | H | $CO_2CH_3$ | H | $OCH_3$ | $CH_3$ | CH |
| N | H | H | $CO_2CH_3$ | H | Cl | $OCH_3$ | CH |
| N | H | H | $CO_2CH_3$ | H | $CH_3$ | $CH_3$ | CH |
| N | H | H | $CO_2CH_3$ | H | $OCH_3$ | $OCH_3$ | N |
| N | H | H | $CO_2CH_3$ | H | $OCH_3$ | $CH_3$ | N |
| N | H | H | $CO_2CH_3$ | H | $OC_2H_5$ | $NHCH_3$ | N |
| N | H | H | $CO_2CH_3$ | $CH_3$ | $OCH_3$ | $CH_3$ | N |
| N | H | H | $CO_2C_2H_5$ | H | $OCH_3$ | $OCH_3$ | CH |
| N | H | H | $CO_2C_2H_5$ | H | $OCH_3$ | $CH_3$ | CH |
| N | H | H | $CO_2C_2H_5$ | H | Cl | $OCH_3$ | CH |
| N | H | H | $CO_2C_2H_5$ | H | $CH_3$ | $CH_3$ | CH |
| N | H | H | $CO_2C_2H_5$ | H | $OCH_3$ | $OCH_3$ | N |
| N | H | H | $CO_2C_2H_5$ | H | $OCH_3$ | $CH_3$ | N |
| N | H | H | $CO_2C_2H_5$ | H | $OC_2H_5$ | $NHCH_3$ | N |
| N | H | H | $CO_2C_2H_5$ | $CH_3$ | $OCH_3$ | $CH_3$ | N |
| N | H | H | $SO_2N(CH_3)_2$ | H | $OCH_3$ | $OCH_3$ | CH |

Table 12 (continued)

General Formula 12

| Z | $R^2$ | $R^3$ | $R^5$ | R | X | Y | $Z^4$ |
|---|---|---|---|---|---|---|---|
| N | H | H | $SO_2N(CH_3)_2$ | H | $OCH_3$ | $CH_3$ | CH |
| N | H | H | $SO_2N(CH_3)_2$ | H | Cl | $OCH_3$ | CH |
| N | H | H | $SO_2N(CH_3)_2$ | H | $CH_3$ | $CH_3$ | CH |
| N | H | H | $SO_2N(CH_3)_2$ | H | $OCH_3$ | $OCH_3$ | N |
| N | H | H | $SO_2N(CH_3)_2$ | H | $OCH_3$ | $CH_3$ | N |
| N | H | H | $SO_2N(CH_3)_2$ | H | $OC_2H_5$ | $NHCH_3$ | N |
| N | H | H | $SO_2N(CH_3)_2$ | $CH_3$ | $OCH_3$ | $CH_3$ | N |

Table 13

General Formula 13

| $R^3$ | $R^5$ | R | X | Y | $Z^4$ |
|---|---|---|---|---|---|
| H | H | H | $OCH_3$ | $OCH_3$ | CH |
| H | H | H | $OCH_3$ | $CH_3$ | CH |
| H | H | H | Cl | $OCH_3$ | CH |
| H | H | H | $CH_3$ | $CH_3$ | CH |
| H | H | H | $OCH_3$ | $OCH_3$ | N |
| H | H | H | $OCH_3$ | $CH_3$ | N |
| H | H | H | $OC_2H_5$ | $NHCH_3$ | N |
| H | H | $CH_3$ | $OCH_3$ | $CH_3$ | N |
| H | Cl | H | $OCH_3$ | $OCH_3$ | CH |
| H | Cl | H | $OCH_3$ | $CH_3$ | CH |
| H | Cl | H | Cl | $OCH_3$ | CH |
| H | Cl | H | $CH_3$ | $CH_3$ | CH |
| H | Cl | H | $OCH_3$ | $OCH_3$ | N |
| H | Cl | H | $OCH_3$ | $CH_3$ | N |
| H | Cl | H | $OC_2H_5$ | $NHCH_3$ | N |
| H | Cl | $CH_3$ | $OCH_3$ | $CH_3$ | N |
| H | $SO_2CH_3$ | H | $OCH_3$ | $OCH_3$ | CH |
| H | $SO_2CH_3$ | H | $OCH_3$ | $CH_3$ | CH |
| H | $SO_2CH_3$ | H | Cl | $OCH_3$ | CH |
| H | $SO_2CH_3$ | H | $CH_3$ | $CH_3$ | CH |
| H | $SO_2CH_3$ | H | $OCH_3$ | $OCH_3$ | N |
| H | $SO_2CH_3$ | H | $OCH_3$ | $CH_3$ | N |
| H | $SO_2CH_3$ | H | $OC_2H_5$ | $NHCH_3$ | N |

Table 13 (continued)

General Formula 13

| $R^3$ | $R^5$ | R | X | Y | $Z^4$ |
|---|---|---|---|---|---|
| H | $SO_2CH_3$ | H | $OCH_3$ | $OCH_3$ | CH |
| H | $SO_2CH_3$ | $CH_3$ | $OCH_3$ | $CH_3$ | N |
| H | $CO_2CH_3$ | H | $OCH_3$ | $OCH_3$ | CH |
| H | $CO_2CH_3$ | H | $OCH_3$ | $CH_3$ | CH |
| H | $CO_2CH_3$ | H | Cl | $OCH_3$ | CH |
| H | $CO_2CH_3$ | H | $CH_3$ | $CH_3$ | CH |
| H | $CO_2CH_3$ | H | $OCH_3$ | $OCH_3$ | N |
| H | $CO_2CH_3$ | H | $OCH_3$ | $CH_3$ | N |
| H | $CO_2CH_3$ | H | $OC_2H_5$ | $NHCH_3$ | N |
| H | $CO_2CH_3$ | $CH_3$ | $OCH_3$ | $CH_3$ | N |
| H | $CO_2C_2H_5$ | H | $OCH_3$ | $OCH_3$ | CH |
| H | $CO_2C_2H_5$ | H | $OCH_3$ | $CH_3$ | CH |
| H | $CO_2C_2H_5$ | H | Cl | $OCH_3$ | CH |
| H | $CO_2C_2H_5$ | H | $CH_3$ | $CH_3$ | CH |
| H | $CO_2C_2H_5$ | H | $OCH_3$ | $OCH_3$ | N |
| H | $CO_2C_2H_5$ | H | $OCH_3$ | $CH_3$ | N |
| H | $CO_2C_2H_5$ | H | $OC_2H_5$ | $NHCH_3$ | N |
| H | $CO_2C_2H_5$ | $CH_3$ | $OCH_3$ | $CH_3$ | N |
| H | $SO_2N(CH_3)_2$ | H | $OCH_3$ | $OCH_3$ | CH |
| H | $SO_2N(CH_3)_2$ | H | $OCH_3$ | $CH_3$ | CH |
| H | $SO_2N(CH_3)_2$ | H | Cl | $OCH_3$ | CH |
| H | $SO_2N(CH_3)_2$ | H | $CH_3$ | $CH_3$ | CH |

Table 13 (continued)

General Formula 13

| $R^3$ | $R^5$ | R | X | Y | $Z^4$ |
| --- | --- | --- | --- | --- | --- |
| H | $SO_2N(CH_3)_2$ | H | $OCH_3$ | $OCH_3$ | N |
| H | $SO_2N(CH_3)_2$ | H | $OCH_3$ | $CH_3$ | N |
| H | $SO_2N(CH_3)_2$ | H | $OC_2H_5$ | $NHCH_3$ | N |
| H | $SO_2N(CH_3)_2$ | $CH_3$ | $OCH_3$ | $CH_3$ | N |

Table 14

General Formula 14

| $R^1$ | $R^2$ | $R^4$ | R | X | Y | $Z^4$ |
|---|---|---|---|---|---|---|
| H | H | H | H | $OCH_3$ | $OCH_3$ | CH |
| H | H | H | H | $OCH_3$ | $CH_3$ | CH |
| H | H | H | H | Cl | $OCH_3$ | CH |
| H | H | H | H | $CH_3$ | $CH_3$ | CH |
| H | H | H | H | $OCH_3$ | $OCH_3$ | N |
| H | H | H | H | $OCH_3$ | $CH_3$ | N |
| H | H | H | H | $OC_2H_5$ | $NHCH_3$ | N |
| H | H | H | $CH_3$ | $OCH_3$ | $CH_3$ | N |
| Cl | H | H | H | $OCH_3$ | $OCH_3$ | CH |
| Cl | H | H | H | $OCH_3$ | $CH_3$ | CH |
| Cl | H | H | H | Cl | $OCH_3$ | CH |
| Cl | H | H | H | $CH_3$ | $CH_3$ | CH |
| Cl | H | H | H | $OCH_3$ | $OCH_3$ | N |
| Cl | H | H | H | $OCH_3$ | $CH_3$ | N |
| Cl | H | H | H | $OC_2H_5$ | $NHCH_3$ | N |
| Cl | H | H | $CH_3$ | $OCH_3$ | $CH_3$ | N |
| $SO_2CH_3$ | H | H | H | $OCH_3$ | $OCH_3$ | CH |
| $SO_2CH_3$ | H | H | H | $OCH_3$ | $CH_3$ | CH |
| $SO_2CH_3$ | H | H | H | Cl | $OCH_3$ | CH |
| $SO_2CH_3$ | H | H | H | $CH_3$ | $CH_3$ | CH |
| $SO_2CH_3$ | H | H | H | $OCH_3$ | $OCH_3$ | N |
| $SO_2CH_3$ | H | H | H | $OCH_3$ | $CH_3$ | N |
| $SO_2CH_3$ | H | H | H | $OC_2H_5$ | $NHCH_3$ | N |

Table 14 (continued)

General Formula 14

| $R^1$ | $R^2$ | $R^4$ | R | X | Y | $Z^4$ |
|---|---|---|---|---|---|---|
| $SO_2CH_3$ | H | H | $CH_3$ | $OCH_3$ | $CH_3$ | N |
| $CO_2CH_3$ | H | H | H | $OCH_3$ | $OCH_3$ | CH |
| $CO_2CH_3$ | H | H | H | $OCH_3$ | $CH_3$ | CH |
| $CO_2CH_3$ | H | H | H | Cl | $OCH_3$ | CH |
| $CO_2CH_3$ | H | H | H | $CH_3$ | $CH_3$ | CH |
| $CO_2CH_3$ | H | H | H | $OCH_3$ | $OCH_3$ | N |
| $CO_2CH_3$ | H | H | H | $OCH_3$ | $CH_3$ | N |
| $CO_2CH_3$ | H | H | H | $OC_2H_5$ | $NHCH_3$ | N |
| $CO_2CH_3$ | H | H | $CH_3$ | $OCH_3$ | $CH_3$ | N |
| $CO_2C_2H_5$ | H | H | H | $OCH_3$ | $OCH_3$ | CH |
| $CO_2C_2H_5$ | H | H | H | $OCH_3$ | $CH_3$ | CH |
| $CO_2C_2H_5$ | H | H | H | Cl | $OCH_3$ | CH |
| $CO_2C_2H_5$ | H | H | H | $CH_3$ | $CH_3$ | CH |
| $CO_2C_2H_5$ | H | H | H | $OCH_3$ | $OCH_3$ | N |
| $CO_2C_2H_5$ | H | H | H | $OCH_3$ | $CH_3$ | N |
| $CO_2C_2H_5$ | H | H | H | $OC_2H_5$ | $NHCH_3$ | N |
| $CO_2C_2H_5$ | H | H | $CH_3$ | $OCH_3$ | $CH_3$ | N |
| $SO_2N(CH_3)_2$ | H | H | H | $OCH_3$ | $OCH_3$ | CH |
| $SO_2N(CH_3)_2$ | H | H | H | $OCH_3$ | $CH_3$ | CH |
| $SO_2N(CH_3)_2$ | H | H | H | Cl | $OCH_3$ | CH |
| $SO_2N(CH_3)_2$ | H | H | H | $CH_3$ | $CH_3$ | CH |
| $SO_2N(CH_3)_2$ | H | H | H | $OCH_3$ | $OCH_3$ | N |

Table 14 (continued)

General Formula 14

| $R^1$ | $R^2$ | $R^4$ | R | X | Y | $Z^4$ |
|---|---|---|---|---|---|---|
| $SO_2N(CH_3)_2$ | H | H | H | $OCH_3$ | $CH_3$ | N |
| $SO_2N(CH_3)_2$ | H | H | H | $OC_2H_5$ | $NHCH_3$ | N |
| $SO_2N(CH_3)_2$ | H | H | $CH_3$ | $OCH_3$ | $CH_3$ | N |



BAD ORIGINAL

Table 15

General Formula 15

| $R^2$ | $R^4$ | R | X | Y | $Z^4$ |
|---|---|---|---|---|---|
| H | H | H | $OCH_3$ | $OCH_3$ | CH |
| H | H | H | $OCH_3$ | $CH_3$ | CH |
| H | H | H | Cl | $OCH_3$ | CH |
| H | H | H | $CH_3$ | $CH_3$ | CH |
| H | H | H | $OCH_3$ | $OCH_3$ | N |
| H | H | H | $OCH_3$ | $CH_3$ | N |
| H | H | H | $OC_2H_5$ | $NHCH_3$ | N |
| H | H | $CH_3$ | $OCH_3$ | $CH_3$ | N |
| $CH_3$ | H | H | $OCH_3$ | $OCH_3$ | CH |
| $CH_3$ | H | H | $OCH_3$ | $CH_3$ | CH |
| $CH_3$ | H | H | Cl | $OCH_3$ | CH |
| $CH_3$ | H | H | $CH_3$ | $CH_3$ | CH |
| $CH_3$ | H | H | $OCH_3$ | $OCH_3$ | N |
| $CH_3$ | H | H | $OCH_3$ | $CH_3$ | N |
| $CH_3$ | H | H | $OC_2H_5$ | $NHCH_3$ | N |
| $CH_3$ | H | $CH_3$ | $OCH_3$ | $CH_3$ | N |
| $OCH_3$ | H | H | $OCH_3$ | $OCH_3$ | CH |
| $OCH_3$ | H | H | $OCH_3$ | $CH_3$ | CH |
| $OCH_3$ | H | H | Cl | $OCH_3$ | CH |
| $OCH_3$ | H | H | $CH_3$ | $CH_3$ | CH |
| $OCH_3$ | H | H | $OCH_3$ | $OCH_3$ | N |
| $OCH_3$ | H | H | $OCH_3$ | $CH_3$ | N |
| $OCH_3$ | H | H | $OC_2H_5$ | $NHCH_3$ | N |

Table 15 (continued)

General Formula 15

| $R^2$ | $R^4$ | R | X | Y | $Z^4$ |
|---|---|---|---|---|---|
| $OCH_3$ | H | CH | $OCH_3$ | $CH_3$ | N |

Table 16

General Formula 16

| $R^1$ | $R^2$ | $R^4$ | R | X | Y | $Z^4$ |
|---|---|---|---|---|---|---|
| H | H | H | H | $OCH_3$ | $OCH_3$ | CH |
| H | H | H | H | $OCH_3$ | $CH_3$ | CH |
| H | H | H | H | Cl | $OCH_3$ | CH |
| H | H | H | H | $CH_3$ | $CH_3$ | CH |
| H | H | H | H | $OCH_3$ | $OCH_3$ | N |
| H | H | H | H | $OCH_3$ | $CH_3$ | N |
| H | H | H | H | $OC_2H_5$ | $NHCH_3$ | N |
| H | H | H | $CH_3$ | $OCH_3$ | $CH_3$ | N |
| Cl | H | H | H | $OCH_3$ | $OCH_3$ | CH |
| Cl | H | H | H | $OCH_3$ | $CH_3$ | CH |
| Cl | H | H | H | Cl | $OCH_3$ | CH |
| Cl | H | H | H | $CH_3$ | $CH_3$ | CH |
| Cl | H | H | H | $OCH_3$ | $OCH_3$ | N |
| Cl | H | H | H | $OCH_3$ | $CH_3$ | N |
| Cl | H | H | H | $OC_2H_5$ | $NHCH_3$ | N |
| Cl | H | H | $CH_3$ | $OCH_3$ | $CH_3$ | N |
| $SO_2CH_3$ | H | H | H | $OCH_3$ | $OCH_3$ | CH |
| $SO_2CH_3$ | H | H | H | $OCH_3$ | $CH_3$ | CH |
| $SO_2CH_3$ | H | H | H | Cl | $OCH_3$ | CH |
| $SO_2CH_3$ | H | H | H | $CH_3$ | $CH_3$ | CH |
| $SO_2CH_3$ | H | H | H | $OCH_3$ | $OCH_3$ | N |
| $SO_2CH_3$ | H | H | H | $OCH_3$ | $CH_3$ | N |
| $SO_2CH_3$ | H | H | H | $OC_2H_5$ | $NHCH_3$ | N |

Table 16 (continued)

General Formula 16

| $R^1$ | $R^2$ | $R^4$ | R | X | Y | $Z^4$ |
|---|---|---|---|---|---|---|
| $SO_2CH_3$ | H | H | $CH_3$ | $OCH_3$ | $CH_3$ | N |
| $CO_2CH_3$ | H | H | H | $OCH_3$ | $OCH_3$ | CH |
| $CO_2CH_3$ | H | H | H | $OCH_3$ | $CH_3$ | CH |
| $CO_2CH_3$ | H | H | H | Cl | $OCH_3$ | CH |
| $CO_2CH_3$ | H | H | H | $CH_3$ | $CH_3$ | CH |
| $CO_2CH_3$ | H | H | H | $OCH_3$ | $OCH_3$ | N |
| $CO_2CH_3$ | H | H | H | $OCH_3$ | $CH_3$ | N |
| $CO_2CH_3$ | H | H | H | $OC_2H_5$ | $NHCH_3$ | N |
| $CO_2CH_3$ | H | H | $CH_3$ | $OCH_3$ | $CH_3$ | N |
| $CO_2C_2H_5$ | H | H | H | $OCH_3$ | $OCH_3$ | CH |
| $CO_2C_2H_5$ | H | H | H | $OCH_3$ | $CH_3$ | CH |
| $CO_2C_2H_5$ | H | H | H | Cl | $OCH_3$ | CH |
| $CO_2C_2H_5$ | H | H | H | $CH_3$ | $CH_3$ | CH |
| $CO_2C_2H_5$ | H | H | H | $OCH_3$ | $OCH_3$ | N |
| $CO_2C_2H_5$ | H | H | H | $OCH_3$ | $CH_3$ | N |
| $CO_2C_2H_5$ | H | H | H | $OC_2H_5$ | $NHCH_3$ | N |
| $CO_2C_2H_5$ | H | H | $CH_3$ | $OCH_3$ | $CH_3$ | N |
| $SO_2N(CH_3)_2$ | H | H | H | $OCH_3$ | $OCH_3$ | CH |
| $SO_2N(CH_3)_2$ | H | H | H | $OCH_3$ | $CH_3$ | CH |
| $SO_2N(CH_3)_2$ | H | H | H | Cl | $OCH_3$ | CH |
| $SO_2N(CH_3)_2$ | H | H | H | $CH_3$ | $CH_3$ | CH |
| $SO_2N(CH_3)_2$ | H | H | H | $OCH_3$ | $OCH_3$ | N |

Table 16 (continued)

General Formula 16

| $R^1$ | $R^2$ | $R^4$ | R | X | Y | $Z^4$ |
|---|---|---|---|---|---|---|
| $SO_2N(CH_3)_2$ | H | H | H | $OCH_3$ | $CH_3$ | N |
| $SO_2N(CH_3)_2$ | H | H | H | $OC_2H_5$ | $NHCH_3$ | N |
| $SO_2N(CH_3)_2$ | H | H | $CH_3$ | $OCH_3$ | $CH_3$ | N |

Table 17

General Formula 17

| $Z^1$ | $R^1$ | $R^2$ | R | X | Y | $Z^4$ |
|---|---|---|---|---|---|---|
| CH | H | H | H | $OCH_3$ | $OCH_3$ | CH |
| CH | H | H | H | $OCH_3$ | $CH_3$ | CH |
| CH | H | H | H | Cl | $OCH_3$ | CH |
| CH | H | H | H | $CH_3$ | $CH_3$ | CH |
| CH | H | H | H | $OCH_3$ | $OCH_3$ | N |
| CH | H | H | H | $OCH_3$ | $CH_3$ | N |
| CH | H | H | H | $OC_2H_5$ | $NHCH_3$ | N |
| CH | H | H | $CH_3$ | $OCH_3$ | $CH_3$ | N |
| CH | Cl | H | H | $OCH_3$ | $OCH_3$ | CH |
| CH | Cl | H | H | $OCH_3$ | $CH_3$ | CH |
| CH | Cl | H | H | Cl | $OCH_3$ | CH |
| CH | Cl | H | H | $CH_3$ | $CH_3$ | CH |
| CH | Cl | H | H | $OCH_3$ | $OCH_3$ | N |
| CH | Cl | H | H | $OCH_3$ | $CH_3$ | N |
| CH | Cl | H | H | $OC_2H_5$ | $NHCH_3$ | N |
| CH | Cl | H | $CH_3$ | $OCH_3$ | $CH_3$ | N |
| CH | $SO_2CH_3$ | H | H | $OCH_3$ | $OCH_3$ | CH |
| CH | $SO_2CH_3$ | H | H | $OCH_3$ | $CH_3$ | CH |
| CH | $SO_2CH_3$ | H | H | Cl | $OCH_3$ | CH |
| CH | $SO_2CH_3$ | H | H | $CH_3$ | $CH_3$ | CH |
| CH | $SO_2CH_3$ | H | H | $OCH_3$ | $OCH_3$ | N |
| CH | $SO_2CH_3$ | H | H | $OCH_3$ | $CH_3$ | N |
| CH | $SO_2CH_3$ | H | H | $OC_2H_5$ | $NHCH_3$ | N |
| CH | $SO_2CH_3$ | H | $CH_3$ | $OCH_3$ | $CH_3$ | N |

Table 17 (continued)

General Formula 17

| $Z^1$ | $R^1$ | $R^2$ | R | X | Y | $Z^2$ |
|---|---|---|---|---|---|---|
| CH | $CO_2CH_3$ | H | H | $OCH_3$ | $OCH_3$ | CH |
| CH | $CO_2CH_3$ | H | H | $OCH_3$ | $CH_3$ | CH |
| CH | $CO_2CH_3$ | H | H | Cl | $OCH_3$ | CH |
| CH | $CO_2CH_3$ | H | H | $CH_3$ | $CH_3$ | CH |
| CH | $CO_2CH_3$ | H | H | $OCH_3$ | $OCH_3$ | N |
| CH | $CO_2CH_3$ | H | H | $OCH_3$ | $CH_3$ | N |
| CH | $CO_2CH_3$ | H | H | $OC_2H_5$ | $NHCH_3$ | N |
| CH | $CO_2CH_3$ | H | $CH_3$ | $OCH_3$ | $CH_3$ | N |
| CH | $CO_2C_2H_5$ | H | H | $OCH_3$ | $OCH_3$ | CH |
| CH | $CO_2C_2H_5$ | H | H | $OCH_3$ | $CH_3$ | CH |
| CH | $CO_2C_2H_5$ | H | H | Cl | $OCH_3$ | CH |
| CH | $CO_2C_2H_5$ | H | H | $CH_3$ | $CH_3$ | CH |
| CH | $CO_2C_2H_5$ | H | H | $OCH_3$ | $OCH_3$ | N |
| CH | $CO_2C_2H_5$ | H | H | $OCH_3$ | $CH_3$ | N |
| CH | $CO_2C_2H_5$ | H | H | $OC_2H_5$ | $NHCH_3$ | N |
| CH | $CO_2C_2H_5$ | H | $CH_3$ | $OCH_3$ | $CH_3$ | N |
| CH | $SO_2N(CH_3)_2$ | H | H | $OCH_3$ | $OCH_3$ | CH |
| CH | $SO_2N(CH_3)_2$ | H | H | $OCH_3$ | $CH_3$ | CH |
| CH | $SO_2N(CH_3)_2$ | H | H | Cl | $OCH_3$ | CH |
| CH | $SO_2N(CH_3)_2$ | H | H | $CH_3$ | $CH_3$ | CH |
| CH | $SO_2N(CH_3)_2$ | H | H | $OCH_3$ | $OCH_3$ | N |
| CH | $SO_2N(CH_3)_2$ | H | H | $OCH_3$ | $CH_3$ | N |
| CH | $SO_2N(CH_3)_2$ | H | H | $OC_2H_5$ | $NHCH_3$ | N |
| CH | $SO_2N(CH_3)_2$ | H | $CH_3$ | $OCH_3$ | $CH_3$ | N |

Table 17 (continued)

General Formula 17

| $Z^1$ | $R^1$ | $R^2$ | R | X | Y | $Z^4$ |
|---|---|---|---|---|---|---|
| N | H | H | H | $OCH_3$ | $OCH_3$ | CH |
| N | H | H | H | $OCH_3$ | $CH_3$ | CH |
| N | H | H | H | Cl | $OCH_3$ | CH |
| N | H | H | H | $CH_3$ | $CH_3$ | CH |
| N | H | H | H | $OCH_3$ | $OCH_3$ | N |
| N | H | H | H | $OCH_3$ | $CH_3$ | N |
| N | H | H | H | $OC_2H_5$ | $NHCH_3$ | N |
| N | H | H | $CH_3$ | $OCH_3$ | $CH_3$ | N |
| N | Cl | H | H | $OCH_3$ | $OCH_3$ | CH |
| N | Cl | H | H | $OCH_3$ | $CH_3$ | CH |
| N | Cl | H | H | Cl | $OCH_3$ | CH |
| N | Cl | H | H | $CH_3$ | $CH_3$ | CH |
| N | Cl | H | H | $OCH_3$ | $OCH_3$ | N |
| N | Cl | H | H | $OCH_3$ | $CH_3$ | N |
| N | Cl | H | H | $OC_2H_5$ | $NHCH_3$ | N |
| N | Cl | H | $CH_3$ | $OCH_3$ | $CH_3$ | N |
| N | $SO_2CH_3$ | H | H | $OCH_3$ | $OCH_3$ | CH |
| N | $SO_2CH_3$ | H | H | $OCH_3$ | $CH_3$ | CH |
| N | $SO_2CH_3$ | H | H | Cl | $OCH_3$ | CH |
| N | $SO_2CH_3$ | H | H | $CH_3$ | $CH_3$ | CH |
| N | $SO_2CH_3$ | H | H | $OCH_3$ | $OCH_3$ | N |
| N | $SO_2CH_3$ | H | H | $OCH_3$ | $CH_3$ | N |
| N | $SO_2CH_3$ | H | H | $OC_2H_5$ | $NHCH_3$ | N |
| N | $SO_2CH_3$ | H | $CH_3$ | $OCH_3$ | $CH_3$ | N |

Table 17 (continued)

General Formula 17

| $Z^1$ | $R^1$ | $R^2$ | R | X | Y | $Z^2$ |
|---|---|---|---|---|---|---|
| N | $CO_2CH_3$ | H | H | $OCH_3$ | $OCH_3$ | CH |
| N | $CO_2CH_3$ | H | H | $OCH_3$ | $CH_3$ | CH |
| N | $CO_2CH_3$ | H | H | Cl | $OCH_3$ | CH |
| N | $CO_2CH_3$ | H | H | $CH_3$ | $CH_3$ | CH |
| N | $CO_2CH_3$ | H | H | $OCH_3$ | $OCH_3$ | N |
| N | $CO_2CH_3$ | H | H | $OCH_3$ | $CH_3$ | N |
| N | $CO_2CH_3$ | H | H | $OC_2H_5$ | $NHCH_3$ | N |
| N | $CO_2CH_3$ | H | $CH_3$ | $OCH_3$ | $CH_3$ | N |
| N | $CO_2C_2H_5$ | H | H | $OCH_3$ | $OCH_3$ | CH |
| N | $CO_2C_2H_5$ | H | H | $OCH_3$ | $CH_3$ | CH |
| N | $CO_2C_2H_5$ | H | H | Cl | $OCH_3$ | CH |
| N | $CO_2C_2H_5$ | H | H | $CH_3$ | $CH_3$ | CH |
| N | $CO_2C_2H_5$ | H | H | $OCH_3$ | $OCH_3$ | N |
| N | $CO_2C_2H_5$ | H | H | $OCH_3$ | $CH_3$ | N |
| N | $CO_2C_2H_5$ | H | H | $OC_2H_5$ | $NHCH_3$ | N |
| N | $CO_2C_2H_5$ | H | $CH_3$ | $OCH_3$ | $CH_3$ | N |
| N | $SO_2N(CH_3)_2$ | H | H | $OCH_3$ | $OCH_3$ | CH |
| N | $SO_2N(CH_3)_2$ | H | H | $OCH_3$ | $CH_3$ | CH |
| N | $SO_2N(CH_3)_2$ | H | H | Cl | $OCH_3$ | CH |
| N | $SO_2N(CH_3)_2$ | H | H | $CH_3$ | $CH_3$ | CH |
| N | $SO_2N(CH_3)_2$ | H | H | $OCH_3$ | $OCH_3$ | N |
| N | $SO_2N(CH_3)_2$ | H | H | $OCH_3$ | $CH_3$ | N |
| N | $SO_2N(CH_3)_2$ | H | H | $OC_2H_5$ | $NHCH_3$ | N |
| N | $SO_2N(CH_3)_2$ | H | $CH_3$ | $OCH_3$ | $CH_3$ | N |

Table 18

General Formula 18

| $Z^1$ | $R^2$ | $R^4$ | R | X | Y | $Z^4$ |
|---|---|---|---|---|---|---|
| CH | H | H | H | $OCH_3$ | $OCH_3$ | CH |
| CH | H | H | H | $OCH_3$ | $CH_3$ | CH |
| CH | H | H | H | Cl | $OCH_3$ | CH |
| CH | H | H | H | $CH_3$ | $CH_3$ | CH |
| CH | H | H | H | $OCH_3$ | $OCH_3$ | N |
| CH | H | H | H | $OCH_3$ | $CH_3$ | N |
| CH | H | H | H | $OC_2H_5$ | $NHCH_3$ | N |
| CH | H | H | $CH_3$ | $OCH_3$ | $CH_3$ | N |
| CH | $CH_3$ | H | H | $OCH_3$ | $OCH_3$ | CH |
| CH | $CH_3$ | H | H | $OCH_3$ | $CH_3$ | CH |
| CH | $CH_3$ | H | H | Cl | $OCH_3$ | CH |
| CH | $CH_3$ | H | H | $CH_3$ | $CH_3$ | CH |
| CH | $CH_3$ | H | H | $OCH_3$ | $OCH_3$ | N |
| CH | $CH_3$ | H | H | $OCH_3$ | $CH_3$ | N |
| CH | $CH_3$ | H | H | $OC_2H_5$ | $NHCH_3$ | N |
| CH | $CH_3$ | H | $CH_3$ | $OCH_3$ | $CH_3$ | N |
| CH | $OCH_3$ | H | H | $OCH_3$ | $OCH_3$ | CH |
| CH | $OCH_3$ | H | H | $OCH_3$ | $CH_3$ | CH |
| CH | $OCH_3$ | H | H | Cl | $OCH_3$ | CH |
| CH | $OCH_3$ | H | H | $CH_3$ | $CH_3$ | CH |
| CH | $OCH_3$ | H | H | $OCH_3$ | $OCH_3$ | N |
| CH | OCH | H | H | $OCH_3$ | $CH_3$ | N |
| CH | $OCH_3$ | H | H | $OC_2H_5$ | $NHCH_3$ | N |
| CH | $OCH_3$ | H | $CH_3$ | $OCH_3$ | $CH_3$ | N |

Table 18 (continued)

General Formula 18

| $Z^1$ | $R^2$ | $R^4$ | R | X | Y | $Z^4$ |
|---|---|---|---|---|---|---|
| N | H | H | H | $OCH_3$ | $OCH_3$ | CH |
| N | H | H | H | $OCH_3$ | $CH_3$ | CH |
| N | H | H | H | Cl | $OCH_3$ | CH |
| N | H | H | H | $CH_3$ | $CH_3$ | CH |
| N | H | H | H | $OCH_3$ | $OCH_3$ | N |
| N | H | H | H | $OCH_3$ | $CH_3$ | N |
| N | H | H | H | $OC_2H_5$ | $NHCH_3$ | N |
| N | H | H | $CH_3$ | $OCH_3$ | $CH_3$ | N |
| N | $CH_3$ | H | H | $OCH_3$ | $OCH_3$ | CH |
| N | $CH_3$ | H | H | $OCH_3$ | $CH_3$ | CH |
| N | $CH_3$ | H | H | Cl | $OCH_3$ | CH |
| N | $CH_3$ | H | H | $CH_3$ | $CH_3$ | CH |
| N | $CH_3$ | H | H | $OCH_3$ | $OCH_3$ | N |
| N | $CH_3$ | H | H | $OCH_3$ | $CH_3$ | N |
| N | $CH_3$ | H | H | $OC_2H_5$ | $NHCH_3$ | N |
| N | $CH_3$ | H | $CH_3$ | $OCH_3$ | $CH_3$ | N |
| N | $OCH_3$ | H | H | $OCH_3$ | $OCH_3$ | CH |
| N | $OCH_3$ | H | H | $OCH_3$ | $CH_3$ | CH |
| N | $OCH_3$ | H | H | Cl | $OCH_3$ | CH |
| N | $OCH_3$ | H | H | $CH_3$ | $CH_3$ | CH |
| N | $OCH_3$ | H | H | $OCH_3$ | $OCH_3$ | N |
| N | $OCH_3$ | H | H | $OCH_3$ | $CH_3$ | N |
| N | $OCH_3$ | H | H | $OC_2H_5$ | $NHCH_3$ | N |
| N | $OCH_3$ | H | $CH_3$ | $OCH_3$ | $CH_3$ | N |

Table 19

General Formula 19

| Z | $Z^2$ | $R^2$ | $R^3$ | R | X | Y | $Z^4$ |
|---|---|---|---|---|---|---|---|
| CH | O | $CH_3$ | $CH_3$ | H | $OCH_3$ | $OCH_3$ | CH |
| CH | O | $CH_3$ | $CH_3$ | H | $OCH_3$ | $CH_3$ | CH |
| CH | O | $CH_3$ | $CH_3$ | H | Cl | $OCH_3$ | CH |
| CH | O | $CH_3$ | $CH_3$ | H | $CH_3$ | $CH_3$ | CH |
| CH | O | $CH_3$ | $CH_3$ | H | $OCH_3$ | $OCH_3$ | N |
| CH | O | $CH_3$ | $CH_3$ | H | $OCH_3$ | $CH_3$ | N |
| CH | O | $CH_3$ | $CH_3$ | H | $OC_2H_5$ | $NHCH_3$ | N |
| CH | O | $CH_3$ | $CH_3$ | $CH_3$ | $OCH_3$ | $CH_3$ | N |
| N | O | $CH_3$ | $CH_3$ | H | $OCH_3$ | $OCH_3$ | CH |
| N | O | $CH_3$ | $CH_3$ | H | $OCH_3$ | $CH_3$ | CH |
| N | O | $CH_3$ | $CH_3$ | H | Cl | $OCH_3$ | CH |
| N | O | $CH_3$ | $CH_3$ | H | $CH_3$ | $CH_3$ | CH |
| N | O | $CH_3$ | $CH_3$ | H | $OCH_3$ | $OCH_3$ | N |
| N | O | $CH_3$ | $CH_3$ | H | $OCH_3$ | $CH_3$ | N |
| N | O | $CH_3$ | $CH_3$ | H | $OC_2H_5$ | $NHCH_3$ | N |
| N | O | $CH_3$ | $CH_3$ | $CH_3$ | $OCH_3$ | $CH_3$ | N |
| CH | S | $CH_3$ | $CH_3$ | H | $OCH_3$ | $OCH_3$ | CH |
| CH | S | $CH_3$ | $CH_3$ | H | $OCH_3$ | $CH_3$ | CH |
| CH | S | $CH_3$ | $CH_3$ | H | Cl | $OCH_3$ | CH |
| CH | S | $CH_3$ | $CH_3$ | H | $CH_3$ | $CH_3$ | CH |
| CH | S | $CH_3$ | $CH_3$ | H | $OCH_3$ | $OCH_3$ | N |
| CH | S | $CH_3$ | $CH_3$ | H | $OCH_3$ | $CH_3$ | N |
| CH | S | $CH_3$ | $CH_3$ | H | $OC_2H_5$ | $NHCH_3$ | N |
| CH | S | $CH_3$ | $CH_3$ | $CH_3$ | $OCH_3$ | $CH_3$ | N |

Table 19 (continued)

General Formula 19

| Z | $Z^2$ | $R^2$ | $R^3$ | R | X | Y | $Z^1$ |
|---|---|---|---|---|---|---|---|
| CH | O | $CH_3$ | H | H | $OCH_3$ | $OCH_3$ | CH |
| CH | O | $CH_3$ | H | H | $OCH_3$ | $CH_3$ | CH |
| CH | O | $CH_3$ | H | H | Cl | $OCH_3$ | CH |
| CH | O | $CH_3$ | H | H | $CH_3$ | $CH_3$ | CH |
| CH | O | $CH_3$ | H | H | $OCH_3$ | $OCH_3$ | N |
| CH | O | $CH_3$ | H | H | $OCH_3$ | $CH_3$ | N |
| CH | O | $CH_3$ | H | H | $OC_2H_5$ | $NHCH_3$ | N |
| CH | O | $CH_3$ | H | $CH_3$ | $OCH_3$ | $CH_3$ | N |
| N | O | $CH_3$ | H | H | $OCH_3$ | $OCH_3$ | CH |
| N | O | $CH_3$ | H | H | $OCH_3$ | $CH_3$ | CH |
| N | O | $CH_3$ | H | H | Cl | $OCH_3$ | CH |
| N | O | $CH_3$ | H | H | $CH_3$ | $CH_3$ | CH |
| N | O | $CH_3$ | H | H | $OCH_3$ | $OCH_3$ | N |
| N | O | $CH_3$ | H | H | $OCH_3$ | $CH_3$ | N |
| N | O | $CH_3$ | H | H | $OC_2H_5$ | $NHCH_3$ | N |
| N | O | $CH_3$ | H | $CH_3$ | $OCH_3$ | $CH_3$ | N |
| CH | S | $CH_3$ | H | H | $OCH_3$ | $OCH_3$ | CH |
| CH | S | $CH_3$ | H | H | $OCH_3$ | $CH_3$ | CH |
| CH | S | $CH_3$ | H | H | Cl | $OCH_3$ | CH |
| CH | S | $CH_3$ | H | H | $CH_3$ | $CH_3$ | CH |
| CH | S | $CH_3$ | H | H | $OCH_3$ | $OCH_3$ | N |
| CH | S | $CH_3$ | H | H | $OCH_3$ | $CH_3$ | N |
| CH | S | $CH_3$ | H | H | $OC_2H_5$ | $NHCH_3$ | N |
| CH | S | $CH_3$ | H | $CH_3$ | $OCH_3$ | $CH_3$ | N |

Table 19 (continued)

General Formula 19

| Z | $Z^2$ | $R^2$ | $R^3$ | R | X | Y | $Z^4$ |
|---|---|---|---|---|---|---|---|
| CH | O | H | $CH_3$ | H | $OCH_3$ | $OCH_3$ | CH |
| CH | O | H | $CH_3$ | H | $OCH_3$ | $CH_3$ | CH |
| CH | O | H | $CH_3$ | H | Cl | $OCH_3$ | CH |
| CH | O | H | $CH_3$ | H | $CH_3$ | $CH_3$ | CH |
| CH | O | H | $CH_3$ | H | $OCH_3$ | $OCH_3$ | N |
| CH | O | H | $CH_3$ | H | $OCH_3$ | $CH_3$ | N |
| CH | O | H | $CH_3$ | H | $OC_2H_5$ | $NHCH_3$ | N |
| CH | O | H | $CH_3$ | $CH_3$ | $OCH_3$ | $CH_3$ | N |
| N | O | H | $CH_3$ | H | $OCH_3$ | $OCH_3$ | CH |
| N | O | H | $CH_3$ | H | $OCH_3$ | $CH_3$ | CH |
| N | O | H | $CH_3$ | H | Cl | $OCH_3$ | CH |
| N | O | H | $CH_3$ | H | $CH_3$ | $CH_3$ | CH |
| N | O | H | $CH_3$ | H | $OCH_3$ | $OCH_3$ | N |
| N | O | H | $CH_3$ | H | $OCH_3$ | $CH_3$ | N |
| N | O | H | $CH_3$ | H | $OC_2H_5$ | $NHCH_3$ | N |
| N | O | H | $CH_3$ | $CH_3$ | $OCH_3$ | $CH_3$ | N |
| CH | S | H | $CH_3$ | H | $OCH_3$ | $OCH_3$ | CH |
| CH | S | H | $CH_3$ | H | $OCH_3$ | $CH_3$ | CH |
| CH | S | H | $CH_3$ | H | Cl | $OCH_3$ | CH |
| CH | S | H | $CH_3$ | H | $CH_3$ | $CH_3$ | CH |
| CH | S | H | $CH_3$ | H | $OCH_3$ | $OCH_3$ | N |
| CH | S | H | $CH_3$ | H | $OCH_3$ | $CH_3$ | N |
| CH | S | H | $CH_3$ | H | $OC_2H_5$ | $NHCH_3$ | N |
| CH | S | H | $CH_3$ | $CH_3$ | $OCH_3$ | $CH_3$ | N |

Table 19 (continued)

General Formula 19

| Z | $Z^2$ | $R^2$ | $R^3$ | R | X | Y | $Z^4$ |
|---|---|---|---|---|---|---|---|
| CH | O | H | H | H | $OCH_3$ | $OCH_3$ | CH |
| CH | O | H | H | H | $OCH_3$ | $CH_3$ | CH |
| CH | O | H | H | H | Cl | $OCH_3$ | CH |
| CH | O | H | H | H | $CH_3$ | $CH_3$ | CH |
| CH | O | H | H | H | $OCH_3$ | $OCH_3$ | N |
| CH | O | H | H | H | $OCH_3$ | $CH_3$ | N |
| CH | O | H | H | H | $OC_2H_5$ | $NHCH_3$ | N |
| CH | O | H | H | $CH_3$ | $OCH_3$ | $CH_3$ | N |
| N | O | H | H | H | $OCH_3$ | $OCH_3$ | CH |
| N | O | H | H | H | $OCH_3$ | $CH_3$ | CH |
| N | O | H | H | H | Cl | $OCH_3$ | CH |
| N | O | H | H | H | $CH_3$ | $CH_3$ | CH |
| N | O | H | H | H | $OCH_3$ | $OCH_3$ | N |
| N | O | H | H | H | $OCH_3$ | $CH_3$ | N |
| N | O | H | H | H | $OC_2H_5$ | $NHCH_3$ | N |
| N | O | H | H | $CH_3$ | $OCH_3$ | $CH_3$ | N |
| CH | S | H | H | H | $OCH_3$ | $OCH_3$ | CH |
| CH | S | H | H | H | $OCH_3$ | $CH_3$ | CH |
| CH | S | H | H | H | Cl | $OCH_3$ | CH |
| CH | S | H | H | H | $CH_3$ | $CH_3$ | CH |
| CH | S | H | H | H | $OCH_3$ | $OCH_3$ | N |
| CH | S | H | H | H | $OCH_3$ | $CH_3$ | N |
| CH | S | H | H | H | $OC_2H_5$ | $NHCH_3$ | N |
| CH | S | H | H | $CH_3$ | $OCH_3$ | $CH_3$ | N |

Table 19 (continued)

General Formula 19

| Z | $Z^2$ | $R^2$ | $R^3$ | R | X | Y | $Z^4$ |
|---|---|---|---|---|---|---|---|
| N | S | $CH_3$ | $CH_3$ | H | $OCH_3$ | $OCH_3$ | CH |
| N | S | $CH_3$ | $CH_3$ | H | $OCH_3$ | $CH_3$ | CH |
| N | S | $CH_3$ | $CH_3$ | H | Cl | $OCH_3$ | CH |
| N | S | $CH_3$ | $CH_3$ | H | $CH_3$ | $CH_3$ | CH |
| N | S | $CH_3$ | $CH_3$ | H | $OCH_3$ | $OCH_3$ | N |
| N | S | $CH_3$ | $CH_3$ | H | $OCH_3$ | $CH_3$ | N |
| N | S | $CH_3$ | $CH_3$ | H | $OC_2H_5$ | $NHCH_3$ | N |
| N | S | $CH_3$ | $CH_3$ | $CH_3$ | $OCH_3$ | $CH_3$ | N |
| CH | $NCH_3$ | $CH_3$ | $CH_3$ | H | $OCH_3$ | $OCH_3$ | CH |
| CH | $NCH_3$ | $CH_3$ | $CH_3$ | H | $OCH_3$ | $CH_3$ | CH |
| CH | $NCH_3$ | $CH_3$ | $CH_3$ | H | Cl | $OCH_3$ | CH |
| CH | $NCH_3$ | $CH_3$ | $CH_3$ | H | $CH_3$ | $CH_3$ | CH |
| CH | $NCH_3$ | $CH_3$ | $CH_3$ | H | $OCH_3$ | $OCH_3$ | N |
| CH | $NCH_3$ | $CH_3$ | $CH_3$ | H | $OCH_3$ | $CH_3$ | N |
| CH | $NCH_3$ | $CH_3$ | $CH_3$ | H | $OC_2H_5$ | $NHCH_3$ | N |
| CH | $NCH_3$ | $CH_3$ | $CH_3$ | $CH_3$ | $OCH_3$ | $CH_3$ | N |
| N | $NCH_3$ | $CH_3$ | $CH_3$ | H | $OCH_3$ | $OCH_3$ | CH |
| N | $NCH_3$ | $CH_3$ | $CH_3$ | H | $OCH_3$ | $CH_3$ | CH |
| N | $NCH_3$ | $CH_3$ | $CH_3$ | H | Cl | $OCH_3$ | CH |
| N | $NCH_3$ | $CH_3$ | $CH_3$ | H | $CH_3$ | $CH_3$ | CH |
| N | $NCH_3$ | $CH_3$ | $CH_3$ | H | $OCH_3$ | $OCH_3$ | N |
| N | $NCH_3$ | $CH_3$ | $CH_3$ | H | $OCH_3$ | $CH_3$ | N |
| N | $NCH_3$ | $CH_3$ | $CH_3$ | H | $OC_2H_5$ | $NHCH_3$ | N |
| N | $NCH_3$ | $CH_3$ | $CH_3$ | $CH_3$ | $OCH_3$ | $CH_3$ | N |

Table 19 (continued)

General Formula 19

| Z | $Z^2$ | $R^2$ | $R^3$ | R | X | Y | $Z^4$ |
|---|---|---|---|---|---|---|---|
| N | S | $CH_3$ | H | H | $OCH_3$ | $OCH_3$ | CH |
| N | S | $CH_3$ | H | H | $OCH_3$ | $CH_3$ | CH |
| N | S | $CH_3$ | H | H | Cl | $OCH_3$ | CH |
| N | S | $CH_3$ | H | H | $CH_3$ | $CH_3$ | CH |
| N | S | $CH_3$ | H | H | $OCH_3$ | $OCH_3$ | N |
| N | S | $CH_3$ | H | H | $OCH_3$ | $CH_3$ | N |
| N | S | $CH_3$ | H | H | $OC_2H_5$ | $NHCH_3$ | N |
| N | S | $CH_3$ | H | $CH_3$ | $OCH_3$ | $CH_3$ | N |
| CH | $NCH_3$ | $CH_3$ | H | H | $OCH_3$ | $OCH_3$ | CH |
| CH | $NCH_3$ | $CH_3$ | H | H | $OCH_3$ | $CH_3$ | CH |
| CH | $NCH_3$ | $CH_3$ | H | H | Cl | $OCH_3$ | CH |
| CH | $NCH_3$ | $CH_3$ | H | H | $CH_3$ | $CH_3$ | CH |
| CH | $NCH_3$ | $CH_3$ | H | H | $OCH_3$ | $OCH_3$ | N |
| CH | $NCH_3$ | $CH_3$ | H | H | $OCH_3$ | $CH_3$ | N |
| CH | $NCH_3$ | $CH_3$ | H | H | $OC_2H_5$ | $NHCH_3$ | N |
| CH | $NCH_3$ | $CH_3$ | H | $CH_3$ | $OCH_3$ | $CH_3$ | N |
| N | $NCH_3$ | $CH_3$ | H | H | $OCH_3$ | $OCH_3$ | CH |
| N | $NCH_3$ | $CH_3$ | H | H | $OCH_3$ | $CH_3$ | CH |
| N | $NCH_3$ | $CH_3$ | H | H | Cl | $OCH_3$ | CH |
| N | $NCH_3$ | $CH_3$ | H | H | $CH_3$ | $CH_3$ | CH |
| N | $NCH_3$ | $CH_3$ | H | H | $OCH_3$ | $OCH_3$ | N |
| N | $NCH_3$ | $CH_3$ | H | H | $OCH_3$ | $CH_3$ | N |
| N | $NCH_3$ | $CH_3$ | H | H | $OC_2H_5$ | $NHCH_3$ | N |
| N | $NCH_3$ | $CH_3$ | H | $CH_3$ | $OCH_3$ | $CH_3$ | N |

Table 19 (continued)

General Formula 19

| Z | $Z^2$ | $R^2$ | $R^3$ | R | X | Y | $Z^4$ |
|---|---|---|---|---|---|---|---|
| N | S | H | $CH_3$ | H | $OCH_3$ | $OCH_3$ | CH |
| N | S | H | $CH_3$ | H | $OCH_3$ | $CH_3$ | CH |
| N | S | H | $CH_3$ | H | Cl | $OCH_3$ | CH |
| N | S | H | $CH_3$ | H | $CH_3$ | $CH_3$ | CH |
| N | S | H | $CH_3$ | H | $OCH_3$ | $OCH_3$ | N |
| N | S | H | $CH_3$ | H | $OCH_3$ | $CH_3$ | N |
| N | S | H | $CH_3$ | H | $OC_2H_5$ | $NHCH_3$ | N |
| N | S | H | $CH_3$ | $CH_3$ | $OCH_3$ | $CH_3$ | N |
| CH | $NCH_3$ | H | $CH_3$ | H | $OCH_3$ | $OCH_3$ | CH |
| CH | $NCH_3$ | H | $CH_3$ | H | $OCH_3$ | $CH_3$ | CH |
| CH | $NCH_3$ | H | $CH_3$ | H | Cl | $OCH_3$ | CH |
| CH | $NCH_3$ | H | $CH_3$ | H | $CH_3$ | $CH_3$ | CH |
| CH | $NCH_3$ | H | $CH_3$ | H | $OCH_3$ | $OCH_3$ | N |
| CH | $NCH_3$ | H | $CH_3$ | H | $OCH_3$ | $CH_3$ | N |
| CH | $NCH_3$ | H | $CH_3$ | H | $OC_2H_5$ | $NHCH_3$ | N |
| CH | $NCH_3$ | H | $CH_3$ | $CH_3$ | $OCH_3$ | $CH_3$ | N |
| N | $NCH_3$ | H | $CH_3$ | H | $OCH_3$ | $OCH_3$ | CH |
| N | $NCH_3$ | H | $CH_3$ | H | $OCH_3$ | $CH_3$ | CH |
| N | $NCH_3$ | H | $CH_3$ | H | Cl | $OCH_3$ | CH |
| N | $NCH_3$ | H | $CH_3$ | H | $CH_3$ | $CH_3$ | CH |
| N | $NCH_3$ | H | $CH_3$ | H | $OCH_3$ | $OCH_3$ | N |
| N | $NCH_3$ | H | $CH_3$ | H | $OCH_3$ | $CH_3$ | N |
| N | $NCH_3$ | H | $CH_3$ | H | $OC_2H_5$ | $NHCH_3$ | N |
| N | $NCH_3$ | H | $CH_3$ | $CH_3$ | $OCH_3$ | $CH_3$ | N |

Table 19 (continued)

General Formula 19

| Z | $Z^2$ | $R^2$ | $R^3$ | R | X | Y | $Z^4$ |
|---|---|---|---|---|---|---|---|
| N | S | H | H | H | $OCH_3$ | $OCH_3$ | CH |
| N | S | H | H | H | $OCH_3$ | $CH_3$ | CH |
| N | S | H | H | H | Cl | $OCH_3$ | CH |
| N | S | H | H | H | $CH_3$ | $CH_3$ | CH |
| N | S | H | H | H | $OCH_3$ | $OCH_3$ | N |
| N | S | H | H | H | $OCH_3$ | $CH_3$ | N |
| N | S | H | H | H | $OC_2H_5$ | $NHCH_3$ | N |
| N | S | H | H | $CH_3$ | $OCH_3$ | $CH_3$ | N |
| CH | $NCH_3$ | H | H | H | $OCH_3$ | $OCH_3$ | CH |
| CH | $NCH_3$ | H | H | H | $OCH_3$ | $CH_3$ | CH |
| CH | $NCH_3$ | H | H | H | Cl | $OCH_3$ | CH |
| CH | $NCH_3$ | H | H | H | $CH_3$ | $CH_3$ | CH |
| CH | $NCH_3$ | H | H | H | $OCH_3$ | $OCH_3$ | N |
| CH | $NCH_3$ | H | H | H | $OCH_3$ | $CH_3$ | N |
| CH | $NCH_3$ | H | H | H | $OC_2H_5$ | $NHCH_3$ | N |
| CH | $NCH_3$ | H | H | $CH_3$ | $OCH_3$ | $CH_3$ | N |
| N | $NCH_3$ | H | H | H | $OCH_3$ | $OCH_3$ | CH |
| N | $NCH_3$ | H | H | H | $OCH_3$ | $CH_3$ | CH |
| N | $NCH_3$ | H | H | H | Cl | $OCH_3$ | CH |
| N | $NCH_3$ | H | H | H | $CH_3$ | $CH_3$ | CH |
| N | $NCH_3$ | H | H | H | $OCH_3$ | $OCH_3$ | N |
| N | $NCH_3$ | H | H | H | $OCH_3$ | $CH_3$ | N |
| N | $NCH_3$ | H | H | H | $OC_2H_5$ | $NHCH_3$ | N |
| N | $NCH_3$ | H | H | $CH_3$ | $OCH_3$ | $CH_3$ | N |

Table 20

General Formula 20

| Z | $Z^3$ | $R^2$ | $R^3$ | R | X | Y | $Z^4$ |
|---|---|---|---|---|---|---|---|
| CH | O | $CH_3$ | $CH_3$ | H | $OCH_3$ | $OCH_3$ | CH |
| CH | O | $CH_3$ | $CH_3$ | H | $OCH_3$ | $CH_3$ | CH |
| CH | O | $CH_3$ | $CH_3$ | H | Cl | $OCH_3$ | CH |
| CH | O | $CH_3$ | $CH_3$ | H | $CH_3$ | $CH_3$ | CH |
| CH | O | $CH_3$ | $CH_3$ | H | $OCH_3$ | $OCH_3$ | N |
| CH | O | $CH_3$ | $CH_3$ | H | $OCH_3$ | $CH_3$ | N |
| CH | O | $CH_3$ | $CH_3$ | H | $OC_2H_5$ | $NHCH_3$ | N |
| CH | O | $CH_3$ | $CH_3$ | $CH_3$ | $OCH_3$ | $CH_3$ | N |
| N | O | $CH_3$ | $CH_3$ | H | $OCH_3$ | $OCH_3$ | CH |
| N | O | $CH_3$ | $CH_3$ | H | $OCH_3$ | $CH_3$ | CH |
| N | O | $CH_3$ | $CH_3$ | H | Cl | $OCH_3$ | CH |
| N | O | $CH_3$ | $CH_3$ | H | $CH_3$ | $CH_3$ | CH |
| N | O | $CH_3$ | $CH_3$ | H | $OCH_3$ | $OCH_3$ | N |
| N | O | $CH_3$ | $CH_3$ | H | $OCH_3$ | $CH_3$ | N |
| N | O | $CH_3$ | $CH_3$ | H | $OC_2H_5$ | $NHCH_3$ | N |
| N | O | $CH_3$ | $CH_3$ | $CH_3$ | $OCH_3$ | $CH_3$ | N |
| CH | S | $CH_3$ | $CH_3$ | H | $OCH_3$ | $OCH_3$ | CH |
| CH | S | $CH_3$ | $CH_3$ | H | $OCH_3$ | $CH_3$ | CH |
| CH | S | $CH_3$ | $CH_3$ | H | Cl | $OCH_3$ | CH |
| CH | S | $CH_3$ | $CH_3$ | H | $CH_3$ | $CH_3$ | CH |
| CH | S | $CH_3$ | $CH_3$ | H | $OCH_3$ | $OCH_3$ | N |
| CH | S | $CH_3$ | $CH_3$ | H | $OCH_3$ | $CH_3$ | N |
| CH | S | $CH_3$ | $CH_3$ | H | $OC_2H_5$ | $NHCH_3$ | N |
| CH | S | $CH_3$ | $CH_3$ | $CH_3$ | $OCH_3$ | $CH_3$ | N |

Table 20 (continued)

General Formula 20

| Z | $Z^3$ | $R^2$ | $R^3$ | R | X | Y | $Z^4$ |
|---|---|---|---|---|---|---|---|
| CH | O | $CH_3$ | H | H | $OCH_3$ | $OCH_3$ | CH |
| CH | O | $CH_3$ | H | H | $OCH_3$ | $CH_3$ | CH |
| CH | O | $CH_3$ | H | H | Cl | $OCH_3$ | CH |
| CH | O | $CH_3$ | H | H | $CH_3$ | $CH_3$ | CH |
| CH | O | $CH_3$ | H | H | $OCH_3$ | $OCH_3$ | N |
| CH | O | $CH_3$ | H | H | $OCH_3$ | $CH_3$ | N |
| CH | O | $CH_3$ | H | H | $OC_2H_5$ | $NHCH_3$ | N |
| CH | O | $CH_3$ | H | $CH_3$ | $OCH_3$ | $CH_3$ | N |
| N | O | $CH_3$ | H | H | $OCH_3$ | $OCH_3$ | CH |
| N | O | $CH_3$ | H | H | $OCH_3$ | $CH_3$ | CH |
| N | O | $CH_3$ | H | H | Cl | $OCH_3$ | CH |
| N | O | $CH_3$ | H | H | $CH_3$ | $CH_3$ | CH |
| N | O | $CH_3$ | H | H | $OCH_3$ | $OCH_3$ | N |
| N | O | $CH_3$ | H | H | $OCH_3$ | $CH_3$ | N |
| N | O | $CH_3$ | H | H | $OC_2H_5$ | $NHCH_3$ | N |
| N | O | $CH_3$ | H | $CH_3$ | $OCH_3$ | $CH_3$ | N |
| CH | S | $CH_3$ | H | H | $OCH_3$ | $OCH_3$ | CH |
| CH | S | $CH_3$ | H | H | $OCH_3$ | $CH_3$ | CH |
| CH | S | $CH_3$ | H | H | Cl | $OCH_3$ | CH |
| CH | S | $CH_3$ | H | H | $CH_3$ | $CH_3$ | CH |
| CH | S | $CH_3$ | H | H | $OCH_3$ | $OCH_3$ | N |
| CH | S | $CH_3$ | H | H | $OCH_3$ | $CH_3$ | N |
| CH | S | $CH_3$ | H | H | $OC_2H_5$ | $NHCH_3$ | N |
| CH | S | $CH_3$ | H | $CH_3$ | $OCH_3$ | $CH_3$ | N |

Table 20 (continued)

General Formula 20

| Z | $Z^3$ | $R^2$ | $R^3$ | R | X | Y | $Z^4$ |
|---|---|---|---|---|---|---|---|
| CH | O | H | $CH_3$ | H | $OCH_3$ | $OCH_3$ | CH |
| CH | O | H | $CH_3$ | H | $OCH_3$ | $CH_3$ | CH |
| CH | O | H | $CH_3$ | H | Cl | $OCH_3$ | CH |
| CH | O | H | $CH_3$ | H | $CH_3$ | $CH_3$ | CH |
| CH | O | H | $CH_3$ | H | $OCH_3$ | $OCH_3$ | N |
| CH | O | H | $CH_3$ | H | $OCH_3$ | $CH_3$ | N |
| CH | O | H | $CH_3$ | H | $OC_2H_5$ | $NHCH_3$ | N |
| CH | O | H | $CH_3$ | $CH_3$ | $OCH_3$ | $CH_3$ | N |
| N | O | H | $CH_3$ | H | $OCH_3$ | $OCH_3$ | CH |
| N | O | H | $CH_3$ | H | $OCH_3$ | $CH_3$ | CH |
| N | O | H | $CH_3$ | H | Cl | $OCH_3$ | CH |
| N | O | H | $CH_3$ | H | $CH_3$ | $CH_3$ | CH |
| N | O | H | $CH_3$ | H | $OCH_3$ | $OCH_3$ | N |
| N | O | H | $CH_3$ | H | $OCH_3$ | $CH_3$ | N |
| N | O | H | $CH_3$ | H | $OC_2H_5$ | $NHCH_3$ | N |
| N | O | H | $CH_3$ | $CH_3$ | $OCH_3$ | $CH_3$ | N |
| CH | S | H | $CH_3$ | H | $OCH_3$ | $OCH_3$ | CH |
| CH | S | H | $CH_3$ | H | $OCH_3$ | $CH_3$ | CH |
| CH | S | H | $CH_3$ | H | Cl | $OCH_3$ | CH |
| CH | S | H | $CH_3$ | H | $CH_3$ | $CH_3$ | CH |
| CH | S | H | $CH_3$ | H | $OCH_3$ | $OCH_3$ | N |
| CH | S | H | $CH_3$ | H | $OCH_3$ | $CH_3$ | N |
| CH | S | H | $CH_3$ | H | $OC_2H_5$ | $NHCH_3$ | N |
| CH | S | H | $CH_3$ | $CH_3$ | $OCH_3$ | $CH_3$ | N |

Table 20 (continued)

General Formula 20

| Z | $Z^3$ | $R^2$ | $R^3$ | R | X | Y | $Z^4$ |
|---|---|---|---|---|---|---|---|
| CH | O | H | H | H | $OCH_3$ | $OCH_3$ | CH |
| CH | O | H | H | H | $OCH_3$ | $CH_3$ | CH |
| CH | O | H | H | H | Cl | $OCH_3$ | CH |
| CH | O | H | H | H | $CH_3$ | $CH_3$ | CH |
| CH | O | H | H | H | $OCH_3$ | $OCH_3$ | N |
| CH | O | H | H | H | $OCH_3$ | $CH_3$ | N |
| CH | O | H | H | H | $OC_2H_5$ | $NHCH_3$ | N |
| CH | O | H | H | $CH_3$ | $OCH_3$ | $CH_3$ | N |
| N | O | H | H | H | $OCH_3$ | $OCH_3$ | CH |
| N | O | H | H | H | $OCH_3$ | $CH_3$ | CH |
| N | O | H | H | H | Cl | $OCH_3$ | CH |
| N | O | H | H | H | $CH_3$ | $CH_3$ | CH |
| N | O | H | H | H | $OCH_3$ | $OCH_3$ | N |
| N | O | H | H | H | $OCH_3$ | $CH_3$ | N |
| N | O | H | H | H | $OC_2H_5$ | $NHCH_3$ | N |
| N | O | H | H | $CH_3$ | $OCH_3$ | $CH_3$ | N |
| CH | S | H | H | H | $OCH_3$ | $OCH_3$ | CH |
| CH | S | H | H | H | $OCH_3$ | $CH_3$ | CH |
| CH | S | H | H | H | Cl | $OCH_3$ | CH |
| CH | S | H | H | H | $CH_3$ | $CH_3$ | CH |
| CH | S | H | H | H | $OCH_3$ | $OCH_3$ | N |
| CH | S | H | H | H | $OCH_3$ | $CH_3$ | N |
| CH | S | H | H | H | $OC_2H_5$ | $NHCH_3$ | N |
| CH | S | H | H | $CH_3$ | $OCH_3$ | $CH_3$ | N |

Table 20 (continued)

General Formula 20

| Z | $Z^3$ | $R^2$ | $R^3$ | R | X | Y | $Z^4$ |
|---|---|---|---|---|---|---|---|
| N | S | $CH_3$ | $CH_3$ | H | $OCH_3$ | $OCH_3$ | CH |
| N | S | $CH_3$ | $CH_3$ | H | $OCH_3$ | $CH_3$ | CH |
| N | S | $CH_3$ | $CH_3$ | H | Cl | $OCH_3$ | CH |
| N | S | $CH_3$ | $CH_3$ | H | $CH_3$ | $CH_3$ | CH |
| N | S | $CH_3$ | $CH_3$ | H | $OCH_3$ | $OCH_3$ | N |
| N | S | $CH_3$ | $CH_3$ | H | $OCH_3$ | $CH_3$ | N |
| N | S | $CH_3$ | $CH_3$ | H | $OC_2H_5$ | $NHCH_3$ | N |
| N | S | $CH_3$ | $CH_3$ | $CH_3$ | $OCH_3$ | $CH_3$ | N |
| CH | $NCH_3$ | $CH_3$ | $CH_3$ | H | $OCH_3$ | $OCH_3$ | CH |
| CH | $NCH_3$ | $CH_3$ | $CH_3$ | H | $OCH_3$ | $CH_3$ | CH |
| CH | $NCH_3$ | $CH_3$ | $CH_3$ | H | Cl | $OCH_3$ | CH |
| CH | $NCH_3$ | $CH_3$ | $CH_3$ | H | $CH_3$ | $CH_3$ | CH |
| CH | $NCH_3$ | $CH_3$ | $CH_3$ | H | $OCH_3$ | $OCH_3$ | N |
| CH | $NCH_3$ | $CH_3$ | $CH_3$ | H | $OCH_3$ | $CH_3$ | N |
| CH | $NCH_3$ | $CH_3$ | $CH_3$ | H | $OC_2H_5$ | $NHCH_3$ | N |
| CH | $NCH_3$ | $CH_3$ | $CH_3$ | $CH_3$ | $OCH_3$ | $CH_3$ | N |
| N | $NCH_3$ | $CH_3$ | $CH_3$ | H | $OCH_3$ | $OCH_3$ | CH |
| N | $NCH_3$ | $CH_3$ | $CH_3$ | H | $OCH_3$ | $CH_3$ | CH |
| N | $NCH_3$ | $CH_3$ | $CH_3$ | H | Cl | $OCH_3$ | CH |
| N | $NCH_3$ | $CH_3$ | $CH_3$ | H | $CH_3$ | $CH_3$ | CH |
| N | $NCH_3$ | $CH_3$ | $CH_3$ | H | $OCH_3$ | $OCH_3$ | N |
| N | $NCH_3$ | $CH_3$ | $CH_3$ | H | $OCH_3$ | $CH_3$ | N |
| N | $NCH_3$ | $CH_3$ | $CH_3$ | H | $OC_2H_5$ | $NHCH_3$ | N |
| N | $NCH_3$ | $CH_3$ | $CH_3$ | $CH_3$ | $OCH_3$ | $CH_3$ | N |

Table 20 (continued)

General Formula 20

| Z | $Z^3$ | $R^2$ | $R^3$ | R | X | Y | $Z^4$ |
|---|---|---|---|---|---|---|---|
| N | S | H | $CH_3$ | H | $OCH_3$ | $OCH_3$ | CH |
| N | S | H | $CH_3$ | H | $OCH_3$ | $CH_3$ | CH |
| N | S | H | $CH_3$ | H | Cl | $OCH_3$ | CH |
| N | S | H | $CH_3$ | H | $CH_3$ | $CH_3$ | CH |
| N | S | H | $CH_3$ | H | $OCH_3$ | $OCH_3$ | N |
| N | S | H | $CH_3$ | H | $OCH_3$ | $CH_3$ | N |
| N | S | H | $CH_3$ | H | $OC_2H_5$ | $NHCH_3$ | N |
| N | S | H | $CH_3$ | $CH_3$ | $OCH_3$ | $CH_3$ | N |
| CH | $NCH_3$ | H | $CH_3$ | H | $OCH_3$ | $OCH_3$ | CH |
| CH | $NCH_3$ | H | $CH_3$ | H | $OCH_3$ | $CH_3$ | CH |
| CH | $NCH_3$ | H | $CH_3$ | H | Cl | $OCH_3$ | CH |
| CH | $NCH_3$ | H | $CH_3$ | H | $CH_3$ | $CH_3$ | CH |
| CH | $NCH_3$ | H | $CH_3$ | H | $OCH_3$ | $OCH_3$ | N |
| CH | $NCH_3$ | H | $CH_3$ | H | $OCH_3$ | $CH_3$ | N |
| CH | $NCH_3$ | H | $CH_3$ | H | $OC_2H_5$ | $NHCH_3$ | N |
| CH | $NCH_3$ | H | $CH_3$ | $CH_3$ | $OCH_3$ | $CH_3$ | N |
| N | $NCH_3$ | H | $CH_3$ | H | $OCH_3$ | $OCH_3$ | CH |
| N | $NCH_3$ | H | $CH_3$ | H | $OCH_3$ | $CH_3$ | CH |
| N | $NCH_3$ | H | $CH_3$ | H | Cl | $OCH_3$ | CH |
| N | $NCH_3$ | H | $CH_3$ | H | $CH_3$ | $CH_3$ | CH |
| N | $NCH_3$ | H | $CH_3$ | H | $OCH_3$ | $OCH_3$ | N |
| N | $NCH_3$ | H | $CH_3$ | H | $OCH_3$ | $CH_3$ | N |
| N | $NCH_3$ | H | $CH_3$ | H | $OC_2H_5$ | $NHCH_3$ | N |
| N | $NCH_3$ | H | $CH_3$ | $CH_3$ | $OCH_3$ | $CH_3$ | N |

Table 20 (continued)

General Formula 20

| Z | $Z^3$ | $R^2$ | $R^3$ | R | X | Y | $Z^4$ |
|---|---|---|---|---|---|---|---|
| N | S | H | H | H | $OCH_3$ | $OCH_3$ | CH |
| N | S | H | H | H | $OCH_3$ | $CH_3$ | CH |
| N | S | H | H | H | Cl | $OCH_3$ | CH |
| N | S | H | H | H | $CH_3$ | $CH_3$ | CH |
| N | S | H | H | H | $OCH_3$ | $OCH_3$ | N |
| N | S | H | H | H | $OCH_3$ | $CH_3$ | N |
| N | S | H | H | H | $OC_2H_5$ | $NHCH_3$ | N |
| N | S | H | H | $CH_3$ | $OCH_3$ | $CH_3$ | N |
| CH | $NCH_3$ | H | H | H | $OCH_3$ | $OCH_3$ | CH |
| CH | $NCH_3$ | H | H | H | $OCH_3$ | $CH_3$ | CH |
| CH | $NCH_3$ | H | H | H | Cl | $OCH_3$ | CH |
| CH | $NCH_3$ | H | H | H | $CH_3$ | $CH_3$ | CH |
| CH | $NCH_3$ | H | H | H | $OCH_3$ | $OCH_3$ | N |
| CH | $NCH_3$ | H | H | H | $OCH_3$ | $CH_3$ | N |
| CH | $NCH_3$ | H | H | H | $OC_2H_5$ | $NHCH_3$ | N |
| CH | $NCH_3$ | H | H | $CH_3$ | $OCH_3$ | $CH_3$ | N |
| N | $NCH_3$ | H | H | H | $OCH_3$ | $OCH_3$ | CH |
| N | $NCH_3$ | H | H | H | $OCH_3$ | $CH_3$ | CH |
| N | $NCH_3$ | H | H | H | Cl | $OCH_3$ | CH |
| N | $NCH_3$ | H | H | H | $CH_3$ | $CH_3$ | CH |
| N | $NCH_3$ | H | H | H | $OCH_3$ | $OCH_3$ | N |
| N | $NCH_3$ | H | H | H | $OCH_3$ | $CH_3$ | N |
| N | $NCH_3$ | H | H | H | $OC_2H_5$ | $NHCH_3$ | N |
| N | $NCH_3$ | H | H | $CH_3$ | $OCH_3$ | $CH_3$ | N |

Table 20 (continued)

General Formula 20

| Z | $Z^3$ | $R^2$ | $R^3$ | R | X | Y | $Z^4$ |
|---|---|---|---|---|---|---|---|
| N | S | $CH_3$ | H | H | $OCH_3$ | $OCH_3$ | CH |
| N | S | $CH_3$ | H | H | $OCH_3$ | $CH_3$ | CH |
| N | S | $CH_3$ | H | H | Cl | $OCH_3$ | CH |
| N | S | $CH_3$ | H | H | $CH_3$ | $CH_3$ | CH |
| N | S | $CH_3$ | H | H | $OCH_3$ | $OCH_3$ | N |
| N | S | $CH_3$ | H | H | $OCH_3$ | $CH_3$ | N |
| N | S | $CH_3$ | H | H | $OC_2H_5$ | $NHCH_3$ | N |
| N | S | $CH_3$ | H | $CH_3$ | $OCH_3$ | $CH_3$ | N |
| CH | $NCH_3$ | $CH_3$ | H | H | $OCH_3$ | $OCH_3$ | CH |
| CH | $NCH_3$ | $CH_3$ | H | H | $OCH_3$ | $CH_3$ | CH |
| CH | $NCH_3$ | $CH_3$ | H | H | Cl | $OCH_3$ | CH |
| CH | $NCH_3$ | $CH_3$ | H | H | $CH_3$ | $CH_3$ | CH |
| CH | $NCH_3$ | $CH_3$ | H | H | $OCH_3$ | $OCH_3$ | N |
| CH | $NCH_3$ | $CH_3$ | H | H | $OCH_3$ | $CH_3$ | N |
| CH | $NCH_3$ | $CH_3$ | H | H | $OC_2H_5$ | $NHCH_3$ | N |
| CH | $NCH_3$ | $CH_3$ | H | $CH_3$ | $OCH_3$ | $CH_3$ | N |
| N | $NCH_3$ | $CH_3$ | H | H | $OCH_3$ | $OCH_3$ | CH |
| N | $NCH_3$ | $CH_3$ | H | H | $OCH_3$ | $CH_3$ | CH |
| N | $NCH_3$ | $CH_3$ | H | H | Cl | $OCH_3$ | CH |
| N | $NCH_3$ | $CH_3$ | H | H | $CH_3$ | $CH_3$ | CH |
| N | $NCH_3$ | $CH_3$ | H | H | $OCH_3$ | $OCH_3$ | N |
| N | $NCH_3$ | $CH_3$ | H | H | $OCH_3$ | $CH_3$ | N |
| N | $NCH_3$ | $CH_3$ | H | H | $OC_2H_5$ | $NHCH_3$ | N |
| N | $NCH_3$ | $CH_3$ | H | $CH_3$ | $OCH_3$ | $CH_3$ | N |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s). More specifically, they will contain these ingredients in the following approximate proportions:

| | Weight Percent* | | |
|---|---|---|---|
| | Active Ingredients | Diluent(s) | Surfactant(s) |
| Wettable powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant of a Diluent equals 100 weight percent.

Lower or higher levels of active ingredient can, of course, be present depending on the intended use of the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactered, may be used. The more absorptive diluents are preferred for wettable powders and the denser ones for dusts. Typical liquid diluents and solvents are described in Marsden, "Solvents Guide," 2nd Ed., Interscience, New York, 1985. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luchenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

## Example 3

Wettable Powder

N-[(4,6-dimethoxypyrimidin-2-yl)aminocarbonyl]-5,7-dimethylpyrazolo[1,5-A]pyrimidine-3-sulfonamide 80%
sodium alkylnaphthalenesulfonate 2%
sodium ligninsulfonate 2%
synthetic amorphous silica 3%
kaolinite 13%

The ingredients are blended, hammer-milled until all the solids are essentially under 50 microns, reblended, and packaged.

## Example 4

Wettable Powder

N-[(4,6-dimethoxypyrimidin-2-yl)aminocarbonyl]-5,7-dimethylpyrazolo[1,5-A]pyrimidine-3-sulfonamide 50%
sodium alkylnaphthalenesulfonate 2%
low viscosity methyl cellulose 2%
diatomaceous earth 46%

The ingredients are blended, coarsely hammer-milled and then air-milled to produce particles essentially all below 10 microns in diameter. The product is reblended before packaging.

## Example 5

Granule

Wettable Powder of Example 4 5%
attapulgite granules 95%
(U.S.S. 20-40 mesh; 0.84-0.42mm)

A slurry of wettable powder containing 25% solids is sprayed on the surfact of attapulgite granules in a double-cone blender. The granules are dried and packaged.

## Example 6

Extruded Pellet

N-[(4,6-dimethoxytriazin-2-yl)aminocarbonyl]-5,7-dimethylpyrazolo[1,5-A]pyrimidine-3-sulfonamide 25%
anhydrous sodium sulfate 10%
crude calcium ligninsulfonate 5%
sodium alkylnaphthalenesulfonate 1%
calcium/magnesium bentonite 59%

The ingredients are blended, hammer-milled and then moistened with about 12% water. The mixture is

extruded as cylinders about 3 mm diameter which are cut to produce pellets about 3 mm long. These may be used directly after drying, or the dried pellets may be crushed to pass a U.S.S. No. 20 sieve (0.84MM openings). The granules held on a U.S.S. No. 40 sieve (0.42 mm openings) may be packaged for use and the fines recycled.

## Example 7

### Oil Suspension

N-[(4, 6-dimethoxypyrimidin-2-yl)aminocarbonyl]-5,7-dimethylpyrazolo[1,5-A]pyrimidine-3-sulfonamide 25%
polyoxyethylene sorbitol hexaoleate · 5%
highly aliphatic hydrocarbon oil 70%

The ingredients are ground together in a sand mill until the solid particles have been reduced to under about 5 microns. The resulting thick suspension may be applied directly, but preferably after being extended with oils or emulsified in water.

## Example 8

### Wettable Powder

N-[(4,6-dimethoxytriazin-2-yl)aminocarbonyl]-5,7-dimethylpyrazolo[1,5-A]pyrimidine-3-sulfonamide 20%
sodium alkylnaphthalenesulfonate 4%
sodium ligninsulfonate 4%
low viscosity methyl cellulose 3%
attapulgite 69%

The ingredients are thoroughly blended. After grinding in a hammer-mill to produce particles essentially all below 100 microns, the material is reblended and sifted through a U.S.S. No. 50 sieve (0.3 mm opening) and packaged.

## Example 9

### Low Strength Granule

N-[(4,6-dimethoxypyrimidin-2-yl)aminocarbonyl]-5,7-dimethylpyrazolo[1,5-A]pyrimidine-3-sulfonamide 1%
N,N-dimethylformamide 9%
attapulgite granules 90%
(U.S.S. 20-2-40 sieve)

The active ingredient is dissolved in the solvent and the solution is sprayed upon dedusted granules in a double cone blender. After spraying of the solution has been completed, the blender is allowed to run for a short period and then the granules are packaged.

## Example 10

### Aqueous Suspension

N-[(4,6-dimethoxytriazin-2-yl)aminocarbonyl]-5,7-dimethylpyrazolo[1,5-A]pyrimidine-3-sulfonamide 40%
polyacrylic acid thickener 0.3%
dodecylphenol polyethylene glycol ether 0.5%
disodium phosphate 1%

monosodium phosphate 0.5%
polyvinyl alcohol 1.0%
water 56.7%

The ingredients are blended and ground together in a sand mill to produce particles essentially all under 5 microns in size.

Example 11

Solution

N-[(4,6-dimethoxypyrimidin-2-yl)aminocarbonyl]-5,7-dimethylpyrazolo[1,5-A]pyrimidine-3-sulfonamide 5%
water 95%

The salt is added directly to the water with stirring to produce the solution, which may then be package for use.

Example 12

Low Strength Granule

N-[(4,6-dimethoxytriazin-2-yl)aminocarbonyl]-5,7-dimethylpyrazolo[1,5-A]pyrimidine-3-sulfonamide 0.1%
attapulgite granules 99.9%
(U.S.S. 20-40 mesh)

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 13

Granule

N-[(4,6-dimethoxypyrimidin-2-yl)aminocarbonyl]-5,7-dimethylpyrazolo[1,5-A]pyrimidine-3-sulfonamide 80%
wetting agent 1%
crude ligninsulfonate salt (containing 5-20% of the natural sugars) 10%
attapulgite clay 9%

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 14

High Strength Concentrate

N-[(4,6-dimethoxytriazin-2-yl)aminocarbonyl]-5,7-dimethylpyrazolo[1,5-A]pyrimidine-3-sulfonamide 99%
silica aerogel 0.5%
synthetic amorphous silica 0.5%

The ingredients are blended and ground in a hammer-mill to produce a material essentially all passing a

U.S.S. No. 50 screen (0.3 mm opening). The concentrate may be formulated further if necessary.

Example 15

Wettable Powder

N-[(4,6-dimethoxypyrimidin-2-yl)aminocarbonyl]-5,7-dimethylpyrazolo[1,5-A]pyrimidine-3-sulfonamide 90%
dioctyl sodium sulfosuccinate 0.1%
synthetic fine silica 9.9%

The ingredients are blended and ground in a hammer-mill to produce particles essentially all below 100 microns. The material is sifted through a U.S.S. No. 50 screen and then packaged.

Example 16

Wettable Powder

N-[(4,6-dimethoxytriazin-2-yl)aminocarbonyl]-5,7-dimethylpyrazolo[1,5-A]pyrimidine-3-sulfonamide 40%
sodium ligninsulfonate 20%
montmorillonite clay 40%

The ingredients are thoroughly blended, coarsely hammer-milled and then air-milled to produce particles essentially all blow 10 microns in size. The material is reblended and then packaged.

Example 17

Oil Suspension

N-[(4,6-dimethoxypyrimidin-2-yl)aminocarbonyl]-5,7-dimethylpyrazolo[1,5-A]pyrimidine-3-sulfonamide 35%
blend of polyalcohol carboxylic 6%
esters and oil soluble petroleum sulfonates
xylene 59%

The ingredients are combined and ground together in a sand mill to produce particles essentially all below 5 microns. The product can be used directly, extended with oils, or emulsified in water.

Example 18

Dust

N-[(4,6-dimethoxytriazin-2-yl)aminocarbonyl]-5,7-dimethylpyrazolo[1,5-A]pyrimidine-3-sulfonamide 10%
attapulgite 10%
Pyrophyllite 80%

The active ingredient is blended with attapulgite and then passed through a hammer-mill to produce particles substantially all below 200 microns. The ground concentrate is then blended with powdered pyrophyllite until homogeneous.

Example 19

Emulsifiable Concentrate

N-[(4,6-dimethoxypyrimidin-2-yl)aminocarbonyl]-5,7-dimethylpyrazolo[1,5-A]pyrimidine-3-sulfonamide 10%
chlorobenzene 84%
sorbitan monostearate and polyoxyethylene condensates thereof 6%

The ingredients are combined and stirred to produce a solution which can be emulsified in water for application.

Example 20

Wettable Powder

N-[[(4,6-dimethoxy-2-pyrimidinyl)amino]carbonyl]-2,4-dimethylpyrazolo[1,5-a]-1,3,5-triazine-8-sulfonamide 80%
sodium alkylnaphthalenesulfonate 2%
sodium ligninsulfonate 2%
synthetic amorphous silica 3%
kaolinite 13%

The ingredients are blended, hammer-milled until all the solids are essentially under 50 microns, reblended, and packaged.

Example 21

Wettable Powder

N-[[(4-methoxy-6-methyl-2-pyrimidinyl)amino]carbonyl]isoxazolo[4,5-b]pyridine-3-sulfonamide 50%
sodium alkylnaphthalenesulfonate 2%
low viscosity methyl cellulose 2%
diatomaceous earth 46%

The ingredients are blended, coarsely hammer-milled and then air-milled to produce particles essentially all below 10 microns in diameter. The product is reblended before packaging.

Example 22

Granule

Wettable Powder of Example 21 5%
attapulgite granules 95%
(U.S.S. 20-40 mesh; 0.84-0.42mm)

A slurry of wettable powder containing 25% solids is sprayed on the surfact of attapulgite granules in a double-cone blender. The granules are dried and packaged.

Example 23

Extruded Pellet

N-[[(4,6-dimethoxy-2-pyrimidinyl)amino]carbonyl]-2,4-dimethylpyrazolo[1,5-a]-1,3,5-triazine-8-sulfonamide 25%
anhydrous sodium sulfate 10%
crude calcium ligninsulfonate 5%
sodium alkylnaphthalenesulfonate 1%
calcium/magnesium bentonite 59%

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84MM openings). The granules held on a U.S.S. No. 40 sieve (0.42 mm openings) may be packaged for use and the fines recycled.

## Example 24

### Oil Suspension

N-[[(4-methoxy-6-methyl-2-pyrimidinyl)aminocarbonyl]-isoxazolo[4,5-b]pyridine-3-sulfonamide 25%
polyoxyethylene sorbitol hexaoleate 5%
highly aliphatic hydrocarbon oil 70%

The ingredients are ground together in a sand mill until the solid particles have been reduced to under about 5 microns. The resulting thick suspension may be applied directly, but preferably after being extended with oils or emulsified in water.

## Example 25

### Wettable Powder

N-[[(4,6-dimethoxy-2-pyrimidinyl)amino]carbonyl]-2,4-dimethylpyrazolo[1,5-a]-1,3,5-triazine-8-sulfonamide 20%
sodium alkylnaphthalenesulfonate 4%
sodium ligninsulfonate 4%
low viscosity methyl cellulose 3%
attapulgite 69%

The ingredients are thoroughly blended. After grinding in a hammer-mill to produce particles essentially all below 100 microns, the material is reblended and sifted through a U.S.S. No. 50 sieve (0.3 mm opening) and packaged.

## Example 26

### Low Strength Granule

N-[[(4-methoxy-6-methyl-2-pyrimidinyl)amino]carbonyl]-isoxazolo[4,5-b]pyridine-3-sulfonamide 1%
N,N-dimethylformamide 9%
attapulgite granules 90%
(U.S.S. 20-2-40 sieve)

The active ingredient is dissolved in the solvent and the solution is sprayed upon dedusted granules in a double cone blender. After spraying of the solution has been completed, the blender is allowed to run for a short period and then the granules are packaged.

## Example 27

### Aqueous Suspension

N-[[(4,6-dimethoxy-2-pyrimidinyl)amino]carbonyl]-2,4-dimethylpyrazolo[1,5-a]-1,3,5-triazine-8-sulfonamide 40%
polyacrylic acid thickener 0.3%
dodecylphenol polyethylene glycol ether 0.5%
disodium phosphate 1%
monosodium phosphate 0.5%

polyvinyl alcohol 1.0%
water 56.7%

The ingredients are blended and ground together in a sand mill to produce particles essentially all under 5 microns in size.

## Example 28

### Solution

N-[[(4-methoxy-6-methyl-2-pyrimidinyl)amino]carbonyl]isoxazolo[4,5-b]pyridine-3-sulfonamide 5%
water 95%

The salt is added directly to the water with stirring to produce the solution, which may then be package for use.

## Example 29

### Low Strength Granule

N-[[(4,6-dimethoxy-2-pyrimidinyl)amino]carbonyl]-2,4-dimethylpyrazolo[1,5-a]-1,3,5-triazine-8-sulfonamide 0.1%
attapulgite granules 99.9%
(U.S.S. 20-40 mesh)

The active ingredient is dissolved in a solvent and the solution is sprayed upon dedusted granules in a double-cone blender. After spraying of the solution has been completed. the material is warmed to evaporate the solvent. The material is allowed to cool and then packaged.

## Example 30

### Granule

N-[[(4-methoxy-6-methyl-2-pyrimidinyl)amino]carbonyl]isoxazolo[4,5-b]pyridine-3-sulfonamide 80%
wetting agent 1%
crude ligninsulfonate salt (containing 5-20% of the natural sugars) 10%
attapulgite clay 9%

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

## Example 31

### High Strength Concentrate

N-[[(4,6-dimethoxy-2-pyrimidinyl)amino]carbonyl]-2,4-dimethylpyrazolo[1,5-a]-1,3,5-triazine-8-sulfonamide 99%
silica aerogel 0.5%
synthetic amorphous silica 0.5%

The ingredients are blended and ground in a hammer-mill to produce a material essentially all passing a U.S.S. No. 50 screen (0.3 mm opening). The concentrate may be formulated further if necessary.

Example 32

Wettable Powder

N-[[(4-methoxy-6-methyl-2-pyrimidinyl)amino]carbonyl]isoxazolo[4,5-b]pyridine-3-sulfonamide 90%
dioctyl sodium sulfosuccinate 0.1%
synthetic fine silica 9.9%

The ingredients are blended and ground in a hammer-mill to produce particles essentially all below 100 microns. The material is sifted through a U.S.S. No. 50 screen and then packaged.

Example 33

Wettable Powder

N-[[(4,6-dimethoxy-2-pyrimidinyl)amino]carbonyl]-2,4-dimethylpyrazolo[1,5-a]-1,3,5-triazine-8-sulfonamide 40%
sodium ligninsulfonate 20%
montmorillonite clay 40%

The ingredients are thoroughly blended, coarsely hammer-milled and then air-milled to produce particles essentially all blow 10 microns in size. The material is reblended and then packaged.

Example 34

Oil Suspension

N-[[(4-methoxy-6-methyl-2-pyrimidinyl)amino]carbonyl]isoxazolo[4,5-b]pyridine-3-sulfonamide 35%
blend of polyalcohol carboxylic esters and oil soluble petroleum sulfonates 6%
xylene 59%

The ingredients are combined and ground together in a sand mill to produce particles essentially all below 5 microns. The product can be used directly, extended with oils, or emulsified in water.

Example 35

Dust

N-[[(4,6-dimethoxy-2-pyrimidinyl)amino]carbonyl]-2,4-dimethylpyrazolo[1,5-a]-1,3,5-triazine-8-sulfonamide 10%
attapulgite 10%
Pyrophyllite 80%

The active ingredient is blended with attapulgite and then passed through a hammer-mill to produce particles substantially all below 200 microns. The ground concentrate is then blended with powdered pyrophyllite until homogeneous.

Example 36

Emulsifiable Concentrate

N-[[(4-methoxy-6-methyl-2-pyrimidinyl)amino]carbonyl]isoxazolo[4,5-b]pyridine-3-sulfonamide 10%
chlorobenzene 84%
sorbitan monostearate and polyoxyethylene condensates thereof 6%

The ingredients are combined and stirred to produce a solution which can be emulsified in water for application.

Utility

Test results indicate that the compounds of the present invention are highly active preemergent or postemergent herbicides or plant growth regulants. Many of them have utility for broad-spectrum pre- and/or postemergence weed control in areas where complete control of all vegetation is desired, such as around industrial storage areas, parking lots, drive-in theaters, around billboards, highway and railroad structures. Some of the compounds have utility for selective weed control in crops such as wheat, barley and cotton. Alternatively, the subject compounds are useful to modify plant growth.

The rates of application for the compounds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of around 0.001 to 20 kg/ha, the lower rates being suggested for use on lighter soils and/or those having a low organic matter content, for plant growth modification or for situations where only short-term persistence is required, such as a herbicide for fallow land.

The compounds of the invention may be used in combination with any other commercial herbicide, non-limiting examples of which are listed below:

| Common Name | Chemical Name |
|---|---|
| acetochlor | 2-chloro-N-(ethoxymethyl)-N-(2-ethyl-6-methylphenyl)acetamide |
| acifluorfen | 5-[2-chloro-4-(trifluoromethyl)phenoxy]-2-nitrobenzoic acid |
| acrolein | 2-propenal |
| alachlor | 2-chloro-N-(2,6-diethylphenyl-N-methoxy-methyl)acetamide |
| ametryn | N-ethyl-N'(1-methylethyl-6-(methylthio-1,3,5-triazine-2,4-diamine |
| amitrole | 1H-1,2,4-triazol-3-amine |
| AMS | ammonium sulfamate |
| asulam | methyl[(4-aminophenyl)sulfonyl]carbamate |
| atrazine | 6-chloro-N-ethyl-N'-(1-methylethyl)-1,3,5-triazine-2,4-diamine |
| barban | 4-chloro-2-butynyl 3-chlorocarbamate |
| benefin | N-butyl-N-ethyl-2,6-dinitro-4-(tri-fluoromethyl)benzenamine |
| bensulfuron methyl | 2-[[[[[4,6-dimethoxy-2-pyrimidinyl)amino]-carbonyl]amino]sulfonyl]methyl]benzoic acid, methyl ester |
| bensulide | O,O-bis(1-methylethyl)S-[2-[(phenylsul-fonyl)amino]ethyl]phosphorodithioate |
| bentazon | 3-(1-methylethyl)-(1H)-2,2,3-benzothia-diazin-4(3H)-one 2,2-dioxide |
| benzofluor | N-[4-(ethylthio)-2-(trifluoromethyl)-phenyl]methanesulfonamide |
| benzoylprop | N-benzoyl-N-(3,4-dichlorophenyl)-DL-alanine |
| bifenox | methyl 5-(2,4-dichlorophenoxy)-2-nitrobenzoate |
| bromacil | 5-bromo-6-methyl-3-(1-methylpropyl)-2,4(1H,3H)pyrimidinedione |

| Common Name | Chemical Name |
|---|---|
| bromoxynil | 3,5-dibromo-4-hydroxybenzonitrile |
| butachlor | N-(butoxymethyl)-2-chloro-N-(2,6-diethylphenyl)acetamide |
| buthidazole | 3-[5-(1,1-dimethylethyl)-1,3,4-thiadiazol-2-yl]-4-hydroxy-1-methyl-2-imidazolidinone |
| butralin | 4-(1,1-dimethylethyl)-N-(1-methylpropyl)-2,6-dinitrobenzenamine |
| butylate | S-ethyl bis(2-methylpropyl)carbamothioate |
| cacodylic acid | dimethyl arsinic oxide |
| CDAA | 2-chloro-N,N-di-2-propenylacetamide |
| CDEC | 2-chloroallyl diethyldithiocarbamate |
| chloramben | 3-amino-2,5-dichlorobenzoic acid |
| chlorbromuron | 3-(4-bromo-3-chlorophenyl)-1-methoxy-1-methylurea |
| chlorimuron ethyl | 2-[[[[4-chloro-6-methoxy-2-pyrimidinyl)-amino]carbonyl]amino]sulfonyl]benzoic acid, ethyl ester |
| chloroxuron | N'-[4-(4-chlorophenoxy)phenyl]-N,N-dimethylurea |
| chlorpropham | 1-methylethyl 3-chlorophenylcarbamate |
| chlorsulfuron | 2-chloro-N-[[(4-methoxy-6-methyl-1,3,5-triazin-2-yl)amino]carbonyl]benzenesulfonamide |
| chlortoluron | N'-(3-chloro-4-methylphenyl-N',N'-dimethylurea |
| cinmethylin | exo-1-methyl-4-(1-methylethyl)-2-[(2-methylphenyl)methoxy]-7-oxabicyclo-[2.2.1]heptane |
| clethodim | (E,E)-(±)-2-[1-[[(3-chloro-2-propenyl)-oxy]imino]propyl]-5-[2-(ethylthio)-propyl]-3-hydroxy-2-cyclohexen-1-one |

| Common Name | Chemical Name |
|---|---|
| clomazone | 2-[(2-chlorophenyl)methyl]-4,4-dimethyl-3-isoxazolidinone |
| cloproxydim | (E,E)-2-[1-[[(3-chloro-2-propenyl)oxy)-imino]butyl]-5-[2-(ethylthio)propyl]-3-hydroxy-2-cyclohexen-1-one |
| clopyralid | 3,6-dichloro-2-pyridinecarboxylic acid |
| CMA | calcium salt of MAA |
| cyanazine | 2-[[4-chloro-6-(ethylamino)-1,3,5-triazin-3-yl]amino]-2-methylpropanenitrile |
| cycloate | S-ethyl cyclohexanecarbamothioate |
| cycluron | 3-cyclooctyl-1,1-dimethylurea |
| cyperquat | 1-methyl-4-phenylpyridinium |
| cyprazine | 2-chloro-4-(cyclopropylamino)-6-(isopropylamino)-s-triazine |
| cyprazole | N-[5-(2-chloro-1,1-dimethylethyl)-1,3,4-thiadiazol-2-yl] cyclopropanecarboxamide |
| cypromid | 3',4'-dichlorocyclopropanecarboxanilide |
| dalapon | 2,2-dichloropropionic acid |
| dazomet | tetrahydro-3,5-dimethyl-2H-1,3,5-thiadiazine-2-thione |
| DCPA | dimethyl 2,3,5,6-tetrachloro-1,4-benzenedicarboxylate |
| desmediphan | ethyl [3-[[(phenylamino)carbonyl]oxy]-phenyl]carbamate |
| desmetryn | 2-(isopropylamino)-4-(methylamino)-6-methylthio)-s-triazine |
| diallate | S-(2,3-dichlor-2-propenyl)bis(1-methylethyl)carbamothioate |
| dicamba | 3,6-dichloro-2-methoxybenzoic acid |
| dichlobenil | 2,6-dichlorobenzonitrile |

| Common Name | Chemical Name |
|---|---|
| dichlorprop | (±)-2-(2,4-dichlorophenoxy)propanoic acid |
| dichlofop | (±)-2-[4-(2,4-dichlorophenoxy)phenoxy]-propanoic acid |
| diethatyl | N-(chloroacetyl)-N-(2,6-diethylphenyl)-glycine |
| difenzoquat | 1,2-dimethyl-3,5-diphenyl-1H-pyrazolium |
| dinitramine | $N^3$,$N^3$-diethyl-2,4-dinitro-6-(trifluoromethyl)-1,3-benzenediamine |
| dinoseb | 2-(1-methylpropyl)-4,6-dinitrophenol |
| diphenamid | N,N-dimethyl-α-phenylbenzeneacetamide |
| dipropetryn | 6-(ethylthio)-N,N'-bis(1-methylethyl)-1,3,5-triazine-2,4-diamine |
| diquat | 6,7-dihydrodipyrido[1,2-α-2',1'-c]-pyrazinediium ion |
| diuron | N'-(3,4-dichlorophenyl)-N,N-dimethylurea |
| DNOC | 2-methyl-4,6-dinitrophenol |
| DPX-M6316 | 3-[[[[(4-methoxy-6-methyl-1,3,5-triazin-2-yl)amino]carbonyl]amino]sulfonyl]-2-thiophenecarboxylic acid, methyl ester |
| DSMA | disodium salt of MAA |
| endothall | 7-oxabicyclo[2.2.1]heptane-2,3-dicarboxylic acid |
| EPTC | S-ethyl dipropylcarbamothioate |
| ethalfluralin | N-ethyl-N-(2-methyl-2-propenyl)-2,6-dinitro-4-(trifluoromethyl)-benzenamine |

| Common Name | Chemical Name |
|---|---|
| ethofumesate | (±)-2-ethoxy-2,3-dihydro-3,3-dimethyl-5-benzofuranyl methanesulfonate |
| Express® | 2-[[N-(4-methoxy-6-methyl-1,3,5-triazine-2-yl)-N-methylaminocarbonyl]aminosulfonyl]benzoic acid, methyl ester |
| fenac | 2,3,6-trichlorobenzeneacetic acid |
| fenoxaprop | (±)-2-[4-[(6-chloro-2-benzoxazolyl)oxy]-phenoxy]propanoic acid |
| fenuron | N,N-dimethyl-N'-phenylurea |
| fenuron TCA | Salt of fenuron and TCA |
| flamprop | N-benzoyl-N-(3-chloro-4-fluorophenyl)-DL-alanine |
| fluazifop | (±)2-[4-[[5-(trifluoromethyl)-2-pyridinyl]oxy]phenoxy]propanoic acid |
| fluazifop-P | (R)-2-[4-[[5-(trifluoromethyl)-2-pyridinyl]oxy]phenoxy]propanoic acid |
| fluchloralin | N-(2-chloroethyl)-2,6-dinitro-N-propyl-4-(trifluoromethyl)benzenamine |
| fluometuron | N,N-dimethyl-N'-(3-(trifluoromethyl)-phenyl]urea |
| fluorochloridone | 3-chloro-4-(chloromethyl)-1-[3-trifluoromethyl)phenyl]-2-pyrrolidinone |
| fluorodifen | p-nitrophenyl α,α,α-trifluoro-2-nitro-p-tolyl ether |
| fluoroglycofen | carboxymethyl 5-[2-chloro-4-(trifluoromethyl)phenoxy]-2-nitrobenzoate |
| fluridone | 1-methyl-3-phenyl-5-[3-(trifluoromethyl)phenyl]-4(1H)-pyridinone |
| fomesafen | 5-(2-chloro-4-trifluoromethylphenoxy)-N-methylsulfonyl-2-nitrobenzamide |

| Common Name | Chemical Name |
|---|---|
| fosaminc | ethyl hydrogen (aminocarbonyl)-phosphate |
| glyphosate | N-(phosphonomethyl)glycine |
| haloxyfop | 2-[4-[[3-chloro-5-(trifluoromethyl)-2-pyridinyl]oxy]phenoxy]propanoic acid |
| hexaflurate | potassium hexafluoroarsenate |
| hexazinone | 3-cyclohexyl-6-(dimethylamino)-1-methyl-1,3,5-triazine-2,4(1H,3H)-dione |
| imazamethabenz | 6-(4-isopropyl-4-methyl-5-oxo-2-imidazolin-2-yl)-m-toluic acid, methyl ester and 6-(4-isopropyl)-4-methyl-5-oxo-2-imidazolin-2-yl-p-toluic acid, methyl ester |
| imazapyr | (±)2-[4,5-dihydro-4-methyl-4-(1-methylethyl)-5-oxo-1H-imidazol-2-yl]-3-pyridinecarboxylic acid |
| imazaquin | 2-(4,5-dihydro-4-methyl-4-(1-methylethyl)-5-oxo-1H-imidazol-2-yl)-3-quinolinecarboxylic acid |
| imazethapyr | (±)-2-[4,5-dihydro-4-methyl-4-(1-methylethyl)-5-oxo-1H-imidazol-2-yl]-5-ethyl-3-pyridinecarboxylic acid |
| ioxynil | 4-hydroxy-3,5-diiodobenzonitrile |
| isopropalin | 4-(1-methylethyl)-2,6-dinitro-N,N-dipropylbenzenamine |
| isoproturon | N-(4-isopropylphenyl)-N',N'-dimethylurea |
| isouron | N'-[5-(1,1-dimethylethyl)-3-isoxazolyl]-N,N-dimethylurea |
| isoxaben | N-[3-(1-ethyl-1-methylpropyl)-5-isoxazolyl]-2,6-dimethoxybenzamide |
| karbutilate | 3-[[(dimethylamino)carbonyl]amino]phenyl-(1,1-dimethylethyl)carbamate |

| Common Name | Chemical Name |
|---|---|
| lactofen | (±)-2-ethoxy-1-methyl-2-oxoethyl-5-[2-chloro-4-(trifluoromethyl)phenoxy]-2-nitrobenzoate |
| lenacil | 3-cyclohexyl-6,7-dihydro-1H-cyclopentapyrimidine-2,4(3H,5H)-dione |
| linuron | N'-(3,4-dichlorophenyl)-N-methoxy-N-methylurea |
| MAA | methylarsonic acid |
| MAMA | monoammonium salt of MAA |
| MCPA | (4-chloro-2-methyl)phenoxy)acetic acid |
| MCPB | 4-(4-chloro-2-methylphenoxy)butanoic acid |
| mecoprop | (±)-2-(4-chloro-2-methylphenoxy)-propanoic acid |
| mefluidide | N-[(2,4-dimethyl-5-[[(trifluoromethyl)-sulfonyl]amino]phenyl]acetamide |
| methal-propalin | N-(2-methyl-2-propenyl)-2,6-dinitro-N-propyl-4-(trifluoromethyl)benzenamide |
| methabenz-thiazuron | 1,3-dimethyl-3-(2-benzothiazolyl)urea |
| metham | methylcarbamodithioic acid |
| methazole | 2-(3,4-dichlorophenyl)-4-methyl-1,2,4-oxadiazolidine-3,5-dione |
| methoxuron | N'-(3-chloro-4-methoxyphenyl)N,N-dimethylurea |
| metolachlor | 2-chloro-N-(2-ethyl-6-methylphenyl)-N-(2-methoxy-1-methylethyl)acetamide |

| Common Name | Chemical Name |
|---|---|
| metribuzin | 4-amino-6-(1,1-dimethylethyl)-3-(methylthio)-1,2,4-triazin-5(4H)-one |
| metsulfuron methyl | 2-[[[[(4-methoxy-6-methyl-1,3,5-triazine-methyl-2-yl)amino]carbonyl]-amino]sulfonyl]benzoic acid, methyl ester |
| MH | 1,2-dihydro-3,6-pyridazinedione |
| molinate | S-ethyl hexahydro-1H-azepine-1-carbothioate |
| monolinuron | 3-(p-chlorophenyl)-1-methoxy-1-methylurea |
| monuron | N'-(4-chlorophenyl)-N,N-dimethylurea |
| monuron TCA | Salt of monuron and TCA |
| MSMA | monosodium salt of MAA |
| napropamide | N,N-diethyl-2-(1-naphthalenyloxy)-propanamide |
| naptalam | 2-[(1-naphthalenylamino)carbonyl]-benzoic acid |
| neburon | 1-butyl-3-(3,4-dichlorophenyl)-1-methylurea |
| nitralin | 4-(methylsulfonyl)-2,6-dinitro-N,N-dipropylaniline |
| nitrofen | 2,4-dichloro-1-(4-nitrophenoxy)benzene |
| nitrofluorfen | 2-chloro-1-(4-nitrophenoxy)-4-(trifluoromethyl)benzene |
| norea | N,N-dimethyl-N'-(octahydro-4,7-methano-1H-inden-5-yl)urea 3aα,-4aα,5α,7α,7aα-isomer |
| norflurazon | 4-chloro-5-(methylamino)-2-(3-trifluoromethyl)phenyl)-3(2H)-pyridazinone |
| oryzalin | 4-(dipropylamino)-3,5-dinitrobenzenesulfonamide |

| Common Name | Chemical Name |
|---|---|
| oxadiazon | 3-[2,4-dichloro-5-(1-methylethoxy)-phenyl]-5-(1,1-dimethylethyl)-1,3,4-oxadiazol-2-(3H)-one |
| oxyfluorfen | 2-chloro-1-(3-ethoxy-4-nitrophenoxy)-4-(trifluoromethyl)benzene |
| paraquat | 1,1'-dimethyl-4,4'-bipyridinium ion |
| pebulate | S-propyl butylethylcarbamothioate |
| pendimethalin | N-(1-ethylpropyl)-3,4-dimethyl-2,6-dinitrobenzenamine |
| perfluidone | 1,1,1-trifluoro-N-[2-methyl-4-(phenyl-sulfonyl)phenyl]methanesulfonamide |
| phenmedipham | 3-[(methoxycarbonyl)amino]phenyl(3-methylphenyl)carbamate |
| picloram | 4-amino-3,5,6-trichloro-2-pyridine-carboxylic acid |
| PPG-1013 | 5-(2-chloro-4-(trifluoromethyl)-phenoxy)-2-nitroacetophenone oxime-O-acetic acid, methyl ester |
| procyazine | 2-[[4-chloro-6-(cyclopropylamino)-1,3,5-triazine-2-yl]amino]-2-methylpropane-nitrile |
| profluralin | N-(cyclopropylmethyl)-2,6-dinitro-N-propyl-4-(trifluoromethyl)benzenamine |
| prometon | 6-methoxy-N,N'-bis(1-methylethyl)-1,3,5-triazine-2,4-diamine |
| prometryn | N,N'-bis(1-methylethyl)-6-(methylthio)-1,3,5-triazine-2,4-diamine |
| pronamide | 3,5-dichloro-N-(1,1-dimethyl-2-propyn-yl)benzamide |
| propachlor | 2-chloro-N-(1-methylethyl)-N-phenylacetamide |
| propanil | N-(3',4'-dichlorophenyl)propanamide |
| propazine | 6-chloro-N,N'-bis(1-methylethyl)-1,3,5-triazine-2,4-diamine |

| Common Name | Chemical Name |
|---|---|
| propham | 1-methylethyl phenylcarbamate |
| prosulfalin | N-[[4-(dipropylamino)-3,5-dinitrophenyl]sulfonyl]-S,S-dimethylsulfilimine |
| prynachlor | 2-chloro-N-(1-methyl-2-propynyl)acetanilide |
| pyrazon | 5-amino-4-chloro-2-phenyl-3(2H)-pyridazinone |
| quizalofop ethyl | 2-[4-(6-chloroquinoxalin-2-yloxy)phenoxypropanoic acid, ethyl ester |
| quizalofop | (±)-2-[4-[(6-chloro-2-quinoxalinyl)-oxy]phenoxy]propanoic acid |
| secbumeton | N-ethyl-6-methoxy-N'(1-methylpropyl)-1,3,5-triazine-2,4-diamine |
| sethoxydim | 2-[1-(ethoxyimino)butyl]-5-[2-(ethylthio)propyl]-3-hydroxy-2-cyclohexene-1-one |
| siduron | N-(2-methylcyclohexyl)-N'-phenylurea |
| simazine | 6-chloro-N,N'-diethyl-1,3,5-triazine-2,4-diamine |
| sulfometuron methyl | 2-[[[[(4,6-dimethyl-2-pyrimidinyl)-amino]carbonyl]amino]sulfonyl]-benzoic acid, methyl ester |
| TCA | trichloroacetic acid |
| tebuthiuron | N-[5-(1,1-dimethylethyl)-1,3,4-thiadiazol-2-yl]-N,N'-dimethylurea |
| terbacil | 5-chloro-3-(1,1-dimethylethyl)-6-methyl-2,4(1H,3H)-pyrimidinedione |
| terbuchlor | N-(butoxymethyl)-2-chloro-N-[2-(1,1-dimethylethyl)-6-methylphenyl]-acetamide |

| Common Name | Chemical Name |
|---|---|
| terbuthyl-azine | 2-(*tert*-butylamino)-4-chloro-6-(ethyl-amino)-*s*-triazine |
| terbutol | 2,6-di-*tert*-butyl-*p*-tolyl methylcar-bamate |
| terbutryn | N-(1,1-dimethylethyl)-N'-ethyl-6-(methylthio)-1,3,5-triazine-2,4-diamine |
| thiameturon methyl | 3-[[[[(4-methoxy-6-methyl-1,3,5-triazin-2-yl)amino]carbonyl]-amino]sulfonyl]-2-thiophenecarboxylic acid, methyl ester |
| thiobencarb | S-[(4-chlorophenyl)methyl] diethylcar-bamothioate |
| triallate | S-(2,3,3-trichloro-2-propenyl)bis(1-methylethyl)carbamothioate |
| triclopyr | [(3,5,6-trichloro-2-pyridinyl)-oxy]acetic acid |
| tridiphane | 1-(3,5-dichlorophenyl)-2-(2,2,2-trichloroethyl)oxirane |
| trifluralin | 2,6-dinitro-N,N-dipropyl-4-(tri-fluoromethyl)benzenamine |
| trimeturon | 1-(*p*-chlorophenyl)-2,3,3-trimethylpseu-dourea |
| 2,4-D | (2,4-dichlorophenoxy)acetic acid |
| 2,4-DB | 4-(2,4-dichlorophenoxy)butanoic acid |
| vernolate | S-propyl dipropylthiocarbamothioate |
| xylachlor | 2-chloro-N-(2,3-dimethylphenyl)-N-(1-methylethyl)acetamide |

The herbicidal properties of the subject compounds were discovered in a number of greenhouse tests. The test procedures and results follow.

COMPOUNDS

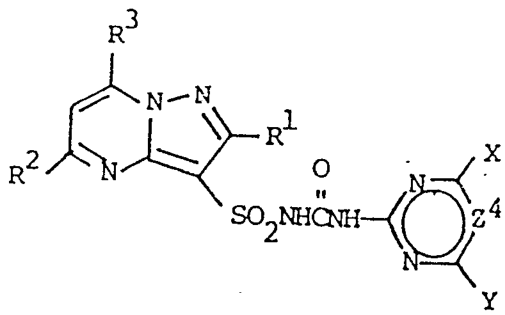

| CMPD | $R^1$ | $R^2$ | $R^3$ | X | Y | $Z^4$ | m.p.(°C) |
|---|---|---|---|---|---|---|---|
| 1 | H | $CH_3$ | $CH_3$ | $OCH_3$ | $OCH_3$ | CH | 166-168 |
| 2 | H | $CH_3$ | $CH_3$ | $OCH_3$ | $OCH_3$ | N | 188-190 |
| 3 | H | $CH_3$ | $CH_3$ | Cl | $OCH_3$ | CH | 199-201 |
| 4 | H | $CH_3$ | $CH_3$ | $CH_3$ | $OCH_3$ | N | 190-193 |
| 5 | H | $CH_3$ | $CH_3$ | $CH_3$ | $CH_3$ | CH | 231-232 |
| 6 | Cl | $CH_3$ | $CH_3$ | $OCH_3$ | $OCH_3$ | CH | 220-221(d) |
| 7 | Cl | $CH_3$ | $CH_3$ | $OCH_3$ | $OCH_3$ | N | 140-141(d) |
| 8 | Cl | $CH_3$ | $CH_3$ | Cl | $OCH_3$ | CH | 187-188(d) |
| 9 | Cl | $CH_3$ | $CH_3$ | $CH_3$ | $OCH_3$ | N | 181-182(d) |
| 10 | Cl | $CH_3$ | $CH_3$ | $CH_3$ | $OCH_3$ | CH | 215-216(d) |
| 11 | Cl | $CH_3$ | $CH_3$ | $CH_3$ | $CH_3$ | CH | 221-222(d) |
| 12 | H | H | H | $OCH_3$ | $OCH_3$ | CH | 225-226(d) |
| 13 | H | H | H | $CH_3$ | $OCH_3$ | CH | 204-205(d) |
| 14 | H | H | H | Cl | $OCH_3$ | CH | 222(d) |
| 15 | H | H | H | $CH_3$ | $CH_3$ | CH | 210-211(d) |
| 16 | H | H | H | $OCH_3$ | $OCH_3$ | N | 205-207(d) |
| 17 | H | H | H | $CH_3$ | $OCH_3$ | N | 197-198(d) |
| 18 | Cl | $CH_3$ | $CH_3$ | $NHCH_3$ | $OC_2H_5$ | N | 200(d) |
| 19 | H | H | H | $NHCH_3$ | $OC_2H_5$ | N | 230-232 |
| 20 | Cl | $CH_3$ | $CH_3$ | $OC_2H_5$ | $OC_2H_5$ | N | 111-113(d) |
| 21 | Cl | $CH_3$ | $CH_3$ | $N(CH_3)_2$ | $OCH_3$ | N | 201-202(d) |
| 22 | Cl | $CH_3$ | $CH_3$ | $N(CH_3)_2$ | $OC_2H_5$ | N | 181-182(d) |
| 23 | Cl | $CH_3$ | $CH_3$ | $NHCH_3$ | $OCH_2CF_3$ | N | 198-201(d) |

COMPOUNDS (continued)

| CMPD | $R^1$ | $R^2$ | $R^3$ | X | Y | $Z^4$ | m.p.(°C) |
|---|---|---|---|---|---|---|---|
| 24 | Cl | $CH_3$ | $CH_3$ | $N(CH_3)_2$ | $OCH_2CF_3$ | N | 150-155(d) |
| 25 | $SCH_3$ | $CH_3$ | $CH_3$ | $CH_3$ | $OCH_3$ | N | 181-182(d) |
| 26 | $SCH_3$ | $CH_3$ | $CH_3$ | $OCH_3$ | $OCH_3$ | N | 177-179(d) |
| 27 | $SCH_3$ | $CH_3$ | $CH_3$ | Cl | $OCH_3$ | CH | 118-119(d) |
| 28 | $SCH_3$ | $CH_3$ | $CH_3$ | $CH_3$ | $OCH_3$ | CH | 150-151 |
| 29 | $SCH_3$ | $CH_3$ | $CH_3$ | $OCH_3$ | $OCH_3$ | CH | 160-161 |
| 30 | $S(CH_2)_2CH_3$ | $CH_3$ | $CH_3$ | $OCH_3$ | $OCH_3$ | CH | 130-135 |
| 31 | $S(CH_2)_2CH_3$ | $CH_3$ | $CH_3$ | $CH_3$ | $OCH_3$ | CH | 120-125 |
| 32 | $S(CH_2)_2CH_3$ | $CH_3$ | $CH_3$ | Cl | $OCH_3$ | CH | 124-130 |
| 33 | $S(CH_2)_2CH_3$ | $CH_3$ | $CH_3$ | $CH_3$ | $CH_3$ | CH | 140-150 |
| 34 | $S(CH_2)_2CH_3$ | $CH_3$ | $CH_3$ | $CH_3$ | $OCH_3$ | N | 123-130 |
| 35 | $S(O)_2(CH_2)_2CH_3$ | $CH_3$ | $CH_3$ | $OCH_3$ | $OCH_3$ | CH | 195-196(d) |
| 36 | $S(O)_2(CH_2)_2CH_3$ | $CH_3$ | $CH_3$ | $CH_3$ | $OCH_3$ | CH | 140-141(d) |
| 37 | $S(O)_2(CH_2)_2CH_3$ | $CH_3$ | $CH_3$ | Cl | $OCH_3$ | CH | 137-138(d) |
| 38 | $S(O)_2(CH_2)_2CH_3$ | $CH_3$ | $CH_3$ | $CH_3$ | $CH_3$ | CH | 134-135(d) |
| 39 | $S(O)_2(CH_2)_2CH_3$ | $CH_3$ | $CH_3$ | $OCH_3$ | $OCH_3$ | N | 142-143(d) |
| 40 | $S(O)_2(CH_2)_2CH_3$ | $CH_3$ | $CH_3$ | $CH_3$ | $OCH_3$ | N | 134-135(d) |
| 41 | $S(O)_2CH_3$ | $CH_3$ | $CH_3$ | $OCH_3$ | $OCH_3$ | CH | 185(d) |
| 42 | $S(O)_2CH_3$ | $CH_3$ | $CH_3$ | $CH_3$ | $OCH_3$ | CH | 217-218(d) |
| 43 | $S(O)_2CH_3$ | $CH_3$ | $CH_3$ | Cl | $OCH_3$ | CH | 158-160(d) |
| 44 | $S(O)_2CH_3$ | $CH_3$ | $CH_3$ | $OCH_3$ | $OCH_3$ | N | 187-190(d) |
| 45 | $S(O)_2CH_3$ | $CH_3$ | $CH_3$ | $CH_3$ | $OCH_3$ | N | 217-218(d) |
| 46 | $S(O)_2CH_3$ | $CH_3$ | $CH_3$ | $CH_3$ | $CH_3$ | CH | 236-237(d) |
| 47 | $SCH_2CH_3$ | $CH_3$ | $CH_3$ | $OCH_3$ | $OCH_3$ | CH | 177(d) |
| 48 | $SCH_2CH_3$ | $CH_3$ | $CH_3$ | $CH_3$ | $OCH_3$ | CH | 153(d) |
| 49 | $SCH_2CH_3$ | $CH_3$ | $CH_3$ | Cl | $OCH_3$ | CH | 135(d) |
| 50 | $SCH_2CH_3$ | $CH_3$ | $CH_3$ | $CH_3$ | $CH_3$ | CH | 147(d) |
| 51 | $SCH_2CH_3$ | $CH_3$ | $CH_3$ | $OCH_3$ | $OCH_3$ | N | 185(d) |
| 52 | $SCH_2CH_3$ | $CH_3$ | $CH_3$ | $CH_3$ | $OCH_3$ | N | 153(d) |
| 53 | $S(O)_2CH_2CH_3$ | $CH_3$ | $CH_3$ | $OCH_3$ | $OCH_3$ | CH | 225-226(d) |
| 54 | $S(O)_2CH_2CH_3$ | $CH_3$ | $CH_3$ | $CH_3$ | $OCH_3$ | CH | 219-221(d) |

COMPOUNDS (continued)

| CMPD | $R^1$ | $R^2$ | $R^3$ | X | Y | $Z^4$ | m.p. (°C) |
|---|---|---|---|---|---|---|---|
| 55 | $S(O)_2CH_2CH_3$ | $CH_3$ | $CH_3$ | Cl | $OCH_3$ | CH | 215-216(d) |
| 56 | $S(O)_2CH_2CH_3$ | $CH_3$ | $CH_3$ | $OCH_3$ | $OCH_3$ | N | 186-187(d) |
| 57 | $S(O)_2CH_2CH_3$ | $CH_3$ | $CH_3$ | $CH_3$ | $OCH_3$ | N | 176-177(d) |
| 58 | $S(O)_2CH_2CH_3$ | $CH_3$ | $CH_3$ | $CH_3$ | $CH_3$ | CH | 204-205(d) |

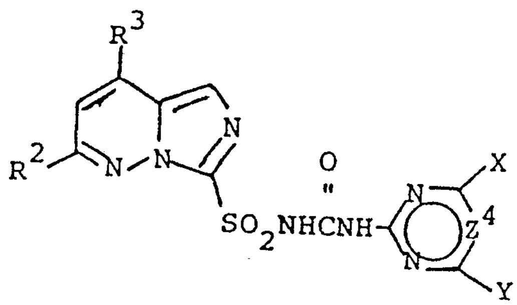

| CMPD | $R^2$ | $R^3$ | X | Y | $Z^4$ | m.p. (°C) |
|---|---|---|---|---|---|---|
| 59 | $CH_3$ | H | $OCH_3$ | $OCH_3$ | CH | 121-124(d) |

Test A

Seeds of crabgrass (Digitaria spp.), barnyardgrass (Echinochloa crus-galli), giant foxtail Setaria faberii), wild oats (Avena fatua), cheatgrass (Bromus secalinus), velvetleaf (Abutilon theophrasti), morningglory (Ipomoea spp.), cocklebur (Xanthium pensylvanicum), sorghum, corn, soybean, sugarbeet, cotton, rice, wheat, barley and purple nutsedge (Cyperus rotundus) tubers were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings: C = chlorosis/necrosis;

B = burn;
D = defoliation;
E = emergence inhibition;
G = growth retardation;
H = formative effective
U = unusual pigmentation;
X = axillary stimulation;
S = albinism; and
Y = abscised buds or flowers.

TABLE A

| | CMPD 1 | | CMPD 2 | |
|---|---|---|---|---|
| RATE=KG/HA | 0.4 | 0.05 | 0.4 | 0.05 |
| POSTEMERGENCE | | | | |
| COTTON | 9C | 9C | 4C,9H | 4C,9G |
| MORNINGGLORY | 10C | 10C | 9C | 5C,8H |
| COCKLEBUR | 10C | 10C | 4G | 0 |
| NUTSEDGE | 10C | 9C | 5C,9G | - |
| CRABGRASS | 9C | 3C,9G | 4C,9G | 3C,8G |
| BARNYARDGRASS | 9C | 9C | 9C | 5C,9G |
| WILD OATS | 4C,9G | 5C,9G | 5C,9G | 2C,9G |
| WHEAT | 9G | 9G | 9C | 9G |
| CORN | 9C | 9C | 4U,9C | 5C,9G |
| SOYBEANS | 9C | 5C,9G | 5C,9G | 4C,8H |
| RICE | 9C | 5C,9G | 9C | 9C |
| SORGHUM | 9C | 9C | 5C,9G | 4C,9G |
| CHEATGRASS | 9C | 9C | 5C,9G | 4C,9G |
| SUGARBEETS | 9C | 9C | 10C | 9C |
| VELVETLEAF | 10C | 10C | 9C | 4C,9H |
| GIANT FOXTAIL | 4C,9G | 4C,8G | 5C,9G | 5C,9G |
| BARLEY | 4C,9G | 3C,9G | 5C,9G | 3C,9G |

TABLE A

| | CMPD 1 | | CMPD 2 | |
|---|---|---|---|---|
| RATE=KG/HA | 0.4 | 0.05 | 0.4 | 0.05 |
| PREEMERGENCE | | | | |
| COTTON | 9G | 0 | 5G | 0 |
| MORNINGGLORY | 9H | 6G | 7G | 0 |
| COCKLEBUR | 3C,6H | 0 | 0 | 0 |
| NUTSEDGE | 10E | 5G | 10E | 0 |
| CRABGRASS | 5C,9G | 5G | 3C,6G | 3G |
| BARNYARDGRASS | 9H | 9H | 3C,8H | 0 |
| WILD OATS | 3C,8G | 2C,3G | 3C,7G | 0 |
| WHEAT | 9H | 8G | 9H | 7G |
| CORN | 4C,9H | 3C,9H | 3C,9G | 2G |
| SOYBEANS | 9H | 3C,6H | 3C,7G | 0 |
| RICE | 9H | 3C,9H | 9H | 7G |
| SORGHUM | 9H | 9H | 9H | 2G |
| CHEATGRASS | 9H | 8G | 8G | 5G |
| SUGARBEETS | 4C,9G | 3C,7H | 9G | 7G |
| VELVETLEAF | 2C,8G | 7G | 2C,3H | 0 |
| GIANT FOXTAIL | 9H | 1H | 9H | 5G |
| BARLEY | 9G | 5G | 9G | 0 |

TABLE A

| | CMPD 3 | | CMPD 4 | |
|---|---|---|---|---|
| RATE=KG/HA | 0.4 | 0.05 | 0.4 | 0.05 |
| POSTEMERGENCE | | | | |
| COTTON | 10C | 3C,7H | 3C,9H | 4G |
| MORNINGGLORY | 3C,7G | 3C,6G | 9C | 3C,8H |
| COCKLEBUR | 5C,9G | 3C,7H | 3C,7H | 1C,1H |
| NUTSEDGE | 4C,9G | 3C,8G | - | 0 |
| CRABGRASS | 3C,4G | 2G | 5C,9G | 3C,8G |
| BARNYARDGRASS | 4C,9H | 3C,8H | 5C,9G | 4C,9G |
| WILD OATS | 0 | 0 | 4C,9G | 9G |
| WHEAT | 3G | 0 | 3C,9G | 9G |
| CORN | 3C,9G | 5C,9G | 5C,9G | 5C,9G |
| SOYBEANS | 3C,7G | 3C,5G | 4C,9G | 3C,5G |
| RICE | 4C,8G | 6G | 9C | 5C,9G |
| SORGHUM | 3C,9G | 3C,9H | 5C,9G | 4C,9G |
| CHEATGRASS | 5G | 0 | 9C | 4C,9G |
| SUGARBEETS | 4C,9H | 3C,8G | 9C | 3C,6G |
| VELVETLEAF | 9C | 3C,7H | 8G | 5G |
| GIANT FOXTAIL | 3C,6G | 2G | 5C,9G | 3C,8G |
| BARLEY | 3G | 0 | 8G | 5G |

TABLE A

| | CMPD 3 | | CMPD 4 | |
|---|---|---|---|---|
| RATE=KG/HA | 0.4 | 0.05 | 0.4 | 0.05 |
| PREEMERGENCE | | | | |
| COTTON | 2C,7G | 0 | 5G | 0 |
| MORNINGGLORY | 0 | 0 | 4G | 0 |
| COCKLEBUR | 0 | 0 | 0 | 0 |
| NUTSEDGE | 7G | 0 | 10E | 0 |
| CRABGRASS | 3G | 0 | 3C,7G | 0 |
| BARNYARDGRASS | 3C,8H | 0 | 3C,8H | 0 |
| WILD OATS | 0 | 0 | 5G | 0 |
| WHEAT | 2G | 0 | 9G | 8G |
| CORN | 3C,9G | 0 | 3C,9H | 3C,5G |
| SOYBEANS | 2C,2H | 0 | 3C,6G | 2G |
| RICE | 8H | 3G | 9H | 2G |
| SORGHUM | 3C,9H | 3C,3G | 3C,9H | 3C,4G |
| CHEATGRASS | 0 | 0 | 8H | 0 |
| SUGARBEETS | 3C,8G | 2H | 7H | 2G |
| VELVETLEAF | 6G | 3G | 2C,5G | 0 |
| GIANT FOXTAIL | 4G | 0 | 9H | 0 |
| BARLEY | 0 | 0 | 8G | 0 |

TABLE A

| CMPD 6 | | |
|---|---|---|
| RATE=KG/HA | 0.05 | 0.01 |
| POSTEMERGENCE | | |
| COTTON | 10C | 5C,9G |
| MORNINGGLORY | 10C | 10C |
| COCKLEBUR | 5C,9G | 10C |
| NUTSEDGE | 10C | 10C |
| CRABGRASS | 10C | 9C |
| BARNYARDGRASS | 10C | 5C,9G |
| WILD OATS | 5C,9G | 3C,8G |
| WHEAT | 5C,9G | 3C,8G |
| CORN | 10C | 9C |
| SOYBEANS | 9C | 9C |
| RICE | 5C,9G | 3C,9G |
| SORGHUM | 9C | 5C,9G |
| CHEATGRASS | - | - |
| SUGARBEETS | 9C | 9C |
| VELVETLEAF | 10C | 10C |
| GIANT FOXTAIL | 9C | 9C |
| BARLEY | 4C,8G | 3C,6G |
| DOWNY BROME | 9C | 9C |

TABLE A

| CMPD 6 | | |
|---|---|---|
| RATE=KG/HA | 0.05 | 0.01 |
| PREEMERGENCE | | |
| COTTON | 9H | 8G |
| MORNINGGLORY | 9G | 3C,7H |
| COCKLEBUR | 3C,9H | 0 |
| NUTSEDGE | 10E | 7G |
| CRABGRASS | 3C,9G | 0 |
| BARNYARDGRASS | 9H | 8H |
| WILD OATS | 3C,9H | 3C,7G |
| WHEAT | 3C,9H | 7G |
| CORN | 3C,9G | 3C,6G |
| SOYBEANS | 3C,8H | 3C,7H |
| RICE | 9H | 9H |
| SORGHUM | 9H | 3C,9H |
| CHEATGRASS | - | - |
| SUGARBEETS | 4C,9G | 9G |
| VELVETLEAF | 3C,9G | 7H |
| GIANT FOXTAIL | 9H | 3C,7G |
| BARLEY | 6G | 6G |
| DOWNY BROME | 9H | 9H |

TABLE A

| | CMPD 7 | | CMPD 8 | |
|---|---|---|---|---|
| RATE=KG/HA | 0.05 | 0.01 | 0.05 | 0.01 |
| POSTEMERGENCE | | | | |
| COTTON | 9C | 4C,9H | 4C,9G | 4C,8G |
| MORNINGGLORY | 4C,9G | 2H | 3C,6G | 3C,3G |
| COCKLEBUR | 4C,8H | 1C | 5C,9G | 5C,9G |
| NUTSEDGE | 9C | 1C | - | 3C,5G |
| CRABGRASS | 4C,8G | 2C,4G | 0 | 0 |
| BARNYARDGRASS | 5C,9H | 1C | 3C,5G | 0 |
| WILD OATS | 5C,9G | 4C,9G | 0 | 0 |
| WHEAT | 9C | 4C,9G | 3G | 0 |
| CORN | 9C | 9C | 3C,9G | 9G |
| SOYBEANS | 5C,9G | 4C,9G | 3C,8G | 3H |
| RICE | 9C | 9C | 3C,8G | 2C,5G |
| SORGHUM | 9C | 5C,9G | 3C,9G | 3C,7G |
| SUGARBEETS | 9C | 4C,8H | 9C | 3G |
| VELVETLEAF | 4C,9H | 1C | 3C,8H | 3C,6G |
| GIANT FOXTAIL | 9C | 5C,9G | 3C,7G | 1C |
| BARLEY | 9C | 5C,9G | 2C,2G | 0 |
| DOWNY BROME | 5C,9G | 5C,9G | 4G | 4G |

TABLE A

| | CMPD 7 | | CMPD 8 | |
|---|---|---|---|---|
| RATE=KG/HA | 0.05 | 0.01 | 0.05 | 0.01 |
| PREEMERGENCE | | | | |
| COTTON | 3C,5G | 0 | 0 | 0 |
| MORNINGGLORY | 0 | 0 | 0 | 0 |
| COCKLEBUR | 8G | 0 | 1C | 0 |
| NUTSEDGE | 0 | 0 | 0 | 0 |
| CRABGRASS | 2C,3G | 0 | 0 | 0 |
| BARNYARDGRASS | 0 | 0 | 0 | 0 |
| WILD OATS | 3G | 0 | 0 | 0 |
| WHEAT | 9H | 3G | 0 | 0 |
| CORN | 3C,5G | 0 | 3C,8H | 0 |
| SOYBEANS | 3C,6H | 0 | 2C,2H | 0 |
| RICE | 8H | 0 | 7G | 0 |
| SORGHUM | 4C,8H | 3C,7G | 3C,9H | 0 |
| SUGARBEETS | 3C,7H | 2G | 2H | 0 |
| VELVETLEAF | 0 | 0 | 3H | 0 |
| GIANT FOXTAIL | 3C,7G | 3G | 3G | 0 |
| BARLEY | 3C,8H | 3G | 0 | 0 |
| DOWNY BROME | 4C,9G | 4G | 3G | 0 |

TABLE A

| | CMPD 9 | | CMPD 10 | |
|---|---|---|---|---|
| RATE=KG/HA | 0.05 | 0.01 | 0.05 | 0.01 |
| POSTEMERGENCE | | | | |
| COTTON | 5C,9G | 3C,8G | 5C,9G | 4C,9G |
| MORNINGGLORY | 4C,8G | 1C | 9C | 4C,9G |
| COCKLEBUR | 2C,5G | 0 | 9C | 5C,9G |
| NUTSEDGE | 3C,8G | 3C,8G | 4C,9G | - |
| CRABGRASS | 3C,7G | 3G | 3C,7G | 3C,5G |
| BARNYARDGRASS | 3C,7G | 0 | 4C,8H | 3C,7G |
| WILD OATS | 4C,8G | 2G | 4C,9G | 3C,8G |
| WHEAT | 9G | 8G | 5C,9G | 3C,8G |
| CORN | 9C | 9C | 5C,9G | 3C,9G |
| SOYBEANS | 9C | 4C,9G | 5C,9G | 5C,9G |
| RICE | 9C | 5C,9G | 4C,9G | 3C,8G |
| SORGHUM | 9C | 4C,9H | 9C | 5C,9G |
| SUGARBEETS | 9C | 3C,7H | 9C | 9C |
| VELVETLEAF | 3G | 3C,7G | 10C | 5C,9G |
| GIANT FOXTAIL | 9C | 3C,9G | 5C,9G | 4C,9G |
| BARLEY | 9C | 9C | 2C,8G | 2C,4G |
| DOWNY BROME | 3C,8G | 3C,8G | 5C,9G | 3C,9G |

TABLE A

| | CMPD 9 | | CMPD 10 | |
|---|---|---|---|---|
| RATE=KG/HA | 0.05 | 0.01 | 0.05 | 0.01 |
| PREEMERGENCE | | | | |
| COTTON | 2C,6G | 0 | 6G | 3G |
| MORNINGGLORY | 0 | 0 | 3C,6H | 1C |
| COCKLEBUR | 1C | 0 | 2C | 0 |
| NUTSEDGE | 0 | 0 | 0 | 0 |
| CRABGRASS | 2C,3G | 0 | 3C,7G | 3C,5G |
| BARNYARDGRASS | 2C | 0 | 3C,6G | 0 |
| WILD OATS | 2G | 0 | 3C,7G | 0 |
| WHEAT | 9H | 7G | 2C,9G | 4G |
| CORN | 3C,8H | 2G | 3C,8H | 2C,3G |
| SOYBEANS | 3C,5H | 0 | 3C,7H | 3C,3H |
| RICE | 8H | 0 | 3C,6G | 0 |
| SORGHUM | 9H | 3C,7G | 3C,8G | 3C,4G |
| SUGARBEETS | 3H | 2H | 6H | 2H |
| VELVETLEAF | 1C | 0 | 3C,6G | 2C,2H |
| GIANT FOXTAIL | 5C,9G | 7G | 3C,7G | 3C,5G |
| BARLEY | 9G | 2C,7G | 3C,7H | 0 |
| DOWNY BROME | 8G | 3G | 4C,8G | 3G |

TABLE A

| CMPD 12 | | |
|---|---|---|
| RATE=KG/HA | 0.05 | 0.01 |
| POSTEMERGENCE | | |
| COTTON | 4C,9G | 4C,9H |
| MORNINGGLORY | 1C | 3C,3H |
| COCKLEBUR | 4C,9G | 3C,7H |
| NUTSEDGE | 2C,5G | 3C,5G |
| CRABGRASS | 0 | 0 |
| BARNYARDGRASS | 0 | 0 |
| WILD OATS | 3C,6G | 3C,6G |
| WHEAT | 2G | 2G |
| CORN | 4C,9H | 4C,9G |
| SOYBEANS | 4C,9G | 4C,8H |
| RICE | 4C,9G | 5C,9G |
| SORGHUM | 3C,9H | 3C,7G |
| SUGARBEETS | 4C,8H | 9C |
| VELVETLEAF | 4C,9H | 3C,8H |
| GIANT FOXTAIL | 0 | 0 |
| BARLEY | 3C,5G | 2G |
| DOWNY BROME | 3C,7G | 2C,5G |

TABLE A

| CMPD 12 | | |
|---|---|---|
| RATE=KG/HA | 0.05 | 0.01 |
| PREEMERGENCE | | |
| COTTON | 2C,5G | 2G |
| MORNINGGLORY | 0 | 0 |
| COCKLEBUR | 4G | 0 |
| NUTSEDGE | 0 | 0 |
| CRABGRASS | 1C | 0 |
| BARNYARDGRASS | 2C | 0 |
| WILD OATS | 3G | 0 |
| WHEAT | 3G | 0 |
| CORN | 3C,3G | 0 |
| SOYBEANS | 3C,4H | 0 |
| RICE | 7G | 1C |
| SORGHUM | 3C,8G | 2C,2G |
| SUGARBEETS | 0 | 0 |
| VELVETLEAF | 2H | 0 |
| GIANT FOXTAIL | 2G | 0 |
| BARLEY | 3C,6G | 0 |
| DOWNY BROME | 8G | 2G |

TABLE A

CMPD 13

| RATE=KG/HA | 0.05 | 0.01 |
|---|---|---|
| POSTEMERGENCE | | |
| COTTON | 4C,9H | 4C,6H |
| MORNINGGLORY | 3C,4G | 1C |
| COCKLEBUR | 4C,9H | 3C,5H |
| NUTSEDGE | 3C,5G | 0 |
| CRABGRASS | 0 | 0 |
| BARNYARDGRASS | 0 | 0 |
| WILD OATS | 3C,5G | 0 |
| WHEAT | 3G | 0 |
| CORN | 3C,9G | 3C,7H |
| SOYBEANS | 3C,8H | 3C,7H |
| RICE | 5C,9G | 3C,7G |
| SORGHUM | 2C,9G | 3C,7G |
| SUGARBEETS | 5C,9G | 4C,7H |
| VELVETLEAF | 3C,9G | 3C,7H |
| GIANT FOXTAIL | 1C | 0 |
| BARLEY | 2C,3G | 1C |
| DOWNY BROME | 3C,7G | 2C,5G |

TABLE A

CMPD 13

| RATE=KG/HA | 0.05 | 0.01 |
|---|---|---|
| PREEMERGENCE | | |
| COTTON | 0 | 0 |
| MORNINGGLORY | 0 | 0 |
| COCKLEBUR | 0 | 0 |
| NUTSEDGE | 0 | 0 |
| CRABGRASS | 0 | 0 |
| BARNYARDGRASS | 0 | 0 |
| WILD OATS | 0 | 0 |
| WHEAT | 0 | 0 |
| CORN | 0 | 0 |
| SOYBEANS | 0 | 0 |
| RICE | 0 | 0 |
| SORGHUM | 2C | 0 |
| SUGARBEETS | 2G | 0 |
| VELVETLEAF | 0 | 0 |
| GIANT FOXTAIL | 0 | 0 |
| BARLEY | 0 | 0 |
| DOWNY BROME | 0 | 0 |

TABLE A

CMPD 18

| RATE=KG/HA | 0.05 | 0.01 |
|---|---|---|
| POSTEMERGENCE | | |
| COTTON | 2G | 0 |
| MORNINGGLORY | 0 | 0 |
| COCKLEBUR | 0 | 0 |
| NUTSEDGE | 3G | 0 |
| CRABGRASS | 3G | 0 |
| BARNYARDGRASS | 0 | 0 |
| WILD OATS | 2G | 0 |
| WHEAT | 2G | 0 |
| CORN | 2C,5G | 0 |
| SOYBEANS | 3C,3H | 0 |
| RICE | 2C,5G | 0 |
| SORGHUM | 3C,8G | 2G |
| CHEATGRASS | 9G | 2G |
| SUGARBEETS | 2H | 0 |
| VELVETLEAF | 3G | 0 |
| GIANT FOXTAIL | 4G | 0 |
| BARLEY | 0 | 0 |
| DOWNY BROME | - | - |

TABLE A

CMPD 18

| RATE=KG/HA | 0.05 | 0.01 |
|---|---|---|
| PREEMERGENCE | | |
| COTTON | 0 | 0 |
| MORNINGGLORY | 0 | 0 |
| COCKLEBUR | 0 | 0 |
| NUTSEDGE | 0 | 0 |
| CRABGRASS | 0 | 0 |
| BARNYARDGRASS | 0 | 0 |
| WILD OATS | 0 | 0 |
| WHEAT | 0 | 0 |
| CORN | 0 | 0 |
| SOYBEANS | 0 | 0 |
| RICE | 0 | 0 |
| SORGHUM | 0 | 0 |
| CHEATGRASS | 0 | 0 |
| SUGARBEETS | 2G | 0 |
| VELVETLEAF | 0 | 0 |
| GIANT FOXTAIL | 0 | 0 |
| BARLEY | 0 | 0 |
| DOWNY BROME | - | - |

TABLE A

| | CMPD 21 | | CMPD 22 | |
|---|---|---|---|---|
| RATE=KG/HA | 0.05 | 0.01 | 0.05 | 0.01 |
| POSTEMERGENCE | | | | |
| COTTON | 7G | 8G | 6G | 1H |
| MORNINGGLORY | 2C,4G | 0 | 2C,3G | 1H |
| COCKLEBUR | 4G | 0 | 2G | 0 |
| NUTSEDGE | 2C,8G | – | 0 | 0 |
| CRABGRASS | 3C,7G | 2G | 3C,5G | 0 |
| BARNYARDGRASS | 2H | 0 | 0 | 0 |
| WILD OATS | 2C,6G | 0 | 2G | 0 |
| WHEAT | 8G | 4G | 8G | 4G |
| CORN | 3C,9H | 2G | 3C,9H | 5G |
| SOYBEANS | 3C,7H | 2H | 3C,4G | 1C |
| RICE | 9C | 3C,9G | 5C,9G | 8G |
| SORGHUM | 4C,9H | 3C,8G | 4C,9H | 5G |
| CHEATGRASS | 9G | 8G | 9G | 6G |
| SUGARBEETS | 3C,7H | 4H | 2H | 1H |
| VELVETLEAF | 5G | 0 | 4G | 0 |
| GIANT FOXTAIL | 3C,8G | 4G | 3C,7G | 3G |
| BARLEY | 7G | 0 | 3G | 0 |

TABLE A

| | CMPD 21 | | CMPD 22 | |
|---|---|---|---|---|
| RATE=KG/HA | 0.05 | 0.01 | 0.05 | 0.01 |
| PREEMERGENCE | | | | |
| COTTON | 0 | 0 | 0 | 0 |
| MORNINGGLORY | 0 | 0 | 0 | 0 |
| COCKLEBUR | 2H | 0 | 0 | 0 |
| NUTSEDGE | 0 | 0 | 0 | 0 |
| CRABGRASS | 0 | 0 | 0 | 0 |
| BARNYARDGRASS | 0 | 0 | 0 | 0 |
| WILD OATS | 0 | 0 | 0 | 0 |
| WHEAT | 0 | 0 | 0 | 0 |
| CORN | 0 | 0 | 0 | 0 |
| SOYBEANS | 1H | 0 | 0 | 0 |
| RICE | 2G | 0 | 0 | 0 |
| SORGHUM | 4G | 0 | 0 | 0 |
| CHEATGRASS | 0 | 0 | 0 | 0 |
| SUGARBEETS | 3G | 0 | 0 | 0 |
| VELVETLEAF | 0 | 0 | 0 | 0 |
| GIANT FOXTAIL | 0 | 0 | 0 | 0 |
| BARLEY | 0 | 0 | 0 | 0 |

TABLE A

| RATE=KG/HA | CMPD 25 0.05 | CMPD 25 0.01 | CMPD 26 0.05 | CMPD 26 0.01 |
|---|---|---|---|---|
| POSTEMERGENCE | | | | |
| COTTON | 9C | 3C,8G | 4C,9H | 4C,9G |
| MORNINGGLORY | 6C,9G | 3C,8H | 9C | 4C,9G |
| COCKLEBUR | 4C,9G | 2C,6G | 5C,9G | 3C,8H |
| NUTSEDGE | 8G | 5G | – | 0 |
| CRABGRASS | 2C,5G | 0 | 3G | 2G |
| BARNYARDGRASS | 4C,9H | 3C,7G | 3C,7H | 3C,6G |
| WILD OATS | 2C,9G | 2C,8G | 3C,9G | 2C,7G |
| WHEAT | 9G | 4G | 9G | 8G |
| CORN | 3C,9G | 4C,9G | 4C,9H | 3C,7G |
| SOYBEANS | 4C,9G | 3C,7G | 4C,9G | 3C,8G |
| RICE | 5C,9G | 4C,8G | 5C,9G | 3C,8G |
| SORGHUM | 4C,9G | 3C,7G | 4C,9H | 3C,7G |
| CHEATGRASS | 3C,8G | 4G | 4C,9G | 7G |
| SUGARBEETS | 9C | 5C,9G | 3C,7H | 3C,6H |
| VELVETLEAF | 5C,9H | 2C,5G | 3C,8H | 6G |
| GIANT FOXTAIL | 4C,9G | 5G | 3C,7G | 3G |
| BARLEY | 3C,8G | 6G | 3C,9G | 2C,7G |

TABLE A

| RATE=KG/HA | CMPD 25 0.05 | CMPD 25 0.01 | CMPD 26 0.05 | CMPD 26 0.01 |
|---|---|---|---|---|
| PREEMERGENCE | | | | |
| COTTON | 7G | 2G | 7G | 0 |
| MORNINGGLORY | 2C,5G | 2H | 5G | 2G |
| COCKLEBUR | 2G | 2G | 1C,1H | 0 |
| NUTSEDGE | 0 | 0 | 0 | – |
| CRABGRASS | 0 | 0 | 0 | 0 |
| BARNYARDGRASS | 3G | 0 | 0 | 0 |
| WILD OATS | 2C,3G | 0 | 2C,2G | 0 |
| WHEAT | 8G | 0 | 5G | 0 |
| CORN | 2C,9G | 2G | 3C,5G | 0 |
| SOYBEANS | 3C,7H | 2C,1H | 3C,5G | 2G |
| RICE | 3C,7G | 0 | 3C,5G | 0 |
| SORGHUM | 3C,7H | 2C,3G | 3C,3G | 0 |
| CHEATGRASS | 7G | 0 | 5G | 0 |
| SUGARBEETS | 7G | 4G | 3H | 2G |
| VELVETLEAF | 2C,2H | 1H | 1C,1H | 0 |
| GIANT FOXTAIL | 4G | 0 | 3G | 0 |
| BARLEY | 2C,8H | 3G | 6G | 0 |

TABLE A

| | CMPD 28 | |
|---|---|---|
| RATE=KG/HA | 0.05 | 0.01 |
| POSTEMERGENCE | | |
| COTTON | 4C,9G | 3C,8G |
| MORNINGGLORY | 5C,9G | 4C,9H |
| COCKLEBUR | 4C,8H | 4G |
| NUTSEDGE | 6G | – |
| CRABGRASS | 5G | 3G |
| BARNYARDGRASS | 4C,9H | 3C,8H |
| WILD OATS | 2C,6G | 2G |
| WHEAT | 9G | 6G |
| CORN | 4C,9G | 3C,8H |
| SOYBEANS | 3C,7G | 3C,6H |
| RICE | 3C,6G | 1C,2G |
| SORGHUM | 5C,9G | 3C,9H |
| CHEATGRASS | 3C,7G | 5G |
| SUGARBEETS | 5C,9G | 3C,7H |
| VELVETLEAF | 2C,6G | 6G |
| GIANT FOXTAIL | 3C,8G | 3C,7G |
| BARLEY | 3C,5G | 3G |

TABLE A

| | CMPD 28 | |
|---|---|---|
| RATE=KG/HA | 0.05 | 0.01 |
| PREEMERGENCE | | |
| COTTON | 0 | 0 |
| MORNINGGLORY | 2C,2G | 0 |
| COCKLEBUR | 0 | 0 |
| NUTSEDGE | 0 | 0 |
| CRABGRASS | 0 | 0 |
| BARNYARDGRASS | 2G | 0 |
| WILD OATS | 0 | 0 |
| WHEAT | 0 | 0 |
| CORN | 2C,3G | 0 |
| SOYBEANS | 3C,5G | 0 |
| RICE | 0 | 0 |
| SORGHUM | 3C,3G | 0 |
| CHEATGRASS | 3G | 0 |
| SUGARBEETS | 5G | 0 |
| VELVETLEAF | 1H | 0 |
| GIANT FOXTAIL | 2G | 0 |
| BARLEY | 0 | 0 |

TABLE A

| | CMPD 29 | | CMPD 30 | |
|---|---|---|---|---|
| RATE=KG/HA | 0.05 | 0.01 | 0.05 | 0.01 |
| POSTEMERGENCE | | | | |
| COTTON | 9C | 5C,9G | 9C | 3C,9H |
| MORNINGGLORY | 10C | 9C | 10C | 5C,9G |
| COCKLEBUR | 9C | 5C,9G | 5C,9G | 4C,9H |
| NUTSEDGE | 3C,8G | 8G | 2C,5G | 4G |
| CRABGRASS | 8G | 2C,5G | 3C,9G | 2C,6G |
| BARNYARDGRASS | 5C,9G | 4C,9G | 5C,9G | 3C,8H |
| WILD OATS | 4C,9G | 3C,9G | 3C,9G | 3C,8G |
| WHEAT | 3C,9G | 8G | 3C,9G | 9G |
| CORN | 4C,9G | 4C,9G | 9C | 3C,9G |
| SOYBEANS | 5C,9G | 4C,9G | 6C,9G | 4C,8G |
| RICE | 9C | 5C,9G | 9C | 4C,9G |
| SORGHUM | 5C,9G | 4C,9G | 3C,8G | 4C,8G |
| CHEATGRASS | 6C,9G | 4C,8G | 3C,9G | 9G |
| SUGARBEETS | 9C | 9C | 9C | 9C |
| VELVETLEAF | 10C | 4C,8H | 10C | 3C,8H |
| GIANT FOXTAIL | 4C,9G | 3C,8G | 5C,9G | 3C,7G |
| BARLEY | 5C,9G | 3C,9G | 5C,9G | 3C,9G |

TABLE A

| | CMPD 29 | | CMPD 30 | |
|---|---|---|---|---|
| RATE=KG/HA | 0.05 | 0.01 | 0.05 | 0.01 |
| PREEMERGENCE | | | | |
| COTTON | 8G | 2G | 0 | 0 |
| MORNINGGLORY | 6G | 3G | 0 | 0 |
| COCKLEBUR | 2C,2H | 0 | 0 | 0 |
| NUTSEDGE | 0 | 0 | 0 | 0 |
| CRABGRASS | 7G | 0 | 2G | 0 |
| BARNYARDGRASS | 3C,6H | 0 | 0 | 0 |
| WILD OATS | 2C,4G | 2G | 0 | 0 |
| WHEAT | 7G | 0 | 0 | 0 |
| CORN | 3C,8G | 0 | 1C,2G | 0 |
| SOYBEANS | 3C,6G | 0 | 1C,2G | 0 |
| RICE | 3C,7G | 0 | 2C,2G | 0 |
| SORGHUM | 4C,9H | 0 | 2C,4G | 0 |
| CHEATGRASS | 8G | 5G | 2C,5G | 0 |
| SUGARBEETS | 3C,7G | 2H | 5H | 0 |
| VELVETLEAF | 3C,8H | 1H | 0 | 0 |
| GIANT FOXTAIL | 3C,8G | 0 | 3G | 0 |
| BARLEY | 7G | 0 | 7G | 0 |

TABLE A

CMPD 31

| RATE=KG/HA | 0.4 | 0.05 |
|---|---|---|
| POSTEMERGENCE | | |
| COTTON | 9C | 9C |
| MORNINGGLORY | 9C | 5C,9G |
| COCKLEBUR | 5C,9G | 4C,8H |
| NUTSEDGE | 3C,7G | 5G |
| CRABGRASS | 3C,7G | 6G |
| BARNYARDGRASS | 4C,8H | 3C,5H |
| WILD OATS | 5C,9G | 8G |
| WHEAT | 9G | 9G |
| CORN | 3C,9G | 4C,9G |
| SOYBEANS | 9C | 4C,9G |
| RICE | 5C,9G | 2C,6G |
| SORGHUM | 6C,9H | 3C,7G |
| CHEATGRASS | 3C,9G | 9G |
| SUGARBEETS | 9C | 5C,8H |
| VELVETLEAF | 9C | 4C,9H |
| GIANT FOXTAIL | 4C,9G | 3C,8G |
| BARLEY | 4C,9G | 8G |

TABLE A

CMPD 31

| RATE=KG/HA | 0.4 | 0.05 |
|---|---|---|
| PREEMERGENCE | | |
| COTTON | 3C,6G | 0 |
| MORNINGGLORY | 2C,4G | 2G |
| COCKLEBUR | 2C,2G | 0 |
| NUTSEDGE | 10E | 0 |
| CRABGRASS | 6H | 0 |
| BARNYARDGRASS | 3C,6H | 2G |
| WILD OATS | 3C,7G | 0 |
| WHEAT | 7G | 0 |
| CORN | 4C,8G | 1C |
| SOYBEANS | 4C,7H | 2C,4H |
| RICE | 3C,5H | 4G |
| SORGHUM | 4C,8H | 2C,2G |
| CHEATGRASS | 4C,8H | 0 |
| SUGARBEETS | 3C,9G | 7G |
| VELVETLEAF | 4C,9G | 4G |
| GIANT FOXTAIL | 3C,9H | 4G |
| BARLEY | 7G | 0 |

TABLE A

| | CMPD 34 | |
|---|---|---|
| RATE=KG/HA | 0.4 | 0.05 |
| POSTEMERGENCE | | |
| COTTON | 4C,9G | 2C,6G |
| MORNINGGLORY | 10C | 3C,9G |
| COCKLEBUR | 10C | 5C,9G |
| NUTSEDGE | 3C,9G | 6G |
| CRABGRASS | 6G | 2G |
| BARNYARDGRASS | 4C,9G | 3C,6H |
| WILD OATS | 9G | 2C,5G |
| WHEAT | 9G | 9G |
| CORN | 3C,9G | 2C,9G |
| SOYBEANS | 9C | 5C,9G |
| RICE | 9C | 3C,8G |
| SORGHUM | 3C,9G | 3C,7H |
| CHEATGRASS | 4C,9G | 8G |
| SUGARBEETS | 9C | 9C |
| VELVETLEAF | 10C | 5C,9H |
| GIANT FOXTAIL | 5C,9G | 2C,6G |
| BARLEY | 9G | 8G |

TABLE A

| | CMPD 34 | |
|---|---|---|
| RATE=KG/HA | 0.4 | 0.05 |
| PREEMERGENCE | | |
| COTTON | 2G | 0 |
| MORNINGGLORY | 9G | 2C,2G |
| COCKLEBUR | 8H | 2C,3H |
| NUTSEDGE | 3C,8G | 0 |
| CRABGRASS | 2G | 0 |
| BARNYARDGRASS | 4H | 2G |
| WILD OATS | 2C,5G | 2G |
| WHEAT | 6G | 0 |
| CORN | 3C,9G | 2C,2G |
| SOYBEANS | 9H | 2C,3G |
| RICE | 4C,7H | 0 |
| SORGHUM | 3C,9H | 2G |
| CHEATGRASS | 6G | 0 |
| SUGARBEETS | 9C | 8G |
| VELVETLEAF | 4C,9G | 3C,6H |
| GIANT FOXTAIL | 3C,6G | 2G |
| BARLEY | 3C,8H | 3G |

TABLE A

| | CMPD 35 | | CMPD 36 | |
|---|---|---|---|---|
| RATE=KG/HA | 0.4 | 0.05 | 0.4 | 0.05 |
| POSTEMERGENCE | | | | |
| COTTON | 10C | 5C,9G | 4C,9G | 3C,8G |
| MORNINGGLORY | 10C | 10C | 5C,9G | 3C,8H |
| COCKLEBUR | 10C | 9C | 5C,9G | 4C,9G |
| NUTSEDGE | 9C | 9C | 4C,9G | 3C,5G |
| CRABGRASS | 8G | 4C,8G | 3C,6G | 2C,4G |
| BARNYARDGRASS | 5C,9G | 3C,9H | 5C,9H | 3C,7H |
| WILD OATS | 4C,9G | 3C,7G | 4C,9G | 3C,4G |
| WHEAT | 4C,9G | 8G | 3C,9G | 9G |
| CORN | 4C,9G | 4C,9G | 4C,9H | 3C,8G |
| SOYBEANS | 5C,9G | 3C,9G | 3C,9G | 3C,7H |
| RICE | 9C | 9C | 9C | 5C,9G |
| SORGHUM | 5C,9G | 4C,9G | 5C,9G | 4C,9G |
| CHEATGRASS | 5C,9G | 3C,8G | 4C,9G | 3C,8G |
| SUGARBEETS | 10C | 9C | 4C,9G | 4C,9G |
| VELVETLEAF | 10C | 6C,9G | 4C,8H | 2C,5G |
| GIANT FOXTAIL | 4C,9G | 5C,9G | 4C,9G | 4C,9G |
| BARLEY | 5C,9G | 4C,9G | 3C,9G | 3C,9G |

TABLE A

| | CMPD 35 | | CMPD 36 | |
|---|---|---|---|---|
| RATE=KG/HA | 0.4 | 0.05 | 0.4 | 0.05 |
| PREEMERGENCE | | | | |
| COTTON | 3C,8G | 0 | 2C,3G | 0 |
| MORNINGGLORY | 9H | 3C,5H | 3C,7H | 1C |
| COCKLEBUR | 8H | 3C,4H | 3C,5H | 1C |
| NUTSEDGE | 7G | 0 | 0 | 0 |
| CRABGRASS | 2G | 0 | 2G | 0 |
| BARNYARDGRASS | 3C,7G | 1C | 2C,2G | 0 |
| WILD OATS | 3C,6G | 2C,2G | 3C,6G | 0 |
| WHEAT | 3C,9G | 6G | 3C,9H | 3G |
| CORN | 9H | 3C,8H | 4C,9H | 3C,5G |
| SOYBEANS | 3C,9H | 3C,5G | 3C,8H | 2C,2H |
| RICE | 9H | 3C,3G | 3C,8H | 0 |
| SORGHUM | 5C,9H | 3C,8H | 3C,6H | 0 |
| CHEATGRASS | 9H | 7G | 3C,8G | 3G |
| SUGARBEETS | 8G | 3C,7H | 4C,9G | 5G |
| VELVETLEAF | 4C,8H | 2C,2H | 3C,3H | 1C |
| GIANT FOXTAIL | 3C,8H | 2G | 3C,7H | 5G |
| BARLEY | 3C,9H | 8G | 2C,9H | 2C,2G |

TABLE A

| RATE=KG/HA | CMPD 37 0.4 | CMPD 37 0.05 | CMPD 38 0.4 | CMPD 38 0.05 |
|---|---|---|---|---|
| POSTEMERGENCE | | | | |
| COTTON | 4C,9G | 4C,9G | 3C,9H | 3G |
| MORNINGGLORY | 4C,9G | 4C,9H | 5G | 1C |
| COCKLEBUR | 4C,9G | 4C,8H | 2C,4G | 1C |
| NUTSEDGE | 5G | 2C,4G | 5G | 0 |
| CRABGRASS | 5C,9G | 2C,5G | 5G | 0 |
| BARNYARDGRASS | 4C,9H | 3C,7G | 3C,7G | 2C,3H |
| WILD OATS | 3C,6G | 2C,2G | 3C,6G | 0 |
| WHEAT | 4C,9G | 8G | 8G | 6G |
| CORN | 4C,9G | 4C,9H | 2C,4G | 1C |
| SOYBEANS | 3C,5H | 3H | 2C,3G | 0 |
| RICE | 4C,8G | 3C,5G | 4C,9G | 3C,9G |
| SORGHUM | 4C,9G | 5C,9G | 4C,7G | 3C,7G |
| CHEATGRASS | 3C,6G | 3C,6G | 3C,7G | 6G |
| SUGARBEETS | 4C,7H | 3C,6G | 4C,7H | 3C,3H |
| VELVETLEAF | 3C,7H | 3C,5G | 2C,2G | 0 |
| GIANT FOXTAIL | 5C,9G | 4C,8G | 4C,9G | 3C,5G |
| BARLEY | 3C,8G | 3C,7G | 3C,9G | 3G |

TABLE A

| RATE=KG/HA | CMPD 37 0.4 | CMPD 37 0.05 | CMPD 38 0.4 | CMPD 38 0.05 |
|---|---|---|---|---|
| PREEMERGENCE | | | | |
| COTTON | 3C,6G | 2C | 0 | 0 |
| MORNINGGLORY | 9G | 2C,5G | 2G | 0 |
| COCKLEBUR | 5H | 3H | 2G | 0 |
| NUTSEDGE | 3C,5G | 0 | 0 | 0 |
| CRABGRASS | 0 | 0 | 0 | 0 |
| BARNYARDGRASS | 3C,7G | 0 | 0 | 0 |
| WILD OATS | 3C,4G | 0 | 2C,2G | 0 |
| WHEAT | 8G | 2G | 3G | 0 |
| CORN | 4C,9G | 3C,5G | 3C,3G | 0 |
| SOYBEANS | 4C,7H | 1C,1H | 0 | 0 |
| RICE | 9H | 0 | 1C | 0 |
| SORGHUM | 3C,9H | 3C,6G | 2C,3G | 0 |
| CHEATGRASS | 7G | 0 | 0 | 0 |
| SUGARBEETS | 9G | 3C,5G | 3G | 0 |
| VELVETLEAF | 3C,4G | 2C,2H | 0 | 0 |
| GIANT FOXTAIL | 4C,8H | 5G | 0 | 0 |
| BARLEY | 3C,9H | 3C,7G | 0 | 0 |

TABLE A

CMPD 39

| RATE=KG/HA | 0.4 | 0.05 |
|---|---|---|
| POSTEMERGENCE | | |
| COTTON | 4C,9G | 4G |
| MORNINGGLORY | 1C | 0 |
| COCKLEBUR | 2C,5G | 0 |
| NUTSEDGE | 0 | 0 |
| CRABGRASS | 4C,9G | 5G |
| BARNYARDGRASS | 2C,3G | 0 |
| WILD OATS | 6G | 0 |
| WHEAT | 9G | 0 |
| CORN | 3C,3G | 2G |
| SOYBEANS | 3G | 0 |
| RICE | 5C,9G | 5G |
| SORGHUM | 3C,6G | 4G |
| CHEATGRASS | 3C,9G | 0 |
| SUGARBEETS | 3C,7G | 0 |
| VELVETLEAF | 0 | 0 |
| GIANT FOXTAIL | 4C,9G | 0 |
| BARLEY | 9G | 5G |

TABLE A

CMPD 39

| RATE=KG/HA | 0.4 | 0.05 |
|---|---|---|
| PREEMERGENCE | | |
| COTTON | 0 | 0 |
| MORNINGGLORY | 0 | 0 |
| COCKLEBUR | 0 | 0 |
| NUTSEDGE | 0 | 0 |
| CRABGRASS | 2G | 0 |
| BARNYARDGRASS | 2G | 0 |
| WILD OATS | 0 | 0 |
| WHEAT | 5G | 0 |
| CORN | 4G | 0 |
| SOYBEANS | 2G | 0 |
| RICE | 3G | 0 |
| SORGHUM | 3G | 0 |
| CHEATGRASS | 2G | 0 |
| SUGARBEETS | 2G | 0 |
| VELVETLEAF | 0 | 0 |
| GIANT FOXTAIL | 0 | 0 |
| BARLEY | 6G | 0 |

TABLE A

| | CMPD 41 | | CMPD 42 | |
|---|---|---|---|---|
| RATE=KG/HA | 0.05 | 0.01 | 0.05 | 0.01 |
| POSTEMERGENCE | | | | |
| COTTON | 10C | 3C,9G | 7G | 5G |
| MORNINGGLORY | 10C | 10C | 2C,6G | 1C |
| COCKLEBUR | 9C | 5C,9G | 3C,8G | 0 |
| NUTSEDGE | 9G | – | 8G | 0 |
| CRABGRASS | 5C,9G | 2C,7G | 3C,7G | 0 |
| BARNYARDGRASS | 5C,9G | 3C,9H | 3C,9G | 2G |
| WILD OATS | 4C,9G | 5C,9G | 3C,7G | 6G |
| WHEAT | 3C,9G | 3C,8G | 9G | 9G |
| CORN | 5C,9G | 3C,9G | 3C,8H | 0 |
| SOYBEANS | 5C,8G | 3C,7G | 0 | 0 |
| RICE | 9C | 9C | 5C,9G | 4C,9G |
| SORGHUM | 5C,9G | 4C,8H | 2G | 0 |
| CHEATGRASS | 9C | 4C,9G | 3C,9G | 8G |
| SUGARBEETS | 9C | 9C | 5C,8G | 4C,7G |
| VELVETLEAF | 10C | 5C,8H | 2G | 0 |
| GIANT FOXTAIL | 9C | 9C | 4C,9G | 3C,7G |
| BARLEY | 5C,9G | 8G | 2C,7G | 7G |

TABLE A

| | CMPD 41 | | CMPD 42 | |
|---|---|---|---|---|
| RATE=KG/HA | 0.05 | 0.01 | 0.05 | 0.01 |
| PREEMERGENCE | | | | |
| COTTON | 4G | 0 | 0 | 0 |
| MORNINGGLORY | 3C,8H | 0 | 0 | 0 |
| COCKLEBUR | 3C,7H | 0 | 0 | 0 |
| NUTSEDGE | 7G | 0 | – | 0 |
| CRABGRASS | 4C,8H | 0 | 4G | 0 |
| BARNYARDGRASS | 3H | 0 | 0 | 0 |
| WILD OATS | 2C,7G | 0 | 0 | 0 |
| WHEAT | 8G | 0 | 0 | 0 |
| CORN | 4C,8G | 0 | 0 | 0 |
| SOYBEANS | 3C,8G | 0 | 0 | 0 |
| RICE | 10H | 0 | 0 | 0 |
| SORGHUM | 3C,9H | 0 | 0 | 0 |
| CHEATGRASS | 9H | 5G | 7G | 0 |
| SUGARBEETS | 4C,9G | 4G | 2C | 0 |
| VELVETLEAF | 3C,6H | 0 | 0 | 0 |
| GIANT FOXTAIL | 4C,9H | 4G | 4G | 0 |
| BARLEY | 3C,8G | 0 | 0 | 0 |

TABLE A

CMPD 43

| RATE=KG/HA | 0.05 | 0.01 |
|---|---|---|
| POSTEMERGENCE | | |
| COTTON | 9H | 2G |
| MORNINGGLORY | 3C,3H | 0 |
| COCKLEBUR | 2C,5H | 0 |
| NUTSEDGE | 4G | 0 |
| CRABGRASS | 4G | 0 |
| BARNYARDGRASS | 3C,7H | 2G |
| WILD OATS | 6G | 2C,4G |
| WHEAT | 8G | 5G |
| CORN | 4C,8H | 2C,5G |
| SOYBEANS | 5G | 0 |
| RICE | 3C,9G | 0 |
| SORGHUM | 4C,7H | 0 |
| CHEATGRASS | 9G | 6G |
| SUGARBEETS | 5C,8H | 3C,5H |
| VELVETLEAF | 0 | 0 |
| GIANT FOXTAIL | 5C,9G | 2C,7G |
| BARLEY | 9G | 4G |

TABLE A

CMPD 43

| RATE=KG/HA | 0.05 | 0.01 |
|---|---|---|
| PREEMERGENCE | | |
| COTTON | 0 | 0 |
| MORNINGGLORY | 0 | 0 |
| COCKLEBUR | 0 | 0 |
| NUTSEDGE | 0 | 0 |
| CRABGRASS | 0 | 0 |
| BARNYARDGRASS | 0 | 0 |
| WILD OATS | 0 | 0 |
| WHEAT | 0 | 0 |
| CORN | 0 | 0 |
| SOYBEANS | 0 | 0 |
| RICE | 0 | 0 |
| SORGHUM | 2C,4G | 0 |
| CHEATGRASS | 3G | 0 |
| SUGARBEETS | 0 | 0 |
| VELVETLEAF | 0 | 0 |
| GIANT FOXTAIL | 2C,5H | 0 |
| BARLEY | 0 | 0 |

TABLE A

| | CMPD 47 | | CMPD 48 | |
|---|---|---|---|---|
| RATE=KG/HA | 0.4 | 0.05 | 0.4 | 0.05 |
| POSTEMERGENCE | | | | |
| COTTON | 9C | 10C | 10C | 10C |
| MORNINGGLORY | 10C | 10C | 10C | 4C,7G |
| COCKLEBUR | 10C | 5C,9G | 5C,9G | 4C,8H |
| NUTSEDGE | - | 4C,9G | 3C,9G | 7G |
| CRABGRASS | 9C | 4C,9G | 4C,8G | 6G |
| BARNYARDGRASS | 9C | 5C,9G | 5C,9G | 3C,7H |
| WILD OATS | 5C,9G | 3C,9G | 4C,9G | 6G |
| WHEAT | 4C,9G | 9G | 3C,9G | 7G |
| CORN | 5C,9G | 3C,9G | 3C,9G | 3C,7H |
| SOYBEANS | 5C,9G | 5C,9G | 5C,9G | 4C,7H |
| RICE | 9C | 9C | 9C | 3C,8G |
| SORGHUM | 4C,9G | 3C,9G | 4C,9G | 3C,9H |
| CHEATGRASS | 5C,9G | 4C,9G | 3C,9G | 7G |
| SUGARBEETS | 9C | 9C | 5C,9G | 5C,8H |
| VELVETLEAF | 10C | 10C | 10C | 2C,6G |
| GIANT FOXTAIL | 9C | 9C | 5C,9G | 3C,8G |
| BARLEY | 4C,9G | 4C,9G | 9G | 6G |

TABLE A

| | CMPD 47 | | CMPD 48 | |
|---|---|---|---|---|
| RATE=KG/HA | 0.4 | 0.05 | 0.4 | 0.05 |
| PREEMERGENCE | | | | |
| COTTON | 8G | 2G | 7G | 0 |
| MORNINGGLORY | 9G | 6G | 4G | 0 |
| COCKLEBUR | 8H | 2C,4H | 3C,4H | 2G |
| NUTSEDGE | 10E | 0 | 4G | 0 |
| CRABGRASS | 5C,8H | 2G | 2C,5H | 0 |
| BARNYARDGRASS | 9H | 0 | 9H | 3H |
| WILD OATS | 3C,8H | 2C,6G | 2C,5G | 2G |
| WHEAT | 8H | 8G | 8H | 3G |
| CORN | 9H | 3C,8G | 9G | 3C,6G |
| SOYBEANS | 9H | 2C,5G | 4C,8H | 2G |
| RICE | 10H | 7H | 3C,7H | 2G |
| SORGHUM | 3C,9H | 8H | 9H | 2C,6H |
| CHEATGRASS | 9H | 7H | 8H | 0 |
| SUGARBEETS | 4C,9G | 8G | 3C,8G | 3G |
| VELVETLEAF | 4C,8G | 3H | 4C,8G | 4H |
| GIANT FOXTAIL | 6C,9H | 2C,7G | 3C,8H | 2H |
| BARLEY | 9H | 7G | 2C,7G | 0 |

TABLE A

| | CMPD 49 | | CMPD 50 | |
|---|---|---|---|---|
| RATE=KG/HA | 0.4 | 0.05 | 0.4 | 0.05 |
| POSTEMERGENCE | | | | |
| COTTON | 5C,9G | 4C,9G | 3C,9G | 2G |
| MORNINGGLORY | 9C | 4C,8H | 2C,4G | 0 |
| COCKLEBUR | 4C,9H | 3C,7H | 4C,6G | 4G |
| NUTSEDGE | 9C | 3C,8G | 3G | 0 |
| CRABGRASS | 3C,7G | 2G | 2G | 0 |
| BARNYARDGRASS | 4C,9H | 3C,8H | 4C,8H | 2C,5H |
| WILD OATS | 5G | 0 | 3C,9G | 4G |
| WHEAT | 5G | 0 | 7G | 0 |
| CORN | 4C,9G | 3C,7G | 3C,9H | 4C,8H |
| SOYBEANS | 3C,6G | 6H | 4C,8G | 3C,4G |
| RICE | 4C,9G | 6G | 3C,7G | 2G |
| SORGHUM | 5C,9G | 3C,8G | 4C,9H | 3C,8H |
| CHEATGRASS | 7G | 0 | 0 | 0 |
| SUGARBEETS | 9C | 3C,7H | 4C,7H | 2C,2G |
| VELVETLEAF | 9C | 8G | 4C,7H | 2C,6H |
| GIANT FOXTAIL | 3C,8G | 2G | 3C,6G | 3G |
| BARLEY | 8G | 5G | 2G | 0 |

TABLE A

| | CMPD 49 | | CMPD 50 | |
|---|---|---|---|---|
| RATE=KG/HA | 0.4 | 0.05 | 0.4 | 0.05 |
| PREEMERGENCE | | | | |
| COTTON | 6G | 0 | 0 | 0 |
| MORNINGGLORY | 1H | 0 | 0 | 0 |
| COCKLEBUR | 2C,3H | 0 | 0 | 0 |
| NUTSEDGE | 3C,5G | 0 | 0 | 0 |
| CRABGRASS | 4C,9G | 0 | 0 | 0 |
| BARNYARDGRASS | 2C,2G | 0 | 0 | 0 |
| WILD OATS | 0 | 0 | 2G | 0 |
| WHEAT | 0 | 0 | 0 | 0 |
| CORN | 4C,9G | 3G | 2C,3G | 0 |
| SOYBEANS | 2C,3G | 0 | 1C,2G | 0 |
| RICE | 7G | 0 | 0 | 0 |
| SORGHUM | 4C,9G | 3C,8G | 4C,9H | 0 |
| CHEATGRASS | 5G | 0 | 0 | 0 |
| SUGARBEETS | 2C,3G | 2G | 3G | 0 |
| VELVETLEAF | 3C,8G | 1H | 0 | 0 |
| GIANT FOXTAIL | 3C,6G | 0 | 0 | 0 |
| BARLEY | 0 | 0 | 0 | 0 |

TABLE A

| | CMPD 51 | | CMPD 52 | |
|---|---|---|---|---|
| RATE=KG/HA | 0.4 | 0.05 | 0.05 | 0.01 |
| POSTEMERGENCE | | | | |
| COTTON | 5C,9G | 3C,9G | 4C,9G | 3C,7G |
| MORNINGGLORY | 9C | 5C,9G | 3C,8G | 3C,5G |
| COCKLEBUR | 10C | 5C,9G | 5C,9G | 5C,9G |
| NUTSEDGE | 4C,9G | 2C,8G | 5C,9G | 5G |
| CRABGRASS | 5G | 2G | 2C,5G | 2G |
| BARNYARDGRASS | 3C,8H | 3C,7H | 3C,7H | 3C,7H |
| WILD OATS | 5C,9G | 8G | 3C,6G | 3C,6G |
| WHEAT | 9C | 9G | 4C,9G | 3C,5G |
| CORN | 3C,9G | 3C,9H | 3C,9H | 3C,9G |
| SOYBEANS | 9C | 5C,9G | 4C,9G | 4C,9G |
| RICE | 9C | 5C,9G | 5C,9G | 5C,9G |
| SORGHUM | 4C,9G | 3C,9G | 4C,9G | 4C,9H |
| CHEATGRASS | 9C | 8G | 3G | 2G |
| SUGARBEETS | 9C | 9C | 9C | 10C |
| VELVETLEAF | 9C | 4C,9G | 5C,9H | 4C,8H |
| GIANT FOXTAIL | 4C,9G | 3C,7G | 3C,6G | 2G |
| BARLEY | 4C,9G | 2C,9G | 6G | 2G |

TABLE A

| | CMPD 51 | | CMPD 52 | |
|---|---|---|---|---|
| RATE=KG/HA | 0.4 | 0.05 | 0.05 | 0.01 |
| PREEMERGENCE | | | | |
| COTTON | 4C,8G | 4G | 2C,3G | 0 |
| MORNINGGLORY | 3C,8G | 3H | 0 | 0 |
| COCKLEBUR | 3C,8H | 3C,6H | 3C,3H | 0 |
| NUTSEDGE | 9G | 0 | 0 | 0 |
| CRABGRASS | 4C,9G | 7G | 5G | 2G |
| BARNYARDGRASS | 3C,7H | 1H | 1C,1H | 0 |
| WILD OATS | 4C,8G | 2C,4G | 2C,5G | 2G |
| WHEAT | 9G | 6G | 4G | 0 |
| CORN | 3C,9H | 5G | 3C,7G | 2C,4G |
| SOYBEANS | 4C,8H | 3C,6G | 3C,7G | 3G |
| RICE | 4C,9H | 3C,7G | 4C,7G | 3G |
| SORGHUM | 9G | 5G | 3C,9H | 3G |
| CHEATGRASS | 4C,8G | 7G | 3C,6G | 2G |
| SUGARBEETS | 5C,9G | 3C,8G | 4C,9G | 7G |
| VELVETLEAF | 4C,9G | 3C,7H | 3C,7H | 3C,3H |
| GIANT FOXTAIL | 3C,8H | 1C | 3G | 0 |
| BARLEY | 2C,8G | 3G | 3G | 0 |

TABLE A

| | CMPD 53 | | CMPD 54 | |
|---|---|---|---|---|
| RATE=KG/HA | 0.05 | 0.01 | 0.05 | 0.01 |
| POSTEMERGENCE | | | | |
| COTTON | 5C,9G | 9G | 9H | 7H |
| MORNINGGLORY | 10C | 6C,9G | 4C,8H | 1C,1H |
| COCKLEBUR | 5C,9G | 5C,9G | 4C,8H | 3C,5H |
| NUTSEDGE | 0 | - | 0 | 0 |
| CRABGRASS | 5C,9G | 2C,6G | 0 | 0 |
| BARNYARDGRASS | 4C,9G | 3C,9G | 4C,9H | 3C,5H |
| WILD OATS | 9G | 4G | 3C,9G | 6G |
| WHEAT | 9C | 8G | 9G | 9G |
| CORN | 3C,9G | 3C,9G | 3C,9G | 2C,5H |
| SOYBEANS | 4C,9G | 4C,9G | 3C,6H | 1C |
| RICE | 9C | 9C | 3C,8G | 3C,8G |
| SORGHUM | 5C,9G | 3C,9G | 2C,5H | 2C,2H |
| CHEATGRASS | 9G | 4C,9G | 7G | 8G |
| SUGARBEETS | 9C | 5C,9H | 9C | 4C,7H |
| VELVETLEAF | 4C,9H | 4C,8H | 2C,3H | 2H |
| GIANT FOXTAIL | 5C,9G | 3C,9G | 3C,9G | 3C,8G |
| BARLEY | 9G | 9G | 9G | 7G |

TABLE A

| | CMPD 53 | | CMPD 54 | |
|---|---|---|---|---|
| RATE=KG/HA | 0.05 | 0.01 | 0.05 | 0.01 |
| PREEMERGENCE | | | | |
| COTTON | 2C,2G | 0 | 0 | 0 |
| MORNINGGLORY | 3C,5H | 0 | 3G | 0 |
| COCKLEBUR | 3C | 2C | 0 | 0 |
| NUTSEDGE | 10E | 0 | 0 | 0 |
| CRABGRASS | 2C,6G | 0 | 0 | 0 |
| BARNYARDGRASS | 4C,9H | 0 | 0 | 0 |
| WILD OATS | 2C,6G | 0 | 2G | 0 |
| WHEAT | 8G | 2G | 2G | 0 |
| CORN | 3C,9G | 0 | 0 | 0 |
| SOYBEANS | 4C,7H | 2C,3G | 1C | 0 |
| RICE | 4C,8H | 2G | 0 | 0 |
| SORGHUM | 9G | 2C,5H | 2C,2G | 0 |
| CHEATGRASS | 9H | 2G | 2C,6H | 0 |
| SUGARBEETS | 9G | 9G | 6G | 0 |
| VELVETLEAF | 3C,6H | 0 | 0 | 0 |
| GIANT FOXTAIL | 3C,9H | 7H | 3C,7H | 0 |
| BARLEY | 9G | 5G | 0 | 0 |

TABLE A

| CMPD 55 | | |
|---|---|---|
| RATE=KG/HA | 0.05 | 0.01 |
| POSTEMERGENCE | | |
| COTTON | 9H | 8H |
| MORNINGGLORY | 4C,7H | 1C,1H |
| COCKLEBUR | 4C,8H | 2C,3H |
| NUTSEDGE | 2C,6G | 0 |
| CRABGRASS | 4G | 2G |
| BARNYARDGRASS | 5C,9G | 3C,6H |
| WILD OATS | 2C,5G | 2G |
| WHEAT | 3C,6G | 7G |
| CORN | 9G | 9G |
| SOYBEANS | 2C,3H | 0 |
| RICE | 3C,7G | 3G |
| SORGHUM | 4C,9G | 4C,8H |
| CHEATGRASS | 7G | 7G |
| SUGARBEETS | 9C | 5C,8H |
| VELVETLEAF | 2C,2H | 0 |
| GIANT FOXTAIL | 3C,9G | 2C,7G |
| BARLEY | 9G | 8G |

TABLE A

| CMPD 55 | | |
|---|---|---|
| RATE=KG/HA | 0.05 | 0.01 |
| PREEMERGENCE | | |
| COTTON | 0 | 0 |
| MORNINGGLORY | 3G | 0 |
| COCKLEBUR | 0 | 0 |
| NUTSEDGE | 0 | 0 |
| CRABGRASS | 2C,7H | 0 |
| BARNYARDGRASS | 0 | 0 |
| WILD OATS | 0 | 0 |
| WHEAT | 2G | 0 |
| CORN | 2C,2G | 0 |
| SOYBEANS | 2C,5G | 0 |
| RICE | 7H | 0 |
| SORGHUM | 3C,6H | 2C |
| CHEATGRASS | 7G | 2G |
| SUGARBEETS | 8G | 0 |
| VELVETLEAF | 0 | 0 |
| GIANT FOXTAIL | 7H | 4H |
| BARLEY | 2C,7G | 0 |

TABLE A

| | CMPD 59 |
|---|---|
| RATE=KG/HA | 0.05 |
| POSTEMERGENCE | |
| COTTON | 4C,9G |
| MORNINGGLORY | 10C |
| COCKLEBUR | 3C,7H |
| NUTSEDGE | 4C,9G |
| CRABGRASS | 3C,4G |
| BARNYARDGRASS | 5C,9G |
| WILD OATS | 3C,7G |
| WHEAT | 7G |
| CORN | 9C |
| SOYBEANS | 4C,9G |
| RICE | 5C,9G |
| SORGHUM | 9C |
| SUGARBEETS | 9C |
| VELVETLEAF | 2C,7G |
| GIANT FOXTAIL | 3C,7G |
| BARLEY | 3C,5G |

TABLE A

| | CMPD 59 |
|---|---|
| RATE=KG/HA | 0.05 |
| PREEMERGENCE | |
| COTTON | 5G |
| MORNINGGLORY | 6G |
| COCKLEBUR | 2G |
| NUTSEDGE | 0 |
| CRABGRASS | 3C,3G |
| BARNYARDGRASS | 0 |
| WILD OATS | 0 |
| WHEAT | 6G |
| CORN | 3C,7G |
| SOYBEANS | 3C,7G |
| RICE | 3C,7G |
| SORGHUM | 4C,8H |
| SUGARBEETS | 3H |
| VELVETLEAF | 3C,7H |
| GIANT FOXTAIL | 0 |
| BARLEY | 3C,5G |
| DOWNY BROME | 7G |

Test B

Post-emergence

Three round pans (25 cm diameter by 12.5 cm deep) were filled with Sassafras loam soil. One pan was planted with nutsedge (Cyperus rotundus) rubers, crabgrass (Digitaria sanguinalis), sicklepod (Cassia obtusifolia), jimsonweed (Datura stramonium), velvetleaf (Abutilon theophrasti), lambsquarters (Chenopodium album), rice (Oryza sativa), and teaweed (Sida spinosa). The second pot was planted with green foxtail (Setaria viridis), cocklebur (Xanthium pensylvanicum), morningglory (Ipomoea hederacea), cotton (Gossypium hirsutum), johnsongrass (Sorghum halepense), barnyardgrass (Echinochloa crus-galli), corn (Zea mays), soybean (Glycine max), and giant foxtail (Setaria faberi). The third pot was planted with wehat (Triticum aestivum), barley (Hordeum vulgare), wild buckwheat (Polygonum convolvulus L.), cheat grass (Bromus secalinus L.), sugarbeet (Beta vulgaris), wild oat (Avena fatua L.), common chickweed (Stellaria media), blackgrass (Alopecurus myosuroides), and rape (Brassica napus). The plants were grown for approximately fourteen days, then sprayed postemergence with the chemicals dissolved in a non-phytotoxic solvent.

Pre-emergence

Three round pans (25 cm diameter by 12.5 cm deep) were filled with Sassafras loam soil. One pan was planted with nutsedge tubers, crabgrass, sicklepod, jimsonweed, velvetleaf, lambsquarters, rice, and teaweed. The second pot was planted with gree foxtail, cocklebur, morningglory, cotton, johnsongrass, barnyardgrass, corn, soybean, and giant foxtail. The third pot was planted with wheat, barley, wild buckwheat, cheatgrass, sugarbeet, wild oat, viola, blackgrass, and rape. The three pans were sprayed pre-emergence with the chemicals dissolved in a non-phytotoxic solvent.

Treated plants and controls were maintained in the greenhouse for approximately 24 days, then all rated plants were compared to controls and visually rated for plant response.

Response ratings are based on a scale of 0 to 100 where 0 = no effect, and 100 - complete control. A dash (-) response means no test.

Response ratings are contained in Table B.

TABLE B

CMPD 1

| RATE=G/HA | 62 | 16 | 4 | 1 |
|---|---|---|---|---|
| POSTEMERGENCE | | | | |
| GIANT FOXTAIL | 30 | 0 | 0 | 0 |
| VELVETLEAF | 100 | 100 | 70 | 50 |
| SUGARBEETS | 100 | 100 | 100 | 60 |
| CRABGRASS | 100 | 40 | 0 | 0 |
| TEAWEED | 80 | 60 | 40 | 0 |
| JIMSONWEED | 100 | 90 | 60 | 30 |
| RICE | 70 | 60 | 50 | 0 |
| COCKLEBUR | 100 | 50 | 30 | 0 |
| COTTON | 80 | 60 | 30 | 0 |
| SOYBEANS | 100 | 100 | 70 | 50 |
| BARNYARDGRASS | 80 | 70 | 50 | 30 |
| WILD OATS | 90 | 70 | 50 | 30 |
| MORNINGGLORY | 100 | 70 | 50 | 30 |
| WHEAT | 70 | 50 | 30 | 0 |
| SICKLEPOD | 70 | 50 | 30 | 0 |
| JOHNSONGRASS | 100 | 80 | 60 | 40 |
| NUTSEDGE | 90 | 60 | 30 | 0 |
| CORN | 100 | 100 | 80 | 60 |
| WILD BUCKWHEAT | 90 | 70 | 50 | 30 |
| BLACKGRASS | 90 | 70 | 50 | 30 |
| RAPE | 100 | 100 | 90 | 60 |
| BARLEY | 60 | 30 | 0 | 0 |
| GREEN FOXTAIL | 50 | 30 | 0 | 0 |
| CHEATGRASS | 90 | 70 | 50 | 30 |
| LAMBSQUARTER | 90 | 60 | 0 | 0 |
| CHICKWEED | 90 | 70 | 50 | 30 |

TABLE B

| CMPD 1 | | | |
|---|---|---|---|
| RATE=G/HA | 250 | 62 | 16 |
| PREEMERGENCE | | | |
| GIANT FOXTAIL | 70 | 30 | 0 |
| VELVETLEAF | 90 | 70 | 60 |
| SUGARBEETS | 100 | 100 | 60 |
| CRABGRASS | 80 | 50 | 30 |
| TEAWEED | 90 | 70 | 50 |
| JIMSONWEED | 70 | 30 | 0 |
| RICE | 90 | 80 | 50 |
| COCKLEBUR | 50 | 0 | 0 |
| COTTON | 60 | 30 | 0 |
| SOYBEANS | 90 | 50 | 0 |
| BARNYARDGRASS | 100 | 70 | 30 |
| WILD OATS | 60 | 30 | 0 |
| MORNINGGLORY | 70 | 30 | 0 |
| WHEAT | 60 | 30 | 0 |
| SICKLEPOD | 80 | 30 | 0 |
| JOHNSONGRASS | 100 | 90 | 60 |
| NUTSEDGE | 90 | 30 | 0 |
| CORN | 90 | 30 | 0 |
| WILD BUCKWHEAT | 80 | 60 | 40 |
| BLACKGRASS | 80 | 60 | 40 |
| RAPE | 70 | 50 | 0 |
| BARLEY | 50 | 30 | 0 |
| GREEN FOXTAIL | 70 | 30 | 0 |
| CHEATGRASS | 90 | 80 | 60 |
| LAMBSQUARTER | 100 | 70 | 50 |
| CHICKWEED | 90 | 70 | 50 |

TABLE B

CMPD 2

| RATE=G/HA | 250 | 62 | 16 | 4 |
|---|---|---|---|---|
| POSTEMERGENCE | | | | |
| GIANT FOXTAIL | 70 | 60 | 30 | 0 |
| VELVETLEAF | 80 | 50 | 30 | 0 |
| SUGARBEETS | 100 | 90 | 60 | 30 |
| CRABGRASS | 30 | 0 | 0 | 0 |
| TEAWEED | 60 | 30 | 0 | 0 |
| JIMSONWEED | 70 | 60 | 50 | 30 |
| RICE | 100 | 90 | 60 | 30 |
| COCKLEBUR | 0 | 0 | 0 | 0 |
| COTTON | 0 | 0 | 0 | 0 |
| SOYBEANS | 90 | 70 | 30 | 0 |
| BARNYARDGRASS | 70 | 60 | 50 | 30 |
| WILD OATS | 70 | 50 | 30 | 0 |
| MORNINGGLORY | 60 | 50 | 30 | 0 |
| WHEAT | 90 | 60 | 30 | 0 |
| SICKLEPOD | 30 | 0 | 0 | 0 |
| JOHNSONGRASS | 90 | 70 | 50 | 30 |
| NUTSEDGE | 0 | 0 | 0 | 0 |
| CORN | 70 | 60 | 40 | 30 |
| WILD BUCKWHEAT | 90 | 60 | 30 | 0 |
| BLACKGRASS | 70 | 50 | 30 | 0 |
| RAPE | 60 | 50 | 30 | 0 |
| BARLEY | 70 | 50 | 30 | 0 |
| GREEN FOXTAIL | 70 | 60 | 50 | 40 |
| CHEATGRASS | 70 | 50 | 30 | 0 |
| LAMBSQUARTER | 0 | 0 | 0 | 0 |
| CHICKWEED | 30 | 0 | 0 | 0 |

TABLE B

| CMPD 2 | | | |
|---|---|---|---|
| RATE=G/HA | 250 | 62 | 16 |
| PREEMERGENCE | | | |
| GIANT FOXTAIL | 80 | 30 | 0 |
| VELVETLEAF | 90 | 80 | 70 |
| SUGARBEETS | 90 | 60 | 30 |
| CRABGRASS | 80 | 30 | 0 |
| TEAWEED | 30 | 0 | 0 |
| JIMSONWEED | 30 | 0 | 0 |
| RICE | 90 | 80 | 30 |
| COCKLEBUR | 30 | 0 | 0 |
| COTTON | 50 | 0 | 0 |
| SOYBEANS | 0 | 0 | 0 |
| BARNYARDGRASS | 90 | 30 | 0 |
| WILD OATS | 0 | 0 | 0 |
| MORNINGGLORY | 70 | 30 | 0 |
| WHEAT | 70 | 0 | 0 |
| SICKLEPOD | 30 | 0 | 0 |
| JOHNSONGRASS | 90 | 70 | 50 |
| NUTSEDGE | 0 | 0 | 0 |
| CORN | 70 | 0 | 0 |
| WILD BUCKWHEAT | 70 | 0 | 0 |
| BLACKGRASS | 60 | 30 | 0 |
| RAPE | 0 | 0 | 0 |
| BARLEY | 30 | 0 | 0 |
| GREEN FOXTAIL | 90 | 50 | 0 |
| CHEATGRASS | 90 | 60 | 30 |
| LAMBSQUARTER | 100 | 100 | 50 |
| CHICKWEED | 50 | 30 | 0 |

TABLE B

CMPD 6

| RATE=G/HA | 62 | 16 | 4 | 1 |
|---|---|---|---|---|
| POSTEMERGENCE | | | | |
| GIANT FOXTAIL | 100 | 100 | 100 | 70 |
| VELVETLEAF | 100 | 100 | 100 | 50 |
| SUGARBEETS | 100 | 100 | 100 | 100 |
| CRABGRASS | 100 | 100 | 100 | 40 |
| TEAWEED | 100 | 100 | 70 | 30 |
| JIMSONWEED | 100 | 100 | 90 | 50 |
| RICE | 100 | 80 | 80 | 20 |
| COCKLEBUR | 100 | 100 | 100 | 40 |
| COTTON | 100 | 100 | 40 | 10 |
| SOYBEANS | 100 | 100 | 90 | 80 |
| BARNYARDGRASS | 100 | 100 | 100 | 40 |
| WILD OATS | 90 | 90 | 70 | 40 |
| MORNINGGLORY | 100 | 100 | 100 | 80 |
| WHEAT | 90 | 60 | 20 | 10 |
| SICKLEPOD | 100 | 100 | 100 | 90 |
| JOHNSONGRASS | 100 | 100 | 100 | 50 |
| NUTSEDGE | 100 | 100 | 80 | 40 |
| CORN | 100 | 100 | 100 | 100 |
| WILD BUCKWHEAT | 90 | 90 | 40 | 0 |
| BLACKGRASS | 90 | 70 | 30 | 0 |
| RAPE | 100 | 100 | 100 | 70 |
| BARLEY | 60 | 30 | 10 | 0 |
| GREEN FOXTAIL | 100 | 100 | 100 | 90 |
| LAMBSQUARTER | 100 | 100 | 100 | 40 |
| CHICKWEED | 100 | 90 | 70 | - |
| DOWNY BROME | 100 | 90 | 90 | 30 |

TABLE B

CMPD 6

| RATE=G/HA | 62 | 16 | 4 | 1 |
|---|---|---|---|---|
| PREEMERGENCE | | | | |
| GIANT FOXTAIL | 100 | 100 | 20 | 0 |
| VELVETLEAF | 90 | 60 | 40 | 20 |
| SUGARBEETS | 90 | 90 | 40 | 0 |
| CRABGRASS | 100 | 80 | 50 | 30 |
| TEAWEED | 90 | 40 | 30 | 0 |
| JIMSONWEED | 70 | 50 | 20 | 0 |
| RICE | 90 | 50 | 20 | 0 |
| COCKLEBUR | 90 | 30 | 0 | 0 |
| COTTON | 90 | 90 | 40 | 10 |
| SOYBEANS | 80 | 50 | 30 | 20 |
| BARNYARDGRASS | 100 | 80 | 20 | 0 |
| WILD OATS | 80 | 30 | 20 | 0 |
| MORNINGGLORY | 100 | 40 | 0 | 0 |
| WHEAT | 40 | 10 | 10 | 0 |
| SICKLEPOD | 70 | 40 | 0 | 0 |
| JOHNSONGRASS | 100 | 70 | 30 | 20 |
| NUTSEDGE | 100 | 40 | 20 | 0 |
| CORN | 100 | 30 | 20 | 20 |
| WILD BUCKWHEAT | 60 | 50 | 30 | 0 |
| BLACKGRASS | 100 | 100 | 90 | 30 |
| RAPE | 40 | 20 | 10 | 0 |
| BARLEY | 20 | 0 | 0 | 0 |
| GREEN FOXTAIL | 100 | 100 | 40 | 0 |
| LAMBSQUARTER | 100 | 90 | 40 | 30 |
| CHICKWEED | 100 | 100 | 90 | 60 |
| DOWNY BROME | 90 | 80 | 30 | - |

TABLE B

CMPD 7

| RATE=G/HA | 250 | 62 | 16 | 4 |
|---|---|---|---|---|
| POSTEMERGENCE | | | | |
| GIANT FOXTAIL | 100 | 100 | 100 | 90 |
| VELVETLEAF | 100 | 70 | 60 | 30 |
| SUGARBEETS | 100 | 100 | 90 | 60 |
| CRABGRASS | 100 | 90 | 40 | 0 |
| TEAWEED | 90 | 90 | 40 | 20 |
| JIMSONWEED | 100 | 100 | 90 | 80 |
| RICE | 100 | 100 | 100 | 90 |
| COCKLEBUR | 40 | 30 | 30 | 0 |
| COTTON | 80 | 70 | 50 | 0 |
| SOYBEANS | 100 | 100 | 70 | 30 |
| BARNYARDGRASS | 100 | 100 | 40 | 20 |
| WILD OATS | 100 | 100 | 90 | 40 |
| MORNINGGLORY | 90 | 90 | 70 | 40 |
| WHEAT | 100 | 100 | 90 | 50 |
| SICKLEPOD | 70 | 70 | 30 | 20 |
| JOHNSONGRASS | 100 | 100 | 90 | 90 |
| NUTSEDGE | 70 | 40 | 20 | 0 |
| CORN | 100 | 100 | 100 | 90 |
| WILD BUCKWHEAT | 100 | 90 | 40 | 0 |
| BLACKGRASS | 100 | 90 | 40 | 30 |
| RAPE | 100 | 100 | 100 | 90 |
| BARLEY | 100 | 100 | 100 | 90 |
| GREEN FOXTAIL | 100 | 100 | 100 | 90 |
| LAMBSQUARTER | 70 | 20 | 0 | 0 |
| CHICKWEED | 100 | 90 | 80 | 40 |
| DOWNY BROME | 100 | 100 | 100 | 60 |

TABLE B

CMPD 7

| RATE=G/HA | 250 | 62 | 16 |
|---|---|---|---|
| PREEMERGENCE | | | |
| GIANT FOXTAIL | 100 | 80 | 30 |
| VELVETLEAF | 80 | 60 | 50 |
| SUGARBEETS | 100 | 90 | 70 |
| CRABGRASS | 90 | 40 | 0 |
| TEAWEED | 80 | 70 | 40 |
| JIMSONWEED | 30 | 20 | 0 |
| RICE | 100 | 90 | 30 |
| COCKLEBUR | 30 | 0 | 0 |
| COTTON | 90 | 40 | 10 |
| SOYBEANS | 90 | 50 | 40 |
| BARNYARDGRASS | 100 | 30 | 30 |
| WILD OATS | 70 | 40 | 0 |
| MORNINGGLORY | 70 | 30 | - |
| WHEAT | 100 | 40 | 0 |
| SICKLEPOD | 50 | 50 | 30 |
| JOHNSONGRASS | 100 | 90 | 70 |
| NUTSEDGE | 70 | 20 | 10 |
| CORN | 100 | 30 | 10 |
| WILD BUCKWHEAT | 60 | 50 | 30 |
| BLACKGRASS | 100 | 90 | 40 |
| RAPE | 80 | 40 | 0 |
| BARLEY | 100 | 30 | 0 |
| GREEN FOXTAIL | 100 | 100 | 40 |
| LAMBSQUARTER | 100 | 80 | 40 |
| CHICKWEED | 90 | 70 | 30 |
| DOWNY BROME | 100 | 90 | 60 |

TABLE B

CMPD 10

| RATE=G/HA | 250 | 62 | 16 | 4 |
|---|---|---|---|---|
| POSTEMERGENCE | | | | |
| GIANT FOXTAIL | 100 | 100 | 90 | 70 |
| VELVETLEAF | 100 | 90 | 80 | 80 |
| SUGARBEETS | 100 | 100 | 100 | 100 |
| CRABGRASS | 100 | 100 | 40 | 0 |
| TEAWEED | 100 | 100 | 90 | 50 |
| JIMSONWEED | 100 | 100 | 90 | 70 |
| RICE | 100 | 90 | 60 | 10 |
| COCKLEBUR | 100 | 100 | 90 | 40 |
| COTTON | 90 | 90 | 90 | 60 |
| SOYBEANS | 100 | 90 | 90 | 80 |
| BARNYARDGRASS | 90 | 40 | 30 | 0 |
| WILD OATS | 100 | 90 | 60 | 40 |
| MORNINGGLORY | 100 | 100 | 100 | 90 |
| WHEAT | 80 | 70 | 70 | 50 |
| SICKLEPOD | 100 | 100 | 90 | 60 |
| JOHNSONGRASS | 100 | 60 | 30 | 20 |
| NUTSEDGE | 100 | 90 | 80 | 20 |
| CORN | 100 | 100 | 100 | 90 |
| WILD BUCKWHEAT | 100 | 100 | 60 | - |
| BLACKGRASS | 100 | 60 | - | 20 |
| RAPE | 100 | 100 | 100 | 90 |
| BARLEY | 80 | 70 | 40 | 10 |
| GREEN FOXTAIL | 100 | 100 | 90 | 50 |
| LAMBSQUARTER | 100 | 100 | 100 | 100 |
| CHICKWEED | 100 | 100 | 80 | - |
| DOWNY BROME | 100 | 100 | 70 | 40 |

TABLE B

CMPD 10

| RATE=G/HA | 250 | 62 | 16 |
|---|---|---|---|
| PREEMERGENCE | | | |
| GIANT FOXTAIL | 100 | 70 | 60 |
| VELVETLEAF | 90 | 60 | 50 |
| SUGARBEETS | 100 | 90 | 70 |
| CRABGRASS | 90 | 80 | 30 |
| TEAWEED | 90 | 60 | 20 |
| JIMSONWEED | 90 | 90 | 50 |
| RICE | 100 | 90 | 40 |
| COCKLEBUR | 40 | 10 | 0 |
| COTTON | 90 | - | 0 |
| SOYBEANS | 90 | 60 | 30 |
| BARNYARDGRASS | 100 | 50 | 0 |
| WILD OATS | 80 | 40 | 0 |
| MORNINGGLORY | 90 | 70 | 40 |
| WHEAT | 90 | 30 | 10 |
| SICKLEPOD | 90 | 70 | 30 |
| JOHNSONGRASS | 100 | 100 | 50 |
| NUTSEDGE | 40 | 0 | 0 |
| CORN | 100 | 30 | 0 |
| WILD BUCKWHEAT | 90 | 40 | 0 |
| BLACKGRASS | 70 | 40 | 40 |
| RAPE | 30 | 20 | 20 |
| BARLEY | 60 | 40 | 10 |
| GREEN FOXTAIL | 100 | 100 | 70 |
| LAMBSQUARTER | 100 | 100 | 80 |
| CHICKWEED | 90 | 90 | 60 |
| DOWNY BROME | 100 | 90 | 40 |

TABLE B

CMPD 25

| RATE=G/HA | 250 | 62 | 16 | 4 |
|---|---|---|---|---|
| POSTEMERGENCE | | | | |
| GIANT FOXTAIL | 100 | 90 | 70 | 40 |
| VELVETLEAF | 100 | 100 | 90 | 40 |
| SUGARBEETS | 100 | 100 | 100 | 90 |
| CRABGRASS | 50 | 40 | 30 | 0 |
| TEAWEED | 90 | 70 | 50 | 0 |
| JIMSONWEED | 90 | 80 | 60 | 40 |
| RICE | 90 | 80 | 70 | 50 |
| COCKLEBUR | 70 | 50 | 30 | 0 |
| COTTON | 100 | 60 | 50 | 40 |
| SOYBEANS | 100 | 90 | 80 | 70 |
| BARNYARDGRASS | 100 | 80 | 70 | 50 |
| WILD OATS | 100 | 100 | 80 | 60 |
| MORNINGGLORY | 100 | 95 | 90 | 60 |
| WHEAT | 100 | 100 | 70 | 50 |
| SICKLEPOD | 80 | 70 | 30 | 0 |
| JOHNSONGRASS | 100 | 90 | 80 | 70 |
| NUTSEDGE | 90 | 70 | 50 | 30 |
| CORN | 70 | 60 | 50 | 0 |
| WILD BUCKWHEAT | 100 | 95 | 90 | 85 |
| BLACKGRASS | 100 | 70 | 50 | 30 |
| RAPE | 90 | 70 | 50 | 30 |
| BARLEY | 90 | 80 | 60 | 40 |
| GREEN FOXTAIL | 100 | 100 | 80 | 30 |
| LAMBSQUARTER | 95 | 90 | 80 | 50 |
| CHICKWEED | 100 | 90 | 80 | 70 |
| DOWNY BROME | 90 | 80 | 60 | 50 |

TABLE B

CMPD 25

| RATE=G/HA | 250 | 62 | 16 |
|---|---|---|---|
| PREEMERGENCE | | | |
| GIANT FOXTAIL | 100 | 100 | 70 |
| VELVETLEAF | 90 | 60 | 30 |
| SUGARBEETS | 90 | 80 | 60 |
| CRABGRASS | 70 | 50 | 30 |
| TEAWEED | 90 | 60 | 30 |
| JIMSONWEED | 50 | 0 | 0 |
| RICE | 100 | 70 | 0 |
| COCKLEBUR | 70 | 50 | 0 |
| COTTON | 50 | 0 | 0 |
| SOYBEANS | 50 | 30 | 0 |
| BARNYARDGRASS | 90 | 50 | 0 |
| WILD OATS | 30 | 0 | 0 |
| MORNINGGLORY | 70 | 50 | 30 |
| WHEAT | 80 | 30 | 0 |
| SICKLEPOD | 50 | 30 | 0 |
| JOHNSONGRASS | 70 | 60 | 50 |
| NUTSEDGE | 90 | 70 | 50 |
| CORN | 90 | 60 | 0 |
| WILD BUCKWHEAT | 90 | 70 | 50 |
| BLACKGRASS | 70 | 50 | 0 |
| RAPE | 80 | 0 | 0 |
| BARLEY | 70 | 30 | 0 |
| GREEN FOXTAIL | 100 | 90 | 60 |
| LAMBSQUARTER | 100 | 90 | 70 |
| CHICKWEED | 90 | 60 | 30 |
| DOWNY BROME | 90 | 70 | 30 |

TABLE B

CMPD 28

| RATE=G/HA | 250 | 62 | 16 | 4 |
|---|---|---|---|---|
| POSTEMERGENCE | | | | |
| GIANT FOXTAIL | 90 | 80 | 70 | 50 |
| VELVETLEAF | 100 | 80 | 60 | 40 |
| SUGARBEETS | 100 | 100 | 70 | 60 |
| CRABGRASS | 80 | 50 | 30 | 0 |
| TEAWEED | 80 | 50 | 30 | 0 |
| JIMSONWEED | 100 | 90 | 70 | 40 |
| RICE | 70 | 50 | 30 | 0 |
| COCKLEBUR | 80 | 70 | 40 | 30 |
| COTTON | 90 | 80 | 70 | 40 |
| SOYBEANS | 80 | 70 | 60 | 30 |
| BARNYARDGRASS | 90 | 80 | 70 | 50 |
| WILD OATS | 100 | 70 | 50 | 30 |
| MORNINGGLORY | 100 | 100 | 90 | 60 |
| WHEAT | 90 | 70 | 50 | 30 |
| SICKLEPOD | 70 | 50 | 30 | 0 |
| JOHNSONGRASS | 100 | 90 | 70 | 50 |
| NUTSEDGE | 70 | 50 | 30 | 0 |
| CORN | 70 | 60 | 50 | 40 |
| WILD BUCKWHEAT | 100 | 90 | 70 | 60 |
| BLACKGRASS | 100 | 100 | 80 | 60 |
| RAPE | 90 | 70 | 50 | 30 |
| BARLEY | 90 | 70 | 50 | 30 |
| GREEN FOXTAIL | 100 | 90 | 70 | 50 |
| LAMBSQUARTER | 100 | 90 | 80 | 70 |
| CHICKWEED | 90 | 70 | 50 | 30 |
| DOWNY BROME | 80 | 70 | 60 | 50 |

TABLE B

CMPD 28

| RATE=G/HA | 250 | 62 | 16 | 4 |
|---|---|---|---|---|
| PREEMERGENCE | | | | |
| GIANT FOXTAIL | 90 | 60 | 0 | 0 |
| VELVETLEAF | 90 | 60 | 30 | 0 |
| SUGARBEETS | 90 | 60 | 30 | 0 |
| CRABGRASS | 90 | 70 | 50 | 0 |
| TEAWEED | 90 | 60 | 30 | 0 |
| JIMSONWEED | 30 | 0 | 0 | 0 |
| RICE | 90 | 0 | 0 | 0 |
| COCKLEBUR | 30 | 0 | 0 | 0 |
| COTTON | 0 | 0 | 0 | 0 |
| SOYBEANS | 50 | 20 | 0 | 0 |
| BARNYARDGRASS | 90 | 50 | 0 | 0 |
| WILD OATS | 60 | 30 | 0 | 0 |
| MORNINGGLORY | 80 | 50 | 30 | 20 |
| WHEAT | 60 | 0 | 0 | 0 |
| SICKLEPOD | 80 | 30 | 0 | 0 |
| JOHNSONGRASS | 70 | 50 | 0 | 0 |
| NUTSEDGE | 30 | 0 | 0 | 0 |
| CORN | 70 | 0 | 0 | 0 |
| WILD BUCKWHEAT | 50 | 0 | 0 | 0 |
| BLACKGRASS | 70 | 50 | 0 | 0 |
| RAPE | 90 | 40 | 0 | 0 |
| BARLEY | 50 | 30 | 0 | 0 |
| GREEN FOXTAIL | 100 | 90 | 60 | 30 |
| LAMBSQUARTER | 90 | 80 | 70 | 50 |
| CHICKWEED | 70 | 50 | 30 | 0 |
| DOWNY BROME | 90 | 60 | 30 | 0 |

TABLE B

CMPD 29

| RATE=G/HA | 250 | 62 | 16 | 4 |
|---|---|---|---|---|
| POSTEMERGENCE | | | | |
| GIANT FOXTAIL | 100 | 100 | 100 | 70 |
| VELVETLEAF | 100 | 100 | 90 | 80 |
| SUGARBEETS | 100 | 100 | 100 | 100 |
| CRABGRASS | 90 | 80 | 60 | 40 |
| TEAWEED | 90 | 80 | 70 | 50 |
| JIMSONWEED | 100 | 100 | 100 | 90 |
| RICE | 80 | 70 | 50 | 40 |
| COCKLEBUR | 100 | 90 | 80 | 50 |
| COTTON | 100 | 100 | 90 | 40 |
| SOYBEANS | 100 | 100 | 100 | 60 |
| BARNYARDGRASS | 100 | 100 | 90 | 80 |
| WILD OATS | 100 | 100 | 90 | 70 |
| MORNINGGLORY | 100 | 100 | 90 | 70 |
| WHEAT | 100 | 100 | 100 | 50 |
| SICKLEPOD | 80 | 60 | 50 | 30 |
| JOHNSONGRASS | 100 | 100 | 90 | 60 |
| NUTSEDGE | 100 | 90 | 80 | 50 |
| CORN | 80 | 70 | 60 | 50 |
| WILD BUCKWHEAT | 100 | 100 | 100 | 100 |
| BLACKGRASS | 100 | 100 | 100 | 100 |
| RAPE | 100 | 100 | 90 | 60 |
| BARLEY | 90 | 80 | 70 | 60 |
| GREEN FOXTAIL | 100 | 100 | 100 | 90 |
| LAMBSQUARTER | 100 | 100 | 90 | 60 |
| CHICKWEED | 100 | 90 | 70 | 50 |
| DOWNY BROME | 90 | 80 | 70 | 60 |

TABLE B

CMPD 29

| RATE=G/HA | 250 | 62 | 16 |
|---|---|---|---|
| PREEMERGENCE | | | |
| GIANT FOXTAIL | 100 | 100 | 100 |
| VELVETLEAF | 90 | 70 | 50 |
| SUGARBEETS | 90 | 70 | 50 |
| CRABGRASS | 100 | 90 | 70 |
| TEAWEED | 90 | 70 | 50 |
| JIMSONWEED | 90 | 70 | 50 |
| RICE | 90 | 70 | 50 |
| COCKLEBUR | 70 | 50 | 30 |
| COTTON | 70 | 0 | 0 |
| SOYBEANS | 80 | 30 | 0 |
| BARNYARDGRASS | 100 | 100 | 0 |
| WILD OATS | 80 | 50 | 30 |
| MORNINGGLORY | 80 | 50 | 30 |
| WHEAT | 70 | 50 | 30 |
| SICKLEPOD | 100 | 100 | 100 |
| JOHNSONGRASS | 80 | 50 | 0 |
| NUTSEDGE | 90 | 60 | 30 |
| CORN | 90 | 70 | 50 |
| WILD BUCKWHEAT | 90 | 80 | 70 |
| BLACKGRASS | 90 | 70 | 50 |
| RAPE | 90 | 60 | 30 |
| BARLEY | 80 | 50 | 30 |
| GREEN FOXTAIL | 100 | 100 | 100 |
| LAMBSQUARTER | 100 | 100 | 90 |
| CHICKWEED | 70 | 30 | 0 |
| DOWNY BROME | 90 | 80 | 70 |

TABLE B

CMPD 53

| RATE=G/HA | 62 | 16 | 4 | 1 |
|---|---|---|---|---|
| POSTEMERGENCE | | | | |
| GIANT FOXTAIL | 100 | 90 | 80 | 70 |
| VELVETLEAF | 90 | 80 | 70 | 30 |
| SUGARBEETS | 100 | 100 | 100 | 60 |
| CRABGRASS | 60 | 50 | 40 | 30 |
| TEAWEED | 80 | 70 | 50 | 30 |
| JIMSONWEED | 100 | 100 | 90 | 80 |
| RICE | 70 | 50 | 30 | 0 |
| COCKLEBUR | 80 | 60 | 40 | 30 |
| COTTON | 70 | 60 | 50 | 30 |
| SOYBEANS | 100 | 100 | 70 | 40 |
| BARNYARDGRASS | 100 | 90 | 80 | 60 |
| WILD OATS | 100 | 100 | 90 | 60 |
| MORNINGGLORY | 70 | 50 | 30 | 0 |
| WHEAT | 90 | 80 | 60 | 50 |
| SICKLEPOD | 30 | 0 | 0 | 0 |
| JOHNSONGRASS | 90 | 80 | 70 | 50 |
| NUTSEDGE | 80 | 70 | 50 | 30 |
| CORN | 60 | 30 | 0 | 0 |
| WILD BUCKWHEAT | 100 | 90 | 30 | 0 |
| BLACKGRASS | 100 | 90 | 80 | 70 |
| RAPE | 100 | 100 | 100 | 70 |
| BARLEY | 90 | 70 | 50 | 40 |
| GREEN FOXTAIL | 100 | 70 | 30 | 0 |
| LAMBSQUARTER | 70 | 60 | 50 | 30 |
| CHICKWEED | 100 | 100 | 90 | 80 |
| DOWNY BROME | 100 | 100 | 90 | 60 |

TABLE B

CMPD 53

| RATE=G/HA | 62 | 16 | 4 | 1 |
|---|---|---|---|---|
| PREEMERGENCE | | | | |
| GIANT FOXTAIL | 100 | 90 | 70 | 60 |
| VELVETLEAF | 70 | 50 | 30 | 0 |
| SUGARBEETS | 90 | 80 | 70 | 50 |
| CRABGRASS | 100 | 90 | 80 | 50 |
| TEAWEED | 70 | 50 | 30 | 0 |
| JIMSONWEED | 70 | 30 | 0 | 0 |
| RICE | 80 | 30 | 0 | 0 |
| COCKLEBUR | 70 | 50 | 30 | 0 |
| COTTON | 40 | 20 | 0 | 0 |
| SOYBEANS | 60 | 0 | 0 | 0 |
| BARNYARDGRASS | 90 | 30 | 0 | 0 |
| WILD OATS | 70 | 60 | 50 | 30 |
| MORNINGGLORY | 60 | 50 | 40 | 30 |
| WHEAT | 50 | 30 | 0 | 0 |
| SICKLEPOD | 90 | 80 | 70 | 60 |
| JOHNSONGRASS | 80 | 30 | 0 | 0 |
| NUTSEDGE | 90 | 70 | 50 | 30 |
| CORN | 50 | 0 | 0 | 0 |
| WILD BUCKWHEAT | 100 | 90 | 60 | 30 |
| BLACKGRASS | 90 | 70 | 50 | 30 |
| RAPE | 90 | 80 | 30 | 0 |
| BARLEY | 70 | 50 | 30 | 0 |
| GREEN FOXTAIL | 100 | 90 | 70 | 50 |
| LAMBSQUARTER | 100 | 100 | 100 | 90 |
| CHICKWEED | 100 | 90 | 60 | 30 |
| DOWNY BROME | 80 | 50 | 30 | 0 |

TABLE B

CMPD 59

| RATE=G/HA | 250 | 62 | 16 | 4 |
|---|---|---|---|---|
| POSTEMERGENCE | | | | |
| GIANT FOXTAIL | 70 | 30 | 0 | 0 |
| VELVETLEAF | 100 | 100 | 100 | 90 |
| SUGARBEETS | 100 | 100 | 100 | 100 |
| CRABGRASS | 60 | 50 | 40 | 30 |
| TEAWEED | 90 | 80 | 60 | 30 |
| JIMSONWEED | 100 | 100 | 100 | 90 |
| RICE | 100 | 100 | 100 | 70 |
| COCKLEBUR | 100 | 70 | 30 | 0 |
| COTTON | 90 | 70 | 50 | 30 |
| SOYBEANS | 100 | 100 | 100 | 100 |
| BARNYARDGRASS | 100 | 100 | 100 | 60 |
| WILD OATS | 90 | 60 | 0 | 0 |
| MORNINGGLORY | 100 | 100 | 100 | 90 |
| WHEAT | 60 | 30 | 0 | 0 |
| SICKLEPOD | 100 | 90 | 70 | 50 |
| JOHNSONGRASS | 100 | 100 | 70 | 60 |
| NUTSEDGE | 100 | 100 | 70 | 30 |
| CORN | 100 | 100 | 100 | 70 |
| WILD BUCKWHEAT | 100 | 100 | 100 | 50 |
| BLACKGRASS | 90 | 70 | 50 | 30 |
| RAPE | 100 | 100 | 100 | 100 |
| BARLEY | 40 | 0 | 0 | 0 |
| GREEN FOXTAIL | 90 | 60 | 30 | 0 |
| LAMBSQUARTER | 100 | 100 | 100 | 90 |
| CHICKWEED | 100 | 100 | 100 | 70 |
| DOWNY BROME | 90 | 70 | 50 | 30 |

TABLE B

CMPD 59

| RATE=G/HA | 250 | 62 | 16 |
|---|---|---|---|
| PREEMERGENCE | | | |
| GIANT FOXTAIL | 80 | 30 | 0 |
| VELVETLEAF | 90 | 60 | 30 |
| SUGARBEETS | 90 | 70 | 50 |
| CRABGRASS | 70 | 50 | 30 |
| TEAWEED | 90 | 70 | 50 |
| JIMSONWEED | 70 | 50 | 30 |
| RICE | 100 | 90 | 60 |
| COCKLEBUR | 30 | 0 | 0 |
| COTTON | 50 | 30 | 0 |
| SOYBEANS | 60 | 20 | 0 |
| BARNYARDGRASS | 80 | 50 | 30 |
| WILD OATS | 30 | 0 | 0 |
| MORNINGGLORY | 70 | 30 | 0 |
| WHEAT | 40 | 0 | 0 |
| SICKLEPOD | 90 | 60 | 30 |
| JOHNSONGRASS | 100 | 100 | 100 |
| NUTSEDGE | 100 | 50 | - |
| CORN | 80 | 30 | 0 |
| WILD BUCKWHEAT | 100 | 60 | 30 |
| BLACKGRASS | 90 | 70 | 50 |
| RAPE | 90 | 70 | 50 |
| BARLEY | 30 | 0 | 0 |
| GREEN FOXTAIL | 90 | 60 | 30 |
| LAMBSQUARTER | 90 | 70 | 50 |
| CHICKWEED | 80 | 50 | 30 |
| DOWNY BROME | 80 | 50 | 30 |

Test C

The following plants were used:
The species below were planted.

| Name | Genus Species | Size (cm) |
|---|---|---|
| Blackgrass, Stage | Alopecurus myosuroides | 14.0 |
| Lambsquarters, Common | Chenopodium album | 4.0 |
| Catchweed Bedstraw | Galium aparine | 9.0 |
| Black Nightshade, Eastern | Solanum nigrum | 2.0 |
| Field Pennycress | Thlaspi arvense | 2.5 |
| Ivyleaf Speedwell | Veronica hederafolia | 5.0 |
| Field Speedwell, Common | Veronica persica | 6.0 |
| Field Pansy | Viola arvensis | 1.5 |

Two days later, a second series were planted using the following species.

| Name | Genus Species | Size (cm) |
|---|---|---|
| Blackgrass, Stage I | Alopecurus myosuroides | 6.0 |
| Wild Oat Stage II | Avena fatua | 22.0 |
| Sugar Beet | Beta vulgaris | 8.0 |
| Oilseed Rape | Brassica napus | 13.0 |
| Kochia | Kochia scoparia | 5.0 |
| Mayweed, Scentless | Matricaria inodora | 4.0 |
| Annual Bluegrass | Poa annua | 7.0 |
| Wild Buckwheat | Polygonum convolvulus | 7.0 |

Seven days following the first plants, a third pot was prepared with the following species.

| Name | Genus Species | Size (cm) |
|---|---|---|
| Jointed Goatgrass | Aegilops cylindrica | 12.0 |
| Wild Oat Stage I | Avena fatua | 15.0 |
| Cheatgrass | Bromus secalinus | 8.0 |
| Downy Brome | Bromus tectorum | 8.0 |
| Winter Barley 'cv. Igri' | Hordeum vulgare | 18.0 |
| Spring Barley 'cv. Klages' | Hordeum vulgare | 24.0 |
| Annual Ryegrass | Lolium multiflorum | 11.0 |
| Green Foxtail | Setaria virdis | 6.0 |
| Winter Wheat 'cv. Centurk' | Triticum aestivum | 23.0 |
| Spring Wheat 'cv. Era' | Triticum aestivum | 16.0 |

All post-emergence plants were grown in the greenhouse until herbicide application. Pre-emergence treatments were prepared immediately before herbicide application in the same manner as described for the post-emergence treatments. Application of the herbicide was accomplished by first diluting the technical material in a non-phytotoxic solvent and applying over the surface of the plants and soil of all six components using a belt sprayer.

After treatment, all components were removed to the greenhouse where they were maintained for 21 days at temperatures of 19°C night and 30°C day with a 16-hour photoperiod and a relative humidity of 45 to 80 percent. At this time, all species were rated using a visual scale of 0 representing no control and 100 representing complete control.

Response ratings are contained in Table C.

TABLE C

CMPD 6

| RATE G/HA | 64 | 32 | 16 | 8 | 4 | 2 |
|---|---|---|---|---|---|---|
| PREEMERGENCE | | | | | | |
| SPRING WHEAT | 70 | 60 | 40 | 40 | 30 | 20 |
| WINTER WHEAT | 60 | 60 | 50 | 30 | 20 | 20 |
| SPRING BARLEY | 90 | 70 | 50 | 30 | 20 | 0 |
| WINTER BARLEY | 90 | 90 | 90 | 70 | 40 | 20 |
| WILD OATS | 80 | 80 | 50 | 20 | 0 | 0 |
| DOWNY BROME | 90 | 70 | 50 | 20 | 0 | 0 |
| CHEAT GRASS | 90 | 90 | 80 | 60 | 60 | 20 |
| BLACKGRASS | 70 | 50 | 40 | 40 | 10 | 0 |
| ANN. BLUEGRASS | 30 | 10 | 10 | 0 | 0 | 0 |
| GREEN FOXTAIL | 100 | 100 | 70 | 20 | 0 | 0 |
| ANNUAL RYEGRASS | 80 | 30 | 10 | 10 | 0 | 0 |
| RAPE | 80 | 70 | 70 | 40 | 20 | 10 |
| JOINT GOATGRASS | 20 | 20 | 0 | 0 | 0 | 0 |
| CTCHWD BEDSTRAW | 40 | 30 | 20 | 0 | 0 | 0 |
| WILD BUCKWHEAT | 20 | 20 | 10 | 0 | 0 | 0 |
| KOCHIA | 0 | 0 | 0 | 0 | 0 | 0 |
| SNTLS MAYWEED | 90 | 80 | 80 | 70 | 70 | 50 |
| BLACK NIGHTSHAD | 10 | 0 | 0 | 0 | 0 | 0 |
| FIELD SPEEDWELL | 20 | 0 | 0 | 0 | 0 | 0 |
| IVYLF SPEEDWELL | 10 | 0 | 0 | 0 | 0 | 0 |
| SUGARBEET | 80 | 80 | 70 | 70 | 60 | 50 |
| LAMBSQUARTER | 90 | 90 | 70 | 50 | 30 | 10 |
| FIELD PENNYCRES | 90 | 80 | 70 | 40 | 30 | 20 |
| FIELD PANSY | 70 | 60 | 30 | 0 | 0 | 0 |

TABLE C

CMPD 6

| RATE G/HA | 64 | 32 | 16 | 8 | 4 | 2 |
|---|---|---|---|---|---|---|
| POSTEMERGENCE | | | | | | |
| SPRING WHEAT | 70 | 70 | 60 | 40 | 20 | 10 |
| WINTER WHEAT | 80 | 80 | 70 | 60 | 20 | 0 |
| SPRING BARLEY | 80 | 80 | 60 | 40 | 20 | 0 |
| WINTER BARLEY | 80 | 80 | 70 | 50 | 20 | 0 |
| WILD OATS | 100 | 80 | 80 | 80 | 80 | 50 |
| DOWNY BROME | 70 | 70 | 20 | 20 | 10 | 0 |
| CHEAT GRASS | 100 | 60 | 40 | 20 | 0 | 0 |
| BLACKGRASS | 50 | 20 | 0 | 0 | 0 | 0 |
| ANN. BLUEGRASS | 70 | 50 | 20 | 10 | 0 | 0 |
| GREEN FOXTAIL | 100 | 100 | 100 | 100 | 80 | 70 |
| ANNUAL RYEGRASS | 0 | 0 | 0 | 0 | 0 | 0 |
| RAPE | 100 | 100 | 100 | 90 | 90 | 60 |
| JOINT GOATGRASS | 100 | 90 | 80 | 50 | 50 | 20 |
| WILD OATS STG 2 | 100 | 100 | 100 | 100 | 90 | 60 |
| BLACKGRASS STG2 | 60 | 20 | 10 | 0 | 0 | 0 |
| CTCHWD BEDSTRAW | 80 | 70 | 50 | 20 | 10 | 0 |
| WILD BUCKWHEAT | 100 | 40 | 20 | 20 | 0 | 0 |
| KOCHIA | 100 | 80 | 30 | 30 | 20 | 0 |
| SNTLS MAYWEED | 100 | 100 | 90 | 90 | 90 | 70 |
| BLACK NIGHTSHAD | 0 | 0 | 0 | 0 | 0 | 0 |
| FIELD SPEEDWELL | 0 | 0 | 0 | 0 | 0 | 0 |
| IVYLF SPEEDWELL | 30 | 20 | 0 | 0 | 0 | 0 |
| SUGARBEET | 100 | 100 | 100 | 100 | 100 | 100 |
| LAMBSQUARTER | 90 | 90 | 90 | 80 | 70 | 40 |
| FIELD PENNYCRES | 100 | 100 | 90 | 90 | 80 | 70 |
| FIELD PANSY | 70 | 70 | 60 | 60 | 50 | 30 |

## Claims

1. Compounds of the formula

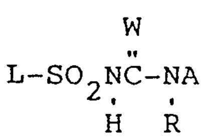

I

wherein
L is

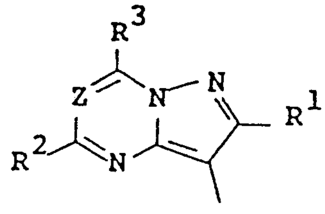,
L-1

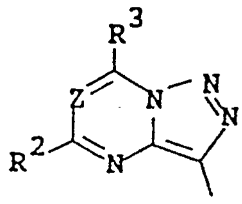,
L-2

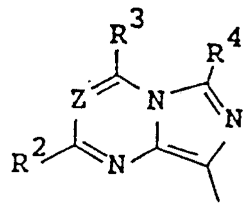,
L-3

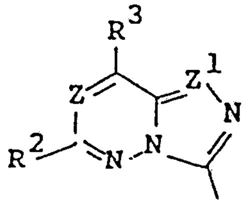,
L-4

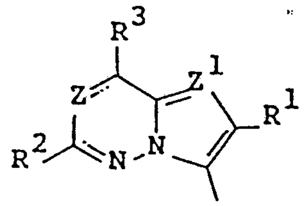,
L-5

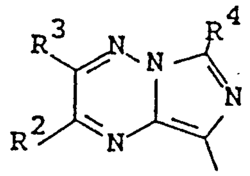,
L-6

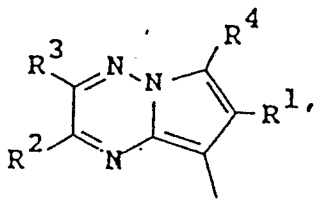
L-7

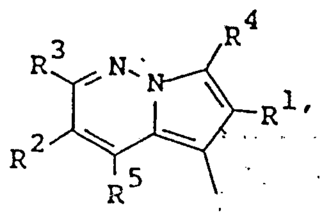
L-8

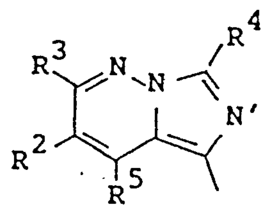
L-9

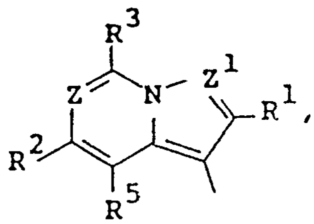,

L-10

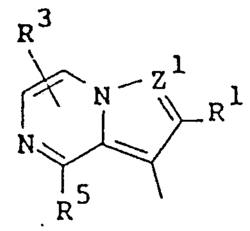,

L-11

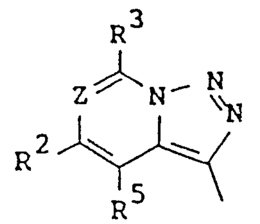,

L-12

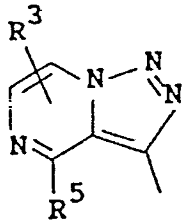,

L-13

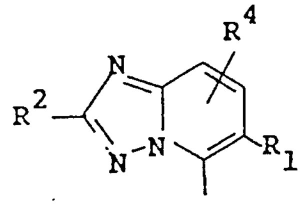,

L-14

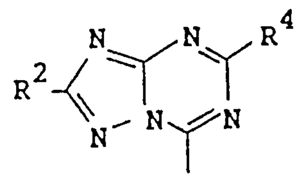,

L-15

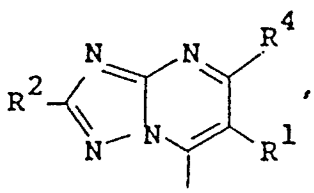,

L-16

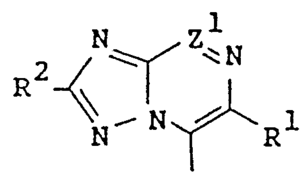,

L-17

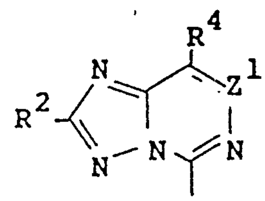,

L-18

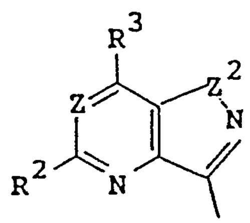

L-19

or

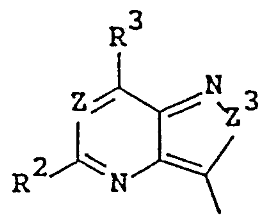;

L-20

$R^1$ is H, halogen, $C_1$-$C_3$ alkyl, $C_1$-$C_3$ haloalkyl, $OR^6$, $SR^6$, $S(O)R^6$, $S(O)_2R^6$, $CO_2R^7$, $C(O)NR^8R^9$, $S(O)_2NR^{10}R^{11}$, $C_2$-$C_3$ alkenyl, $C_2$-$C_3$ haloalkenyl, $C_2$-$C_3$ alkynyl, $NO_2$, CN, $C(O)R^{12}$, $C(R^{13})=NOR^{14}$ or $C_1$-$C_2$ alkyl substituted by $C_1$-$C_2$ alkoxy, $C_1$-$C_2$ alkylthio or CN;

$R^2$ is H, $C_1$-$C_3$ alkyl, $C_1$-$C_3$ alkoxy, $C_1$-$C_2$ haloalkoxy or $C_1$-$C_2$ alkyl substituted with $OCH_3$, $SCH_3$ or CN;

$R^3$ is H, $CH_3$ or $OCH_3$;

$R^4$ is H or $CH_3$;

$R^5$ is H, halogen, $C_1$-$C_3$ alkyl, $C_1$-$C_3$ haloalkyl, $OR^6$, $SR^6$, $S(O)R^6$, $S(O)_2R^6$, $CO_2R^6$, $C(O)NR^8R^9$, $S(O)_2NR^{10}R^{11}$, $C_2$-$C_3$ alkenyl, $C_2$-$C_3$ haloalkenyl, $C_2$-$C_3$ alkynyl, $NO_2$, CN, $C(O)R^{12}$, or $C_1$-$C_2$ alkyl substituted by $C_1$-$C_2$ alkoxy, $C_1$-$C_2$ alkylthio or CN;

$R^6$ is $C_1$-$C_3$ alkyl, $C_1$-$C_3$ haloalkyl, $C_3$ alkenyl, $C_2$-$C_3$ haloalkenyl, propargyl, cyclopropyl, cyclopropylmethyl or $-CH_2CH_2-$ substituted by OH, $C_1$-$C_2$ alkoxy, SH, $C_1$-$C_2$ thioalkyl or CN;

$R^7$ is $C_1$-$C_4$ alkyl, $C_2$-$C_3$ haloalkyl, allyl, propargyl, cyclopropyl, cyclopropylmethyl, or $-CH_2CH_2-$ substituted by OH, $OCH_3$, $SCH_3$ or CN;

$R^8$ is H or $C_1$-$C_2$ alkyl;

$R^9$ is $C_1$-$C_3$ alkyl, $C_1$-$C_3$ alkoxy, allyl, propargyl, cyclopropyl, $CH_2CN$, $CH_2CH_2CN$ or $CH_2CH_2OCH_3$;

$R^{10}$ is H or $C_1$-$C_3$ alkyl;

$R^{11}$ is $C_1$-$C_3$ alkyl, $C_1$-$C_3$ alkoxy, allyl, propargyl, cyclopropyl, cyclopropylmethyl, $CH_2CN$, $CH_2CH_2CN$ or $CH_2CH_2OCH_3$;

$R^{10}$ and $R^{11}$ can be taken together to form a ring consisting of $(-CH_2-)_4$, $(-CH_2-)_5$ or $(-CH_2CH_2-)_2O$;

$R^{12}$ is H, $C_1$-$C_3$ alkyl or cyclopropyl;
$R^{13}$ is H, $C_1$-$C_3$ alkyl, cyclopropyl, Cl, CN, $OCH_3$, $SCH_3$ or $N(CH_3)_2$;
$R^{14}$ is H or $C_1$-$C_3$ alkyl;
Z is N or CH;
$Z^1$ is N, CH or $CCH_3$;
$Z^2$ is O, S or $NCH_3$;
$Z^3$ is O, S or N-$R^{15}$;
$R^{15}$ is H, $C_1$-$C_2$ alkyl, $C_1$-$C_2$ haloalkyl, $CH_2CH_2OCH_3$, $CH_2CH_2SCH_3$, $CH_2CN$ or $CO_2CH_3$;
W is O or S;
R is H or $CH_3$;

A is 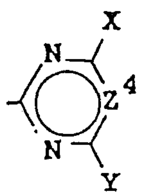 , 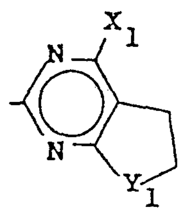 , 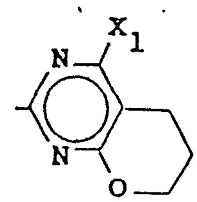 ,

A-1 A-2 A-3

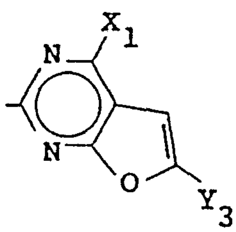 , 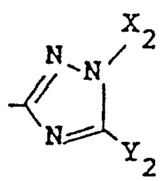 , 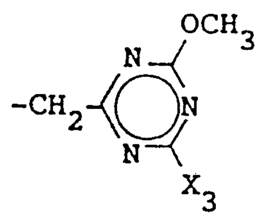

A-4 A-5 A-6

or 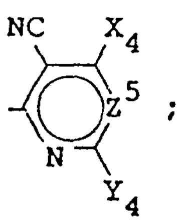 ;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino or di($C_1$-$C_3$ alkyl)amino;
Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkyl thioalkyl, $C_2$-$C_5$ alkylsulfinylalkyl, $C_2$-$C_5$ alkylsulfonylalkyl, $C_1$-$C_4$ haloalkyl, $C_2$-$C_4$ alkynyl, $C_3$-$C_5$ cycloalkyl, azido, cyano,

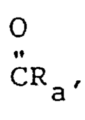 , 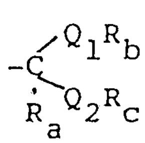 , 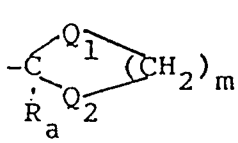 , 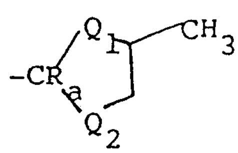

or $N(OCH_3)CH_3$;
m is 2 or 3;
$Q^1$ and $Q^2$ are independently O or S;
$R_a$ is H or $C_1$-$C_3$ alkyl;
$R_b$ and $R_c$ are independently $C_1$-$C_3$ alkyl;
$Z^4$ is CH, N, $CCH_3$, $CC_2H_5$, CCl or CBr;
$Z^5$ is CH or N;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl;
$Y_4$ is $CH_3$, $OCH_3$, $OC_2H_5$ or Cl;
and their agriculturally suitable salts;
provided that

1) when X is halogen, then $Z^4$ is CH and Y is $OCH_3$, $OC_2H_5$, $NH_2$, $NHCH_3$, $N(CH_3)_2$, $OCF_2H$, $OCF_2Br$ or $N(OCH_3)CH_3$;

2) when X or Y is $C_1$ haloalkoxy, then $Z^4$ is CH;

3) when W is S, then R is H, A is A-1, $Z^4$ is CH or N, and Y is $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$,

$CH(OCH_3)_2$ or 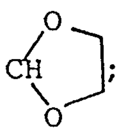;

4) when the total number of carbon atoms of X and Y is greater than four, then the greatest combined number of carbons of any two substituents on an L, selected from $R^1$, $R^2$, $R^3$, $R^4$, $R^5$ and $R^{15}$ is less than or equal to six;

5) $X_4$ and $Y_4$ are not simultaneously Cl;

6) the total number of carbon atoms of $R^{10}$ and $R^{11}$ is less than or equal to five.

2. Compounds of Claim 1 where L is L-1, L-3, L-4, L-5, L-6, L-7, L-8, L-9, L-10, L-11, L-14, L-19 or L-20.

3. Compounds of Claim 2 where W is O and $Z^4$ is CH or N.

4. Compounds of Claim 3 where

X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$;

Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C\equiv CH$, $OCH_2CH_2OCH_3$,

$CH_2SCH_3$, 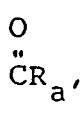, 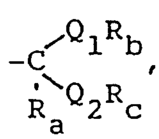, 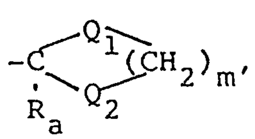

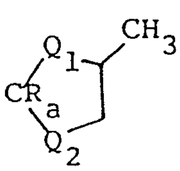, $OCF_2H$, $OCF_2Br$, $SCF_2H$, cyclopropyl, $C\equiv CH$ or $C\equiv CCH_3$;

$R_a$ is H or $CH_3$; and

$R_b$ and $R_c$ are independently $CH_3$ or $CH_2CH_3$.

5. Compounds of Claim 4 where

$R^1$ is H, halogen, $C_1$-$C_2$ alkyl, $C_1$-$C_2$ haloalkyl, $OR^6$, $SR^6$, $S(O)R^6$, $S(O)_2R^6$, $CO_2R^7$, $C(O)NR^8R^9$, $S(O)_2NR^{10}R^{11}$, $C_2$-$C_3$ alkenyl, $C_2$-$C_3$ haloalkenyl, $NO_2$, CN, $C(O)R^{12}$, $C(R^{13})=NOR^{14}$ or $C_1$-$C_2$ alkyl

substituted by $C_1$-$C_2$ alkoxy, $C_1$-$C_2$ alkylthio or CN;
$R^2$ is H, $CH_3$ or $OCH_3$;
$R^5$ is H, halogen, $C_1$-$C_2$ alkyl, $C_1$-$C_2$ haloalkyl, $OR^6$, $SR^6$, $S(O)R^6$, $S(O)_2R^6$, $CO_2R^7$, $C(O)NR^8R^9$, $S(O)_2NR^{10}R^{11}$, $C_2$-$C_3$ alkenyl, $C_2$-$C_3$ haloalkenyl, $NO_2$, CN, $C(O)R^{12}$, or $C_1$-$C_2$ alkyl substituted by $C_1$-$C_2$ alkoxy, $C_1$-$C_2$ alkylthio or CN;
$R^6$ is $C_1$-$C_3$ alkyl, $C_1$-$C_3$ haloalkyl, $C_3$ alkenyl, $C_2$-$C_3$ haloalkenyl, propargyl, cyclopropyl or cyclopropylmethyl.

6. Compounds of Claim 5 where
A is A-1;
X is $CH_3$, $OCH_3$, $OCH_2CH_3$, Cl or $OCF_2H$;
Y is $CH_3$, $OCH_3$, $C_2H_5$, $CH_2OCH_3$, $NHCH_3$, $CH(OCH_3)_2$ or cyclopropyl; and
R is H.

7. Compounds of Claim 6 where L is L-1.

8. Compounds of Claim 6 where L is L-3.

9. Compounds of Claim 6 where L is L-4.

10. Compounds of Claim 6 where L is L-5.

11. The compound of Claim 1 which is N-[(4,6-dimethoxypyrimidin-2-yl)aminocarbonyl]-5,7-dimethylpyrazolo[1,5-A]-pyrimidine-3-sulfonamide.

12. The compound of Claim 1 which is N-[(4,6-dimethoxytriazin-2-yl)aminocarbonyl]-5,7-dimethylpyrazolo[1,5-A]-pyrimidine-3-sulfonamide.

13. An agriculturally suitable composition for controlling the growth of undesired vegetation or for use as a plant growth regulant comprising an effective amount of a compound of any of Claims 1 to 12 and at least one of the following: surfactant, solid diluent or liquid diluent.

14. A composition of Claim 13 comprising a compound of Claim 11 or an agriculturally suitable salt thereof.

15. A composition of Claim 13 comprising a compound of Claim 12 or an agriculturally suitable salt thereof.

16. A method for controlling the growth of undesired vegetation which comprises applying to the locus to be protected an effective amount of a compound of any of Claims 1 to 12.

17. A method of Claim 16 wherein the compound of Claim 11 is applied or an agriculturally suitable salt thereof.

18. A method of Claim 16 wherein the compound of Claim 12 is applied or an agriculturally suitable salt thereof.

19. A method for regulating the growth of plants which comprises applying to the locus of such plants an effective but substantially non-phytotoxic amount of a plant growth regulant selected from compounds of any of Claims 1 to 12.